# EUROPEAN PATENT APPLICATION

(11) **EP 4 789 717 A2**
(43) Date of publication of application: **12.08.2026**
(21) Application number: 26171663.3
(22) Date of filing: 21.09.2018
(51) Int. Cl.: A61P 21/00

(54) **NUCLEIC ACID-POLYPEPTIDE COMPOSITIONS AND METHODS OF INDUCING EXON SKIPPING**

(30) Priority: 22.09.2017 US 201762561939 P; 11.07.2018 US 201862696766 P
(62) Document number(s) of the earlier application(s) in accordance with Art. 76 EPC: 18858928.7 / 3 684 376
(71) Applicant: Avidity Biosciences, Inc., San Diego, CA 92121 (US)
(72) Inventor: LEVIN, Arthur A, La Jolla, 92037 (US); GEALL, Andrew John, La Jolla, 92037 (US); COCHRAN, Michael Caramian, La Jolla, 92037 (US); HUANG, Hanhua, La Jolla, 92037 (US); DOPPALAPUDI, Venkata Ramana, La Jolla, 92037 (US); BURKE, Rob, La Jolla, 92037 (US); DARIMONT, Beatrice Diana, La Jolla, 92037 (US); SHI, Yunyu, La Jolla, 92037 (US); JOHNS, Rachel, La Jolla, 92037 (US)
(74) Representative: Mewburn Ellis LLP

(57) **Abstract**

Disclosed herein are molecules and pharmaceutical compositions that induce an insertion, deletion, duplication, or alteration in an incorrectly spliced mRNA transcript to induce exon skipping or exon inclusion. Also described herein include methods for treating a disease or disorder that comprises a molecule or a pharmaceutical composition that induces an insertion, deletion, duplication, or alteration in an incorrectly spliced mRNA transcript to induce exon skipping or exon inclusion.

## Description

### CROSS-REFERENCE

This application claims the benefit of U.S. Provisional Application No. 62/561,939, filed September 22, 2017, and U.S. Provisional Application No. 62/696,766, filed July 11, 2018, each of which is incorporated herein by reference in its entirety.

### BACKGROUND OF THE DISCLOSURE

Modulation of RNA function is a developing area of therapeutic interest. Drugs that affect mRNA stability like antisense oligonucleotides and short interfering RNAs are one way to modulate RNA function. Another group of oligonucleotides can modulate RNA function by altering the processing of pre-mRNA to include or exclude specific regions of pre-mRNAs from the ultimate gene product: the encoded protein. As such, oligonucleotide therapeutics represent a means of modulating protein expression in disease states and as such have utility as therapeutics.

### SUMMARY OF THE DISCLOSURE

Disclosed herein, in certain embodiments, are molecules and pharmaceutical compositions for modulating RNA processing. In some embodiments, also disclosed herein are molecules and pharmaceutical compositions for the treatment of a muscular dystrophy.

Disclosed herein, in certain embodiments, are methods of treating a disease or disorder caused by an incorrectly spliced mRNA transcript in a subject in need thereof, the method comprising: administering to the subject a polynucleic acid molecule conjugate; wherein the polynucleic acid molecule conjugate is conjugated to a cell targeting binding moiety; wherein the polynucleotide optionally comprises at least one 2' modified nucleotide, at least one modified internucleotide linkage, or at least one inverted abasic moiety; wherein the polynucleic acid molecule conjugate induces insertion, deletion, duplication, or alteration in the incorrectly spliced mRNA transcript to induce exon skipping or exon inclusion in the incorrectly spliced mRNA transcript to generate a fully processed mRNA transcript; and wherein the fully processed mRNA transcript encodes a functional protein, thereby treating the disease or disorder in the subject. In some embodiments, the disease or disorder is further characterized by one or more mutations in the mRNA. In some embodiments, the disease or disorder comprises a neuromuscular disease, a genetic disease, cancer, a hereditary disease, or a cardiovascular disease. In some embodiments, the disease or disorder is muscular dystrophy. In some embodiments, the disease or disorder is Duchenne muscular dystrophy. In some embodiments, the exon skipping is of exon 8, 23, 35, 43, 44, 45, 50, 51, 52, 53, or 55 of the *DMD* gene. In some embodiments, the exon skipping is of exon 23 of the *DMD* gene. In some embodiments, the polynucleic acid molecule conjugate is of Formula (I):

A-X-B Formula I

wherein,
A is a binding moiety;
B is a polynucleotide; and
X is a bond or first linker.

In some embodiments, the polynucleic acid molecule conjugate is of Formula (II):

A-X-B-Y-C Formula II

wherein,
A is a binding moiety;
B is a polynucleotide;
C is a polymer;
X is a bond or first linker; and
Y is a bond or second linker.

In some embodiments, the polynucleic acid molecule conjugate is of Formula (III):

A-X-C-Y-B Formula III

wherein,
A is a binding moiety;
B is a polynucleotide;
C is a polymer;
X is a bond or first linker; and
Y is a bond or second linker.

In some embodiments, the at least one 2' modified nucleotide comprises a morpholino, 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified nucleotide. In some embodiments, the at least one 2' modified nucleotide comprises locked nucleic acid (LNA), ethylene nucleic acid (ENA), or a peptide nucleic acid (PNA). In some embodiments, the at least one 2' modified nucleotide comprises a morpholino. In some embodiments, the at least one inverted basic moiety is at least one terminus. In some embodiments, the at least one modified internucleotide linkage comprises a phosphorothioate linkage or a phosphorodithioate linkage. In some embodiments, the polynucleic acid molecule is at least from about 10 to about 30 nucleotides in length. In some embodiments, the polynucleic acid molecule is at least one of: from about 15 to about 30, from about 18 to about 25, from about 18 to about 24, from about 19 to about 23, or from about 20 to about 22 nucleotides in length. In some embodiments, the polynucleic acid molecule is at least about 16, 17, 18, 19, 20, 21, 22, 23, 24, or 25 nucleotides in length. In some embodiments, the polynucleic acid molecule comprises at least one of: from about 5% to about 100% modification, from about 10% to about 100% modification, from about 20% to about 100% modification, from about 30% to about 100% modification, from about 40% to about 100% modification, from about 50% to about 100% modification, from about 60% to about 100% modification, from about 70% to about 100% modification, from about 80% to about 100% modification, and from about 90% to about 100% modification. In some embodiments, the polynucleic acid molecule comprises at least one of: from about 10% to about 90% modification, from about 20% to about 90% modification, from about 30% to about 90% modification, from about 40% to about 90% modification, from about 50% to about 90% modification, from about 60% to about 90% modification, from about 70% to about 90% modification, and from about 80% to about 100% modification. In some embodiments, the polynucleic acid molecule comprises at least one of: from about 10% to about 80% modification, from about 20% to about 80% modification, from about 30% to about 80% modification, from about 40% to about 80% modification, from about 50% to about 80% modification, from about 60% to about 80% modification, and from about 70% to about 80% modification. In some embodiments, the polynucleic acid molecule comprises at least one of: from about 10% to about 70% modification, from about 20% to about 70% modification, from about 30% to about 70% modification, from about 40% to about 70% modification, from about 50% to about 70% modification, and from about 60% to about 70% modification. In some embodiments, the polynucleic acid molecule comprises at least one of: from about 10% to about 60% modification, from about 20% to about 60% modification, from about 30% to about 60% modification, from about 40% to about 60% modification, and from about 50% to about 60% modification. In some embodiments, the polynucleic acid molecule comprises at least one of: from about 10% to about 50% modification, from about 20% to about 50% modification, from about 30% to about 50% modification, and from about 40% to about 50% modification. In some embodiments, the polynucleic acid molecule comprises at least one of: from about 10% to about 40% modification, from about 20% to about 40% modification, and from about 30% to about 40% modification. In some embodiments, the polynucleic acid molecule comprises at least one of: from about 10% to about 30% modification, and from about 20% to about 30% modification. In some embodiments, the polynucleic acid molecule comprises from about 10% to about 20% modification. In some embodiments, the polynucleic acid molecule comprises from about 15% to about 90%, from about 20% to about 80%, from about 30% to about 70%, or from about 40% to about 60% modifications. In some embodiments, the polynucleic acid molecule comprises at least about 15%, 20%, 30%, 40%, 50%, 60%, 70%, 80%, 90%, 95%, or 99% modification. In some embodiments, the polynucleic acid molecule comprises at least about 3, about 4, about 5, about 6, about 7, about 8, about 9, about 10, about 11, about 12, about 13, about 14, about 15, about 16, about 17, about 18, about 19, about 20, about 21, about 22 or more modifications. In some embodiments, the polynucleic acid molecule comprises at least about 1, about 2, about 3, about 4, about 5, about 6, about 7, about 8, about 9, about 10, about 11, about 12, about 13, about 14, about 15, about 16, about 17, about 18, about 19, about 20, about 21, about 22 or more modified nucleotides. In some embodiments, the polynucleic acid molecule comprises a single strand.

In some embodiments, the polynucleic acid molecule comprises two or more strands. In some embodiments, the polynucleic acid molecule comprises a first polynucleotide and a second polynucleotide hybridized to the first polynucleotide to form a double-stranded polynucleic acid molecule. In some embodiments, the second polynucleotide comprises at least one modification. In some embodiments, the first polynucleotide and the second polynucleotide are RNA molecules. In some embodiments, the first polynucleotide and the second polynucleotide are siRNA molecules. In some embodiments, X and Y are independently a bond, a degradable linker, a non-degradable linker, a cleavable linker, or a non-polymeric linker group. In some embodiments, X is a bond. In some embodiments, X is a C₁-C₆ alkyl group. In some embodiments, Y is a C₁-C₆ alkyl group. In some embodiments, X is a homobifuctional linker or a heterobifunctional linker, optionally conjugated to a C₁-C₆ alkyl group. In some embodiments, Y is a homobifuctional linker or a heterobifunctional linker. In some embodiments, the binding moiety is an antibody or binding fragment thereof. In some embodiments, the antibody or binding fragment thereof comprises a humanized antibody or binding fragment thereof, chimeric antibody or binding fragment thereof, monoclonal antibody or binding fragment thereof, monovalent Fab', divalent Fab2, single-chain variable fragment (scFv), diabody, minibody, nanobody, single-domain antibody (sdAb), or camelid antibody or binding fragment thereof. In some embodiments, C is polyethylene glycol. In some embodiments, C has a molecular weight of about 5000 Da. In some embodiments, A-X is conjugated to the 5' end of B and Y-C is conjugated to the 3' end of B. In some embodiments, Y-C is conjugated to the 5' end of B and A-X is conjugated to the 3' end of B. In some embodiments, A-X, Y-C or a combination thereof is conjugated to an internucleotide linkage group. In some embodiments, methods further comprise D. In some embodiments, D is conjugated to C or to A. In some embodiments, D is conjugated to the molecule conjugate of Formula (II) according to Formula (IV):

(A-X-B-Y-C_{c})-L-D Formula IV

wherein,
A is a binding moiety;
B is a polynucleotide;
C is a polymer;
X is a bond or first linker;
Y is a bond or second linker;
L is a bond or third linker;
D is an endosomolytic moiety; and
c is an integer between 0 and 1; and
wherein the polynucleotide comprises at least one 2' modified nucleotide, at least one modified internucleotide linkage, or an inverted abasic moiety; and D is conjugated anywhere on A, B, or C.

In some embodiments, D is INF7 or melittin. In some embodiments, L is a C₁-C₆ alkyl group. In some embodiments, L is a homobifuctional linker or a heterobifunctional linker. In some embodiments, methods further comprise at least a second binding moiety A. In some embodiments, the at least second binding moiety A is conjugated to A, to B, or to C.

Disclosed herein, in some embodiments, are methods of inducing an insertion, deletion, duplication, or alteration in the incorrectly spliced mRNA transcript to induce exon skipping or exon inclusion in the incorrectly spliced mRNA transcript, the method comprising: contacting a target cell with a polynucleic acid molecule conjugate, wherein the polynucleotide comprises at least one 2' modified nucleotide, at least one modified internucleotide linkage, or at least one inverted abasic moiety; hybridizing the polynucleic acid molecule conjugate to the incorrectly spliced mRNA transcript within the target cell to induce an insertion, deletion, duplication, or alteration in the incorrectly spliced mRNA transcript to induce exon skipping or exon inclusion, wherein the incorrectly spliced mRNA transcript is capable of encoding a functional form of a protein; and translating the functional form of a protein from a fully processed mRNA transcript of the previous step. In some embodiments, the target cell is a target cell of a subject. In some embodiments, the incorrectly spliced mRNA transcript further induces a disease or disorder. In some embodiments, the disease or disorder is further characterized by one or more mutations in the mRNA. In some embodiments, the disease or disorder comprises a neuromuscular disease, a genetic disease, cancer, a hereditary disease, or a cardiovascular disease. In some embodiments, the disease or disorder is muscular dystrophy. In some embodiments, the disease or disorder is Duchenne muscular dystrophy. In some embodiments, the exon skipping is of exon 8, 23, 35, 43, 44, 45, 50, 51, 52, 53, or 55 of the *DMD* gene. In some embodiments, the exon skipping is of exon 23 of the *DMD* gene. In some embodiments, the polynucleic acid molecule conjugate is of Formula (I):

A-X-B Formula I

wherein,
A is a binding moiety;
B is a polynucleotide; and
X is a bond or first linker.

In some embodiments, the polynucleic acid molecule conjugate is of Formula (II):

A-X-B-Y-C Formula II

wherein,
A is a binding moiety;
B is a polynucleotide;
C is a polymer;
X is a bond or first linker; and
Y is a bond or second linker.

In some embodiments, the polynucleic acid molecule conjugate is of Formula (III):

A-X-C-Y-B Formula III

wherein,
A is a binding moiety;
B is a polynucleotide;
C is a polymer;
X is a bond or first linker; and
Y is a bond or second linker.

In some embodiments, the at least one 2' modified nucleotide comprises a morpholino, 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified nucleotide. In some embodiments, the at least one 2' modified nucleotide comprises locked nucleic acid (LNA), ethylene nucleic acid (ENA), peptide nucleic acid (PNA). In some embodiments, the at least one 2' modified nucleotide comprises a morpholino. In some embodiments, the at least one inverted basic moiety is at least one terminus. In some embodiments, the at least one modified internucleotide linkage comprises a phosphorothioate linkage or a phosphorodithioate linkage. In some embodiments, the polynucleic acid molecule is at least from about 10 to about 30 nucleotides in length. In some embodiments, the polynucleic acid molecule is at least one of: from about 15 to about 30, from about 18 to about 25, from about 18 to about 24, from about 19 to about 23, or from about 20 to about 22 nucleotides in length. In some embodiments, the polynucleic acid molecule is at least about 16, 17, 18, 19, 20, 21, 22, 23, 24, or 25 nucleotides in length. In some embodiments, the polynucleic acid molecule comprises at least one of: from about 5% to about 100% modification, from about 10% to about 100% modification, from about 20% to about 100% modification, from about 30% to about 100% modification, from about 40% to about 100% modification, from about 50% to about 100% modification, from about 60% to about 100% modification, from about 70% to about 100% modification, from about 80% to about 100% modification, and from about 90% to about 100% modification. In some embodiments, the polynucleic acid molecule comprises at least one of: from about 10% to about 90% modification, from about 20% to about 90% modification, from about 30% to about 90% modification, from about 40% to about 90% modification, from about 50% to about 90% modification, from about 60% to about 90% modification, from about 70% to about 90% modification, and from about 80% to about 100% modification. In some embodiments, the polynucleic acid molecule comprises at least one of: from about 10% to about 80% modification, from about 20% to about 80% modification, from about 30% to about 80% modification, from about 40% to about 80% modification, from about 50% to about 80% modification, from about 60% to about 80% modification, and from about 70% to about 80% modification. In some embodiments, the polynucleic acid molecule comprises at least one of: from about 10% to about 70% modification, from about 20% to about 70% modification, from about 30% to about 70% modification, from about 40% to about 70% modification, from about 50% to about 70% modification, and from about 60% to about 70% modification. In some embodiments, the polynucleic acid molecule comprises at least one of: from about 10% to about 60% modification, from about 20% to about 60% modification, from about 30% to about 60% modification, from about 40% to about 60% modification, and from about 50% to about 60% modification. In some embodiments, the polynucleic acid molecule comprises at least one of: from about 10% to about 50% modification, from about 20% to about 50% modification, from about 30% to about 50% modification, and from about 40% to about 50% modification. In some embodiments, the polynucleic acid molecule comprises at least one of: from about 10% to about 40% modification, from about 20% to about 40% modification, and from about 30% to about 40% modification. In some embodiments, the polynucleic acid molecule comprises at least one of: from about 10% to about 30% modification, and from about 20% to about 30% modification. In some embodiments, the polynucleic acid molecule comprises from about 10% to about 20% modification. In some embodiments, the polynucleic acid molecule comprises from about 15% to about 90%, from about 20% to about 80%, from about 30% to about 70%, or from about 40% to about 60% modifications. In some embodiments, the polynucleic acid molecule comprises at least about 15%, 20%, 30%, 40%, 50%, 60%, 70%, 80%, 90%, 95%, or 99% modification. In some embodiments, the polynucleic acid molecule comprises at least about 3, about 4, about 5, about 6, about 7, about 8, about 9, about 10, about 11, about 12, about 13, about 14, about 15, about 16, about 17, about 18, about 19, about 20, about 21, about 22 or more modifications. In some embodiments, the polynucleic acid molecule comprises at least about 1, about 2, about 3, about 4, about 5, about 6, about 7, about 8, about 9, about 10, about 11, about 12, about 13, about 14, about 15, about 16, about 17, about 18, about 19, about 20, about 21, about 22 or more modified nucleotides. In some embodiments, the polynucleic acid molecule comprises a single strand.

In some embodiments, the polynucleic acid molecule comprises two or more strands. In some embodiments, the polynucleic acid molecule comprises a first polynucleotide and a second polynucleotide hybridized to the first polynucleotide to form a double-stranded polynucleic acid molecule. In some embodiments, the second polynucleotide comprises at least one modification. In some embodiments, the first polynucleotide and the second polynucleotide are RNA molecules. In some embodiments, the first polynucleotide and the second polynucleotide are siRNA molecules. In some embodiments, X and Y are independently a bond, a degradable linker, a non-degradable linker, a cleavable linker, or a non-polymeric linker group. In some embodiments, X is a bond. In some embodiments, X is a C₁-C₆ alkyl group. In some embodiments, Y is a C₁-C₆ alkyl group. In some embodiments, X is a homobifuctional linker or a heterobifunctional linker, optionally conjugated to a C₁-C₆ alkyl group. In some embodiments, Y is a homobifuctional linker or a heterobifunctional linker. In some embodiments, the binding moiety is an antibody or binding fragment thereof. In some embodiments, the antibody or binding fragment thereof comprises a humanized antibody or binding fragment thereof, chimeric antibody or binding fragment thereof, monoclonal antibody or binding fragment thereof, monovalent Fab', divalent Fab2, single-chain variable fragment (scFv), diabody, minibody, nanobody, single-domain antibody (sdAb), or camelid antibody or binding fragment thereof. In some embodiments, C is polyethylene glycol. In some embodiments, C has a molecular weight of about 5000 Da. In some embodiments, A-X is conjugated to the 5' end of B and Y-C is conjugated to the 3' end of B. In some embodiments, Y-C is conjugated to the 5' end of B and A-X is conjugated to the 3' end of B. In some embodiments, A-X, Y-C or a combination thereof is conjugated to an internucleotide linkage group. In some embodiments, methods further comprise D. In some embodiments, D is conjugated to C or to A. In some embodiments, D is conjugated to the molecule conjugate of Formula (II) according to Formula (IV):

(A-X-B-Y-C_{c})-L-D Formula IV

wherein,
A is a binding moiety;
B is a polynucleotide;
C is a polymer;
X is a bond or first linker;
Y is a bond or second linker;
L is a bond or third linker;
D is an endosomolytic moiety; and
c is an integer between 0 and 1; and
wherein the polynucleotide comprises at least one 2' modified nucleotide, at least one modified internucleotide linkage, or an inverted abasic moiety; and D is conjugated anywhere on A, B, or C.

In some embodiments, D is INF7 or melittin. In some embodiments, L is a C₁-C₆ alkyl group. In some embodiments, L is a homobifuctional linker or a heterobifunctional linker. In some embodiments, methods further comprise at least a second binding moiety A. In some embodiments, the at least second binding moiety A is conjugated to A, to B, or to C. In some embodiments, the method is an *in vivo* method. In some embodiments, the method is an *in vitro* method. In some embodiments, the subject is a human.

Disclosed herein, in certain embodiments, are pharmaceutical compositions comprising: a molecule obtained by any one of the methods disclosed herein and a pharmaceutically acceptable excipient. In some embodiments, the pharmaceutical composition is formulated as a nanoparticle formulation. In some embodiments, the pharmaceutical composition is formulated for parenteral, oral, intranasal, buccal, rectal, or transdermal administration.

Disclosed herein, in certain embodiments, are compositions comprising a polynucleic acid molecule conjugate, wherein the polynucleic acid molecule conjugate comprises a polynucleotide comprising a sequence having at least 60%, 65%, 70%, 75%, 80%, 85%, 90%, 95%, 96%, 97%, 98%, 99%, or 100% sequence identity to SEQ ID NOs: 45-963. Disclosed herein, in certain embodiments, are compositions comprising a polynucleic acid molecule conjugate, wherein the polynucleic acid molecule conjugate comprises a polynucleotide comprising a sequence having at least 80%, 85%, 90%, 95%, 96%, 97%, 98%, 99%, or 100% sequence identity to SEQ ID NOs: 45-963. In certain embodiments, the polynucleic acid molecule conjugate is of Formula (I):

A-X-B Formula I

wherein,
A is a binding moiety;
B is the polynucleotide; and
X is a bond or first linker.

In certain embodiments, the polynucleic acid molecule conjugate is of Formula (II):

A-X-B-Y-C Formula II

wherein,
A is a binding moiety;
B is the polynucleotide;
C is a polymer;
X is a bond or first linker; and
Y is a bond or second linker.

In certain embodiments, the polynucleic acid molecule conjugate is of Formula (III):

A-X-C-Y-B Formula III

wherein,
A is a binding moiety;
B is the polynucleotide;
C is a polymer;
X is a bond or first linker; and
Y is a bond or second linker.

In certain embodiments, the at least one 2' modified nucleotide comprises a morpholino, 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA) modified nucleotide. In certain embodiments, the at least one 2' modified nucleotide comprises a morpholino.

Disclosed herein, in certain embodiments, is a polynucleic acid conjugate comprising a target cell binding moiety binding to at least one polynucleic acid molecule that hybridizes to a target region of a pre-mRNA transcript of *DMD* gene, wherein the at least one polynucleic acid molecule induces splicing out of an exon from a pre-mRNA transcript to generate a mRNA transcript that encodes a functional dystrophin protein. In some embodiments, the functional dystrophin protein is a truncated form of the dystrophin protein. In some embodiments, the target region is at an exon-intron junction, wherein the exon is the exon that is to be spliced out. In some embodiments, the exon is exon 8, 23, 35, 43, 44, 45, 50, 51, 52, 53, or 55. In some embodiments, the exon-intron junction is located at the 5' of the exon that is to be spliced out. In some embodiments, the target region is an intronic region upstream of the exon-intron junction. In some embodiments, the target region is about 500, 450, 400, 350, 300, 250, 200, 150, 100, 90, 80, 70, 60, 50, 40, 30, 20, or 10 nucleotides upstream of the exon-intron junction. In some embodiments, the exon-intron junction is located at the 3' of the exon that is to be spliced out. In some embodiments, the target region is an intronic region downstream of the exon-intron junction. In some embodiments, the target region is about 500, 450, 400, 350, 300, 250, 200, 150, 100, 90, 80, 70, 60, 50, 40, 30, 20, or 10 nucleotides downstream of the exon-intron junction. In some embodiments, the target cell binding moiety binds to two or more, three or more, four or more, five or more, six or more, or eight or more polynucleic acid molecules. In some embodiments, the polynucleic acid molecule is from about 10 to about 50 nucleotides in length. In some embodiments, the polynucleic acid molecule comprises about 80%, 85%, 90%, 95%, 96%, 97%, 98%, or 99% sequence identity to a sequence selected from SEQ ID NOs: 964-1285. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 964-1285. In some embodiments, the polynucleic acid molecule further comprises 1, 2, 3, or 4 mismatches. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 1056-1094, 1147-1162, or 1173-1211. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 1056-1076. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 1077-1094. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 1147-1162. In some embodiments, the polynucleic acid molecule comprises at least 10, 15, 16, 17, 18, 19, 20, 21, 22, 23, 24, 25, 26, or more contiguous bases of a base sequence selected from SEQ ID NOs: 1173-1211. In some embodiments, the binding moiety comprises an antibody. In some embodiments, the antibody comprises an anti-transferrin antibody. In some embodiments, the binding moiety comprises a plasma protein. In some embodiments, the polynucleic acid conjugate comprises A-(X¹-B)_{n;} Formula (V), wherein, A comprises the binding moiety; B consists of the polynucleic acid molecule; X¹ consists of a bond or first non-polymeric linker; and n is an averaged value selected from 1-12. In some embodiments, the polynucleic acid molecule comprises a passenger strand and a guide strand. In some embodiments, the guide strand comprises at least one modified internucleotide linkage, at least one inverted abasic moiety, at least one 5'-vinylphosphonate modified non-natural nucleotide, or a combination thereof. In some embodiments, the guide strand comprises about 2, 3, 4, 5, 6, 7, 8, or 9 phosphorothioate-modified non-natural nucleotides. In some embodiments, the guide strand comprises 1 phosphorothioate-modified non-natural nucleotide. In some embodiments, the phosphorothioate modified non-natural nucleotide is located at an internucleotide linkage of the polynucleotide. In some embodiments, the at least one 5'-vinylphosphonate modified non-natural nucleotide is located about 1, 2, 3, 4, or 5 bases away from the 5' terminus of the guide strand. In some embodiments, the at least one 5'-vinylphosphonate modified non-natural nucleotide is further modified at the 2'-position. In some embodiments, the 2'-modification is selected from 2'-O-methyl, 2'-O-methoxyethyl (2'-O-MOE), 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified nucleotide. In some embodiments, the passenger strand comprises at least 6, 7, 8, 9, 10, 11, 12, 13, 14, 15, 16, 17, 18, 19, 20, or more phosphorodiamidate morpholino oligomer-modified non-natural nucleotides. In some embodiments, the passenger strand comprises 100% phosphorodiamidate morpholino oligomer-modified non-natural nucleotides. In some embodiments, the passenger strand is shorter in length than the guide strand, thereby generating a 5' overhang, a 3' overhang, or a combination thereof. In some embodiments, the passenger strand is equal in length to the guide strand, thereby generating a blunt end at each terminus of the polynucleic acid molecule. In some embodiments, the polynucleic acid molecule is a phosphorodiamidate morpholino oligomer/RNA hetero-duplex. In some embodiments, the passenger strand comprises at least 6, 7, 8, 9, 10, 11, 12, 13, 14, 15, 16, 17, 18, 19, 20, or more peptide nucleic acid-modified non-natural nucleotides. In some embodiments, the passenger strand comprises 100% peptide nucleic acid-modified non-natural nucleotides. In some embodiments, the passenger strand is shorter in length than the guide strand, thereby generating a 5' overhang, a 3' overhang, or a combination thereof. In some embodiments, the passenger strand is equal in length to the guide strand, thereby generating a blunt end at each terminus of the polynucleic acid molecule. In some embodiments, the polynucleic acid molecule is a peptide nucleic acid/RNA hetero-duplex. In some embodiments, the passenger strand is conjugated to A- X¹. In some embodiments, A- X¹ is conjugated to the 5' end of the passenger strand. In some embodiments, A- X¹ is conjugated to the 3' end of the passenger strand. In some embodiments, X¹ is a bond. In some embodiments, X¹ is a C₁-C₆ alkyl group. In some embodiments, X¹ is a homobifuctional linker or a heterobifunctional linker, optionally conjugated to a C₁-C₆ alkyl group. In some embodiments, the polynucleic acid conjugate further comprises C. In some embodiments, C is polyethylene glycol. In some embodiments, C is directly conjugated to B via X². In some embodiments, X² consists of a bond or second non-polymeric linker. In some embodiments, X² is a bond. In some embodiments, X² is a C₁-C₆ alkyl group. In some embodiments, X² is a homobifuctional linker or a heterobifunctional linker, optionally conjugated to a C₁-C₆ alkyl group. In some embodiments, the passenger strand is conjugated to A- X¹ and X²-C. In some embodiments, A- X¹ is conjugated to the 5' end of the passenger strand and X²-C is conjugated to the 3' end of the passenger strand. In some embodiments, X²-C is conjugated to the 5' end of the passenger strand and A- X¹ is conjugated to the 3' end of the passenger strand. In some embodiments, the polynucleic acid conjugate comprises: A-X¹-(B-X²-C)ₙ; Formula (VI), wherein, A comprises the binding moiety; B consists of the polynucleic acid molecule; C consists of a polymer; X¹ consists a bond or first non-polymeric linker; X² consists of a bond or second non-polymeric linker; and n is an averaged value selected from 1-12. In some embodiments, the polynucleic acid conjugate further comprises D. In some embodiments, D is an endosomolytic moiety.

Disclosed herein, in certain embodiments, is a polynucleic acid molecule comprising at least 23 contiguous bases of a base sequence selected from SEQ ID NOs: 1056-1058 or 1087-1089, wherein the polynucleic acid molecule comprises no more than 50 nucleotides in length.

Disclosed herein, in certain embodiments, is a polynucleic acid molecule comprising SEQ ID NOs: 1056-1058, wherein the polynucleic acid molecule comprises no more than 50 nucleotides in length.

Disclosed herein, in certain embodiments, is a polynucleic acid molecule comprising SEQ ID NOs: 1087-1089, wherein the polynucleic acid molecule comprises no more than 50 nucleotides in length.

Disclosed herein, in certain embodiments, is a pharmaceutical composition, comprising: a polynucleic acid conjugate described herein or a polynucleic acid molecule described herein; and a pharmaceutically acceptable excipient. In some embodiments, the pharmaceutical composition is formulated for systemic delivery. In some embodiments, the pharmaceutical composition is formulated for parenteral administration.

Disclosed herein, in certain embodiments, is a method of treating a disease or condition characterized with a defective mRNA in a subject in need thereof, comprising: administering to the subject a polynucleic acid conjugate described herein or a polynucleic acid molecule described herein to induce skipping of an exon that leads to the defective mRNA to generate a processed mRNA encoding a functional protein, thereby treating the disease or condition in the subject. In some embodiments, the disease or condition is a neuromuscular disease, a genetic disease, cancer, a hereditary disease, or a cardiovascular disease. In some embodiments, the neuromuscular disease is a muscular dystrophy. In some embodiments, the muscular dystrophy is Duchenne muscular dystrophy, Becker muscular dystrophy, facioscapulohumeral muscular dystrophy, congenital muscular dystrophy, or myotonic dystrophy. In some embodiments, the subject is a human.

Disclosed herein, in certain embodiments, is a method of treating a muscular dystrophy in a subject in need thereof, comprising: administering to the subject a polynucleic acid conjugate described herein or a polynucleic acid molecule described herein, thereby treating the muscular dystrophy in the subject. In some embodiments, the muscular dystrophy is Duchenne muscular dystrophy. In some embodiments, the subject is a human.

Disclosed herein, in certain embodiments, is a kit comprising a polynucleic acid conjugate described herein or a polynucleic acid molecule described herein.

Disclosed herein, in certain embodiments, are kits comprising a molecule obtained by any one of the methods disclosed herein.

### DESCRIPTION OF THE DRAWINGS

**Fig. 1** depicts a phosphorodiamidate morpholino oligomer (PMO) sequence with end nucleotides expanded.
**Fig. 2A** depicts a phosphorothioate antisense oligonucleotide (PS ASO) sequence with end nucleotides expanded.
**Fig. 2B** depicts a fully expanded phosphorothioate antisense oligonucleotide (PS ASO) sequence.
**Fig. 3** depicts methods used to quantify skipped DMD mRNA in total RNA using Taqman qPCR.
**Fig. 4** depicts a chromatogram of anti-CD71 mAb-PMO reaction mixture produced with hydrophobic interaction chromatography (HIC) method 2.
**Fig. 5A** depicts a chromatogram of anti-CD71 mAb produced using size exclusion chromatography (SEC) method 1.
**Fig. 5B** depicts a chromatogram of anti-CD71 mAb-PMO DAR 1,2 produced using size exclusion chromatography (SEC) method 1.
**Fig. 5C** depicts a chromatogram of anti-CD71 mAb-PMO DAR >2 produced using size exclusion chromatography (SEC) method 1.
**Fig. 6A** depicts a chromatogram of anti-CD71 mAb produced using hydrophobic interaction chromatography (HIC) method 2.
**Fig. 6B** depicts a chromatogram of purified anti-CD71 mAb-PMO DAR 1,2 conjugate produced using hydrophobic interaction chromatography (HIC) method 2.
**Fig. 6C** depicts a chromatogram of purified anti-CD71 mAb-PMO DAR >2 conjugate produced using hydrophobic interaction chromatography (HIC) method 2.
**Fig. 7A** depicts a chromatogram of fast protein liquid chromatography (FPLC) purification of anti-CD71 **Fab-PMO** using hydrophobic interaction chromatography (HIC) method 3.
**Fig. 7B** depicts a chromatogram of anti-CD71 Fab produced using SEC method 1.
**Fig. 7C** depicts a chromatogram of anti-CD71 Fab-PMO DAR 1 conjugate produced using SEC method 1.
**Fig. 7D** depicts a chromatogram of anti-CD71 Fab-PMO DAR 2 conjugate produced using SEC method 1.
**Fig. 7E** depicts a chromatogram of anti-CD71 Fab-PMO DAR 3 conjugate produced using SEC method 1.
**Fig. 7F** depicts a chromatogram of anti-CD71 Fab produced using HIC method 4.
**Fig. 7G** depicts a chromatogram of anti-CD71 Fab-PMO DAR 1 conjugate produced using HIC method 4.
**Fig. 7H** depicts a chromatogram of anti-CD71 Fab-PMO DAR 2 conjugate produced using HIC method 4.
**Fig. 7I** depicts a chromatogram of anti-CD71 Fab-PMO DAR 3 conjugate produced using HIC method 4.
**Fig. 8A** depicts a chromatogram of anti-CD71 mAb-PS ASO reaction mixture produced with SAX method 2.
**Fig. 8B** depicts a chromatogram of anti-CD71 mAb produced using SEC method 1.
**Fig. 8C** depicts a chromatogram of anti-CD71 mAb-PS ASO DAR 1 conjugate produced using SEC method 1.
**Fig. 8D** depicts a chromatogram of anti-CD71 mAb-PS ASO DAR 2 conjugate produced using SEC method **1.**
**Fig. 8E** depicts a chromatogram of anti-CD71 mAb-PS ASO DAR 3 conjugate produced using SEC method 1.
**Fig. 8F** depicts a chromatogram of anti-CD71 mAb-PS ASO DAR 1 conjugate produced using SAX **method 2.**
**Fig. 8G** depicts a chromatogram of anti-CD71 mAb-PS ASO DAR 2 conjugate produced using SAX method 2.
**Fig. 8H** depicts a chromatogram of anti-CD71 mAb-PS ASO DAR 3 conjugate produced using SAX method 2.
**Fig. 9** depicts an agarose gel from nested PCR detecting exon 23 skipping in differentiated C2C12 cells using PMO and anti-CD71 mAb-PMO conjugate.
**Fig. 10** depicts an agarose gel from nested PCR detecting exon 23 skipping in differentiated C2C12 cells using PMO, anti-CD71 mAb-PMO, and anti-CD71 Fab-PMO conjugates.
**Fig. 11** depicts an agarose gel from nested PCR detecting exon 23 skipping in differentiated C2C12 cells PMO, ASO, conjugated anti-CD71 mAb-ASO of DAR1 ("ASC-DAR1"), conjugated anti-CD71 mAb-ASO of DAR2 ("ASC-DAR2"), and conjugated anti-CD71 mAb-ASO of DAR3 ("ASC-DAR3").
**Fig. 12A** depicts an agarose gel from nested PCR detecting exon 23 skipping in gastrocnemius muscle of wild- type mice administered a single intravenous injection of anti-CD71 mAb-PMO conjugate.
**Fig. 12B** is a graph of quantification of PCR products from gastrocnemius muscle.
**Fig. 12C** is a graph of quantification of *in vivo* exon skipping using Taqman qPCR from gastrocnemius muscle from wild-type mice.
**Fig. 13A** depicts an agarose gel from nested PCR detecting exon 23 skipping in heart muscle from wild-type mice after a single intravenous injection.
**Fig. 13B** is a graph of quantification of PCR products from heart muscle.
**Fig. 14** depicts sequencing data of DNA fragments from skipped and wild-type PCR products.
**Fig. 15** illustrates exon skipping activity of exon-skipping PMOs at different lengths targeting exon 45 in the human DMD pre-mRNA in transfected primary human skeletal muscle cells.
**Fig. 16** illustrates binding of hTfR1.mAb-PMO conjugates to human Transferrin Receptor in vitro.
**Fig. 17** illustrates exon skipping activity of hTfR1.mAb-PMO conjugates in primary human skeletal muscle cells.
**Fig. 18** illustrates exon skipping activity of hTfR1.mAb-PMO conjugates in myotubes of primary and immortalized human skeletal muscle cells.

### DETAILED DESCRIPTION OF THE DISCLOSURE

Nucleic acid (e.g., RNAi) therapy is a targeted therapy with high selectivity and specificity. However, in some instances, nucleic acid therapy is also hindered by poor intracellular uptake, insufficient intracellular concentrations in target cells, and low efficacy. To address these issues, various modifications of the nucleic acid composition are explored, such as for example, novel linkers for better stabilizing and/or lower toxicity, optimization of binding moiety for increased target specificity and/or target delivery, and nucleic acid polymer modifications for increased stability and/or reduced off-target effect.

In some instances, one such area where oligonucleotide is used is for treating muscular dystrophy. Muscular dystrophy encompasses several diseases that affect the muscle. Duchenne muscular dystrophy is a severe form of muscular dystrophy and caused by mutations in the *DMD* gene. In some instances, mutations in the *DMD* gene disrupt the translational reading frame and results in non-functional dystrophin protein.

Described herein, in certain embodiments, are methods and compositions relating nucleic acid therapy to induce an insertion, deletion, duplication, or alteration in an incorrectly spliced mRNA transcript to induce exon skipping or exon inclusion, which is used to restore the translational reading frame. In some embodiments, also described herein include methods and compositions for treating a disease or disorder characterized by an incorrectly processed mRNA transcript, in which after removal of an exon, the mRNA is capable of encoding a functional protein, thereby treating the disease or disorder. In additional embodiments, described herein include pharmaceutical compositions and kits for treating the same.

### RNA Processing

RNA has a central role in regulation of gene expression and cell physiology. Proper processing of RNA is important for translational of functional protein. Alterations in RNA processing such as a result of incorrect splicing of RNA can result in disease. For example, mutations in a splice site causes exposure of a premature stop codon, a loss of an exon, or inclusion of an intron. In some instances, alterations in RNA processing results in an insertion, deletion, or duplication. In some instances, alterations in RNA processing results in an insertion, deletion, or duplication of an exon. Alterations in RNA processing, in some cases, results in an insertion, deletion, or duplication of an intron.

### Exon Skipping

Exon skipping is a form of RNA splicing. In some cases, exon skipping occurs when an exon is skipped over or is spliced out of the processed mRNA. As a result of exon skipping, the processed mRNA does not contain the skipped exon. In some instances, exon skipping results in expression of an altered product.

In some instances, antisense oligonucleotides (AONs) are used to induce exon skipping. In some instances, AONs are short nucleic acid sequences that bind to specific mRNA or pre-mRNA sequences. For example, AONs bind splice sites or exonic enhancers. In some instances, binding of AONs to specific mRNA or pre-mRNA sequences generates double-stranded regions. In some instances, formation of double-stranded regions occurs at sites where the spliceosome or proteins associated with the spliceosome would normally bind and causes exons to be skipped. In some instances, skipping of exons results in restoration of the transcript reading frame and allows for production of a partially functional protein.

### Exon Inclusion

In some instances, a mutation in RNA results in exon skipping. In some cases, a mutation is at least one of at the splice site, near the splice site, and at a distance from the splice site. In some instances, the mutations result in at least one of inactivating or weakening the splice site, disrupting exon splice enhancer or intron splice enhancer, and creating an exon splice silencer or intron splice enhancer. Mutations in some instances alter RNA secondary structure. In some cases, a mutation alters a RNA secondary structure result in disrupting the accessibility of signals important for exon recognition.

In some instances, use of AONs results in inclusion of the skipped exon. In some instances, the AONs bind to at least one of a splice site, a site near a splice site, and a site distant to a splice site. In some cases, AONs bind at site in the RNA to prevent disruption of an exon splice enhancer or intron splice enhancer. In some instances, AONs bind at site in the RNA to prevent creation of an exon splice silencer or intron splice silencer.

### Indications

In some embodiments, a polynucleic acid molecule or a pharmaceutical composition described herein is used for the treatment of a disease or disorder characterized with a defective mRNA. In some embodiments, a polynucleic acid molecule or a pharmaceutical composition described herein is used for the treatment of disease or disorder by inducing an insertion, deletion, duplication, or alteration in an incorrectly spliced mRNA transcript to induce exon skipping or exon inclusion.

A large percentage of human protein-coding genes are alternatively spliced. In some instances, a mutation results in improperly spliced or partially spliced mRNA. For example, a mutation is in at least one of a splice site in a protein coding gene, a silencer or enhancer sequence, exonic sequences, or intronic sequences. In some instances, a mutation results in gene dysfunction. In some instances, a mutation results in a disease or disorder.

In some instances, a disease or disorder resulting from improperly spliced or partially spliced mRNA includes, but not limited to, a neuromuscular disease, a genetic disease, cancer, a hereditary disease, or a cardiovascular disease.

In some instances, genetic diseases or disorders include an autosomal dominant disorder, an autosomal recessive disorder, X-linked dominant disorder, X-linked recessive disorder, Y-linked disorder, mitochondrial disease, or multifactorial or polygenic disorder.

In some instances, cardiovascular disease such as hypercholesterolemia results from improperly spliced or partially spliced mRNA. In hypercholesterolemia, it has been shown that a single nucleotide polymorphism in exon 12 of the low density lipoprotein receptor (LDLR) promotes exon skipping.

In some instances, improperly spliced or partially spliced mRNA results in cancer. For example, improperly spliced or partially spliced mRNA affects cellular processes involved in cancer including, but not limited to, proliferation, motility, and drug response. In some instances is a solid cancer or a hematologic cancer. In some instances, the cancer is bladder cancer, lung cancer, brain cancer, melanoma, breast cancer, Non-Hodgkin lymphoma, cervical cancer, ovarian cancer, colorectal cancer, pancreatic cancer, esophageal cancer, prostate cancer, kidney cancer, skin cancer, leukemia, thyroid cancer, liver cancer, or uterine cancer.

Improperly spliced or partially spliced mRNA in some instances causes a neuromuscular disease or disorder. Exemplary neuromuscular diseases include muscular dystrophy such as Duchenne muscular dystrophy, Becker muscular dystrophy, facioscapulohumeral muscular dystrophy, congenital muscular dystrophy, or myotonic dystrophy. In some instances, muscular dystrophy is genetic. In some instances, muscular dystrophy is caused by a spontaneous mutation. Becker muscular dystrophy and Duchenne muscular dystrophy have been shown to involve mutations in the *DMD* gene, which encodes the protein dystrophin. Facioscapulohumeral muscular dystrophy has been shown to involve mutations in double homeobox, 4 *(DUX4)* gene.

In some instances, improperly spliced or partially spliced mRNA causes Duchenne muscular dystrophy. Duchenne muscular dystrophy results in severe muscle weakness and is caused by mutations in the *DMD* gene that abolishes the production of functional dystrophin. In some instances, Duchenne muscular dystrophy is a result of a mutation in an exon in the *DMD* gene. In some instances, Duchenne muscular dystrophy is a result of a mutation in at least one of exon 1, 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78 and 79 in the *DMD* gene. In some instances, Duchenne muscular dystrophy is a result of a mutation in at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, and 63 in the *DMD* gene. In some instances, Duchenne muscular dystrophy is a result of a mutation in at least one of exon 8, 23, 35, 43, 44, 45, 50, 51, 52, 53, and 55 in the *DMD* gene. In some instances, multiple exons are mutated. For example, mutation of exons 48-50 is common in Duchenne muscular dystrophy patients. In some instances, Duchenne muscular dystrophy is a result of mutation of exon 51. In some instances, Duchenne muscular dystrophy is a result of mutation of exon 23. In some instances, a mutation involves a deletion of one or multiple exons. In some instances, a mutation involves a duplication of one or multiple exons. In some instances, a mutation involves a point mutation in an exon. For example, it has been shown that some patients have a nonsense point mutation in exon 51 of the *DMD* gene.

In some instances, a polynucleic acid molecule or a pharmaceutical composition described herein is used for the treatment of muscular dystrophy. In some instances, a polynucleic acid molecule or a pharmaceutical composition described herein is used for the treatment of Duchenne muscular dystrophy, Becker muscular dystrophy, facioscapulohumeral muscular dystrophy, congenital muscular dystrophy, or myotonic dystrophy. In some instances, a polynucleic acid molecule or a pharmaceutical composition described herein is used for the treatment of Duchenne muscular dystrophy.

### Polynucleic Acid Molecule

In some embodiments, a polynucleic acid molecule described herein that induces an insertion, deletion, duplication, or alteration in an incorrectly spliced mRNA transcript to induce exon skipping or exon inclusion. In some instances, the polynucleic acid molecule restores the translational reading frame. In some instances, the polynucleic acid molecule results in a functional and truncated protein.

In some instances, a polynucleic acid molecule targets an mRNA sequence. In some instances, the polynucleic acid molecule targets a splice site. In some instances, the polynucleic acid molecule targets a cis-regulatory element. In some instances, the polynucleic molecule targets a *trans-regulatory* element. In some instances, the polynucleic acid molecule targets exonic splice enhancers or intronic splice enhancers. In some instances, the polynucleic acid molecule targets exonic splice silencers or intronic splice silencers.

In some instances, a polynucleic acid molecule targets a sequence found in introns or exons. For example, the polynucleic acid molecule targets a sequence found in an exon that mediates splicing of said exon. In some instances, the polynucleic acid molecule targets an exon recognition sequence. In some instances, the polynucleic acid molecule targets a sequence upstream of an exon. In some instances, the polynucleic acid molecule targets a sequence downstream of an exon.

As described above, a polynucleic acid molecule targets an incorrectly processed mRNA transcript which results in a disease or disorder not limited to a neuromuscular disease, a genetic disease, cancer, a hereditary disease, or a cardiovascular disease. In some cases, a polynucleic acid molecule targets an incorrectly processed mRNA transcript which results in a neuromuscular disease or disorder. In some cases, a neuromuscular disease or disorder is Duchenne muscular dystrophy, Becker muscular dystrophy, facioscapulohumeral muscular dystrophy, congenital muscular dystrophy, or myotonic dystrophy. In some cases, a polynucleic acid molecule targets an incorrectly processed mRNA transcript which results in Duchenne muscular dystrophy, Becker muscular dystrophy, facioscapulohumeral muscular dystrophy, congenital muscular dystrophy, or myotonic dystrophy. In some cases, a polynucleic acid molecule targets an incorrectly processed mRNA transcript which results in Duchenne muscular dystrophy.

In some instances, a polynucleic acid molecule targets an exon that is mutated in the *DMD* gene that causes Duchenne muscular dystrophy. Exemplary exons that are mutated in the *DMD* gene that causes Duchenne muscular dystrophy include, but not limited to, exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, and 78. In some instances, the polynucleic acid molecule targets a sequence adjacent to a mutated exon. For example, if there is a deletion of exon 50, the polynucleic acid molecule targets a sequence in exon 51 so that exon 51 is skipped. In another instance, if there is a mutation in exon 23, the polynucleic acid molecule targets a sequence in exon 22 so that exon 23 is skipped.

In some instances, a polynucleic acid molecule described herein targets a region that is at the exon-intron junction of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, or 78 of the *DMD* gene. In some instances, a polynucleic acid molecule described herein targets a region that is at the exon-intron junction of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, or 63 of the *DMD* gene. In some instances, a polynucleic acid molecule described herein targets a region that is at the exon-intron junction of exon 8, 23, 35, 43, 44, 45, 50, 51, 52, 53, or 55 of the *DMD* gene. In some cases, a polynucleic acid molecule described herein targets a region that is at the exon-intron junction of exon 8 of the *DMD* gene. In some cases, a polynucleic acid molecule described herein targets a region that is at the exon-intron junction of exon 23 of the *DMD* gene. In some cases, a polynucleic acid molecule described herein targets a region that is at the exon-intron junction of exon 35 of the *DMD* gene. In some cases, a polynucleic acid molecule described herein targets a region that is at the exon-intron junction of exon 43 of the *DMD* gene. In some cases, a polynucleic acid molecule described herein targets a region that is at the exon-intron junction of exon 44 of the *DMD* gene. In some cases, a polynucleic acid molecule described herein targets a region that is at the exon-intron junction of exon 45 of the *DMD* gene. In some cases, a polynucleic acid molecule described herein targets a region that is at the exon-intron junction of exon 48 of the *DMD* gene. In some cases, a polynucleic acid molecule described herein targets a region that is at the exon-intron junction of exon 49 of the *DMD* gene. In some cases, a polynucleic acid molecule described herein targets a region that is at the exon-intron junction of exon 50 of the *DMD* gene. In some cases, a polynucleic acid molecule described herein targets a region that is at the exon-intron junction of exon 51 of the *DMD* gene. In some cases, a polynucleic acid molecule described herein targets a region that is at the exon-intron junction of exon 52 of the *DMD* gene. In some cases, a polynucleic acid molecule described herein targets a region that is at the exon-intron junction of exon 53 of the *DMD* gene. In some cases, a polynucleic acid molecule described herein targets a region that is at the exon-intron junction of exon 55 of the *DMD* gene.

In some instances, the polynucleic acid molecule hybridizes to a target region that is at either the 5' intron-exon junction or the 3' exon-intron junction of at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene. In some instances, the polynucleic acid molecule hybridizes to a target region that is at either the 5' intron-exon junction or the 3' exon-intron junction of at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene. In some instances, the polynucleic acid molecule hybridizes to a target region that is at either the 5' intron-exon junction or the 3' exon-intron junction of exon 8, 23, 35, 43, 44, 45, 50, 51, 52, 53, or 55 of the *DMD* gene.

In some cases, the polynucleic acid molecule hybridizes to a target region that is at the 5' intron-exon junction of at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene (e.g., the 5' intron-exon junction of exon 3 is the junction intron 2-exon 3). In some cases, the polynucleic acid molecule hybridizes to a target region that is at the 5' intron-exon junction of at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene (e.g., the 5' intron-exon junction of exon 3 is the junction intron 2-exon 3). In some cases, the polynucleic acid molecule hybridizes to a target region that is at the 5' intron-exon junction of exon 8, 23, 35, 43, 44, 45, 50, 51, 52, 53, or 55 of the *DMD* gene. In some cases, the polynucleic acid molecule hybridizes to a target region that is at the 5' intron-exon junction of exon 8 of the *DMD* gene. In some cases, the polynucleic acid molecule hybridizes to a target region that is at the 5' intron-exon junction of exon 23 of the *DMD* gene. In some cases, the polynucleic acid molecule hybridizes to a target region that is at the 5' intron-exon junction of exon 35 of the *DMD* gene. In some cases, the polynucleic acid molecule hybridizes to a target region that is at the 5' intron-exon junction of exon 43 of the *DMD* gene. In some cases, the polynucleic acid molecule hybridizes to a target region that is at the 5' intron-exon junction of exon 44 of the *DMD* gene. In some cases, the polynucleic acid molecule hybridizes to a target region that is at the 5' intron-exon junction of exon 45 of the *DMD* gene. In some cases, the polynucleic acid molecule hybridizes to a target region that is at the 5' intron-exon junction of exon 50 of the *DMD* gene. In some cases, the polynucleic acid molecule hybridizes to a target region that is at the 5' intron-exon junction of exon 51 of the *DMD* gene. In some cases, the polynucleic acid molecule hybridizes to a target region that is at the 5' intron-exon junction of exon 52 of the *DMD* gene. In some cases, the polynucleic acid molecule hybridizes to a target region that is at the 5' intron-exon junction of exon 53 of the *DMD* gene. In some cases, the polynucleic acid molecule hybridizes to a target region that is at the 5' intron-exon junction of exon 55 of the *DMD* gene.

In some cases, the polynucleic acid molecule hybridizes to a target region that is at the 3' exon-intron junction of at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene (e.g., the 3' exon-intron junction of exon 3 is the junction exon 3-intron 3). In some cases, the polynucleic acid molecule hybridizes to a target region that is at the 3' exon-intron junction of at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene (e.g., the 3' exon-intron junction of exon 3 is the junction exon 3-intron 3). In some cases, the polynucleic acid molecule hybridizes to a target region that is at the 3' exon-intron junction of exon 8, 23, 35, 43, 44, 45, 50, 51, 52, 53, or 55 of the *DMD* gene. In some cases, the polynucleic acid molecule hybridizes to a target region that is at the 3' exon-intron junction of exon 8 of the *DMD* gene. In some cases, the polynucleic acid molecule hybridizes to a target region that is at the 3' exon-intron junction of exon 23 of the *DMD* gene. In some cases, the polynucleic acid molecule hybridizes to a target region that is at the 3' exon-intron junction of exon 35 of the *DMD* gene. In some cases, the polynucleic acid molecule hybridizes to a target region that is at the 3' exon-intron junction of exon 43 of the *DMD* gene. In some cases, the polynucleic acid molecule hybridizes to a target region that is at the 3' exon-intron junction of exon 44 of the *DMD* gene. In some cases, the polynucleic acid molecule hybridizes to a target region that is at the 3' exon-intron junction of exon 45 of the *DMD* gene. In some cases, the polynucleic acid molecule hybridizes to a target region that is at the 3' exon-intron junction of exon 50 of the *DMD* gene. In some cases, the polynucleic acid molecule hybridizes to a target region that is at the 3' exon-intron junction of exon 51 of the *DMD* gene. In some cases, the polynucleic acid molecule hybridizes to a target region that is at the 3' exon-intron junction of exon 52 of the *DMD* gene. In some cases, the polynucleic acid molecule hybridizes to a target region that is at the 3' exon-intron junction of exon 53 of the *DMD* gene. In some cases, the polynucleic acid molecule hybridizes to a target region that is at the 3' exon-intron junction of exon 55 of the *DMD* gene.

In some instances, a polynucleic acid molecule described herein targets a splice site of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene. In some instances, a polynucleic acid molecule described herein targets a splice site of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, or 63 of the *DMD* gene. In some instances, a polynucleic acid molecule described herein targets a splice site of exon 8, 23, 35, 43, 44, 45, 50, 51, 52, 53, or 55 of the *DMD* gene. In some cases, a polynucleic acid molecule described herein targets a splice site of exon 8 of the *DMD* gene. In some instances, a polynucleic acid molecule described herein targets a splice site of exon 23 of the *DMD* gene. In some cases, a polynucleic acid molecule described herein targets a splice site of exon 35 of the *DMD* gene. In some cases, a polynucleic acid molecule described herein targets a splice site of exon 43 of the *DMD* gene. In some cases, a polynucleic acid molecule described herein targets a splice site of exon 44 of the *DMD* gene. In some cases, a polynucleic acid molecule described herein targets a splice site of exon 45 of the *DMD* gene. In some instances, a polynucleic acid molecule described herein targets a splice site of exon 48 of the *DMD* gene. In some instances, a polynucleic acid molecule described herein targets a splice site of exon 49 of the *DMD* gene. In some instances, a polynucleic acid molecule described herein targets a splice site of exon 50 of the *DMD* gene. In some instances, a polynucleic acid molecule described herein targets a splice site of exon 51 of the *DMD* gene. In some cases, a polynucleic acid molecule described herein targets a splice site of exon 52 of the *DMD* gene. In some cases, a polynucleic acid molecule described herein targets a splice site of exon 53 of the *DMD* gene. In some cases, a polynucleic acid molecule described herein targets a splice site of exon 55 of the *DMD* gene. As used herein, a splice site includes a canonical splice site, a cryptic splice site or an alternative splice site that is capable of inducing an insertion, deletion, duplication, or alteration in an incorrectly spliced mRNA transcript to induce exon skipping or exon inclusion.

In some embodiments, a polynucleic acid molecule described herein target a partially spliced mRNA sequence comprising additional exons involved in Duchenne muscular dystrophy such as exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, or 63.

In some instances, the polynucleic acid molecule hybridizes to a target region that is proximal to the exon-intron junction. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, or 78 of the*DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, >8, 59, 60, 61, 62, or 63 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 8, 23, 35, 43, 44, 45, 50, 51, 52, 53, or 55 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 8 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 23 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 35 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 43 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 44 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 45 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 48 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 49 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 50 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 51 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 52 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 53 of the *DMD* gene. In some instances, a polynucleic acid molecule described herein targets a region at least 1000 nt, 500 nt, 400 nt, 300 nt, 200 nt, 100 nt, 80 nt, 60 nt, 50 nt, 40 nt, 30 nt, 20 nt, 10 nt, or 5 nt upstream (or from the 5') of exon 55 of the *DMD* gene.

In some instances, the polynucleic acid molecule hybridizes to a target region that is upstream (or 5') to at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene. In some instances, the polynucleic acid molecule hybridizes to a target region that is upstream (or 5') to at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene. In some instances, the polynucleic acid molecule hybridizes to a target region that is upstream (or 5') to at least one of exon 8, 23, 35, 43, 44, 45, 50, 51, 52, 53, or 55 of the *DMD* gene. In some instances, the polynucleic acid molecule hybridizes to a target region that is about 5, 10, 15, 20, 50, 100, 200, 300, 400 or 500 bp upstream (or 5') to at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene.

In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, or 78 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, or 63 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 8, 23, 35, 43, 44, 45, 50, 51, 52, 53, or 55 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 8 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 23 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 35 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 43 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 44 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 45 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 48 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 49 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 50 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 51 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 52 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 53 of the *DMD* gene. In some instances, a polynucleic acid molecule described herein targets a region at least 1000 nucleotides (nt), 500 nt, 400 nt, 300 nt, 200 nt, 100 nt, 80 nt, 60 nt, 50 nt, 40 nt, 30 nt, 20 nt, 10 nt, or 5 nt downstream (or from the 3') of exon 55 of the *DMD* gene.

In some instances, the polynucleic acid molecule hybridizes to a target region that is downstream (or 3') to at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene. In some instances, the polynucleic acid molecule hybridizes to a target region that is downstream (or 3') to at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene. In some instances, the polynucleic acid molecule hybridizes to a target region that is about 5, 10, 15, 20, 50, 100, 200, 300, 400 or 500 bp downstream (or 3') to at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene. In some instances, the polynucleic acid molecule hybridizes to a target region that is about 5, 10, 15, 20, 50, 100, 200, 300, 400 or 500 bp downstream (or 3') to at least one of exon 8, 23, 35, 43, 44, 45, 50, 51, 52, 53, or 55 of the *DMD* gene.

In some instances, a polynucleic acid molecule described herein targets an internal region within exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, or 78 of the *DMD* gene. In some instances, a polynucleic acid molecule described herein targets an internal region within exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, or 63 of the *DMD* gene. In some instances, a polynucleic acid molecule described herein targets an internal region within exon 8, 23, 35, 43, 44, 45, 50, 51, 52, 53, or 55 of the *DMD* gene. In some instances, a polynucleic acid molecule described herein targets an internal region within exon 8 of the *DMD* gene. In some instances, a polynucleic acid molecule described herein targets an internal region within exon 23 of the *DMD* gene. In some instances, a polynucleic acid molecule described herein targets an internal region within exon 35 of the *DMD* gene. In some instances, a polynucleic acid molecule described herein targets an internal region within exon 43 of the *DMD* gene. In some instances, a polynucleic acid molecule described herein targets an internal region within exon 44 of the *DMD* gene. In some instances, a polynucleic acid molecule described herein targets an internal region within exon 45 of the *DMD* gene. In some instances, a polynucleic acid molecule described herein targets an internal region within exon 48 of the *DMD* gene. In some instances, a polynucleic acid molecule described herein targets an internal region within exon 49 of the *DMD* gene. In some instances, a polynucleic acid molecule described herein targets an internal region within exon 50 of the *DMD* gene. In some instances, a polynucleic acid molecule described herein targets an internal region within exon 51 of the *DMD* gene. In some instances, a polynucleic acid molecule described herein targets an internal region within exon 52 of the *DMD* gene. In some instances, a polynucleic acid molecule described herein targets an internal region within exon 53 of the *DMD* gene. In some instances, a polynucleic acid molecule described herein targets an internal region within exon 55 of the *DMD* gene.

In some instances, the polynucleic acid molecule hybridizes to a target region that is within at least one of exon 2, 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, 63, 64, 65, 66, 67, 68, 69, 70, 71, 72, 73, 74, 75, 76, 77, and 78 of the *DMD* gene. In some instances, the polynucleic acid molecule hybridizes to a target region that is within at least one of exon 3, 4, 5, 6, 7, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 53, 54, 55, 56, 57, 58, 59, 60, 61, 62, and 63 of the *DMD* gene. In some instances, the polynucleic acid molecule hybridizes to a target region that is within at least one of exon 8, 23, 35, 43, 44, 45, 50, 51, 52, 53, or 55 of the *DMD* gene.

In some embodiments, a polynucleic acid molecule described herein targets a partially spliced mRNA sequence comprising exon 44 of the *DMD* gene. In some instances, the polynucleic acid molecule hybridizes to a target region that is upstream (or 5') to exon 44. In some instances, the polynucleic acid molecule hybridizes to a target region that is about 5, 10, 15, 20, 50, 100, 200, 300, 400 or 500 bp upstream (or 5') to exon 44. In some instances, the polynucleic acid molecule hybridizes to a target region that is downstream (or 3') to exon 44. In some instances, the polynucleic acid molecule hybridizes to a target region that is about 5, 10, 15, 20, 50, 100, 200, 300, 400 or 500 bp downstream (or 3') to exon 44.

In some instances, the polynucleic acid molecule hybridizes to a target region that is within exon 44 of the *DMD* gene. In some instances, the polynucleic acid molecule hybridizes to a target region that is at either the 5' intron-exon 44 junction or the 3' exon 44-intron junction.

In some embodiments, a polynucleic acid molecule described herein targets a partially spliced mRNA sequence comprising exon 45 of the *DMD* gene. In some instances, the polynucleic acid molecule hybridizes to a target region that is upstream (or 5') to exon 45. In some instances, the polynucleic acid molecule hybridizes to a target region that is about 5, 10, 15, 20, 50, 100, 200, 300, 400 or 500 bp upstream (or 5') to exon 45. In some instances, the polynucleic acid molecule hybridizes to a target region that is downstream (or 3') to exon 45. In some instances, the polynucleic acid molecule hybridizes to a target region that is about 5, 10, 15, 20, 50, 100, 200, 300, 400 or 500 bp downstream (or 3') to exon 45.

In some instances, the polynucleic acid molecule hybridizes to a target region that is within exon 45 of the *DMD* gene. In some instances, the polynucleic acid molecule hybridizes to a target region that is at either the 5' intron-exon 45 junction or the 3' exon 45-intron junction.

In some embodiments, a polynucleic acid molecule described herein targets a partially spliced mRNA sequence comprising exon 51 of the *DMD* gene. In some instances, the polynucleic acid molecule hybridizes to a target region that is upstream (or 5') to exon 51. In some instances, the polynucleic acid molecule hybridizes to a target region that is about 5, 10, 15, 20, 50, 100, 200, 300, 400 or 500 bp upstream (or 5') to exon 51. In some instances, the polynucleic acid molecule hybridizes to a target region that is downstream (or 3') to exon 51. In some instances, the polynucleic acid molecule hybridizes to a target region that is about 5, 10, 15, 20, 50, 100, 200, 300, 400 or 500 bp downstream (or 3') to exon 51.

In some instances, the polynucleic acid molecule hybridizes to a target region that is within exon 51 of the *DMD* gene. In some instances, the polynucleic acid molecule hybridizes to a target region that is at either the 5' intron-exon 51 junction or the 3' exon 51-intron junction.

In some embodiments, a polynucleic acid molecule described herein targets a partially spliced mRNA sequence comprising exon 53 of the *DMD* gene. In some instances, the polynucleic acid molecule hybridizes to a target region that is upstream (or 5') to exon 53. In some instances, the polynucleic acid molecule hybridizes to a target region that is about 5, 10, 15, 20, 50, 100, 200, 300, 400 or 500 bp upstream (or 5') to exon 53. In some instances, the polynucleic acid molecule hybridizes to a target region that is downstream (or 3') to exon 53. In some instances, the polynucleic acid molecule hybridizes to a target region that is about 5, 10, 15, 20, 50, 100, 200, 300, 400 or 500 bp downstream (or 3') to exon 53.

In some instances, the polynucleic acid molecule hybridizes to a target region that is within exon 53 of the *DMD* gene. In some instances, the polynucleic acid molecule hybridizes to a target region that is at either the 5' intron-exon 53 junction or the 3' exon 53-intron junction.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to a target sequence of interest. In some embodiments, the polynucleic acid molecule comprises a sequence having at least 50% sequence identity to a target sequence of interest. In some embodiments, the polynucleic acid molecule comprises a sequence having at least 60% sequence identity to a target sequence of interest. In some embodiments, the polynucleic acid molecule comprises a sequence having at least 70% sequence identity to a target sequence of interest. In some embodiments, the polynucleic acid molecule comprises a sequence having at least 75% sequence identity to a target sequence of interest. In some embodiments, the polynucleic acid molecule comprises a sequence having at least 80% sequence identity to a target sequence of interest. In some embodiments, the polynucleic acid molecule comprises a sequence having at least 85% sequence identity to a target sequence of interest. In some embodiments, the polynucleic acid molecule comprises a sequence having at least 90% sequence identity to a target sequence of interest. In some embodiments, the polynucleic acid molecule comprises a sequence having at least 95% sequence identity to a target sequence of interest. In some embodiments, the polynucleic acid molecule comprises a sequence having at least 96% sequence identity to a target sequence of interest. In some embodiments, the polynucleic acid molecule comprises a sequence having at least 97% sequence identity to a target sequence of interest. In some embodiments, the polynucleic acid molecule comprises a sequence having at least 98% sequence identity to a target sequence of interest. In some embodiments, the polynucleic acid molecule comprises a sequence having at least 99% sequence identity to a target sequence of interest. In some embodiments, the polynucleic acid molecule consists of a target sequence of interest.

In some embodiments, the polynucleic acid molecule comprises a first polynucleotide and a second polynucleotide. In some instances, the first polynucleotide comprises a sequence having at least 50%, 55%, 60%, 65%, 70%, 75%, 80%, 85%, 90%, 95%, 96%, 97%, 98%, 99%, or 100% sequence identity to a target sequence of interest. In some cases, the second polynucleotide comprises a sequence having at least 50%, 55%, 60%, 65%, 70%, 75%, 80%, 85%, 90%, 95%, 96%, 97%, 98%, 99%, or 100% sequence identity to a target sequence of interest. In some cases, the polynucleic acid molecule comprises a first polynucleotide having at least 50%, 55%, 60%, 65%, 70%, 75%, 80%, 85%, 90%, 95%, 96%, 97%, 98%, 99%, or 100% sequence identity to a target sequence of interest and a second polynucleotide having at least 50%, 55%, 60%, 65%, 70%, 75%, 80%, 85%, 90%, 95%, 96%, 97%, 98%, 99%, or 100% sequence identity to a target sequence of interest.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 964-1285. In some embodiments, the polynucleic acid molecule comprises a sequence having at least 50% sequence identity to SEQ ID NOs: 964-1285. In some embodiments, the polynucleic acid molecule comprises a sequence having at least 60% sequence identity to SEQ ID NOs: 964-1285. In some embodiments, the polynucleic acid molecule comprises a sequence having at least 70% sequence identity to SEQ ID NOs: 964-1285. In some embodiments, the polynucleic acid molecule comprises a sequence having at least 75% sequence identity to SEQ ID NOs: 964-1285. In some embodiments, the polynucleic acid molecule comprises a sequence having at least 80% sequence identity to SEQ ID NOs: 964-1285. In some embodiments, the polynucleic acid molecule comprises a sequence having at least 85% sequence identity to SEQ ID NOs: 964-1285. In some embodiments, the polynucleic acid molecule comprises a sequence having at least 90% sequence identity to SEQ ID NOs: 964-1285. In some embodiments, the polynucleic acid molecule comprises a sequence having at least 95% sequence identity to SEQ ID NOs: 964-1285. In some embodiments, the polynucleic acid molecule comprises a sequence having at least 96% sequence identity to SEQ ID NOs: 964-1285. In some embodiments, the polynucleic acid molecule comprises a sequence having at least 97% sequence identity to SEQ ID NOs: 964-1285. In some embodiments, the polynucleic acid molecule comprises a sequence having at least 98% sequence identity to SEQ ID NOs: 964-1285. In some embodiments, the polynucleic acid molecule comprises a sequence having at least 99% sequence identity to SEQ ID NOs: 964-1285. In some embodiments, the polynucleic acid molecule consists of SEQ ID NOs: 964-1285.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1056-1094. In some embodiments, the polynucleic acid molecule comprises a sequence having at least 50% sequence identity to SEQ ID NOs: 1056-1094. In some embodiments, the polynucleic acid molecule comprises a sequence having at least 60% sequence identity to SEQ ID NOs: 1056-1094. In some embodiments, the polynucleic acid molecule comprises a sequence having at least 70% sequence identity to SEQ ID NOs: 1056-1094. In some embodiments, the polynucleic acid molecule comprises a sequence having at least 75% sequence identity to SEQ ID NOs: 1056-1094. In some embodiments, the polynucleic acid molecule comprises a sequence having at least 80% sequence identity to SEQ ID NOs: 1056-1094. In some embodiments, the polynucleic acid molecule comprises a sequence having at least 85% sequence identity to SEQ ID NOs: 1056-1094. In some embodiments, the polynucleic acid molecule comprises a sequence having at least 90% sequence identity to SEQ ID NOs: 1056-1094. In some embodiments, the polynucleic acid molecule comprises a sequence having at least 95% sequence identity to SEQ ID NOs: 1056-1094. In some embodiments, the polynucleic acid molecule comprises a sequence having at least 96% sequence identity to SEQ ID NOs: 1056-1094. In some embodiments, the polynucleic acid molecule comprises a sequence having at least 97% sequence identity to SEQ ID NOs: 1056-1094. In some embodiments, the polynucleic acid molecule comprises a sequence having at least 98% sequence identity to SEQ ID NOs: 1056-1094. In some embodiments, the polynucleic acid molecule comprises a sequence having at least 99% sequence identity to SEQ ID NOs: 1056-1094. In some embodiments, the polynucleic acid molecule consists of SEQ ID NOs: 1056-1094.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1147-1162. In some embodiments, the polynucleic acid molecule comprises a sequence having at least 50% sequence identity to SEQ ID NOs: 1147-1162. In some embodiments, the polynucleic acid molecule comprises a sequence having at least 60% sequence identity to SEQ ID NOs: 1147-1162. In some embodiments, the polynucleic acid molecule comprises a sequence having at least 70% sequence identity to SEQ ID NOs: 1147-1162. In some embodiments, the polynucleic acid molecule comprises a sequence having at least 75% sequence identity to SEQ ID NOs: 1147-1162. In some embodiments, the polynucleic acid molecule comprises a sequence having at least 80% sequence identity to SEQ ID NOs: 1147-1162. In some embodiments, the polynucleic acid molecule comprises a sequence having at least 85% sequence identity to SEQ ID NOs: 1147-1162. In some embodiments, the polynucleic acid molecule comprises a sequence having at least 90% sequence identity to SEQ ID NOs: 1147-1162. In some embodiments, the polynucleic acid molecule comprises a sequence having at least 95% sequence identity to SEQ ID NOs: 1147-1162. In some embodiments, the polynucleic acid molecule comprises a sequence having at least 96% sequence identity to SEQ ID NOs: 1147-1162. In some embodiments, the polynucleic acid molecule comprises a sequence having at least 97% sequence identity to SEQ ID NOs: 1147-1162. In some embodiments, the polynucleic acid molecule comprises a sequence having at least 98% sequence identity to SEQ ID NOs: 1147-1162. In some embodiments, the polynucleic acid molecule comprises a sequence having at least 99% sequence identity to SEQ ID NOs: 1147-1162. In some embodiments, the polynucleic acid molecule consists of SEQ ID NOs: 1147-1162.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1173-1211. In some embodiments, the polynucleic acid molecule comprises a sequence having at least 50% sequence identity to SEQ ID NOs: 1173-1211. In some embodiments, the polynucleic acid molecule comprises a sequence having at least 60% sequence identity to SEQ ID NOs: 1173-1211. In some embodiments, the polynucleic acid molecule comprises a sequence having at least 70% sequence identity to SEQ ID NOs: 1173-1211. In some embodiments, the polynucleic acid molecule comprises a sequence having at least 75% sequence identity to SEQ ID NOs: 1173-1211. In some embodiments, the polynucleic acid molecule comprises a sequence having at least 80% sequence identity to SEQ ID NOs: 1173-1211. In some embodiments, the polynucleic acid molecule comprises a sequence having at least 85% sequence identity to SEQ ID NOs: 1173-1211. In some embodiments, the polynucleic acid molecule comprises a sequence having at least 90% sequence identity to SEQ ID NOs: 1173-1211. In some embodiments, the polynucleic acid molecule comprises a sequence having at least 95% sequence identity to SEQ ID NOs: 1173-1211. In some embodiments, the polynucleic acid molecule comprises a sequence having at least 96% sequence identity to SEQ ID NOs: 1173-1211. In some embodiments, the polynucleic acid molecule comprises a sequence having at least 97% sequence identity to SEQ ID NOs: 1173-1211. In some embodiments, the polynucleic acid molecule comprises a sequence having at least 98% sequence identity to SEQ ID NOs: 1173-1211. In some embodiments, the polynucleic acid molecule comprises a sequence having at least 99% sequence identity to SEQ ID NOs: 1173-1211. In some embodiments, the polynucleic acid molecule consists of SEQ ID NOs: 1173-1211.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1056-1076. In some embodiments, the polynucleic acid molecule comprises a sequence having at least 50% sequence identity to SEQ ID NOs: 1056-1076. In some embodiments, the polynucleic acid molecule comprises a sequence having at least 60% sequence identity to SEQ ID NOs: 1056-1076. In some embodiments, the polynucleic acid molecule comprises a sequence having at least 70% sequence identity to SEQ ID NOs: 1056-1076. In some embodiments, the polynucleic acid molecule comprises a sequence having at least 75% sequence identity to SEQ ID NOs: 1056-1076. In some embodiments, the polynucleic acid molecule comprises a sequence having at least 80% sequence identity to SEQ ID NOs: 1056-1076. In some embodiments, the polynucleic acid molecule comprises a sequence having at least 85% sequence identity to SEQ ID NOs: 1056-1076. In some embodiments, the polynucleic acid molecule comprises a sequence having at least 90% sequence identity to SEQ ID NOs: 1056-1076. In some embodiments, the polynucleic acid molecule comprises a sequence having at least 95% sequence identity to SEQ ID NOs: 1056-1076. In some embodiments, the polynucleic acid molecule comprises a sequence having at least 96% sequence identity to SEQ ID NOs: 1056-1076. In some embodiments, the polynucleic acid molecule comprises a sequence having at least 97% sequence identity to SEQ ID NOs: 1056-1076. In some embodiments, the polynucleic acid molecule comprises a sequence having at least 98% sequence identity to SEQ ID NOs: 1056-1076. In some embodiments, the polynucleic acid molecule comprises a sequence having at least 99% sequence identity to SEQ ID NOs: 1056-1076. In some embodiments, the polynucleic acid molecule consists of SEQ ID NOs: 1056-1076.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1077-1094. In some embodiments, the polynucleic acid molecule comprises a sequence having at least 50% sequence identity to SEQ ID NOs: 1077-1094. In some embodiments, the polynucleic acid molecule comprises a sequence having at least 60% sequence identity to SEQ ID NOs: 1077-1094. In some embodiments, the polynucleic acid molecule comprises a sequence having at least 70% sequence identity to SEQ ID NOs: 1077-1094. In some embodiments, the polynucleic acid molecule comprises a sequence having at least 75% sequence identity to SEQ ID NOs: 1077-1094. In some embodiments, the polynucleic acid molecule comprises a sequence having at least 80% sequence identity to SEQ ID NOs: 1077-1094. In some embodiments, the polynucleic acid molecule comprises a sequence having at least 85% sequence identity to SEQ ID NOs: 1077-1094. In some embodiments, the polynucleic acid molecule comprises a sequence having at least 90% sequence identity to SEQ ID NOs: 1077-1094. In some embodiments, the polynucleic acid molecule comprises a sequence having at least 95% sequence identity to SEQ ID NOs: 1077-1094. In some embodiments, the polynucleic acid molecule comprises a sequence having at least 96% sequence identity to SEQ ID NOs: 1077-1094. In some embodiments, the polynucleic acid molecule comprises a sequence having at least 97% sequence identity to SEQ ID NOs: 1077-1094. In some embodiments, the polynucleic acid molecule comprises a sequence having at least 98% sequence identity to SEQ ID NOs: 1077-1094. In some embodiments, the polynucleic acid molecule comprises a sequence having at least 99% sequence identity to SEQ ID NOs: 1077-1094. In some embodiments, the polynucleic acid molecule consists of SEQ ID NOs: 1077-1094.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1056-1058. In some embodiments, the polynucleic acid molecule comprises a sequence having at least 50% sequence identity to SEQ ID NOs: 1056-1058. In some embodiments, the polynucleic acid molecule comprises a sequence having at least 60% sequence identity to SEQ ID NOs: 1056-1058. In some embodiments, the polynucleic acid molecule comprises a sequence having at least 70% sequence identity to SEQ ID NOs: 1056-1058. In some embodiments, the polynucleic acid molecule comprises a sequence having at least 75% sequence identity to SEQ ID NOs: 1056-1058. In some embodiments, the polynucleic acid molecule comprises a sequence having at least 80% sequence identity to SEQ ID NOs: 1056-1058. In some embodiments, the polynucleic acid molecule comprises a sequence having at least 85% sequence identity to SEQ ID NOs: 1056-1058. In some embodiments, the polynucleic acid molecule comprises a sequence having at least 90% sequence identity to SEQ ID NOs: 1056-1058. In some embodiments, the polynucleic acid molecule comprises a sequence having at least 95% sequence identity to SEQ ID NOs: 1056-1058. In some embodiments, the polynucleic acid molecule comprises a sequence having at least 96% sequence identity to SEQ ID NOs: 1056-1058. In some embodiments, the polynucleic acid molecule comprises a sequence having at least 97% sequence identity to SEQ ID NOs: 1056-1058. In some embodiments, the polynucleic acid molecule comprises a sequence having at least 98% sequence identity to SEQ ID NOs: 1056-1058. In some embodiments, the polynucleic acid molecule comprises a sequence having at least 99% sequence identity to SEQ ID NOs: 1056-1058. In some embodiments, the polynucleic acid molecule consists of SEQ ID NOs: 1056-1058.

In some embodiments, the polynucleic acid molecule comprises a sequence having at least 50%, 55%, 60%, 65%, 70%, 75%, 80%, 85%, 90%, 95%, 96%, 97%, 98%, 99%, or 100% sequence identity to SEQ ID NOs: 1087-1089. In some embodiments, the polynucleic acid molecule comprises a sequence having at least 50% sequence identity to SEQ ID NOs: 1087-1089. In some embodiments, the polynucleic acid molecule comprises a sequence having at least 60% sequence identity to SEQ ID NOs: 1087-1089. In some embodiments, the polynucleic acid molecule comprises a sequence having at least 70% sequence identity to SEQ ID NOs: 1087-1089. In some embodiments, the polynucleic acid molecule comprises a sequence having at least 75% sequence identity to SEQ ID NOs: 1087-1089. In some embodiments, the polynucleic acid molecule comprises a sequence having at least 80% sequence identity to SEQ ID NOs: 1087-1089. In some embodiments, the polynucleic acid molecule comprises a sequence having at least 85% sequence identity to SEQ ID NOs: 1087-1089. In some embodiments, the polynucleic acid molecule comprises a sequence having at least 90% sequence identity to SEQ ID NOs: 1087-1089. In some embodiments, the polynucleic acid molecule comprises a sequence having at least 95% sequence identity to SEQ ID NOs: 1087-1089. In some embodiments, the polynucleic acid molecule comprises a sequence having at least 96% sequence identity to SEQ ID NOs: 1087-1089. In some embodiments, the polynucleic acid molecule comprises a sequence having at least 97% sequence identity to SEQ ID NOs: 1087-1089. In some embodiments, the polynucleic acid molecule comprises a sequence having at least 98% sequence identity to SEQ ID NOs: 1087-1089. In some embodiments, the polynucleic acid molecule comprises a sequence having at least 99% sequence identity to SEQ ID NOs: 1087-1089. In some embodiments, the polynucleic acid molecule consists of SEQ ID NOs: 1087-1089.

In some embodiments, the polynucleic acid molecule at least 10, 15, 16, 17, 18, 19, 20, 21, 22, 23, 24, 25, 26, or more contiguous bases of a base sequence selected from SEQ ID NOs: 964-1285. In some instances, the polynucleic acid molecule at least 10, 15, 16, 17, 18, 19, 20, 21, 22, 23, 24, 25, 26, or more contiguous bases of a base sequence selected from SEQ ID NOs: 1056-1094, 1147-1162, or 1173-1211. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1056-1076. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1077-1094. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1147-1162. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1173-1211. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1056-1058. In some instances, the polynucleic acid molecule comprises at least 10, 15, 16, 17, 18, 19, 20, 21, 22, 23, 24, 25, 26, or more contiguous bases of a base sequence selected from SEQ ID NOs: 1087-1089. In some cases, the polynucleic acid molecule further comprises 1, 2, 3, or 4 mismatches.

In some embodiments, the polynucleic acid molecule comprises a guide strand and a passenger strand. In some instances, the guide strand comprises a sequence having at least 50%, 55%, 60%, 65%, 70%, 75%, 80%, 85%, 90%, 95%, 96%, 97%, 98%, 99%, or 100% sequence identity to SEQ ID NOs: 964-1285. In some cases, the guide strand comprises a sequence selected from SEQ ID NOs: 964-1285.

In some embodiments, the polynucleic acid molecule described herein comprises RNA or DNA. In some cases, the polynucleic acid molecule comprises RNA. In some instances, RNA comprises short interfering RNA (siRNA), short hairpin RNA (shRNA), microRNA (miRNA), double-stranded RNA (dsRNA), transfer RNA (tRNA), ribosomal RNA (rRNA), or heterogeneous nuclear RNA (hnRNA). In some instances, RNA comprises shRNA. In some instances, RNA comprises miRNA. In some instances, RNA comprises dsRNA. In some instances, RNA comprises tRNA. In some instances, RNA comprises rRNA. In some instances, RNA comprises hnRNA. In some instances, the RNA comprises siRNA. In some instances, the polynucleic acid molecule comprises siRNA. In some instances, the polynucleic acid molecule is an antisense oligonucleotide (ASO).

In some embodiments, the polynucleic acid molecule is from about 10 to about 50 nucleotides in length. In some instances, the polynucleic acid molecule is from about 10 to about 30, from about 15 to about 30, from about 18 to about 30, from about 18 to about 25, form about 18 to about 24, from about 19 to about 23, from about 19 to about 30, from about 19 to about 25, form about 19 to about 24, from about 19 to about 23, from about 20 to about 30, from about 20 to about 25, from about 20 to about 24, from about 20 to about 23, or from about 20 to about 22 nucleotides in length.

In some embodiments, the polynucleic acid molecule is about 50 nucleotides in length. In some instances, the polynucleic acid molecule is about 45 nucleotides in length. In some instances, the polynucleic acid molecule is about 40 nucleotides in length. In some instances, the polynucleic acid molecule is about 35 nucleotides in length. In some instances, the polynucleic acid molecule is about 30 nucleotides in length. In some instances, the polynucleic acid molecule is about 25 nucleotides in length. In some instances, the polynucleic acid molecule is about 20 nucleotides in length. In some instances, the polynucleic acid molecule is about 19 nucleotides in length. In some instances, the polynucleic acid molecule is about 18 nucleotides in length. In some instances, the polynucleic acid molecule is about 17 nucleotides in length. In some instances, the polynucleic acid molecule is about 16 nucleotides in length. In some instances, the polynucleic acid molecule is about 15 nucleotides in length. In some instances, the polynucleic acid molecule is about 14 nucleotides in length. In some instances, the polynucleic acid molecule is about 13 nucleotides in length. In some instances, the polynucleic acid molecule is about 12 nucleotides in length. In some instances, the polynucleic acid molecule is about 11 nucleotides in length. In some instances, the polynucleic acid molecule is about 10 nucleotides in length. In some instances, the polynucleic acid molecule is between about 10 and about 50 nucleotides in length. In some instances, the polynucleic acid molecule is between about 10 and about 45 nucleotides in length. In some instances, the polynucleic acid molecule is between about 10 and about 40 nucleotides in length. In some instances, the polynucleic acid molecule is between about 10 and about 35 nucleotides in length. In some instances, the polynucleic acid molecule is between about 10 and about 30 nucleotides in length. In some instances, the polynucleic acid molecule is between about 10 and about 25 nucleotides in length. In some instances, the polynucleic acid molecule is between about 10 and about 20 nucleotides in length. In some instances, the polynucleic acid molecule is between about 15 and about 25 nucleotides in length. In some instances, the polynucleic acid molecule is between about 15 and about 30 nucleotides in length. In some instances, the polynucleic acid molecule is between about 12 and about 30 nucleotides in length. In some instances, the polynucleic acid molecule is between about 19 and about 30 nucleotides in length. In some instances, the polynucleic acid molecule is between about 20 and about 30 nucleotides in length. In some instances, the polynucleic acid molecule is between about 19 and about 25 nucleotides in length. In some instances, the polynucleic acid molecule is between about 20 and about 25 nucleotides in length.

In some embodiments, the polynucleic acid molecule is at least 15, 16, 17, 18, 19, 20, 21, 22, 23, 24, 25, 26, 27, 28, 29, 30, 35, 40, 45, or 50 nucleotides in length. In some instances, the polynucleic acid molecule is at least 15, 16, 17, 18, 19, 20, 21, 22, 23, 24, 25, 26, 27, 28, 29, or 30 nucleotides in length. In some instances, the polynucleic acid molecule is at least 15 nucleotides in length. In some instances, the polynucleic acid molecule is at least 18 nucleotides in length. In some instances, the polynucleic acid molecule is at least 19 nucleotides in length. In some instances, the polynucleic acid molecule is at least 20 nucleotides in length. In some instances, the polynucleic acid molecule is at least 21 nucleotides in length. In some instances, the polynucleic acid molecule is at least 22 nucleotides in length. In some instances, the polynucleic acid molecule is at least 23 nucleotides in length. In some instances, the polynucleic acid molecule is at least 24 nucleotides in length. In some instances, the polynucleic acid molecule is at least 25 nucleotides in length. In some instances, the polynucleic acid molecule is at least 30 nucleotides in length.

In some embodiments, the polynucleic acid molecule is about 50 nucleotides in length. In some instances, the polynucleic acid molecule is about 45 nucleotides in length. In some instances, the polynucleic acid molecule is about 40 nucleotides in length. In some instances, the polynucleic acid molecule is about 35 nucleotides in length. In some instances, the polynucleic acid molecule is about 30 nucleotides in length. In some instances, the polynucleic acid molecule is about 29 nucleotides in length. In some instances, the polynucleic acid molecule is about 28 nucleotides in length. In some instances, the polynucleic acid molecule is about 27 nucleotides in length. In some instances, the polynucleic acid molecule is about 26 nucleotides in length. In some instances, the polynucleic acid molecule is about 25 nucleotides in length. In some instances, the polynucleic acid molecule is about 24 nucleotides in length. In some instances, the polynucleic acid molecule is about 23 nucleotides in length. In some instances, the polynucleic acid molecule is about 22 nucleotides in length. In some instances, the polynucleic acid molecule is about 21 nucleotides in length. In some instances, the polynucleic acid molecule is about 20 nucleotides in length. In some instances, the polynucleic acid molecule is about 19 nucleotides in length. In some instances, the polynucleic acid molecule is about 18 nucleotides in length. In some instances, the polynucleic acid molecule is about 17 nucleotides in length. In some instances, the polynucleic acid molecule is about 16 nucleotides in length. In some instances, the polynucleic acid molecule is about 15 nucleotides in length. In some instances, the polynucleic acid molecule is about 14 nucleotides in length. In some instances, the polynucleic acid molecule is about 13 nucleotides in length. In some instances, the polynucleic acid molecule is about 12 nucleotides in length. In some instances, the polynucleic acid molecule is about 11 nucleotides in length. In some instances, the polynucleic acid molecule is about 10 nucleotides in length.

In some embodiments, the polynucleic acid molecule comprises a first polynucleotide. In some instances, the polynucleic acid molecule comprises a second polynucleotide. In some instances, the polynucleic acid molecule comprises a first polynucleotide and a second polynucleotide. In some instances, the first polynucleotide is a sense strand or passenger strand. In some instances, the second polynucleotide is an antisense strand or guide strand.

In some embodiments, the polynucleic acid molecule is a first polynucleotide. In some embodiments, the first polynucleotide is from about 10 to about 50 nucleotides in length. In some instances, the first polynucleotide is from about 10 to about 30, from about 15 to about 30, from about 18 to about 30, from about 18 to about 25, form about 18 to about 24, from about 19 to about 23, from about 19 to about 30, from about 19 to about 25, form about 19 to about 24, from about 19 to about 23, from about 20 to about 30, from about 20 to about 25, from about 20 to about 24, from about 20 to about 23, or from about 20 to about 22 nucleotides in length.

In some instances, the first polynucleotide is about 50 nucleotides in length. In some instances, the first polynucleotide is about 45 nucleotides in length. In some instances, the first polynucleotide is about 40 nucleotides in length. In some instances, the first polynucleotide is about 35 nucleotides in length. In some instances, the first polynucleotide is about 30 nucleotides in length. In some instances, the first polynucleotide is about 25 nucleotides in length. In some instances, the first polynucleotide is about 20 nucleotides in length. In some instances, the first polynucleotide is about 19 nucleotides in length. In some instances, the first polynucleotide is about 18 nucleotides in length. In some instances, the first polynucleotide is about 17 nucleotides in length. In some instances, the first polynucleotide is about 16 nucleotides in length. In some instances, the first polynucleotide is about 15 nucleotides in length. In some instances, the first polynucleotide is about 14 nucleotides in length. In some instances, the first polynucleotide is about 13 nucleotides in length. In some instances, the first polynucleotide is about 12 nucleotides in length. In some instances, the first polynucleotide is about 11 nucleotides in length. In some instances, the first polynucleotide is about 10 nucleotides in length. In some instances, the first polynucleotide is between about 10 and about 50 nucleotides in length. In some instances, the first polynucleotide is between about 10 and about 45 nucleotides in length. In some instances, the first polynucleotide is between about 10 and about 40 nucleotides in length. In some instances, the first polynucleotide is between about 10 and about 35 nucleotides in length. In some instances, the first polynucleotide is between about 10 and about 30 nucleotides in length. In some instances, the first polynucleotide is between about 10 and about 25 nucleotides in length. In some instances, the first polynucleotide is between about 10 and about 20 nucleotides in length. In some instances, the first polynucleotide is between about 15 and about 25 nucleotides in length. In some instances, the first polynucleotide is between about 15 and about 30 nucleotides in length. In some instances, the first polynucleotide is between about 12 and about 30 nucleotides in length.

In some embodiments, the polynucleic acid molecule is a second polynucleotide. In some embodiments, the second polynucleotide is from about 10 to about 50 nucleotides in length. In some instances, the second polynucleotide is from about 10 to about 30, from about 15 to about 30, from about 18 to about 30, from about 18 to about 25, form about 18 to about 24, from about 19 to about 23, from about 19 to about 30, from about 19 to about 25, form about 19 to about 24, from about 19 to about 23, from about 20 to about 30, from about 20 to about 25, from about 20 to about 24, from about 20 to about 23, or from about 20 to about 22 nucleotides in length.

In some instances, the second polynucleotide is about 50 nucleotides in length. In some instances, the second polynucleotide is about 45 nucleotides in length. In some instances, the second polynucleotide is about 40 nucleotides in length. In some instances, the second polynucleotide is about 35 nucleotides in length. In some instances, the second polynucleotide is about 30 nucleotides in length. In some instances, the second polynucleotide is about 25 nucleotides in length. In some instances, the second polynucleotide is about 20 nucleotides in length. In some instances, the second polynucleotide is about 19 nucleotides in length. In some instances, the second polynucleotide is about 18 nucleotides in length. In some instances, the second polynucleotide is about 17 nucleotides in length. In some instances, the second polynucleotide is about 16 nucleotides in length. In some instances, the second polynucleotide is about 15 nucleotides in length. In some instances, the second polynucleotide is about 14 nucleotides in length. In some instances, the second polynucleotide is about 13 nucleotides in length. In some instances, the second polynucleotide is about 12 nucleotides in length. In some instances, the second polynucleotide is about 11 nucleotides in length. In some instances, the second polynucleotide is about 10 nucleotides in length. In some instances, the second polynucleotide is between about 10 and about 50 nucleotides in length. In some instances, the second polynucleotide is between about 10 and about 45 nucleotides in length. In some instances, the second polynucleotide is between about 10 and about 40 nucleotides in length. In some instances, the second polynucleotide is between about 10 and about 35 nucleotides in length. In some instances, the second polynucleotide is between about 10 and about 30 nucleotides in length. In some instances, the second polynucleotide is between about 10 and about 25 nucleotides in length. In some instances, the second polynucleotide is between about 10 and about 20 nucleotides in length. In some instances, the second polynucleotide is between about 15 and about 25 nucleotides in length. In some instances, the second polynucleotide is between about 15 and about 30 nucleotides in length. In some instances, the second polynucleotide is between about 12 and about 30 nucleotides in length.

In some embodiments, the polynucleic acid molecule comprises a first polynucleotide and a second polynucleotide. In some instances, the polynucleic acid molecule further comprises a blunt terminus, an overhang, or a combination thereof. In some instances, the blunt terminus is a 5' blunt terminus, a 3' blunt terminus, or both. In some cases, the overhang is a 5' overhang, 3' overhang, or both. In some cases, the overhang comprises 1, 2, 3, 4, 5, 6, 7, 8, 9, or 10 non-base pairing nucleotides. In some cases, the overhang comprises 1, 2, 3, 4, 5, or 6 non-base pairing nucleotides. In some cases, the overhang comprises 1, 2, 3, or 4 non-base pairing nucleotides. In some cases, the overhang comprises 1 non-base pairing nucleotide. In some cases, the overhang comprises 2 non-base pairing nucleotides. In some cases, the overhang comprises 3 non-base pairing nucleotides. In some cases, the overhang comprises 4 non-base pairing nucleotides.

In some embodiments, the sequence of the polynucleic acid molecule is at least 40%, 50%, 55%, 60%, 65%, 70%, 75%, 80%, 85%, 90%, 95%, 98%, 99%, or 99.5% complementary to a target sequence described herein. In some embodiments, the sequence of the polynucleic acid molecule is at least 50% complementary to a target sequence described herein. In some embodiments, the sequence of the polynucleic acid molecule is at least 60% complementary to a target sequence described herein. In some embodiments, the sequence of the polynucleic acid molecule is at least 70% complementary to a target sequence described herein. In some embodiments, the sequence of the polynucleic acid molecule is at least 80% complementary to a target sequence described herein. In some embodiments, the sequence of the polynucleic acid molecule is at least 90% complementary to a target sequence described herein. In some embodiments, the sequence of the polynucleic acid molecule is at least 95% complementary to a target sequence described herein. In some embodiments, the sequence of the polynucleic acid molecule is at least 99% complementary to a target sequence described herein. In some instances, the sequence of the polynucleic acid molecule is 100% complementary to a target sequence described herein.

In some embodiments, the sequence of the polynucleic acid molecule has 5 or less mismatches to a target sequence described herein. In some embodiments, the sequence of the polynucleic acid molecule has 4 or less mismatches to a target sequence described herein. In some instances, the sequence of the polynucleic acid molecule has 3 or less mismatches to a target sequence described herein. In some cases, the sequence of the polynucleic acid molecule has 2 or less mismatches to a target sequence described herein. In some cases, the sequence of the polynucleic acid molecule has 1 or less mismatches to a target sequence described herein.

In some embodiments, the specificity of the polynucleic acid molecule that hybridizes to a target sequence described herein is a 95%, 98%, 99%, 99.5% or 100% sequence complementarity of the polynucleic acid molecule to a target sequence. In some instances, the hybridization is a high stringent hybridization condition.

In some embodiments, the polynucleic acid molecule has reduced off-target effect. In some instances, "off-target" or "off-target effects" refer to any instance in which a polynucleic acid polymer directed against a given target causes an unintended effect by interacting either directly or indirectly with another mRNA sequence, a DNA sequence or a cellular protein or other moiety. In some instances, an "off-target effect" occurs when there is a simultaneous degradation of other transcripts due to partial homology or complementarity between that other transcript and the sense and/or antisense strand of the polynucleic acid molecule.

In some embodiments, the polynucleic acid molecule comprises natural or synthetic or artificial nucleotide analogues or bases. In some cases, the polynucleic acid molecule comprises combinations of DNA, RNA and/or nucleotide analogues. In some instances, the synthetic or artificial nucleotide analogues or bases comprise modifications at one or more of ribose moiety, phosphate moiety, nucleoside moiety, or a combination thereof.

In some embodiments, nucleotide analogues or artificial nucleotide base comprise a nucleic acid with a modification at a 2' hydroxyl group of the ribose moiety. In some instances, the modification includes an H, OR, R, halo, SH, SR, NH2, NHR, NR2, or CN, wherein R is an alkyl moiety. Exemplary alkyl moiety includes, but is not limited to, halogens, sulfurs, thiols, thioethers, thioesters, amines (primary, secondary, or tertiary), amides, ethers, esters, alcohols and oxygen. In some instances, the alkyl moiety further comprises a modification. In some instances, the modification comprises an azo group, a keto group, an aldehyde group, a carboxyl group, a nitro group, a nitroso, group, a nitrile group, a heterocycle (e.g., imidazole, hydrazino or hydroxylamino) group, an isocyanate or cyanate group, or a sulfur containing group (e.g., sulfoxide, sulfone, sulfide, or disulfide). In some instances, the alkyl moiety further comprises a hetero substitution. In some instances, the carbon of the heterocyclic group is substituted by a nitrogen, oxygen or sulfur. In some instances, the heterocyclic substitution includes but is not limited to, morpholino, imidazole, and pyrrolidino.

In some instances, the modification at the 2' hydroxyl group is a 2'-O-methyl modification or a 2'-O-methoxyethyl (2'-O-MOE) modification. In some cases, the 2'-O-methyl modification adds a methyl group to the 2' hydroxyl group of the ribose moiety whereas the 2'O-methoxyethyl modification adds a methoxyethyl group to the 2' hydroxyl group of the ribose moiety. Exemplary chemical structures of a 2'-O-methyl modification of an adenosine molecule and 2'O-methoxyethyl modification of an uridine are illustrated below.

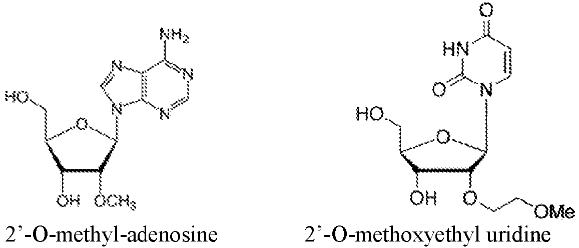

In some instances, the modification at the 2' hydroxyl group is a 2'-O-aminopropyl modification in which an extended amine group comprising a propyl linker binds the amine group to the 2' oxygen. In some instances, this modification neutralizes the phosphate derived overall negative charge of the oligonucleotide molecule by introducing one positive charge from the amine group per sugar and thereby improves cellular uptake properties due to its zwitterionic properties. An exemplary chemical structure of a 2'-O-aminopropyl nucleoside phosphoramidite is illustrated below.

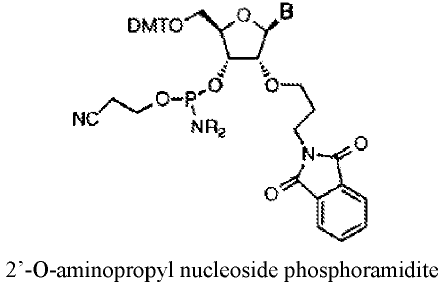

In some instances, the modification at the 2' hydroxyl group is a locked or bridged ribose modification (e.g., locked nucleic acid or LNA) in which the oxygen molecule bound at the 2' carbon is linked to the 4' carbon by a methylene group, thus forming a 2'-C,4'-C-oxy-methylene-linked bicyclic ribonucleotide monomer. Exemplary representations of the chemical structure of LNA are illustrated below. The representation shown to the left highlights the chemical connectivities of an LNA monomer. The representation shown to the right highlights the locked 3'-endo (³E) conformation of the furanose ring of an LNA monomer.

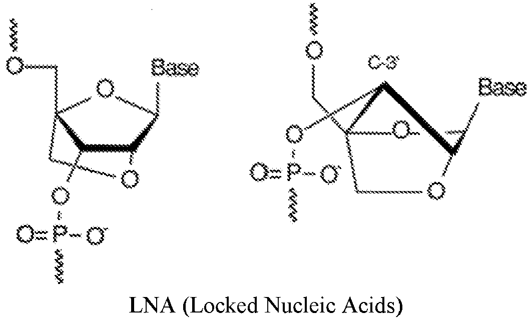

In some instances, the modification at the 2' hydroxyl group comprises ethylene nucleic acids (ENA) such as for example 2'-4'-ethylene-bridged nucleic acid, which locks the sugar conformation into a C₃'-endo sugar puckering conformation. ENA are part of the bridged nucleic acids class of modified nucleic acids that also comprises LNA. Exemplary chemical structures of the ENA and bridged nucleic acids are illustrated below.

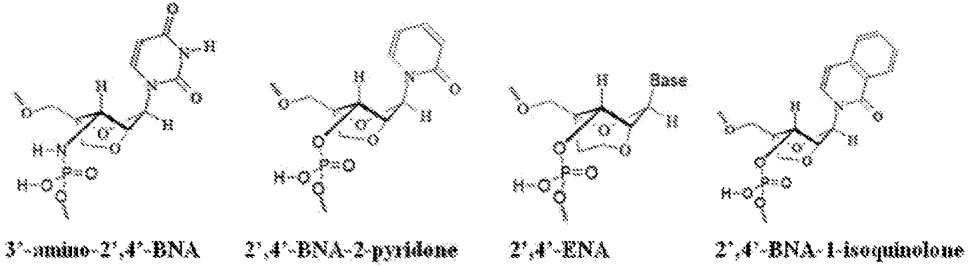

In some embodiments, additional modifications at the 2' hydroxyl group include 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA).

In some embodiments, nucleotide analogues comprise modified bases such as, but not limited to, 5-propynyluridine, 5-propynylcytidine, 6- methyladenine, 6-methylguanine, N, N, -dimethyladenine, 2-propyladenine, 2propylguanine, 2-aminoadenine, 1-methylinosine, 3-methyluridine, 5-methylcytidine, 5-methyluridine and other nucleotides having a modification at the 5 position, 5- (2- amino) propyl uridine, 5-halocytidine, 5-halouridine, 4-acetylcytidine, 1- methyladenosine, 2-methyladenosine, 3-methylcytidine, 6-methyluridine, 2- methylguanosine, 7-methylguanosine, 2, 2-dimethylguanosine, 5-methylaminoethyluridine, 5-methyloxyuridine, deazanucleotides such as 7-deaza- adenosine, 6-azouridine, 6-azocytidine, 6-azothymidine, 5-methyl-2-thiouridine, other thio bases such as 2-thiouridine and 4-thiouridine and 2-thiocytidine, dihydrouridine, pseudouridine, queuosine, archaeosine, naphthyl and substituted naphthyl groups, any O-and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4, 6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyi nucleotides, and alkylcarbonylalkylated nucleotides. Modified nucleotides also include those nucleotides that are modified with respect to the sugar moiety, as well as nucleotides having sugars or analogs thereof that are not ribosyl. For example, the sugar moieties, in some cases are or be based on, mannoses, arabinoses, glucopyranoses, galactopyranoses, 4'-thioribose, and other sugars, heterocycles, or carbocycles. The term nucleotide also includes what are known in the art as universal bases. By way of example, universal bases include but are not limited to 3-nitropyrrole, 5-nitroindole, or nebularine.

In some embodiments, nucleotide analogues further comprise morpholinos, peptide nucleic acids (PNAs), methylphosphonate nucleotides, thiolphosphonate nucleotides, 2'-fluoro N3-P5'-phosphoramidites, 1', 5'- anhydrohexitol nucleic acids (HNAs), or a combination thereof. Morpholino or phosphorodiamidate morpholino oligo (PMO) comprises synthetic molecules whose structure mimics natural nucleic acid structure by deviates from the normal sugar and phosphate structures. In some instances, the five member ribose ring is substituted with a six member morpholino ring containing four carbons, one nitrogen and one oxygen. In some cases, the ribose monomers are linked by a phosphordiamidate group instead of a phosphate group. In such cases, the backbone alterations remove all positive and negative charges making morpholinos neutral molecules capable of crossing cellular membranes without the aid of cellular delivery agents such as those used by charged oligonucleotides.

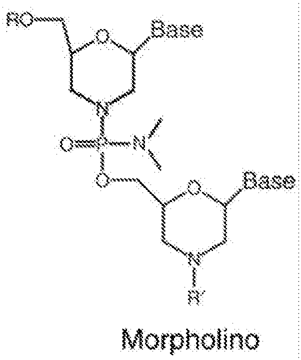

In some embodiments, peptide nucleic acid (PNA) does not contain sugar ring or phosphate linkage and the bases are attached and appropriately spaced by oligoglycine-like molecules, therefore, eliminating a backbone charge.

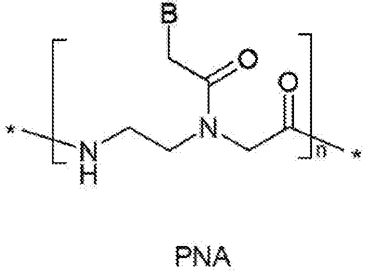

In some embodiments, one or more modifications optionally occur at the internucleotide linkage. In some instances, modified internucleotide linkage include, but is not limited to, phosphorothioates, phosphorodithioates, methylphosphonates, 5'- alkylenephosphonates, 5'-methylphosphonate, 3'-alkylene phosphonates, borontrifluoridates, borano phosphate esters and selenophosphates of 3'-5'linkage or 2'-5'linkage, phosphotriesters, thionoalkylphosphotriesters, hydrogen phosphonate linkages, alkyl phosphonates, alkylphosphonothioates, arylphosphonothioates, phosphoroselenoates, phosphorodiselenoates, phosphinates, phosphoramidates, 3'-alkylphosphoramidates, aminoalkylphosphoramidates, thionophosphoramidates, phosphoropiperazidates, phosphoroanilothioates, phosphoroanilidates, ketones, sulfones, sulfonamides, carbonates, carbamates, methylenehydrazos, methylenedimethylhydrazos, formacetals, thioformacetals, oximes, methyleneiminos, methylenemethyliminos, thioamidates, linkages with riboacetyl groups, aminoethyl glycine, silyl or siloxane linkages, alkyl or cycloalkyl linkages with or without heteroatoms of, for example, 1 to 10 carbons that are saturated or unsaturated and/or substituted and/or contain heteroatoms, linkages with morpholino structures, amides, polyamides wherein the bases are attached to the aza nitrogens of the backbone directly or indirectly, and combinations thereof. Phosphorothioate antisene oligonucleotides (PS ASO) are antisense oligonucleotides comprising a phosphorothioate linkage. An exemplary PS ASO is illustrated below.

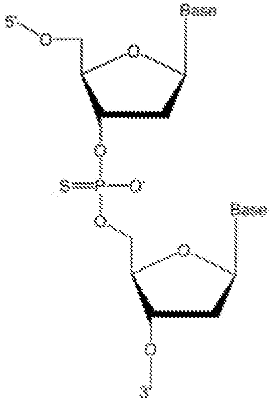

In some instances, the modification is a methyl or thiol modification such as methylphosphonate or thiolphosphonate modification. Exemplary thiolphosphonate nucleotide (left) and methylphosphonate nucleotide (right) are illustrated below.

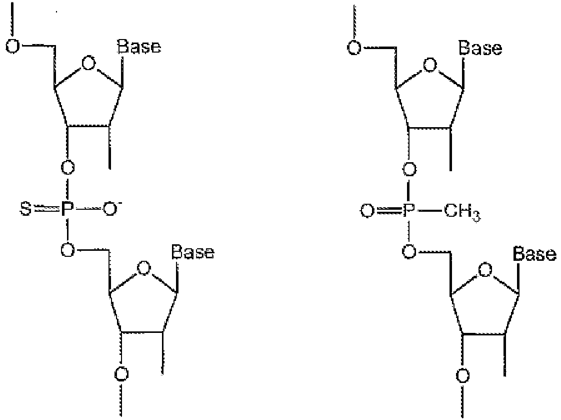

In some instances, a modified nucleotide includes, but is not limited to, 2'-fluoro N3-P5'-phosphoramidites illustrated as:

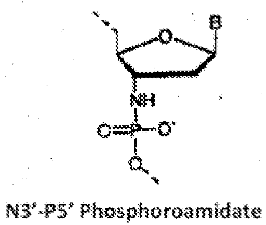

In some instances, a modified nucleotide includes, but is not limited to, hexitol nucleic acid (or 1', 5'- anhydrohexitol nucleic acids (HNA)) illustrated as:

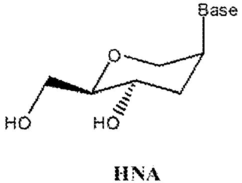

In some embodiments, a nucleotide analogue or artificial nucleotide base described above comprises a 5'-vinylphosphonate modified nucleotide nucleic acid with a modification at a 5' hydroxyl group of the ribose moiety. In some embodiments, the 5'-vinylphosphonate modified nucleotide is selected from the nucleotide provided below, wherein X is O or S; and B is a heterocyclic base moiety.

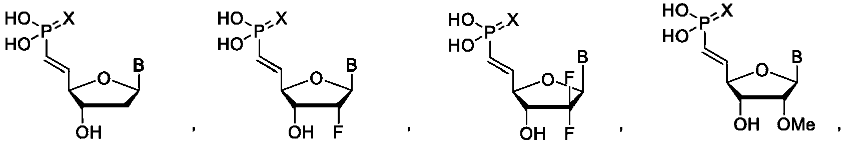

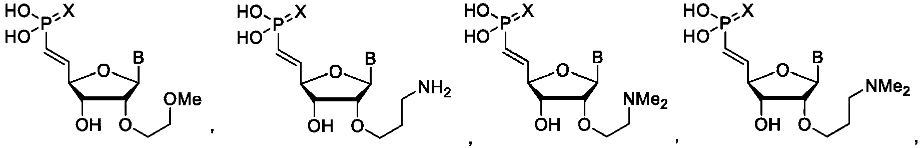

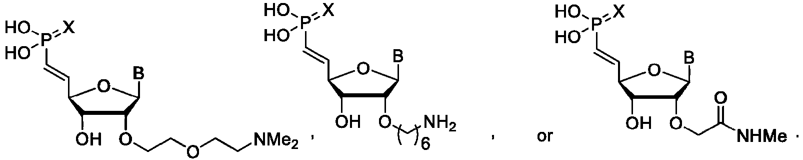

In some instances, the modification at the 2' hydroxyl group is a 2'-O-aminopropyl modification in which an extended amine group comprising a propyl linker binds the amine group to the 2' oxygen. In some instances, this modification neutralizes the phosphate-derived overall negative charge of the oligonucleotide molecule by introducing one positive charge from the amine group per sugar and thereby improves cellular uptake properties due to its zwitterionic properties.

In some instances, the 5'-vinylphosphonate modified nucleotide is further modified at the 2' hydroxyl group in a locked or bridged ribose modification (e.g., locked nucleic acid or LNA) in which the oxygen molecule bound at the 2' carbon is linked to the 4' carbon by a methylene group, thus forming a 2'-C,4'-C-oxy-methylene-linked bicyclic ribonucleotide monomer. Exemplary representations of the chemical structure of 5'-vinylphosphonate modified LNA are illustrated below, wherein X is O or S; B is a heterocyclic base moiety; and J is an internucleotide linking group linking to the adjacent nucleotide of the polynucleotide.

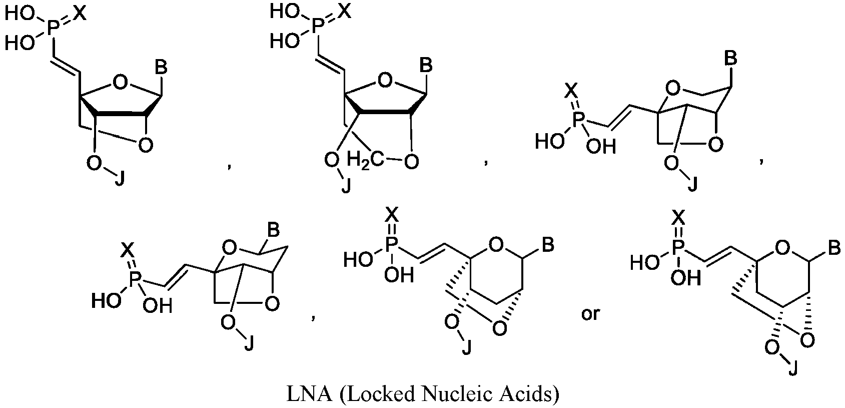

In some embodiments, additional modifications at the 2' hydroxyl group include 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA).

In some embodiments, a nucleotide analogue comprises a modified base such as, but not limited to, 5-propynyluridine, 5-propynylcytidine, 6- methyladenine, 6-methylguanine, N, N, - dimethyladenine, 2-propyladenine, 2propylguanine, 2-aminoadenine, 1-methylinosine, 3-methyluridine, 5-methylcytidine, 5-methyluridine and other nucleotides having a modification at the 5 position, 5- (2-amino) propyl uridine, 5-halocytidine, 5-halouridine, 4-acetylcytidine, 1- methyladenosine, 2-methyladenosine, 3-methylcytidine, 6-methyluridine, 2- methylguanosine, 7-methylguanosine, 2, 2-dimethylguanosine, 5- methylaminoethyluridine, 5-methyloxyuridine, deazanucleotides (such as 7-deaza-adenosine, 6-azouridine, 6-azocytidine, or 6-azothymidine), 5-methyl-2-thiouridine, other thio bases (such as 2-thiouridine, 4-thiouridine, and 2-thiocytidine), dihydrouridine, pseudouridine, queuosine, archaeosine, naphthyl and substituted naphthyl groups, any O-and N-alkylated purines and pyrimidines (such as N6-methyladenosine, 5-methylcarbonylmethyluridine, uridine 5-oxyacetic acid, pyridine-4-one, or pyridine-2-one), phenyl and modified phenyl groups such as aminophenol or 2,4, 6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyi nucleotides, and alkylcarbonylalkylated nucleotides. 5'-Vinylphosphonate modified nucleotides also include those nucleotides that are modified with respect to the sugar moiety, as well as 5'-vinylphosphonate modified nucleotides having sugars or analogs thereof that are not ribosyl. For example, the sugar moieties, in some cases are or are based on, mannoses, arabinoses, glucopyranoses, galactopyranoses, 4'-thioribose, and other sugars, heterocycles, or carbocycles. The term nucleotide also includes what are known in the art as universal bases. By way of example, universal bases include but are not limited to 3-nitropyrrole, 5-nitroindole, or nebularine.

In some embodiments, a 5'-vinylphosphonate modified nucleotide analogue further comprises a morpholino, a peptide nucleic acid (PNA), a methylphosphonate nucleotide, a thiolphosphonate nucleotide, a 2'-fluoro N3-P5'-phosphoramidite, or a 1', 5'- anhydrohexitol nucleic acid (HNA). Morpholino or phosphorodiamidate morpholino oligo (PMO) comprises synthetic molecules whose structure mimics natural nucleic acid structure but deviates from the normal sugar and phosphate structures. In some instances, the five member ribose ring is substituted with a six member morpholino ring containing four carbons, one nitrogen, and one oxygen. In some cases, the ribose monomers are linked by a phosphordiamidate group instead of a phosphate group. In such cases, the backbone alterations remove all positive and negative charges making morpholinos neutral molecules capable of crossing cellular membranes without the aid of cellular delivery agents such as those used by charged oligonucleotides. A non-limiting example of a 5'-vinylphosphonate modified morpholino oligonucleotide is illustrated below, wherein X is O or S; and B is a heterocyclic base moiety.

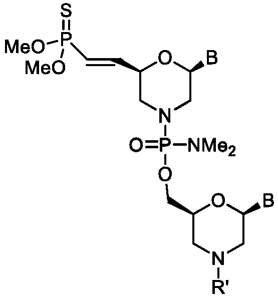

In some embodiments, a 5'-vinylphosphonate modified morpholino or PMO described above is a PMO comprising a positive or cationic charge. In some instances, the PMO is *PMOplus* (Sarepta). *PMOplus* refers to phosphorodiamidate morpholino oligomers comprising any number of (1-piperazino)phosphinylideneoxy, (1-(4-(omega -guanidino-alkanoyl))-piperazino)phosphinylideneoxy linkages (e.g., as such those described in PCT Publication No. WO2008/036127. In some cases, the PMO is a PMO described in U.S. Patent No. 7943762.

In some embodiments, a morpholino or PMO described above is a PMO-X (Sarepta). In some cases, PMO-X refers to phosphorodiamidate morpholino oligomers comprising at least one linkage or at least one of the disclosed terminal modifications, such as those disclosed in PCT Publication No. WO2011/150408 and U.S. Publication No. 2012/0065169.

In some embodiments, a morpholino or PMO described above is a PMO as described in Table 5 of U.S. Publication No. 2014/0296321.

Exemplary representations of the chemical structure of 5'-vinylphosphonate modified nucleic acids are illustrated below, wherein X is O or S; B is a heterocyclic base moiety; and J is an internucleotide linkage.

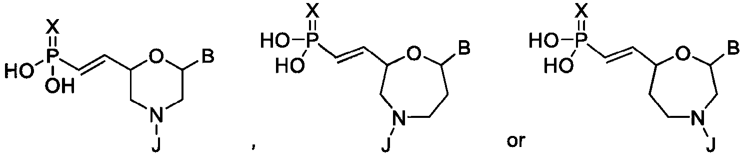

In some embodiments, peptide nucleic acid (PNA) does not contain sugar ring or phosphate linkage and the bases are attached and appropriately spaced by oligoglycine-like molecules, therefore, eliminating a backbone charge.

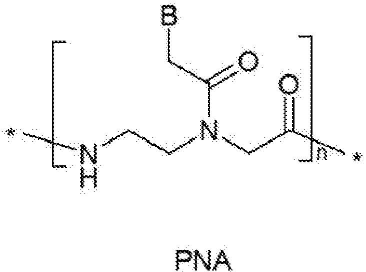

In some embodiments, one or more modifications of the 5'-vinylphosphonate modified oligonucleotide optionally occur at the internucleotide linkage. In some instances, modified internucleotide linkage includes, but is not limited to, phosphorothioates; phosphorodithioates; methylphosphonates; 5'- alkylenephosphonates; 5'-methylphosphonate; 3'-alkylene phosphonates; borontrifluoridates; borano phosphate esters and selenophosphates of 3'-5'linkage or 2'-5'linkage; phosphotriesters; thionoalkylphosphotriesters; hydrogen phosphonate linkages; alkyl phosphonates; alkylphosphonothioates; aryl phosphonothioates; phosphoroselenoates; phosphorodiselenoates; phosphinates; phosphoramidates; 3'- alkylphosphoramidates; aminoalkylphosphoramidates; thionophosphoramidates; phosphoropiperazidates; phosphoroanilothioates; phosphoroanilidates; ketones; sulfones; sulfonamides; carbonates; carbamates; methylenehydrazos; methylenedimethylhydrazos; formacetals; thioformacetals; oximes; methyleneiminos; methylenemethyliminos; thioamidates; linkages with riboacetyl groups; aminoethyl glycine; silyl or siloxane linkages; alkyl or cycloalkyl linkages with or without heteroatoms of, for example, 1 to 10 carbons that are saturated or unsaturated and/or substituted and/or contain heteroatoms; linkages with morpholino structures, amides, or polyamides wherein the bases are attached to the aza nitrogens of the backbone directly or indirectly; and combinations thereof.

In some instances, the modification is a methyl or thiol modification such as methylphosphonate or thiolphosphonate modification. Exemplary thiolphosphonate nucleotide (left), phosphorodithioates (center) and methylphosphonate nucleotide (right) are illustrated below.

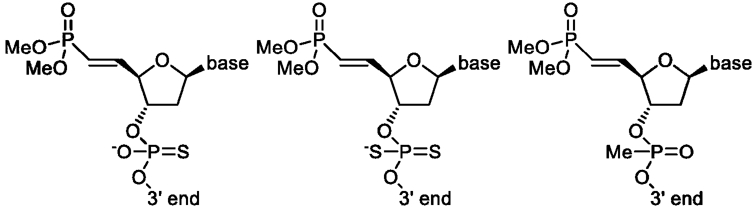

In some instances, a 5'-vinylphosphonate modified nucleotide includes, but is not limited to, phosphoramidites illustrated as:

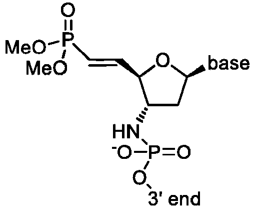

In some instances, the modified internucleotide linkage is a phosphorodiamidate linkage. A non-limiting example of a phosphorodiamidate linkage with a morpholino system is shown below.

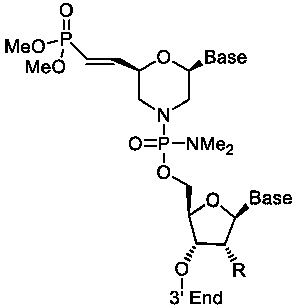

In some instances, the modified internucleotide linkage is a methylphosphonate linkage. A non-limiting example of a methylphosphonate linkage is shown below.

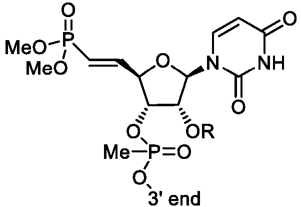

In some instances, the modified internucleotide linkage is a amide linkage. A non-limiting example of an amide linkage is shown below.

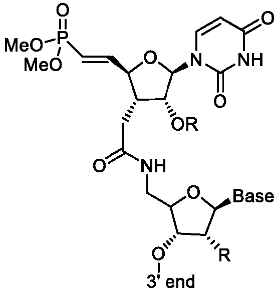

In some instances, a 5'-vinylphosphonate modified nucleotide includes, but is not limited to, the modified nucleic acid illustrated below.

In some embodiments, one or more modifications comprise a modified phosphate backbone in which the modification generates a neutral or uncharged backbone. In some instances, the phosphate backbone is modified by alkylation to generate an uncharged or neutral phosphate backbone. As used herein, alkylation includes methylation, ethylation, and propylation. In some cases, an alkyl group, as used herein in the context of alkylation, refers to a linear or branched saturated hydrocarbon group containing from 1 to 6 carbon atoms. In some instances, exemplary alkyl groups include, but are not limited to, methyl, ethyl, n-propyl, isopropyl, n-butyl, isobutyl, sec-butyl, tert-butyl, n- pentyl, isopentyl, neopentyl, hexyl, isohexyl, 1, 1 -dimethylbutyl, 2,2-dimethylbutyl, 3.3- dimethylbutyl, and 2-ethylbutyl groups. In some cases, a modified phosphate is a phosphate group as described in U.S. Patent No. 9481905.

In some embodiments, additional modified phosphate backbones comprise methylphosphonate, ethylphosphonate, methylthiophosphonate, or methoxyphosphonate. In some cases, the modified phosphate is methylphosphonate. In some cases, the modified phosphate is ethylphosphonate. In some cases, the modified phosphate is methylthiophosphonate. In some cases, the modified phosphate is methoxyphosphonate.

In some embodiments, one or more modifications further optionally include modifications of the ribose moiety, phosphate backbone and the nucleoside, or modifications of the nucleotide analogues at the 3' or the 5' terminus. For example, the 3' terminus optionally include a 3' cationic group, or by inverting the nucleoside at the 3'-terminus with a 3'-3' linkage. In another alternative, the 3'-terminus is optionally conjugated with an aminoalkyl group, e.g., a 3' C5-aminoalkyl dT. In an additional alternative, the 3'-terminus is optionally conjugated with an abasic site, *e.g.,* with an apurinic or apyrimidinic site. In some instances, the 5'-terminus is conjugated with an aminoalkyl group, e.g., a 5'-O-alkylamino substituent. In some cases, the 5'-terminus is conjugated with an abasic site, e.g., with an apurinic or apyrimidinic site.

In some embodiments, the polynucleic acid molecule comprises one or more of the artificial nucleotide analogues described herein. In some instances, the polynucleic acid molecule comprises 1, 2, 3, 4, 5, 6, 7, 8, 9, 10, 11, 12, 13, 14, 15, 16, 17, 18, 20, 25, or more of the artificial nucleotide analogues described herein. In some embodiments, the artificial nucleotide analogues include 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified, LNA, ENA, PNA, HNA, morpholino, methylphosphonate nucleotides, thiolphosphonate nucleotides, 2'-fluoro N3-P5'-phosphoramidites, or a combination thereof. In some instances, the polynucleic acid molecule comprises 1, 2, 3, 4, 5, 6, 7, 8, 9, 10, 11, 12, 13, 14, 15, 16, 17, 18, 20, 25, or more of the artificial nucleotide analogues selected from 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA) modified, LNA, ENA, PNA, HNA, morpholino, methylphosphonate nucleotides, thiolphosphonate nucleotides, 2'-fluoro N3-P5'-phosphoramidites, or a combination thereof. In some instances, the polynucleic acid molecule comprises 1, 2, 3, 4, 5, 6, 7, 8, 9, 10, 11, 12, 13, 14, 15, 16, 17, 18, 20, 25, or more of 2'-O-methyl modified nucleotides. In some instances, the polynucleic acid molecule comprises 1, 2, 3, 4, 5, 6, 7, 8, 9, 10, 11, 12, 13, 14, 15, 16, 17, 18, 20, 25, or more of 2'-O- methoxyethyl (2'-O-MOE) modified nucleotides. In some instances, the polynucleic acid molecule comprises 1, 2, 3, 4, 5, 6, 7, 8, 9, 10, 11, 12, 13, 14, 15, 16, 17, 18, 20, 25, or more of thiolphosphonate nucleotides.

In some embodiments, the polynucleic acid molecule comprises a plurality of phosphorodiamidate morpholino oligomers or a plurality of peptide nucleic acid-modified non-natural nucleotides, and optionally comprises at least one inverted abasic moiety. In some instances, the polynucleic acid molecule comprises at least 6, 7, 8, 9, 10, 11, 12, 13, 14, 15, 16, 17, 18, 19, 20, or more phosphorodiamidate morpholino oligomer-modified non-natural nucleotides. In some instances, the polynucleic acid molecule comprises 100% phosphorodiamidate morpholino oligomer-modified non-natural nucleotides.

In some instances, the polynucleic acid molecule comprises at least 6, 7, 8, 9, 10, 11, 12, 13, 14, 15, 16, 17, 18, 19, 20, or more peptide nucleic acid-modified non-natural nucleotides. In some instances, the polynucleic acid molecule comprises 100% peptide nucleic acid-modified non-natural nucleotides.

In some embodiments, the polynucleic acid molecule comprises one or more nucleotide analogs in which each nucleotide analog is in a stereochemically isomeric form. In such instance, the polynucleic acid molecule is a chiral molecule. In some cases, the nucleotide analog comprises a backbone stereochemistry. In additional cases, the nucleotide analog comprises a chiral analog as described in U.S. Patent 9,982,257, 9,695,211, or 9,605,019.

In some instances, the polynucleic acid molecule comprises at least one of: from about 5% to about 100% modification, from about 10% to about 100% modification, from about 20% to about 100% modification, from about 30% to about 100% modification, from about 40% to about 100% modification, from about 50% to about 100% modification, from about 60% to about 100% modification, from about 70% to about 100% modification, from about 80% to about 100% modification, and from about 90% to about 100% modification.

In some cases, the polynucleic acid molecule comprises at least one of: from about 10% to about 90% modification, from about 20% to about 90% modification, from about 30% to about 90% modification, from about 40% to about 90% modification, from about 50% to about 90% modification, from about 60% to about 90% modification, from about 70% to about 90% modification, and from about 80% to about 100% modification.

In some cases, the polynucleic acid molecule comprises at least one of: from about 10% to about 80% modification, from about 20% to about 80% modification, from about 30% to about 80% modification, from about 40% to about 80% modification, from about 50% to about 80% modification, from about 60% to about 80% modification, and from about 70% to about 80% modification.

In some instances, the polynucleic acid molecule comprises at least one of: from about 10% to about 70% modification, from about 20% to about 70% modification, from about 30% to about 70% modification, from about 40% to about 70% modification, from about 50% to about 70% modification, and from about 60% to about 70% modification.

In some instances, the polynucleic acid molecule comprises at least one of: from about 10% to about 60% modification, from about 20% to about 60% modification, from about 30% to about 60% modification, from about 40% to about 60% modification, and from about 50% to about 60% modification.

In some cases, the polynucleic acid molecule comprises at least one of: from about 10% to about 50% modification, from about 20% to about 50% modification, from about 30% to about 50% modification, and from about 40% to about 50% modification.

In some cases, the polynucleic acid molecule comprises at least one of: from about 10% to about 40% modification, from about 20% to about 40% modification, and from about 30% to about 40% modification.

In some cases, the polynucleic acid molecule comprises at least one of: from about 10% to about 30% modification, and from about 20% to about 30% modification.

In some cases, the polynucleic acid molecule comprises from about 10% to about 20% modification.

In some cases, the polynucleic acid molecule comprises from about 15% to about 90%, from about 20% to about 80%, from about 30% to about 70%, or from about 40% to about 60% modifications.

In additional cases, the polynucleic acid molecule comprises at least about 15%, 20%, 30%, 40%, 50%, 60%, 70%, 80%, 90%, 95%, or 99% modification.

In some embodiments, the polynucleic acid molecule comprises at least about 1, about 2, about 3, about 4, about 5, about 6, about 7, about 8, about 9, about 10, about 11, about 12, about 13, about 14, about 15, about 16, about 17, about 18, about 19, about 20, about 21, about 22 or more modifications.

In some instances, the polynucleic acid molecule comprises at least about 1, about 2, about 3, about 4, about 5, about 6, about 7, about 8, about 9, about 10, about 11, about 12, about 13, about 14, about 15, about 16, about 17, about 18, about 19, about 20, about 21, about 22 or more modified nucleotides.

In some instances, from about 5 to about 100% of the polynucleic acid molecule comprise the artificial nucleotide analogues described herein. In some instances, about 5%, 10%, 15%, 20%, 25%, 30%, 35%, 40%, 45%, 50%, 55%, 60%, 65%, 70%, 75%, 80%, 85%, 90%, 95% or 100% of the polynucleic acid molecule comprise the artificial nucleotide analogues described herein. In some instances, about 5% of a polynucleic acid molecule comprises the artificial nucleotide analogues described herein. In some instances, about 10% of a polynucleic acid molecule comprises the artificial nucleotide analogues described herein. In some instances, about 15% of a polynucleic acid molecule comprises the artificial nucleotide analogues described herein. In some instances, about 20% of a polynucleic acid molecule comprises the artificial nucleotide analogues described herein. In some instances, about 25% of a polynucleic acid molecule comprises the artificial nucleotide analogues described herein. In some instances, about 30% of a polynucleic acid molecule comprises the artificial nucleotide analogues described herein. In some instances, about 35% of a polynucleic acid molecule comprises the artificial nucleotide analogues described herein. In some instances, about 40% of a polynucleic acid molecule comprises the artificial nucleotide analogues described herein. In some instances, about 45% of a polynucleic acid molecule comprises the artificial nucleotide analogues described herein. In some instances, about 50% of a polynucleic acid molecule comprises the artificial nucleotide analogues described herein. In some instances, about 55% of a polynucleic acid molecule comprises the artificial nucleotide analogues described herein. In some instances, about 60% of a polynucleic acid molecule comprises the artificial nucleotide analogues described herein. In some instances, about 65% of a polynucleic acid molecule comprises the artificial nucleotide analogues described herein. In some instances, about 70% of a polynucleic acid molecule comprises the artificial nucleotide analogues described herein. In some instances, about 75% of a polynucleic acid molecule comprises the artificial nucleotide analogues described herein. In some instances, about 80% of a polynucleic acid molecule comprises the artificial nucleotide analogues described herein. In some instances, about 85% of a polynucleic acid molecule comprises the artificial nucleotide analogues described herein. In some instances, about 90% of a polynucleic acid molecule comprises the artificial nucleotide analogues described herein. In some instances, about 95% of a polynucleic acid molecule comprises the artificial nucleotide analogues described herein. In some instances, about 96% of a polynucleic acid molecule comprises the artificial nucleotide analogues described herein. In some instances, about 97% of a polynucleic acid molecule comprises the artificial nucleotide analogues described herein. In some instances, about 98% of a polynucleic acid molecule comprises the artificial nucleotide analogues described herein. In some instances, about 99% of a polynucleic acid molecule comprises the artificial nucleotide analogues described herein. In some instances, about 100% of a polynucleic acid molecule comprises the artificial nucleotide analogues described herein. In some embodiments, the artificial nucleotide analogues include 2'-O-methyl, 2'-O-methoxyethyl (2'-O-MOE), 2'-0-aminopropyl, 2'-deoxy, T-deoxy-2'-fluoro, 2'-0-aminopropyl (2'-O-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA) modified, LNA, ENA, PNA, HNA, morpholino, methylphosphonate nucleotides, thiolphosphonate nucleotides, 2'-fluoro N3-P5'-phosphoramidites, or a combination thereof.

In some embodiments, the polynucleic acid molecule comprises from about 1 to about 25 modifications in which the modification comprises an artificial nucleotide analogues described herein. In some embodiments, a polynucleic acid molecule comprises about 1 modification in which the modification comprises an artificial nucleotide analogue described herein. In some embodiments, a polynucleic acid molecule comprises about 2 modifications in which the modifications comprise an artificial nucleotide analogue described herein. In some embodiments, a polynucleic acid molecule comprises about 3 modifications in which the modifications comprise an artificial nucleotide analogue described herein. In some embodiments, a polynucleic acid molecule comprises about 4 modifications in which the modifications comprise an artificial nucleotide analogue described herein. In some embodiments, a polynucleic acid molecule comprises about 5 modifications in which the modifications comprise an artificial nucleotide analogue described herein. In some embodiments, a polynucleic acid molecule comprises about 6 modifications in which the modifications comprise an artificial nucleotide analogue described herein. In some embodiments, a polynucleic acid molecule comprises about 7 modifications in which the modifications comprise an artificial nucleotide analogue described herein. In some embodiments, a polynucleic acid molecule comprises about 8 modifications in which the modifications comprise an artificial nucleotide analogue described herein. In some embodiments, a polynucleic acid molecule comprises about 9 modifications in which the modifications comprise an artificial nucleotide analogue described herein. In some embodiments, a polynucleic acid molecule comprises about 10 modifications in which the modifications comprise an artificial nucleotide analogue described herein. In some embodiments, a polynucleic acid molecule comprises about 11 modifications in which the modifications comprise an artificial nucleotide analogue described herein. In some embodiments, a polynucleic acid molecule comprises about 12 modifications in which the modifications comprise an artificial nucleotide analogue described herein. In some embodiments, a polynucleic acid molecule comprises about 13 modifications in which the modifications comprise an artificial nucleotide analogue described herein. In some embodiments, a polynucleic acid molecule comprises about 14 modifications in which the modifications comprise an artificial nucleotide analogue described herein. In some embodiments, a polynucleic acid molecule comprises about 15 modifications in which the modifications comprise an artificial nucleotide analogue described herein. In some embodiments, a polynucleic acid molecule comprises about 16 modifications in which the modifications comprise an artificial nucleotide analogue described herein. In some embodiments, a polynucleic acid molecule comprises about 17 modifications in which the modifications comprise an artificial nucleotide analogue described herein. In some embodiments, a polynucleic acid molecule comprises about 18 modifications in which the modifications comprise an artificial nucleotide analogue described herein. In some embodiments, a polynucleic acid molecule comprises about 19 modifications in which the modifications comprise an artificial nucleotide analogue described herein. In some embodiments, a polynucleic acid molecule comprises about 20 modifications in which the modifications comprise an artificial nucleotide analogue described herein. In some embodiments, a polynucleic acid molecule comprises about 21 modifications in which the modifications comprise an artificial nucleotide analogue described herein. In some embodiments, a polynucleic acid molecule comprises about 22 modifications in which the modifications comprise an artificial nucleotide analogue described herein. In some embodiments, a polynucleic acid molecule comprises about 23 modifications in which the modifications comprise an artificial nucleotide analogue described herein. In some embodiments, a polynucleic acid molecule comprises about 24 modifications in which the modifications comprise an artificial nucleotide analogue described herein. In some embodiments, a polynucleic acid molecule comprises about 25 modifications in which the modifications comprise an artificial nucleotide analogue described herein.

In some embodiments, a polynucleic acid molecule is assembled from two separate polynucleotides wherein one polynucleotide comprises the sense strand and the second polynucleotide comprises the antisense strand of the polynucleic acid molecule. In other embodiments, the sense strand is connected to the antisense strand via a linker molecule, which in some instances is a polynucleotide linker or a non-nucleotide linker.

In some embodiments, a polynucleic acid molecule comprises a sense strand and antisense strand, wherein pyrimidine nucleotides in the sense strand comprises 2'-O-methylpyrimidine nucleotides and purine nucleotides in the sense strand comprise 2'-deoxy purine nucleotides. In some embodiments, a polynucleic acid molecule comprises a sense strand and antisense strand, wherein pyrimidine nucleotides present in the sense strand comprise 2'-deoxy-2'-fluoro pyrimidine nucleotides and wherein purine nucleotides present in the sense strand comprise 2'-deoxy purine nucleotides.

In some embodiments, a polynucleic acid molecule comprises a sense strand and antisense strand, wherein the pyrimidine nucleotides when present in said antisense strand are 2'-deoxy-2'-fluoro pyrimidine nucleotides and the purine nucleotides when present in said antisense strand are 2'-O-methyl purine nucleotides.

In some embodiments, a polynucleic acid molecule comprises a sense strand and antisense strand, wherein the pyrimidine nucleotides when present in said antisense strand are 2'-deoxy-2'-fluoro pyrimidine nucleotides and wherein the purine nucleotides when present in said antisense strand comprise 2'-deoxy-purine nucleotides.

In some embodiments, a polynucleic acid molecule comprises a sense strand and antisense strand, wherein the sense strand includes a terminal cap moiety at the 5'-end, the 3'-end, or both of the 5' and 3' ends of the sense strand. In other embodiments, the terminal cap moiety is an inverted deoxy abasic moiety.

In some embodiments, a polynucleic acid molecule comprises a sense strand and an antisense strand, wherein the antisense strand comprises a phosphate backbone modification at the 3' end of the antisense strand. In some instances, the phosphate backbone modification is a phosphorothioate. In some cases, the passenger strand comprises more phosphorothioate modifications than the guide strand. In other cases, the guide strand comprises more phosphorothioate modifications than the passenger strand. In additional cases, the passenger strand comprises about 2, 3, 4, 5, 6, 7, 8, 9, 10, or more phosphorothioate modifications. In additional cases, the guide strand comprises about 2, 3, 4, 5, 6, 7, 8, 9, 10, or more phosphorothioate modifications.

In some embodiments, a polynucleic acid molecule comprises a sense strand and an antisense strand, wherein the antisense strand comprises a glyceryl modification at the 3' end of the antisense strand.

In some embodiments, a polynucleic acid molecule comprises a sense strand and an antisense strand, in which the sense strand comprises one or more, for example, about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or about one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and in which the antisense strand comprises about 1 to about 10 or more, specifically about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In other embodiments, one or more, for example about 1, 2, 3, 4, 5, 6, 7, 8, 9, 10, or more, pyrimidine nucleotides of the sense and/or antisense strand are chemically-modified with 2'-deoxy, 2'-O-methyl and/or 2'-deoxy-2'-fluoro nucleotides, with or without one or more, for example about 1, 2, 3, 4, 5, 6, 7, 8, 9, 10, or more, phosphorothioate internucleotide linkages and/or a terminal cap molecule at the 3'-end, the 5'-end, or both of the 3'- and 5'-ends, being present in the same or different strand.

In some embodiments, a polynucleic acid molecule comprises a sense strand and an antisense strand, in which the sense strand comprises about 1 to about 25, for example, about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10, or more) 2'-deoxy, 2'-O-methyl, 2'-deoxy-2'-fluoro, and/or one or more (e.g., about 1, 2, 3, 4, 5, 6, 7, 8, 9, 10, or more) universal base modified nucleotides, and optionally a terminal cap molecule at the 3-end, the 5'-end, or both of the 3'- and 5'-ends of the sense strand; and in which the antisense strand comprises about 1 to about 25 or more, for example about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In other embodiments, one or more, for example about 1, 2, 3, 4, 5, 6, 7, 8, 9, 10, or more, pyrimidine nucleotides of the sense and/or antisense strand are chemically-modified with 2'-deoxy, 2'-O-methyl and/or 2'-deoxy-2'-fluoro nucleotides, with or without about 1 to about 25 or more, for example about 1, 2, 3, 4, 5, 6, 7, 8, 9, 10, 11, 12, 13, 14, 15, 16, 17, 18, 19, 20, or more phosphorothioate internucleotide linkages and/or a terminal cap molecule at the 3'-end, the 5'-end, or both of the 3'- and 5'-ends, being present in the same or different strand.

In some embodiments, a polynucleic acid molecule comprises a sense strand and an antisense strand, in which the antisense strand comprises one or more, for example, about 1, 2, 3, 4, 5, 6, 7, 8, 9, 10, 11, 12, 13, 14, 15, 16, 17, 18, 19, 20, or more phosphorothioate internucleotide linkages, and/or about one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 10 or more, specifically about 1, 2, 3, 4, 5, 6, 7, 8, 9, 10 or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In other embodiments, one or more, for example about 1, 2, 3, 4, 5, 6, 7, 8, 9, 10, 11, 12, 13, 14, 15, 16, 17, 18, 19, 20, or more pyrimidine nucleotides of the sense and/or antisense strand are chemically-modified with 2'-deoxy, 2'-O-methyl and/or 2'-deoxy-2'-fluoro nucleotides, with or without one or more, for example, about 1, 2, 3, 4, 5, 6, 7, 8, 9, 10 or more phosphorothioate internucleotide linkages and/or a terminal cap molecule at the 3'-end, the 5'-end, or both of the 3' and 5'-ends, being present in the same or different strand.

In some embodiments, a polynucleic acid molecule comprises a sense strand and an antisense strand, in which the antisense strand comprises about 1 to about 25 or more, for example, about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 25 or more, for example about 1, 2, 3, 4, 5, 6, 7, 8, 9, 10, 11, 12, 13, 14, 15, 16, 17, 18, 19, 20, or more phosphorothioate internucleotide linkages, and/or one or more (e.g., about 1, 2, 3, 4, 5, 6, 7, 8, 9, 10 or more) 2'-deoxy, 2'-O-methyl, 2'-deoxy-2'-fluoro, and/or one or more (e.g., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In other embodiments, one or more, for example about 1, 2, 3, 4, 5, 6, 7, 8, 9, 10 or more pyrimidine nucleotides of the sense and/or antisense strand are chemically-modified with 2'-deoxy, 2'-O-methyl and/or 2'-deoxy-2'-fluoro nucleotides, with or without about 1 to about 5, for example about 1, 2, 3, 4, 5 or more phosphorothioate internucleotide linkages and/or a terminal cap molecule at the 3'-end, the 5'-end, or both of the 3'- and 5'-ends, being present in the same or different strand.

In some embodiments, a polynucleic acid molecule is a duplex polynucleic acid molecule with one or more of the following properties: a greater hepatocyte stability, reduced overall charge, reduced hepatocyte uptake, or extended pharmacokinetics. In some embodiments, the duplex polynucleic acid molecule comprises a passenger strand (e.g., a sense strand) and a guide strand (e.g., an antisense strand) comprising a plurality of modifications.

In some embodiments, the duplex polynucleic acid molecule comprises a guide strand (e.g., an antisense strand) with one or more of the modification described above, and a passenger strand (e.g., a sense strand) with a plurality of phosphorodiamidate morpholino oligomers or a plurality of peptide nucleic acid-modified non-natural nucleotides.

In some embodiments, a polynucleic acid molecule described herein is a chemically-modified short interfering nucleic acid molecule having about 1 to about 25, for example, about 1, 2, 3, 4, 5, 6, 7, 8, 9, 10, 11, 12, 13, 14, 15, 16, 17, 18, 19, 20 or more phosphorothioate internucleotide linkages in each strand of the polynucleic acid molecule.

In another embodiment, a polynucleic acid molecule described herein comprises 2'-5' internucleotide linkages. In some instances, the 2'-5' internucleotide linkage(s) is at the 3'-end, the 5'-end, or both of the 3'- and 5'-ends of one or both sequence strands. In addition instances, the 2'-5' internucleotide linkage(s) is present at various other positions within one or both sequence strands, for example, about 1, 2, 3, 4, 5, 6, 7, 8, 9, 10, or more including every internucleotide linkage of a pyrimidine nucleotide in one or both strands of the polynucleic acid molecule comprise a 2'-5' internucleotide linkage, or about 1, 2, 3, 4, 5, 6, 7, 8, 9, 10, or more including every internucleotide linkage of a purine nucleotide in one or both strands of the polynucleic acid molecule comprise a 2'-5' internucleotide linkage.

In some embodiments, a polynucleic acid molecule is a single stranded polynucleic acid molecule that mediates RNAi activity in a cell or reconstituted in vitro system, wherein the polynucleic acid molecule comprises a single stranded polynucleotide having complementarity to a target nucleic acid sequence, and wherein one or more pyrimidine nucleotides present in the polynucleic acid are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the polynucleic acid are 2'-deoxy purine nucleotides (e.g., wherein all purine nucleotides are 2'-deoxy purine nucleotides or alternately a plurality of purine nucleotides are 2'-deoxy purine nucleotides), and a terminal cap modification, that is optionally present at the 3'-end, the 5'-end, or both of the 3' and 5'-ends of the antisense sequence, the polynucleic acid molecule optionally further comprising about 1 to about 4 (e.g., about 1, 2, 3, or 4) terminal 2'-deoxynucleotides at the 3'-end of the polynucleic acid molecule, wherein the terminal nucleotides further comprise one or more (e.g., 1, 2, 3, or 4) phosphorothioate internucleotide linkages, and wherein the polynucleic acid molecule optionally further comprises a terminal phosphate group, such as a 5'-terminal phosphate group.

In some cases, one or more of the artificial nucleotide analogues described herein are resistant toward nucleases such as for example ribonuclease such as RNase H, deoxyribunuclease such as DNase, or exonuclease such as 5'-3' exonuclease and 3'-5' exonuclease when compared to natural polynucleic acid molecules. In some instances, artificial nucleotide analogues comprising 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-0-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified, LNA, ENA, PNA, HNA, morpholino, methylphosphonate nucleotides, thiolphosphonate nucleotides, 2'-fluoro N3-P5'-phosphoramidites, or combinations thereof are resistant toward nucleases such as for example ribonuclease such as RNase H, deoxyribunuclease such as DNase, or exonuclease such as 5'-3' exonuclease and 3'-5' exonuclease. In some instances, 2'-O-methyl modified polynucleic acid molecule is nuclease resistance (e.g., RNase H, DNase, 5'-3' exonuclease or 3'-5' exonuclease resistance). In some instances, 2'O-methoxyethyl (2'-O-MOE) modified polynucleic acid molecule is nuclease resistance (e.g., RNase H, DNase, 5'-3' exonuclease or 3'-5' exonuclease resistance). In some instances, 2'-O-aminopropyl modified polynucleic acid molecule is nuclease resistance (e.g., RNase H, DNase, 5'-3' exonuclease or 3'-5' exonuclease resistance). In some instances, 2'-deoxy modified polynucleic acid molecule is nuclease resistance (e.g., RNase H, DNase, 5'-3' exonuclease or 3'-5' exonuclease resistance). In some instances, T-deoxy-2'-fluoro modified polynucleic acid molecule is nuclease resistance (e.g., RNase H, DNase, 5'-3' exonuclease or 3'-5' exonuclease resistance). In some instances, 2'-O-aminopropyl (2'-O-AP) modified polynucleic acid molecule is nuclease resistance (e.g., RNase H, DNase, 5'-3' exonuclease or 3'-5' exonuclease resistance). In some instances, 2'-O-dimethylaminoethyl (2'-O-DMAOE) modified polynucleic acid molecule is nuclease resistance (e.g., RNase H, DNase, 5'-3' exonuclease or 3'-5' exonuclease resistance). In some instances, 2'-O-dimethylaminopropyl (2'-O-DMAP) modified polynucleic acid molecule is nuclease resistance (e.g., RNase H, DNase, 5'-3' exonuclease or 3'-5' exonuclease resistance). In some instances, T-O- dimethylaminoethyloxyethyl (2'-O-DMAEOE) modified polynucleic acid molecule is nuclease resistance (e.g., RNase H, DNase, 5'-3' exonuclease or 3'-5' exonuclease resistance). In some instances, 2'-O-N-methylacetamido (2'-O-NMA) modified polynucleic acid molecule is nuclease resistance (e.g., RNase H, DNase, 5'-3' exonuclease or 3'-5' exonuclease resistance). In some instances, LNA modified polynucleic acid molecule is nuclease resistance (e.g., RNase H, DNase, 5'-3' exonuclease or 3'-5' exonuclease resistance). In some instances, ENA modified polynucleic acid molecule is nuclease resistance (e.g., RNase H, DNase, 5'-3' exonuclease or 3'-5' exonuclease resistance). In some instances, HNA modified polynucleic acid molecule is nuclease resistance (e.g., RNase H, DNase, 5'-3' exonuclease or 3'-5' exonuclease resistance). In some instances, morpholinos is nuclease resistance (e.g., RNase H, DNase, 5'-3' exonuclease or 3'-5' exonuclease resistance). In some instances, PNA modified polynucleic acid molecule is resistant to nucleases (e.g., RNase H, DNase, 5'-3' exonuclease or 3'-5' exonuclease resistance). In some instances, methylphosphonate nucleotides modified polynucleic acid molecule is nuclease resistance (e.g., RNase H, DNase, 5'-3' exonuclease or 3'-5' exonuclease resistance). In some instances, thiolphosphonate nucleotides modified polynucleic acid molecule is nuclease resistance (e.g., RNase H, DNase, 5'-3' exonuclease or 3'-5' exonuclease resistance). In some instances, polynucleic acid molecule comprising 2'-fluoro N3-P5'-phosphoramidites is nuclease resistance (e.g., RNase H, DNase, 5'-3' exonuclease or 3'-5' exonuclease resistance). In some instances, the 5' conjugates described herein inhibit 5'-3' exonucleolytic cleavage. In some instances, the 3' conjugates described herein inhibit 3'-5' exonucleolytic cleavage.

In some embodiments, one or more of the artificial nucleotide analogues described herein have increased binding affinity toward their mRNA target relative to an equivalent natural polynucleic acid molecule. The one or more of the artificial nucleotide analogues comprising 2'-O-methyl, 2'-O-methoxyethyl (2'-O-MOE), 2'-O-aminopropyl, 2'-deoxy, T-deoxy-2'-fluoro, 2'-O-aminopropyl (2'-0-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified, LNA, ENA, PNA, HNA, morpholino, methylphosphonate nucleotides, thiolphosphonate nucleotides, or 2'-fluoro N3-P5'-phosphoramidites have increased binding affinity toward their mRNA target relative to an equivalent natural polynucleic acid molecule. In some instances, 2'-O-methyl modified polynucleic acid molecule has increased binding affinity toward their mRNA target relative to an equivalent natural polynucleic acid molecule. In some instances, 2'-O-methoxyethyl (2'-O-MOE) modified polynucleic acid molecule has increased binding affinity toward their mRNA target relative to an equivalent natural polynucleic acid molecule. In some instances, 2'-O-aminopropyl modified polynucleic acid molecule has increased binding affinity toward their mRNA target relative to an equivalent natural polynucleic acid molecule. In some instances, 2'-deoxy modified polynucleic acid molecule has increased binding affinity toward their mRNA target relative to an equivalent natural polynucleic acid molecule. In some instances, T-deoxy-2'-fluoro modified polynucleic acid molecule has increased binding affinity toward their mRNA target relative to an equivalent natural polynucleic acid molecule. In some instances, 2'-O-aminopropyl (2'-O-AP) modified polynucleic acid molecule has increased binding affinity toward their mRNA target relative to an equivalent natural polynucleic acid molecule. In some instances, 2'-O-dimethylaminoethyl (2'-O-DMAOE) modified polynucleic acid molecule has increased binding affinity toward their mRNA target relative to an equivalent natural polynucleic acid molecule. In some instances, 2'-O-dimethylaminopropyl (2'-O-DMAP) modified polynucleic acid molecule has increased binding affinity toward their mRNA target relative to an equivalent natural polynucleic acid molecule. In some instances, T-O- dimethylaminoethyloxyethyl (2'-O-DMAEOE) modified polynucleic acid molecule has increased binding affinity toward their mRNA target relative to an equivalent natural polynucleic acid molecule. In some instances, 2'-O-N-methylacetamido (2'-O-NMA) modified polynucleic acid molecule has increased binding affinity toward their mRNA target relative to an equivalent natural polynucleic acid molecule. In some instances, LNA modified polynucleic acid molecule has increased binding affinity toward their mRNA target relative to an equivalent natural polynucleic acid molecule. In some instances, ENA modified polynucleic acid molecule has increased binding affinity toward their mRNA target relative to an equivalent natural polynucleic acid molecule. In some instances, PNA modified polynucleic acid molecule has increased binding affinity toward their mRNA target relative to an equivalent natural polynucleic acid molecule. In some instances, HNA modified polynucleic acid molecule has increased binding affinity toward their mRNA target relative to an equivalent natural polynucleic acid molecule. In some instances, morpholino modified polynucleic acid molecule has increased binding affinity toward their mRNA target relative to an equivalent natural polynucleic acid molecule. In some instances, methylphosphonate nucleotides modified polynucleic acid molecule has increased binding affinity toward their mRNA target relative to an equivalent natural polynucleic acid molecule. In some instances, thiolphosphonate nucleotides modified polynucleic acid molecule has increased binding affinity toward their mRNA target relative to an equivalent natural polynucleic acid molecule. In some instances, polynucleic acid molecule comprising 2'-fluoro N3-P5'-phosphoramidites has increased binding affinity toward their mRNA target relative to an equivalent natural polynucleic acid molecule. In some cases, the increased affinity is illustrated with a lower Kd, a higher melt temperature (Tm), or a combination thereof.

In some embodiments, a polynucleic acid molecule described herein is a chirally pure (or stereo pure) polynucleic acid molecule, or a polynucleic acid molecule comprising a single enantiomer. In some instances, the polynucleic acid molecule comprises L-nucleotide. In some instances, the polynucleic acid molecule comprises D-nucleotides. In some instance, a polynucleic acid molecule composition comprises less than 30%, 25%, 20%, 15%, 10%, 5%, 4%, 3%, 2%, 1%, or less of its mirror enantiomer. In some cases, a polynucleic acid molecule composition comprises less than 30%, 25%, 20%, 15%, 10%, 5%, 4%, 3%, 2%, 1%, or less of a racemic mixture. In some instances, the polynucleic acid molecule is a polynucleic acid molecule described in: U.S. Patent Publication Nos: 2014/194610 and 2015/211006; and PCT Publication No.: WO2015107425.

In some embodiments, a polynucleic acid molecule described herein is further modified to include an aptamer conjugating moiety. In some instances, the aptamer conjugating moiety is a DNA aptamer conjugating moiety. In some instances, the aptamer conjugating moiety is Alphamer (Centauri Therapeutics), which comprises an aptamer portion that recognizes a specific cell-surface target and a portion that presents a specific epitopes for attaching to circulating antibodies. In some instance, a polynucleic acid molecule described herein is further modified to include an aptamer conjugating moiety as described in: U.S. Patent Nos: 8,604,184, 8,591,910, and 7,850,975.

In additional embodiments, a polynucleic acid molecule described herein is modified to increase its stability. In some embodiment, the polynucleic acid molecule is RNA (e.g., siRNA). In some instances, the polynucleic acid molecule is modified by one or more of the modifications described above to increase its stability. In some cases, the polynucleic acid molecule is modified at the 2' hydroxyl position, such as by 2'-O-methyl, 2'-O-methoxyethyl (2'-O-MOE), 2'-O-aminopropyl, 2'-deoxy, T-deoxy-2'-fluoro, 2'-O-aminopropyl (2'-0-AP), 2'-O-dimethylaminoethyl (2'-O-DMAOE), 2'-O-dimethylaminopropyl (2'-O-DMAP), T-O- dimethylaminoethyloxyethyl (2'-O-DMAEOE), or 2'-O-N-methylacetamido (2'-O-NMA) modification or by a locked or bridged ribose conformation (e.g., LNA or ENA). In some cases, the polynucleic acid molecule is modified by 2'-O-methyl and/or 2'-O-methoxyethyl ribose. In some cases, the polynucleic acid molecule also includes morpholinos, PNAs, HNA, methylphosphonate nucleotides, thiolphosphonate nucleotides, and/or 2'-fluoro N3-P5'-phosphoramidites to increase its stability. In some instances, the polynucleic acid molecule is a chirally pure (or stereo pure) polynucleic acid molecule. In some instances, the chirally pure (or stereo pure) polynucleic acid molecule is modified to increase its stability. Suitable modifications to the RNA to increase stability for delivery will be apparent to the skilled person.

In some embodiments, a polynucleic acid molecule describe herein has RNAi activity that modulates expression of RNA encoded by a gene involved in muscular dystrophy such as, but not limited to, *DMD, DUX4, DYSF, EMD,* or *LMNA.* In some instances, a polynucleic acid molecule describe herein is a double-stranded siRNA molecule that down-regulates expression of at least one of *DMD, DUX4, DYSF, EMD,* or *LMNA,* wherein one of the strands of the double-stranded siRNA molecule comprises a nucleotide sequence that is complementary to a nucleotide sequence of at least one of *DMD, DUX4, DYSF, EMD,* or *LMNA* or RNA encoded by at least one of *DMD, DUX4, DYSF, EMD,* or *LMNA* or a portion thereof, and wherein the second strand of the double-stranded siRNA molecule comprises a nucleotide sequence substantially similar to the nucleotide sequence of at least one of *DMD, DUX4, DYSF, EMD,* or *LMNA* or RNA encoded by at least one of *DMD, DUX4, DYSF, EMD,* or *LMNA* or a portion thereof. In some cases, a polynucleic acid molecule describe herein is a double-stranded siRNA molecule that down-regulates expression of at least one of *DMD, DUX4, DYSF, EMD,* or *LMNA,* wherein each strand of the siRNA molecule comprises about 15 to 25, 18 to 24, or 19 to about 23 nucleotides, and wherein each strand comprises at least about 14, 17, or 19 nucleotides that are complementary to the nucleotides of the other strand. In some cases, a polynucleic acid molecule describe herein is a double-stranded siRNA molecule that down-regulates expression of at least one of *DMD, DUX4, DYSF, EMD,* or *LMNA,* wherein each strand of the siRNA molecule comprises about 19 to about 23 nucleotides, and wherein each strand comprises at least about 19 nucleotides that are complementary to the nucleotides of the other strand. In some instances, the RNAi activity occurs within a cell. In other instances, the RNAi activity occurs in a reconstituted in vitro system.

In some embodiments, a polynucleic acid molecule describe herein has RNAi activity that modulates expression of RNA encoded by the *DMD* gene. In some instances, a polynucleic acid molecule describe herein is a single-stranded siRNA molecule that down-regulates expression of *DMD,* wherein the single-stranded siRNA molecule comprises a nucleotide sequence that is complementary to a nucleotide sequence of *DMD* or RNA encoded by *DMD* or a portion thereof. In some cases, a polynucleic acid molecule describe herein is a single-stranded siRNA molecule that down-regulates expression of *DMD,* wherein the siRNA molecule comprises about 15 to 25, 18 to 24, or 19 to about 23 nucleotides. In some cases, a polynucleic acid molecule describe herein is a single-stranded siRNA molecule that down-regulates expression of *DMD,* wherein the siRNA molecule comprises about 19 to about 23 nucleotides. In some instances, the RNAi activity occurs within a cell. In other instances, the RNAi activity occurs in a reconstituted in vitro system.

In some instances, the polynucleic acid molecule is a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. In some instances, the polynucleic acid molecule is assembled from two separate polynucleotides, where one strand is the sense strand and the other is the antisense strand, wherein the antisense and sense strands are self-complementary (e.g., each strand comprises nucleotide sequence that is complementary to nucleotide sequence in the other strand; such as where the antisense strand and sense strand form a duplex or double stranded structure, for example wherein the double stranded region is about 19, 20, 21, 22, 23, or more base pairs); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the polynucleic acid molecule is assembled from a single oligonucleotide, where the self-complementary sense and antisense regions of the polynucleic acid molecule are linked by means of a nucleic acid based or non-nucleic acid-based linker(s).

In some cases, the polynucleic acid molecule is a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. In other cases, the polynucleic acid molecule is a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide is processed either in vivo or in vitro to generate an active polynucleic acid molecule capable of mediating RNAi. In additional cases, the polynucleic acid molecule also comprises a single stranded polynucleotide having nucleotide sequence complementary to nucleotide sequence in a target nucleic acid molecule or a portion thereof (for example, where such polynucleic acid molecule does not require the presence within the polynucleic acid molecule of nucleotide sequence corresponding to the target nucleic acid sequence or a portion thereof), wherein the single stranded polynucleotide further comprises a terminal phosphate group, such as a 5'-phosphate (see for example Martinez et al., 2002, Cell., 110, 563-574 and Schwarz et al., 2002, Molecular Cell, 10, 537-568), or 5',3'-diphosphate.

In some instances, an asymmetric is a linear polynucleic acid molecule comprising an antisense region, a loop portion that comprises nucleotides or non-nucleotides, and a sense region that comprises fewer nucleotides than the antisense region to the extent that the sense region has enough complimentary nucleotides to base pair with the antisense region and form a duplex with loop. For example, an asymmetric hairpin polynucleic acid molecule comprises an antisense region having length sufficient to mediate RNAi in a cell or in vitro system (e.g. about 19 to about 22 nucleotides) and a loop region comprising about 4 to about 8 nucleotides, and a sense region having about 3 to about 18 nucleotides that are complementary to the antisense region. In some cases, the asymmetric hairpin polynucleic acid molecule also comprises a 5'-terminal phosphate group that is chemically modified. In additional cases, the loop portion of the asymmetric hairpin polynucleic acid molecule comprises nucleotides, non-nucleotides, linker molecules, or conjugate molecules.

In some embodiments, an asymmetric duplex is a polynucleic acid molecule having two separate strands comprising a sense region and an antisense region, wherein the sense region comprises fewer nucleotides than the antisense region to the extent that the sense region has enough complimentary nucleotides to base pair with the antisense region and form a duplex. For example, an asymmetric duplex polynucleic acid molecule comprises an antisense region having length sufficient to mediate RNAi in a cell or in vitro system (e.g. about 19 to about 22 nucleotides) and a sense region having about 3 to about 18 nucleotides that are complementary to the antisense region.

In some cases, an universal base refers to nucleotide base analogs that form base pairs with each of the natural DNA/RNA bases with little discrimination between them. Non-limiting examples of universal bases include C-phenyl, C-naphthyl and other aromatic derivatives, inosine, azole carboxamides, and nitroazole derivatives such as 3-nitropyrrole, 4-nitroindole, 5-nitroindole, and 6-nitroindole as known in the art (see for example Loakes, 2001, Nucleic Acids Research, 29, 2437-2447).

### Polynucleic Acid Molecule Synthesis

In some embodiments, a polynucleic acid molecule described herein is constructed using chemical synthesis and/or enzymatic ligation reactions using procedures known in the art. For example, a polynucleic acid molecule is chemically synthesized using naturally occurring nucleotides or variously modified nucleotides designed to increase the biological stability of the molecules or to increase the physical stability of the duplex formed between the polynucleic acid molecule and target nucleic acids. Exemplary methods include those described in: U.S. Patent Nos. 5,142,047; 5,185,444; 5,889,136; 6,008,400; and 6,111,086; PCT Publication No. WO2009099942; or European Publication No. 1579015. Additional exemplary methods include those described in: Griffey et al., "2'-O-aminopropyl ribonucleotides: a zwitterionic modification that enhances the exonuclease resistance and biological activity of antisense oligonucleotides," J. Med. Chem. 39(26):5100-5109 (1997)); Obika, et al. "Synthesis of 2'-0,4'-C-methyleneuridine and -cytidine. Novel bicyclic nucleosides having a fixed C3, -endo sugar puckering". Tetrahedron Letters 38 (50): 8735 (1997); Koizumi, M. "ENA oligonucleotides as therapeutics". Current opinion in molecular therapeutics 8 (2): 144-149 (2006); and Abramova et al., "Novel oligonucleotide analogues based on morpholino nucleoside subunits-antisense technologies: new chemical possibilities," Indian Journal of Chemistry 48B:1721-1726 (2009). Alternatively, the polynucleic acid molecule is produced biologically using an expression vector into which a polynucleic acid molecule has been subcloned in an antisense orientation (i.e., RNA transcribed from the inserted polynucleic acid molecule will be of an antisense orientation to a target polynucleic acid molecule of interest).

In some embodiments, a polynucleic acid molecule is synthesized via a tandem synthesis methodology, wherein both strands are synthesized as a single contiguous oligonucleotide fragment or strand separated by a cleavable linker which is subsequently cleaved to provide separate fragments or strands that hybridize and permit purification of the duplex.

In some instances, a polynucleic acid molecule is also assembled from two distinct nucleic acid strands or fragments wherein one fragment includes the sense region and the second fragment includes the antisense region of the molecule.

Additional modification methods for incorporating, for example, sugar, base and phosphate modifications include: Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci., 1992, 17, 334-339; Usman et al. International Publication PCT No. WO 93/15187; Sproat, U.S. Pat. No. 5,334,711 and Beigelman et al., 1995, J. Biol. Chem., 270, 25702; Beigelman et al., International PCT publication No. WO 97/26270; Beigelman et al., U.S. Pat. No. 5,716,824; Usman et al., U.S. Pat. No. 5,627,053; Woolf et al., International PCT Publication No. WO 98/13526; Thompson et al., U.S. Ser. No. 60/082,404 which was filed on Apr. 20, 1998; Karpeisky et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; and Burlina et al., 1997, Bioorg. Med. Chem., 5, 1999-2010. Such publications describe general methods and strategies to determine the location of incorporation of sugar, base and/or phosphate modifications and the like into nucleic acid molecules without modulating catalysis.

In some instances, while chemical modification of the polynucleic acid molecule internucleotide linkages with phosphorothioate, phosphorodithioate, and/or 5'-methylphosphonate linkages improves stability, excessive modifications sometimes cause toxicity or decreased activity. Therefore, when designing nucleic acid molecules, the amount of these internucleotide linkages in some cases is minimized. In such cases, the reduction in the concentration of these linkages lowers toxicity, increases efficacy and higher specificity of these molecules.

### Nucleic Acid-Polypeptide Conjugate

In some embodiments, a polynucleic acid molecule is further conjugated to a polypeptide A for delivery to a site of interest. In some cases, a polynucleic acid molecule is conjugated to a polypeptide A and optionally a polymeric moiety.

In some instances, at least one polypeptide A is conjugated to at least one B. In some instances, the at least one polypeptide A is conjugated to the at least one B to form an A-B conjugate. In some embodiments, at least one A is conjugated to the 5' terminus of B, the 3' terminus of B, an internal site on B, or in any combinations thereof. In some instances, the at least one polypeptide A is conjugated to at least two B. In some instances, the at least one polypeptide A is conjugated to at least 2, 3, 4, 5, 6, 7, 8, or more B.

In some embodiments, at least one polypeptide A is conjugated at one terminus of at least one B while at least one C is conjugated at the opposite terminus of the at least one B to form an A-B-C conjugate. In some instances, at least one polypeptide A is conjugated at one terminus of the at least one B while at least one of C is conjugated at an internal site on the at least one B. In some instances, at least one polypeptide A is conjugated directly to the at least one C. In some instances, the at least one B is conjugated indirectly to the at least one polypeptide A via the at least one C to form an A-C-B conjugate.

In some instances, at least one B and/or at least one C, and optionally at least one D are conjugated to at least one polypeptide A. In some instances, the at least one B is conjugated at a terminus (e.g., a 5' terminus or a 3' terminus) to the at least one polypeptide A or are conjugated via an internal site to the at least one polypeptide A. In some cases, the at least one C is conjugated either directly to the at least one polypeptide A or indirectly via the at least one B. If indirectly via the at least one B, the at least one C is conjugated either at the same terminus as the at least one polypeptide A on B, at opposing terminus from the at least one polypeptide A, or independently at an internal site. In some instances, at least one additional polypeptide A is further conjugated to the at least one polypeptide A, to B, or to C. In additional instances, the at least one D is optionally conjugated either directly or indirectly to the at least one polypeptide A, to the at least one B, or to the at least one C. If directly to the at least one polypeptide A, the at least one D is also optionally conjugated to the at least one B to form an A-D-B conjugate or is optionally conjugated to the at least one B and the at least one C to form an A-D-B-C conjugate. In some instances, the at least one D is directly conjugated to the at least one polypeptide A and indirectly to the at least one B and the at least one C to form a D-A-B-C conjugate. If indirectly to the at least one polypeptide A, the at least one D is also optionally conjugated to the at least one B to form an A-B-D conjugate or is optionally conjugated to the at least one B and the at least one C to form an A-B-D-C conjugate. In some instances, at least one additional D is further conjugated to the at least one polypeptide A, to B, or to C.

In some embodiments, a polynucleic acid molecule conjugate comprises a construct as illustrated:

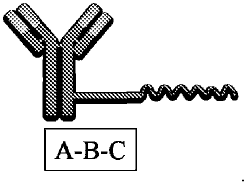

In some embodiments, a polynucleic acid molecule conjugate comprises a construct as illustrated:

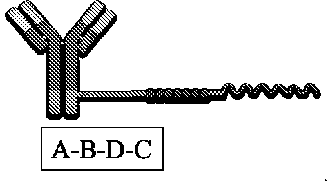

In some embodiments, a polynucleic acid molecule conjugate comprises a construct as illustrated:

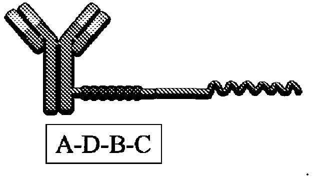

In some embodiments, a polynucleic acid molecule conjugate comprises a construct as illustrated:

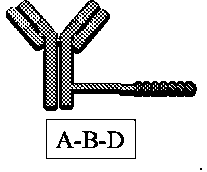

In some embodiments, a polynucleic acid molecule conjugate comprises a construct as illustrated:

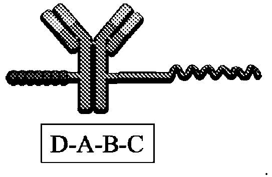

In some embodiments, a polynucleic acid molecule conjugate comprises a construct as illustrated:

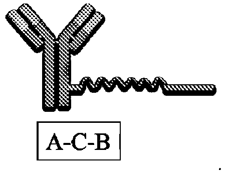

In some embodiments, a polynucleic acid molecule conjugate comprises a construct as illustrated:

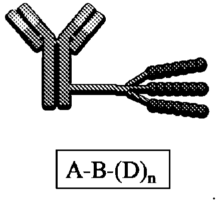

In some embodiments, a polynucleic acid molecule conjugate comprises a construct as illustrated:

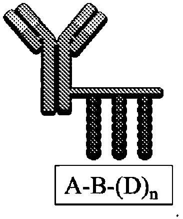

In some embodiments, a polynucleic acid molecule conjugate comprises a construct as illustrated:

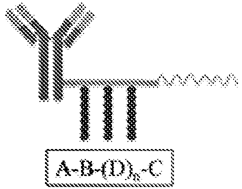

In some embodiments, a polynucleic acid molecule conjugate comprises a construct as illustrated:

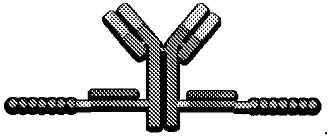

In some embodiments, a polynucleic acid molecule conjugate comprises a construct as illustrated:

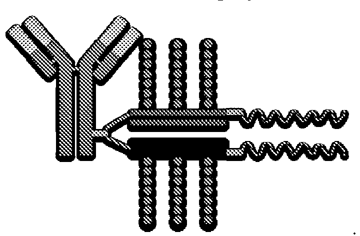

In some embodiments, a polynucleic acid molecule conjugate comprises a construct as illustrated:

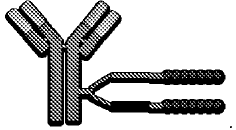

In some embodiments, a polynucleic acid molecule conjugate comprises a construct as illustrated:.

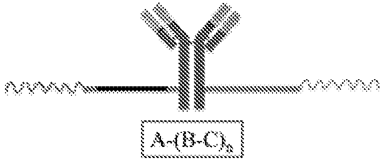

The 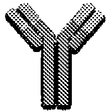 as illustrated above is for representation purposes only and encompasses a humanized antibody or binding fragment thereof, chimeric antibody or binding fragment thereof, monoclonal antibody or binding fragment thereof, monovalent Fab', divalent Fab2, single-chain variable fragment (scFv), diabody, minibody, nanobody, single-domain antibody (sdAb), or camelid antibody or binding fragment thereof.

### Binding Moiety

In some embodiments, the binding moiety A is a polypeptide. In some instances, the polypeptide is an antibody or its fragment thereof. In some cases, the fragment is a binding fragment. In some instances, the antibody or binding fragment thereof comprises a humanized antibody or binding fragment thereof, murine antibody or binding fragment thereof, chimeric antibody or binding fragment thereof, monoclonal antibody or binding fragment thereof, monovalent Fab', divalent Fab₂, F(ab)'₃ fragments, single-chain variable fragment (scFv), bis-scFv, (scFv)₂, diabody, minibody, nanobody, triabody, tetrabody, disulfide stabilized Fv protein (dsFv), single-domain antibody (sdAb), Ig NAR, camelid antibody or binding fragment thereof, bispecific antibody or biding fragment thereof, or a chemically modified derivative thereof.

In some instances, A is an antibody or binding fragment thereof. In some instances, A is a humanized antibody or binding fragment thereof, murine antibody or binding fragment thereof, chimeric antibody or binding fragment thereof, monoclonal antibody or binding fragment thereof, monovalent Fab', divalent Fab₂, F(ab)'₃ fragments, single-chain variable fragment (scFv), bis-scFv, (scFv)₂, diabody, minibody, nanobody, triabody, tetrabody, disulfide stabilized Fv protein ("dsFv"), single-domain antibody (sdAb), Ig NAR, camelid antibody or binding fragment thereof, bispecific antibody or biding fragment thereof, or a chemically modified derivative thereof. In some instances, A is a humanized antibody or binding fragment thereof. In some instances, A is a murine antibody or binding fragment thereof. In some instances, A is a chimeric antibody or binding fragment thereof. In some instances, A is a monoclonal antibody or binding fragment thereof. In some instances, A is a monovalent Fab'. In some instances, A is a diavalent Fab₂. In some instances, A is a single-chain variable fragment (scFv).

In some embodiments, the binding moiety A is a bispecific antibody or binding fragment thereof. In some instances, the bispecific antibody is a trifunctional antibody or a bispecific mini-antibody. In some cases, the bispecific antibody is a trifunctional antibody. In some instances, the trifunctional antibody is a full length monoclonal antibody comprising binding sites for two different antigens.

In some cases, the bispecific antibody is a bispecific mini-antibody. In some instances, the bispecific mini-antibody comprises divalent Fab₂, F(ab)'₃ fragments, bis-scFv, (scFv)₂, diabody, minibody, triabody, tetrabody or a bi-specific T-cell engager (BiTE). In some embodiments, the bi-specific T-cell engager is a fusion protein that contains two single-chain variable fragments (scFvs) in which the two scFvs target epitopes of two different antigens.

In some embodiments, the binding moiety A is a bispecific mini-antibody. In some instances, A is a bispecific Fab₂. In some instances, A is a bispecific F(ab)'₃ fragment. In some cases, A is a bispecific bis-scFv. In some cases, A is a bispecific (scFv)₂. In some embodiments, A is a bispecific diabody. In some embodiments, A is a bispecific minibody. In some embodiments, A is a bispecific triabody. In other embodiments, A is a bispecific tetrabody. In other embodiments, A is a bi-specific T-cell engager (BiTE).

In some embodiments, the binding moiety A is a trispecific antibody. In some instances, the trispecific antibody comprises F(ab)'₃ fragments or a triabody. In some instances, A is a trispecific F(ab)'₃ fragment. In some cases, A is a triabody. In some embodiments, A is a trispecific antibody as described in Dimas, et al., "Development of a trispecific antibody designed to simultaneously and efficiently target three different antigens on tumor cells," Mol. Pharmaceutics, 12(9): 3490-3501 (2015).

In some embodiments, the binding moiety A is an antibody or binding fragment thereof that recognizes a cell surface protein. In some instances, the binding moiety A is an antibody or binding fragment thereof that recognizes a cell surface protein on a muscle cell. Exemplary cell surface proteins recognized by an antibody or binding fragment thereof include, but are not limited to, Sca-1, CD34, Myo-D, myogenin, MRF4, NCAM, CD43, and CD95 (Fas).

In some instances, the cell surface protein comprises clusters of differentiation (CD) cell surface markers. Exemplary CD cell surface markers include, but are not limited to, CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L (L-selectin), CD62P, CD63, CD64, CD65, CD66a, CD66b, CD66c, CD66d, CD66e, CD79 (e.g., CD79a, CD79b), CD90, CD95 (Fas), CD103, CD104, CD125 (IL5RA), CD134 (OX40), CD137 (4-1BB), CD152 (CTLA-4), CD221, CD274, CD279 (PD-1), CD319 (SLAMF7), CD326 (EpCAM), and the like.

In some instances, the binding moiety A is an antibody or binding fragment thereof that recognizes a CD cell surface marker. In some instances, the binding moiety A is an antibody or binding fragment thereof that recognizes CD1, CD2, CD3, CD4, CD5, CD6, CD7, CD8, CD9, CD10, CD11a, CD11b, CD11c, CD11d, CDw12, CD13, CD14, CD15, CD15s, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD45RO, CD45RA, CD45RB, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L (L-selectin), CD62P, CD63, CD64, CD65, CD66a, CD66b, CD66c, CD66d, CD66e, CD79 (e.g., CD79a, CD79b), CD90, CD95 (Fas), CD103, CD104, CD125 (IL5RA), CD134 (OX40), CD137 (4-1BB), CD152 (CTLA-4), CD221, CD274, CD279 (PD-1), CD319 (SLAMF7), CD326 (EpCAM), or a combination thereof.

In some embodiments, the binding moiety A is an anti-myosin antibody, an anti-transferrin antibody, and an antibody that recognizes Muscle-Specific kinase (MuSK).

In some instances, the binding moiety A is an anti-myosin antibody. In some cases, the anti-myosin antibody is a humanized antibody. In other cases, the anti-myosin antibody is a chimeric antibody. In additional cases, the anti-myosin antibody is a monovalent, a divalent, or a multi-valent antibody.

In some instances, the binding moiety A is an anti-transferrin (anti-CD71) antibody. In some cases, the anti-transferrin antibody is a humanized antibody. In other cases, the anti-transferrin antibody is a chimeric antibody. In additional cases, the anti-transferrin antibody is a monovalent, a divalent, or a multi-valent antibody. In some embodiments, exemplary anti-transferrin antibodies include MAB5746 from R&D Systems, AHP858 from Bio-Rad Laboratories, A80-128A from Bethyl Laboratories, Inc., and T2027 from MilliporeSigma.

In some instances, the binding moiety A is an antibody that recognizes MuSK. In some cases, the anti-MuSK antibody is a humanized antibody. In other cases, the anti-MuSK antibody is a chimeric antibody. In additional cases, the anti-MuSK antibody is a monovalent, a divalent, or a multi-valent antibody.

In some embodiments, the binding moiety A is conjugated to a polynucleic acid molecule (B) non-specifically. In some instances, the binding moiety A is conjugated to a polynucleic acid molecule (B) via a lysine residue or a cysteine residue, in a non-site specific manner. In some instances, the binding moiety A is conjugated to a polynucleic acid molecule (B) via a lysine residue in a non-site specific manner. In some cases, the binding moiety A is conjugated to a polynucleic acid molecule (B) via a cysteine residue in a non-site specific manner.

In some embodiments, the binding moiety A is conjugated to a polynucleic acid molecule (B) in a site-specific manner. In some instances, the binding moiety A is conjugated to a polynucleic acid molecule (B) through a lysine residue, a cysteine residue, at the 5'-terminus, at the 3'-terminus, an unnatural amino acid, or an enzyme-modified or enzyme-catalyzed residue, via a site-specific manner. In some instances, the binding moiety A is conjugated to a polynucleic acid molecule (B) through a lysine residue via a site-specific manner. In some instances, the binding moiety A is conjugated to a polynucleic acid molecule (B) through a cysteine residue via a site-specific manner. In some instances, the binding moiety A is conjugated to a polynucleic acid molecule (B) at the 5'-terminus via a site-specific manner. In some instances, the binding moiety A is conjugated to a polynucleic acid molecule (B) at the 3'-terminus via a site-specific manner. In some instances, the binding moiety A is conjugated to a polynucleic acid molecule (B) through an unnatural amino acid via a site-specific manner. In some instances, the binding moiety A is conjugated to a polynucleic acid molecule (B) through an enzyme-modified or enzyme-catalyzed residue via a site-specific manner.

In some embodiments, one or more polynucleic acid molecule (B) is conjugated to a binding moiety A. In some instances, about 1, 2, 3, 4, 5, 6, 7, 8, 9, 10, 11, 12, 13, 14, 15, 16, or more polynucleic acid molecules are conjugated to one binding moiety A. In some instances, about 1 polynucleic acid molecule is conjugated to one binding moiety A. In some instances, about 2 polynucleic acid molecules are conjugated to one binding moiety A. In some instances, about 3 polynucleic acid molecules are conjugated to one binding moiety A. In some instances, about 4 polynucleic acid molecules are conjugated to one binding moiety A. In some instances, about 5 polynucleic acid molecules are conjugated to one binding moiety A. In some instances, about 6 polynucleic acid molecules are conjugated to one binding moiety A. In some instances, about 7 polynucleic acid molecules are conjugated to one binding moiety A. In some instances, about 8 polynucleic acid molecules are conjugated to one binding moiety A. In some instances, about 9 polynucleic acid molecules are conjugated to one binding moiety A. In some instances, about 10 polynucleic acid molecules are conjugated to one binding moiety A. In some instances, about 11 polynucleic acid molecules are conjugated to one binding moiety A. In some instances, about 12 polynucleic acid molecules are conjugated to one binding moiety A. In some instances, about 13 polynucleic acid molecules are conjugated to one binding moiety A. In some instances, about 14 polynucleic acid molecules are conjugated to one binding moiety A. In some instances, about 15 polynucleic acid molecules are conjugated to one binding moiety A. In some instances, about 16 polynucleic acid molecules are conjugated to one binding moiety A. In some cases, the one or more polynucleic acid molecules are the same. In other cases, the one or more polynucleic acid molecules are different.

In some embodiments, the number of polynucleic acid molecule (B) conjugated to a binding moiety A forms a ratio. In some instances, the ratio is referred to as a DAR (drug-to-antibody) ratio, in which the drug as referred to herein is the polynucleic acid molecule (B). In some instances, the DAR ratio of the polynucleic acid molecule (B) to binding moiety A is about 1, 2, 3, 4, 5, 6, 7, 8, 9, 10, 11, 12, 13, 14, 15, 16, or greater. In some instances, the DAR ratio of the polynucleic acid molecule (B) to binding moiety A is about 1 or greater. In some instances, the DAR ratio of the polynucleic acid molecule (B) to binding moiety A is about 2 or greater. In some instances, the DAR ratio of the polynucleic acid molecule (B) to binding moiety A is about 3 or greater. In some instances, the DAR ratio of the polynucleic acid molecule (B) to binding moiety A is about 4 or greater. In some instances, the DAR ratio of the polynucleic acid molecule (B) to binding moiety A is about 5 or greater. In some instances, the DAR ratio of the polynucleic acid molecule (B) to binding moiety A is about 6 or greater. In some instances, the DAR ratio of the polynucleic acid molecule (B) to binding moiety A is about 7 or greater. In some instances, the DAR ratio of the polynucleic acid molecule (B) to binding moiety A is about 8 or greater. In some instances, the DAR ratio of the polynucleic acid molecule (B) to binding moiety A is about 9 or greater. In some instances, the DAR ratio of the polynucleic acid molecule (B) to binding moiety A is about 10 or greater. In some instances, the DAR ratio of the polynucleic acid molecule (B) to binding moiety A is about 11 or greater. In some instances, the DAR ratio of the polynucleic acid molecule (B) to binding moiety A is about 12 or greater.

In some instances, the DAR ratio of the polynucleic acid molecule (B) to binding moiety A is about 1, 2, 3, 4, 5, 6, 7, 8, 9, 10, 11, 12, 13, 14, 15, or 16. In some instances, the DAR ratio of the polynucleic acid molecule (B) to binding moiety A is about 1. In some instances, the DAR ratio of the polynucleic acid molecule (B) to binding moiety A is about 2. In some instances, the DAR ratio of the polynucleic acid molecule (B) to binding moiety A is about 3. In some instances, the DAR ratio of the polynucleic acid molecule (B) to binding moiety A is about 4. In some instances, the DAR ratio of the polynucleic acid molecule (B) to binding moiety A is about 5. In some instances, the DAR ratio of the polynucleic acid molecule (B) to binding moiety A is about 6. In some instances, the DAR ratio of the polynucleic acid molecule (B) to binding moiety A is about 7. In some instances, the DAR ratio of the polynucleic acid molecule (B) to binding moiety A is about 8. In some instances, the DAR ratio of the polynucleic acid molecule (B) to binding moiety A is about 9. In some instances, the DAR ratio of the polynucleic acid molecule (B) to binding moiety A is about 10. In some instances, the DAR ratio of the polynucleic acid molecule (B) to binding moiety A is about 11. In some instances, the DAR ratio of the polynucleic acid molecule (B) to binding moiety A is about 12. In some instances, the DAR ratio of the polynucleic acid molecule (B) to binding moiety A is about 13. In some instances, the DAR ratio of the polynucleic acid molecule (B) to binding moiety A is about 14. In some instances, the DAR ratio of the polynucleic acid molecule (B) to binding moiety A is about 15. In some instances, the DAR ratio of the polynucleic acid molecule (B) to binding moiety A is about 16.

In some instances, the DAR ratio of the polynucleic acid molecule (B) to binding moiety A is 1, 2, 3, 4, 5, 6, 7, 8, 9, 10, 11, 12, 13, 14, 15, or 16. In some instances, the DAR ratio of the polynucleic acid molecule (B) to binding moiety A is 1. In some instances, the DAR ratio of the polynucleic acid molecule (B) to binding moiety A is 2. In some instances, the DAR ratio of the polynucleic acid molecule (B) to binding moiety A is 4. In some instances, the DAR ratio of the polynucleic acid molecule (B) to binding moiety A is 6. In some instances, the DAR ratio of the polynucleic acid molecule (B) to binding moiety A is 8. In some instances, the DAR ratio of the polynucleic acid molecule (B) to binding moiety A is 12.

In some instances, a conjugate comprising polynucleic acid molecule (B) and binding moiety A has improved activity as compared to a conjugate comprising polynucleic acid molecule (B) without a binding moiety A. In some instances, improved activity results in enhanced biologically relevant functions, e.g., improved stability, affinity, binding, functional activity, and efficacy in treatment or prevention of a disease state. In some instancs, the disease state is a result of one or more mutated exons of a gene. In some instances, the conjugate comprising polynucleic acid molecule (B) and binding moiety A results in increased exon skipping of the one or more mutated exons as compared to the conjugate comprising polynucleic acid molecule (B) without a binding moiety A. In some instances, exon skipping is increased by at least or about 5%, 10%, 20%, 25%, 30%, 40%, 50%, 60%, 70%, 80%, 90%, 95%, or more than 95% in the conjugate comprising polynucleic acid molecule (B) and binding moiety A as compared to the conjugate comprising polynucleic acid molecule (B) without a binding moiety A.

In some embodiments, an antibody or its binding fragment is further modified using conventional techniques known in the art, for example, by using amino acid deletion, insertion, substitution, addition, and/or by recombination and/or any other modification (e.g. posttranslational and chemical modifications, such as glycosylation and phosphorylation) known in the art either alone or in combination. In some instances, the modification further comprises a modification for modulating interaction with Fc receptors. In some instances, the one or more modifications include those described in, for example, International Publication No. WO97/34631, which discloses amino acid residues involved in the interaction between the Fc domain and the FcRn receptor. Methods for introducing such modifications in the nucleic acid sequence underlying the amino acid sequence of an antibody or its binding fragment is well known to the person skilled in the art.

In some instances, an antibody binding fragment further encompasses its derivatives and includes polypeptide sequences containing at least one CDR.

In some instances, the term "single-chain" as used herein means that the first and second domains of a bi-specific single chain construct are covalently linked, preferably in the form of a co-linear amino acid sequence encodable by a single nucleic acid molecule.

In some instances, a bispecific single chain antibody construct relates to a construct comprising two antibody derived binding domains. In such embodiments, bi-specific single chain antibody construct is tandem bi-scFv or diabody. In some instances, a scFv contains a VH and VL domain connected by a linker peptide. In some instances, linkers are of a length and sequence sufficient to ensure that each of the first and second domains can, independently from one another, retain their differential binding specificities.

In some embodiments, binding to or interacting with as used herein defines a binding/interaction of at least two antigen-interaction-sites with each other. In some instances, antigen-interaction-site defines a motif of a polypeptide that shows the capacity of specific interaction with a specific antigen or a specific group of antigens. In some cases, the binding/interaction is also understood to define a specific recognition. In such cases, specific recognition refers to that the antibody or its binding fragment is capable of specifically interacting with and/or binding to at least two amino acids of each of a target molecule. For example, specific recognition relates to the specificity of the antibody molecule, or to its ability to discriminate between the specific regions of a target molecule. In additional instances, the specific interaction of the antigen-interaction-site with its specific antigen results in an initiation of a signal, e.g. due to the induction of a change of the conformation of the antigen, an oligomerization of the antigen, etc. In further embodiments, the binding is exemplified by the specificity of a "key-lock-principle". Thus in some instances, specific motifs in the amino acid sequence of the antigen-interaction-site and the antigen bind to each other as a result of their primary, secondary or tertiary structure as well as the result of secondary modifications of said structure. In such cases, the specific interaction of the antigen-interaction-site with its specific antigen results as well in a simple binding of the site to the antigen.

In some instances, specific interaction further refers to a reduced cross-reactivity of the antibody or its binding fragment or a reduced off-target effect. For example, the antibody or its binding fragment that bind to the polypeptide/protein of interest but do not or do not essentially bind to any of the other polypeptides are considered as specific for the polypeptide/protein of interest. Examples for the specific interaction of an antigen-interaction-site with a specific antigen comprise the specificity of a ligand for its receptor, for example, the interaction of an antigenic determinant (epitope) with the antigenic binding site of an antibody.

### Additional Binding Moieties

In some embodiments, the binding moiety is a plasma protein. In some instances, the plasma protein comprises albumin. In some instances, the binding moiety A is albumin. In some instances, albumin is conjugated by one or more of a conjugation chemistry described herein to a polynucleic acid molecule. In some instances, albumin is conjugated by native ligation chemistry to a polynucleic acid molecule. In some instances, albumin is conjugated by lysine conjugation to a polynucleic acid molecule.

In some instances, the binding moiety is a steroid. Exemplary steroids include cholesterol, phospholipids, di-and triacylglycerols, fatty acids, hydrocarbons that are saturated, unsaturated, comprise substitutions, or combinations thereof. In some instances, the steroid is cholesterol. In some instances, the binding moiety is cholesterol. In some instances, cholesterol is conjugated by one or more of a conjugation chemistry described herein to a polynucleic acid molecule. In some instances, cholesterol is conjugated by native ligation chemistry to a polynucleic acid molecule. In some instances, cholesterol is conjugated by lysine conjugation to a polynucleic acid molecule.

In some instances, the binding moiety is a polymer, including but not limited to polynucleic acid molecule aptamers that bind to specific surface markers on cells. In this instance the binding moiety is a polynucleic acid that does not hybridize to a target gene or mRNA, but instead is capable of selectively binding to a cell surface marker similarly to an antibody binding to its specific epitope of a cell surface marker.

In some cases, the binding moiety is a peptide. In some cases, the peptide comprises between about 1 and about 3 kDa. In some cases, the peptide comprises between about 1.2 and about 2.8 kDa, about 1.5 and about 2.5 kDa, or about 1.5 and about 2 kDa. In some instances, the peptide is a bicyclic peptide. In some cases, the bicyclic peptide is a constrained bicyclic peptide. In some instances, the binding moiety is a bicyclic peptide (e.g., bicycles from Bicycle Therapeutics).

In additional cases, the binding moiety is a small molecule. In some instances, the small molecule is an antibody-recruiting small molecule. In some cases, the antibody-recruiting small molecule comprises a target-binding terminus and an antibody-binding terminus, in which the target-binding terminus is capable of recognizing and interacting with a cell surface receptor. For example, in some instances, the target-binding terminus comprising a glutamate urea compound enables interaction with PSMA, thereby, enhances an antibody interaction with a cell that expresses PSMA. In some instances, a binding moiety is a small molecule described in Zhang et al., "A remote arene-binding site on prostate specific membrane antigen revealed by antibody-recruiting small molecules," J Am Chem Soc. 132(36): 12711-12716 (2010); or McEnaney, et al., "Antibody-recruiting molecules: an emerging paradigm for engaging immune function in treating human disease," ACS Chem Biol. 7(7): 1139-1151 (2012).

### Conjugation Chemistry

In some embodiments, a polynucleic acid molecule B is conjugated to a binding moiety. In some instances, the binding moiety comprises amino acids, peptides, polypeptides, proteins, antibodies, antigens, toxins, hormones, lipids, nucleotides, nucleosides, sugars, carbohydrates, polymers such as polyethylene glycol and polypropylene glycol, as well as analogs or derivatives of all of these classes of substances. Additional examples of binding moiety also include steroids, such as cholesterol, phospholipids, di-and triacylglycerols, fatty acids, hydrocarbons (e.g., saturated, unsaturated, or contains substitutions), enzyme substrates, biotin, digoxigenin, and polysaccharides. In some instances, the binding moiety is an antibody or binding fragment thereof. In some instances, the polynucleic acid molecule is further conjugated to a polymer, and optionally an endosomolytic moiety.

In some embodiments, the polynucleic acid molecule is conjugated to the binding moiety by a chemical ligation process. In some instances, the polynucleic acid molecule is conjugated to the binding moiety by a native ligation. In some instances, the conjugation is as described in: Dawson, et al. "Synthesis of proteins by native chemical ligation," Science 1994, 266, 776-779; Dawson, et al. "Modulation of Reactivity in Native Chemical Ligation through the Use of Thiol Additives," J. Am. Chem. Soc. 1997, 119, 4325-4329; Hackeng, et al. "Protein synthesis by native chemical ligation: Expanded scope by using straightforward methodology.," Proc. Natl. Acad. Sci. USA 1999, 96, 10068-10073; or Wu, et al. "Building complex glycopeptides: Development of a cysteine-free native chemical ligation protocol," Angew. Chem. Int. Ed. 2006, 45, 4116-4125. In some instances, the conjugation is as described in U.S. Patent No. 8,936,910. In some embodiments, the polynucleic acid molecule is conjugated to the binding moiety either site-specifically or non-specifically via native ligation chemistry.

In some instances, the polynucleic acid molecule is conjugated to the binding moiety by a site-directed method utilizing a "traceless" coupling technology (Philochem). In some instances, the "traceless" coupling technology utilizes an N-terminal 1,2-aminothiol group on the binding moiety which is then conjugate with a polynucleic acid molecule containing an aldehyde group. *(see* Casi et al., "Site-specific traceless coupling of potent cytotoxic drugs to recombinant antibodies for pharmacodelivery," JACS 134(13): 5887-5892 (2012))

In some instances, the polynucleic acid molecule is conjugated to the binding moiety by a site-directed method utilizing an unnatural amino acid incorporated into the binding moiety. In some instances, the unnatural amino acid comprises p-acetylphenylalanine (pAcPhe). In some instances, the keto group of pAcPhe is selectively coupled to an alkoxy-amine derivatived conjugating moiety to form an oxime bond. *(see* Axup et al., "Synthesis of site-specific antibody-drug conjugates using unnatural amino acids," PNAS 109(40): 16101-16106 (2012)).

In some instances, the polynucleic acid molecule is conjugated to the binding moiety by a site-directed method utilizing an enzyme-catalyzed process. In some instances, the site-directed method utilizes SMARTag^{™} technology (Redwood). In some instances, the SMARTag^{™} technology comprises generation of a formylglycine (FGly) residue from cysteine by formylglycine-generating enzyme (FGE) through an oxidation process under the presence of an aldehyde tag and the subsequent conjugation of FGly to an alkylhydraine-functionalized polynucleic acid molecule via hydrazino-Pictet-Spengler (HIPS) ligation. *(see* Wu et al., "Site-specific chemical modification of recombinant proteins produced in mammalian cells by using the genetically encoded aldehyde tag," PNAS 106(9): 3000-3005 (2009); Agarwal, et al., "A Pictet-Spengler ligation for protein chemical modification," PNAS 110(1): 46-51 (2013))

In some instances, the enzyme-catalyzed process comprises microbial transglutaminase (mTG). In some cases, the polynucleic acid molecule is conjugated to the binding moiety utilizing a microbial transglutaminze catalyzed process. In some instances, mTG catalyzes the formation of a covalent bond between the amide side chain of a glutamine within the recognition sequence and a primary amine of a functionalized polynucleic acid molecule. In some instances, mTG is produced from *Streptomyces mobarensis. (see* Strop et al., "Location matters: site of conjugation modulates stability and pharmacokinetics of antibody drug conjugates," Chemistry and Biology 20(2) 161-167 (2013))

In some instances, the polynucleic acid molecule is conjugated to the binding moiety by a method as described in PCT Publication No. WO2014/140317, which utilizes a sequence-specific transpeptidase.

In some instances, the polynucleic acid molecule is conjugated to the binding moiety by a method as described in U.S. Patent Publication Nos. 2015/0105539 and 2015/0105540.

### Production of Antibodies or Binding Fragments Thereof

In some embodiments, polypeptides described herein (e.g., antibodies and its binding fragments) are produced using any method known in the art to be useful for the synthesis of polypeptides (e.g., antibodies), in particular, by chemical synthesis or by recombinant expression, and are preferably produced by recombinant expression techniques.

In some instances, an antibody or its binding fragment thereof is expressed recombinantly, and the nucleic acid encoding the antibody or its binding fragment is assembled from chemically synthesized oligonucleotides (e.g., as described in Kutmeier et al., 1994, BioTechniques 17:242), which involves the synthesis of overlapping oligonucleotides containing portions of the sequence encoding the antibody, annealing and ligation of those oligonucleotides, and then amplification of the ligated oligonucleotides by PCR.

Alternatively, a nucleic acid molecule encoding an antibody is optionally generated from a suitable source (e.g., an antibody cDNA library, or cDNA library generated from any tissue or cells expressing the immunoglobulin) by PCR amplification using synthetic primers hybridizable to the 3' and 5' ends of the sequence or by cloning using an oligonucleotide probe specific for the particular gene sequence.

In some instances, an antibody or its binding is optionally generated by immunizing an animal, such as a rabbit, to generate polyclonal antibodies or, more preferably, by generating monoclonal antibodies, e.g., as described by Kohler and Milstein (1975, Nature 256:495-497) or, as described by Kozbor et al. (1983, Immunology Today 4:72) or Cole et al. (1985 in Monoclonal Antibodies and Cancer Therapy, Alan R. Liss, Inc., pp. 77-96). Alternatively, a clone encoding at least the Fab portion of the antibody is optionally obtained by screening Fab expression libraries (e.g., as described in Huse et al., 1989, Science 246:1275-1281) for clones of Fab fragments that bind the specific antigen or by screening antibody libraries (See, e.g., Clackson et al., 1991, Nature 352:624; Hane et al., 1997 Proc. Natl. Acad. Sci. USA 94:4937).

In some embodiments, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity are used. A chimeric antibody is a molecule in which different portions are derived from different animal species, such as those having a variable region derived from a murine monoclonal antibody and a human immunoglobulin constant region, e.g., humanized antibodies.

In some embodiments, techniques described for the production of single chain antibodies (U.S. Pat. No. 4,694,778; Bird, 1988, Science 242:423-42; Huston et al., 1988, Proc. Natl. Acad. Sci. USA 85:5879-5883; and Ward et al., 1989, Nature 334:544-54) are adapted to produce single chain antibodies. Single chain antibodies are formed by linking the heavy and light chain fragments of the Fv region via an amino acid bridge, resulting in a single chain polypeptide. Techniques for the assembly of functional Fv fragments in *E. coli* are also optionally used (Skerra et al., 1988, Science 242:1038-1041).

In some embodiments, an expression vector comprising the nucleotide sequence of an antibody or the nucleotide sequence of an antibody is transferred to a host cell by conventional techniques (e.g., electroporation, liposomal transfection, and calcium phosphate precipitation), and the transfected cells are then cultured by conventional techniques to produce the antibody. In specific embodiments, the expression of the antibody is regulated by a constitutive, an inducible or a tissue, specific promoter.

In some embodiments, a variety of host-expression vector systems is utilized to express an antibody or its binding fragment described herein. Such host-expression systems represent vehicles by which the coding sequences of the antibody is produced and subsequently purified, but also represent cells that are, when transformed or transfected with the appropriate nucleotide coding sequences, express an antibody or its binding fragment in situ. These include, but are not limited to, microorganisms such as bacteria (e.g., *E. coli* and *B. subtilis)* transformed with recombinant bacteriophage DNA, plasmid DNA or cosmid DNA expression vectors containing an antibody or its binding fragment coding sequences; yeast (e.g., *Saccharomyces Pichia)* transformed with recombinant yeast expression vectors containing an antibody or its binding fragment coding sequences; insect cell systems infected with recombinant virus expression vectors (e.g., baculovirus) containing an antibody or its binding fragment coding sequences; plant cell systems infected with recombinant virus expression vectors (e.g., cauliflower mosaic virus (CaMV) and tobacco mosaic virus (TMV)) or transformed with recombinant plasmid expression vectors (e.g., Ti plasmid) containing an antibody or its binding fragment coding sequences; or mammalian cell systems (e.g., COS, CHO, BH, 293, 293T, 3T3 cells) harboring recombinant expression constructs containing promoters derived from the genome of mammalian cells (e.g., metallothionein promoter) or from mammalian viruses (e.g. the adenovirus late promoter; the vaccinia virus 7.5K promoter).

For long-term, high-yield production of recombinant proteins, stable expression is preferred. In some instances, cell lines that stably express an antibody are optionally engineered. Rather than using expression vectors that contain viral origins of replication, host cells are transformed with DNA controlled by appropriate expression control elements (e.g., promoter, enhancer, sequences, transcription terminators, polyadenylation sites, etc.), and a selectable marker. Following the introduction of the foreign DNA, engineered cells are then allowed to grow for 1-2 days in an enriched media, and then are switched to a selective media. The selectable marker in the recombinant plasmid confers resistance to the selection and allows cells to stably integrate the plasmid into their chromosomes and grow to form foci that in turn are cloned and expanded into cell lines. This method can advantageously be used to engineer cell lines which express the antibody or its binding fragments.

In some instances, a number of selection systems are used, including but not limited to the herpes simplex virus thymidine kinase (Wigler et al., 1977, Cell 11:223), hypoxanthine-guanine phosphoribosyltransferase (Szybalska & Szybalski, 192, Proc. Natl. Acad. Sci. USA 48:202), and adenine phosphoribosyltransferase (Lowy et al., 1980, Cell 22:817) genes are employed in tk-, hgprt- or aprt-cells, respectively. Also, antimetabolite resistance are used as the basis of selection for the following genes: dhfr, which confers resistance to methotrexate (Wigler et al., 1980, Proc. Natl. Acad. Sci. USA 77:357; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 *(*Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIB TECH 11(5):155-215) and hygro, which confers resistance to hygromycin (Santerre et al., 1984, Gene 30:147). Methods commonly known in the art of recombinant DNA technology which can be used are described in Ausubel et al. (eds., 1993, Current Protocols in Molecular Biology, John Wiley & Sons, NY; Kriegler, 1990, Gene Transfer and Expression, A Laboratory Manual, Stockton Press, NY; and in Chapters 12 and 13, Dracopoli et al. (eds), 1994, Current Protocols in Human Genetics, John Wiley & Sons, NY.; Colberre-Garapin et al., 1981, J. Mol. Biol. 150:1).

In some instances, the expression levels of an antibody are increased by vector amplification (for a review, see Bebbington and Hentschel, The use of vectors based on gene amplification for the expression of cloned genes in mammalian cells in DNA cloning, Vol. 3. (Academic Press, New York, 1987)). When a marker in the vector system expressing an antibody is amplifiable, an increase in the level of inhibitor present in culture of host cell will increase the number of copies of the marker gene. Since the amplified region is associated with the nucleotide sequence of the antibody, production of the antibody will also increase (Crouse et al., 1983, Mol. Cell Biol. 3:257).

In some instances, any method known in the art for purification or analysis of an antibody or antibody conjugates is used, for example, by chromatography (e.g., ion exchange, affinity, particularly by affinity for the specific antigen after Protein A, and sizing column chromatography), centrifugation, differential solubility, or by any other standard technique for the purification of proteins. Exemplary chromatography methods included, but are not limited to, strong anion exchange chromatography, hydrophobic interaction chromatography, size exclusion chromatography, and fast protein liquid chromatography.

### Polymer Conjugating Moiety

In some embodiments, a polymer moiety C is further conjugated to a polynucleic acid molecule described herein, a binding moiety described herein, or in combinations thereof. In some instances, a polymer moiety C is conjugated a polynucleic acid molecule. In some cases, a polymer moiety C is conjugated to a binding moiety. In other cases, a polymer moiety C is conjugated to a polynucleic acid molecule-binding moiety molecule. In additional cases, a polymer moiety C is conjugated, as illustrated *supra.*

In some instances, the polymer moiety C is a natural or synthetic polymer, consisting of long chains of branched or unbranched monomers, and/or cross-linked network of monomers in two or three dimensions. In some instances, the polymer moiety C includes a polysaccharide, lignin, rubber, or polyalkylen oxide (e.g., polyethylene glycol). In some instances, the at least one polymer moiety C includes, but is not limited to, alpha-, omega-dihydroxylpolyethyleneglycol, biodegradable lactone-based polymer, e.g. polyacrylic acid, polylactide acid (PLA), poly(glycolic acid) (PGA), polypropylene, polystyrene, polyolefin, polyamide, polycyanoacrylate, polyimide, polyethylenterephthalat (PET, PETG), polyethylene terephthalate (PETE), polytetramethylene glycol (PTG), or polyurethane as well as mixtures thereof. As used herein, a mixture refers to the use of different polymers within the same compound as well as in reference to block copolymers. In some cases, block copolymers are polymers wherein at least one section of a polymer is build up from monomers of another polymer. In some instances, the polymer moiety C comprises polyalkylene oxide. In some instances, the polymer moiety C comprises PEG. In some instances, the polymer moiety C comprises polyethylene imide (PEI) or hydroxy ethyl starch (HES).

In some instances, C is a PEG moiety. In some instances, the PEG moiety is conjugated at the 5' terminus of the polynucleic acid molecule while the binding moiety is conjugated at the 3' terminus of the polynucleic acid molecule. In some instances, the PEG moiety is conjugated at the 3' terminus of the polynucleic acid molecule while the binding moiety is conjugated at the 5' terminus of the polynucleic acid molecule. In some instances, the PEG moiety is conjugated to an internal site of the polynucleic acid molecule. In some instances, the PEG moiety, the binding moiety, or a combination thereof, are conjugated to an internal site of the polynucleic acid molecule. In some instances, the conjugation is a direct conjugation. In some instances, the conjugation is via native ligation.

In some embodiments, the polyalkylene oxide (e.g., PEG) is a polydispers or monodispers compound. In some instances, polydispers material comprises disperse distribution of different molecular weight of the material, characterized by mean weight (weight average) size and dispersity. In some instances, the monodisperse PEG comprises one size of molecules. In some embodiments, C is poly- or monodispersed polyalkylene oxide (e.g., PEG) and the indicated molecular weight represents an average of the molecular weight of the polyalkylene oxide, e.g., PEG, molecules.

In some embodiments, the molecular weight of the polyalkylene oxide (e.g., PEG) is about 200, 300, 400, 500, 600, 700, 800, 900, 1000, 1100, 1200, 1300, 1400, 1450, 1500, 1600, 1700, 1800, 1900, 2000, 2100, 2200, 2300, 2400, 2500, 2600, 2700, 2800, 2900, 3000, 3250, 3350, 3500, 3750, 4000, 4250, 4500, 4600, 4750, 5000, 5500, 6000, 6500, 7000, 7500, 8000, 10,000, 12,000, 20,000, 35,000, 40,000, 50,000, 60,000, or 100,000 Da.

In some embodiments, C is polyalkylene oxide (e.g., PEG) and has a molecular weight of about 200, 300, 400, 500, 600, 700, 800, 900, 1000, 1100, 1200, 1300, 1400, 1450, 1500, 1600, 1700, 1800, 1900, 2000, 2100, 2200, 2300, 2400, 2500, 2600, 2700, 2800, 2900, 3000, 3250, 3350, 3500, 3750, 4000, 4250, 4500, 4600, 4750, 5000, 5500, 6000, 6500, 7000, 7500, 8000, 10,000, 12,000, 20,000, 35,000, 40,000, 50,000, 60,000, or 100,000 Da. In some embodiments, C is PEG and has a molecular weight of about 200, 300, 400, 500, 600, 700, 800, 900, 1000, 1100, 1200, 1300, 1400, 1450, 1500, 1600, 1700, 1800, 1900, 2000, 2100, 2200, 2300, 2400, 2500, 2600, 2700, 2800, 2900, 3000, 3250, 3350, 3500, 3750, 4000, 4250, 4500, 4600, 4750, 5000, 5500, 6000, 6500, 7000, 7500, 8000, 10,000, 12,000, 20,000, 35,000, 40,000, 50,000, 60,000, or 100,000 Da. In some instances, the molecular weight of C is about 200 Da. In some instances, the molecular weight of C is about 300 Da. In some instances, the molecular weight of C is about 400 Da. In some instances, the molecular weight of C is about 500 Da. In some instances, the molecular weight of C is about 600 Da. In some instances, the molecular weight of C is about 700 Da. In some instances, the molecular weight of C is about 800 Da. In some instances, the molecular weight of C is about 900 Da. In some instances, the molecular weight of C is about 1000 Da. In some instances, the molecular weight of C is about 1100 Da. In some instances, the molecular weight of C is about 1200 Da. In some instances, the molecular weight of C is about 1300 Da. In some instances, the molecular weight of C is about 1400 Da. In some instances, the molecular weight of C is about 1450 Da. In some instances, the molecular weight of C is about 1500 Da. In some instances, the molecular weight of C is about 1600 Da. In some instances, the molecular weight of C is about 1700 Da. In some instances, the molecular weight of C is about 1800 Da. In some instances, the molecular weight of C is about 1900 Da. In some instances, the molecular weight of C is about 2000 Da. In some instances, the molecular weight of C is about 2100 Da. In some instances, the molecular weight of C is about 2200 Da. In some instances, the molecular weight of C is about 2300 Da. In some instances, the molecular weight of C is about 2400 Da. In some instances, the molecular weight of C is about 2500 Da. In some instances, the molecular weight of C is about 2600 Da. In some instances, the molecular weight of C is about 2700 Da. In some instances, the molecular weight of C is about 2800 Da. In some instances, the molecular weight of C is about 2900 Da. In some instances, the molecular weight of C is about 3000 Da. In some instances, the molecular weight of C is about 3250 Da. In some instances, the molecular weight of C is about 3350 Da. In some instances, the molecular weight of C is about 3500 Da. In some instances, the molecular weight of C is about 3750 Da. In some instances, the molecular weight of C is about 4000 Da. In some instances, the molecular weight of C is about 4250 Da. In some instances, the molecular weight of C is about 4500 Da. In some instances, the molecular weight of C is about 4600 Da. In some instances, the molecular weight of C is about 4750 Da. In some instances, the molecular weight of C is about 5000 Da. In some instances, the molecular weight of C is about 5500 Da. In some instances, the molecular weight of C is about 6000 Da. In some instances, the molecular weight of C is about 6500 Da. In some instances, the molecular weight of C is about 7000 Da. In some instances, the molecular weight of C is about 7500 Da. In some instances, the molecular weight of C is about 8000 Da. In some instances, the molecular weight of C is about 10,000 Da. In some instances, the molecular weight of C is about 12,000 Da. In some instances, the molecular weight of C is about 20,000 Da. In some instances, the molecular weight of C is about 35,000 Da. In some instances, the molecular weight of C is about 40,000 Da. In some instances, the molecular weight of C is about 50,000 Da. In some instances, the molecular weight of C is about 60,000 Da. In some instances, the molecular weight of C is about 100,000 Da.

In some embodiments, the polyalkylene oxide (e.g., PEG) is a discrete PEG, in which the discrete PEG is a polymeric PEG comprising more than one repeating ethylene oxide units. In some instances, a discrete PEG (dPEG) comprises from 2 to 60, from 2 to 50, or from 2 to 48 repeating ethylene oxide units. In some instances, a dPEG comprises about 2, 3, 4, 5, 6, 7, 8, 9, 10, 11, 12, 13, 14, 15, 16, 17, 18, 19, 20, 22, 24, 26, 28, 30, 35, 40, 42, 48, 50 or more repeating ethylene oxide units. In some instances, a dPEG comprises about 2 or more repeating ethylene oxide units. In some instances, a dPEG comprises about 3 or more repeating ethylene oxide units. In some instances, a dPEG comprises about 4 or more repeating ethylene oxide units. In some instances, a dPEG comprises about 5 or more repeating ethylene oxide units. In some instances, a dPEG comprises about 6 or more repeating ethylene oxide units. In some instances, a dPEG comprises about 7 or more repeating ethylene oxide units. In some instances, a dPEG comprises about 8 or more repeating ethylene oxide units. In some instances, a dPEG comprises about 9 or more repeating ethylene oxide units. In some instances, a dPEG comprises about 10 or more repeating ethylene oxide units. In some instances, a dPEG comprises about 11 or more repeating ethylene oxide units. In some instances, a dPEG comprises about 12 or more repeating ethylene oxide units. In some instances, a dPEG comprises about 13 or more repeating ethylene oxide units. In some instances, a dPEG comprises about 14 or more repeating ethylene oxide units. In some instances, a dPEG comprises about 15 or more repeating ethylene oxide units. In some instances, a dPEG comprises about 16 or more repeating ethylene oxide units. In some instances, a dPEG comprises about 17 or more repeating ethylene oxide units. In some instances, a dPEG comprises about 18 or more repeating ethylene oxide units. In some instances, a dPEG comprises about 19 or more repeating ethylene oxide units. In some instances, a dPEG comprises about 20 or more repeating ethylene oxide units. In some instances, a dPEG comprises about 22 or more repeating ethylene oxide units. In some instances, a dPEG comprises about 24 or more repeating ethylene oxide units. In some instances, a dPEG comprises about 26 or more repeating ethylene oxide units. In some instances, a dPEG comprises about 28 or more repeating ethylene oxide units. In some instances, a dPEG comprises about 30 or more repeating ethylene oxide units. In some instances, a dPEG comprises about 35 or more repeating ethylene oxide units. In some instances, a dPEG comprises about 40 or more repeating ethylene oxide units. In some instances, a dPEG comprises about 42 or more repeating ethylene oxide units. In some instances, a dPEG comprises about 48 or more repeating ethylene oxide units. In some instances, a dPEG comprises about 50 or more repeating ethylene oxide units. In some cases, a dPEG is synthesized as a single molecular weight compound from pure (e.g., about 95%, 98%, 99%, or 99.5%) staring material in a step-wise fashion. In some cases, a dPEG has a specific molecular weight, rather than an average molecular weight. In some cases, a dPEG described herein is a dPEG from Quanta Biodesign, LMD.

In some embodiments, the polymer moiety C comprises a cationic mucic acid-based polymer (cMAP). In some instances, cMAP comprises one or more subunit of at least one repeating subunit, and the subunit structure is represented as Formula (V):

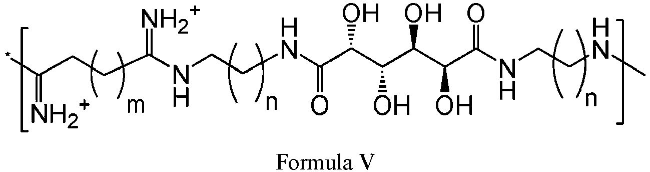

wherein m is independently at each occurrence 1, 2, 3, 4, 5, 6, 7, 8, 9, or 10, preferably 4-6 or 5; and n is independently at each occurrence 1, 2, 3, 4, or 5. In some embodiments, m and n are, for example, about 10.

In some instances, cMAP is further conjugated to a PEG moiety, generating a cMAP-PEG copolymer, an mPEG-cMAP-PEGm triblock polymer, or a cMAP-PEG-cMAP triblock polymer. In some instances, the PEG moiety is in a range of from about 500 Da to about 50,000 Da. In some instances, the PEG moiety is in a range of from about 500 Da to about 1000 Da, greater than 1000 Da to about 5000 Da, greater than 5000 Da to about 10,000 Da, greater than 10,000 to about 25,000 Da, greater than 25,000 Da to about 50,000 Da, or any combination of two or more of these ranges.

In some instances, the polymer moiety C is cMAP-PEG copolymer, an mPEG-cMAP-PEGm triblock polymer, or a cMAP-PEG-cMAP triblock polymer. In some cases, the polymer moiety C is cMAP-PEG copolymer. In other cases, the polymer moiety C is an mPEG-cMAP-PEGm triblock polymer. In additional cases, the polymer moiety C is a cMAP-PEG-cMAP triblock polymer.

In some embodiments, the polymer moiety C is conjugated to the polynucleic acid molecule, the binding moiety, and optionally to the endosomolytic moiety as illustrated *supra.*

### Endosomolytic Moiety

In some embodiments, a molecule of Formula (I): A-X-B-Y-C, further comprises an additional conjugating moiety. In some instances, the additional conjugating moiety is an endosomolytic moiety. In some cases, the endosomolytic moiety is a cellular compartmental release component, such as a compound capable of releasing from any of the cellular compartments known in the art, such as the endosome, lysosome, endoplasmic reticulum (ER), golgi apparatus, microtubule, peroxisome, or other vesicular bodies with the cell. In some cases, the endosomolytic moiety comprises an endosomolytic polypeptide, an endosomolytic polymer, an endosomolytic lipid, or an endosomolytic small molecule. In some cases, the endosomolytic moiety comprises an endosomolytic polypeptide. In other cases, the endosomolytic moiety comprises an endosomolytic polymer.

### Endosomolytic Polypeptides

In some embodiments, a molecule of Formula (I): A-X-B-Y-C, is further conjugated with an endosomolytic polypeptide. In some embodiments, a molecule of Formula (V): A-(X^{l}-B)ₙ or Formula (II): A-X^{I}-(B-X²-C)ₙ is further conjugated with an endosomolytic polypeptide. In some cases, the endosomolytic polypeptide is a pH-dependent membrane active peptide. In some cases, the endosomolytic polypeptide is an amphipathic polypeptide. In additional cases, the endosomolytic polypeptide is a peptidomimetic. In some instances, the endosomolytic polypeptide comprises INF, melittin, meucin, or their respective derivatives thereof. In some instances, the endosomolytic polypeptide comprises INF or its derivatives thereof. In other cases, the endosomolytic polypeptide comprises melittin or its derivatives thereof. In additional cases, the endosomolytic polypeptide comprises meucin or its derivatives thereof.

In some instances, INF7 is a 24 residue polypeptide those sequence comprises CGIFGEIEELIEEGLENLIDWGNA (SEQ ID NO: 1), or GLFEAIEGFIENGWEGMIDGWYGC (SEQ ID NO: 2). In some instances, INF7 or its derivatives comprise a sequence of:
GLFEAIEGFIENGWEGMIWDYGSGSCG (SEQ ID NO: 3), GLFEAIEGFIENGWEGMIDG WYG-(PEG)6-NH2 (SEQ ID NO: 4), or GLFEAIEGFIENGWEGMIWDYG-SGSC-K(GalNAc)2 (SEQ ID NO: 5).

In some cases, melittin is a 26 residue polypeptide those sequence comprises CLIGAILKVLATGLPTLISWIKNKRKQ (SEQ ID NO: 6), or GIGAVLKVLTTGLPALISWIKRKRQQ (SEQ ID NO: 7). In some instances, melittin comprises a polypeptide sequence as described in U.S. Patent No. 8,501,930.

In some instances, meucin is an antimicrobial peptide (AMP) derived from the venom gland of the scorpion Mesobuthus eupeus. In some instances, meucin comprises of meucin-13 those sequence comprises IFGAIAGLLKNIF-NH₂ (SEQ ID NO: 8) and meucin-18 those sequence comprises FFGHLFKLATKIIPSLFQ (SEQ ID NO: 9).

In some instances, the endosomolytic polypeptide comprises a polypeptide in which its sequence is at least 50%, 60%, 70%, 80%, 90%, 95%, or 99% sequence identity to INF7 or its derivatives thereof, melittin or its derivatives thereof, or meucin or its derivatives thereof. In some instances, the endosomolytic moiety comprises INF7 or its derivatives thereof, melittin or its derivatives thereof, or meucin or its derivatives thereof.

In some instances, the endosomolytic moiety is INF7 or its derivatives thereof. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NOs: 1-5. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 1. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 2-5. In some cases, the endosomolytic moiety comprises SEQ ID NO: 1. In some cases, the endosomolytic moiety comprises SEQ ID NO: 2-5. In some cases, the endosomolytic moiety consists of SEQ ID NO: 1. In some cases, the endosomolytic moiety consists of SEQ ID NO: 2-5.

In some instances, the endosomolytic moiety is melittin or its derivatives thereof. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NOs: 6 or 7. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 6. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 7. In some cases, the endosomolytic moiety comprises SEQ ID NO: 6. In some cases, the endosomolytic moiety comprises SEQ ID NO: 7. In some cases, the endosomolytic moiety consists of SEQ ID NO: 6. In some cases, the endosomolytic moiety consists of SEQ ID NO: 7.

In some instances, the endosomolytic moiety is meucin or its derivatives thereof. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NOs: 8 or 9. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 8. In some cases, the endosomolytic moiety comprises a polypeptide having at least 50%, 55%, 60%, 65%, 70%, 75%, 80%, 85%, 90%, 95%, 96%, 97%, 98%, 99%, or 100% sequence identity to SEQ ID NO: 9. In some cases, the endosomolytic moiety comprises SEQ ID NO: 8. In some cases, the endosomolytic moiety comprises SEQ ID NO: 9. In some cases, the endosomolytic moiety consists of SEQ ID NO: 8. In some cases, the endosomolytic moiety consists of SEQ ID NO: 9.

In some instances, the endosomolytic moiety comprises a sequence as illustrated in **Table 1** below.

| **Name** | **Origin** | **Amino Acid Sequence** | **SEQ ID NO:** | **Type** |
|---|---|---|---|---|
| Pep-1 | NLS from Simian Virus 40 large antigen and Reverse transcriptase of HIV | KETWWETWWTEWSQPKKKR KV | 10 | Primary amphipathic |
| pVEC | VE-cadherin | LLIILRRRRIRKQAHAHSK | 11 | Primary amphipathic |
| VT5 | Synthetic peptide | DPKGDPKGVTVTVTVTVTGK GDPKPD | 12 | β-sheet amphipathic |
| C105Y | 1-antitrypsin | CSIPPEVKFNKPFVYLI | 13 | - |
| Transportan | Galanin and mastoparan | GWTLNSAGYLLGKINLKALA ALAKKIL | 14 | Primary amphipathic |
| TP10 | Galanin and mastoparan | AGYLLGKINLKALAALAKKIL | 15 | Primary amphipathic |
| MPG | A hydrofobic domain from the fusion sequence of HIV gp41 and NLS of SV40 T antigen | GALFLGFLGAAGSTMGA | 16 | β-sheet amphipathic |
| gH625 | Glycoprotein gH of HSV type I | HGLASTLTRWAHYNALIRAF | 17 | Secondary amphipathic α-helical |
| CADY | PPTG1 peptide | GLWRALWRLLRSLWRLLWRA | 18 | Secondary amphipathic α-helical |
| GALA | Synthetic peptide | WEAALAEALAEALAEHLAEA LAEALEALAA | 19 | Secondary amphipathic α-helical |
| INF | Influenza HA2 fusion | GLFEAIEGFIENGWEGMIDGW | 20 | Secondary |
| | peptide | YGC | | amphipathic α-helical/ pH-dependent membrane active peptide |
| HA2E5-TAT | Influenza HA2 subunit of influenza virus X31 strain fusion peptide | GLFGAIAGFIENGWEGMIDGW YG | 21 | Secondary amphipathic α-helical/ pH-dependent membrane active peptide |
| HA2-penetratin | Influenza HA2 subunit of influenza virus X31 strain fusion peptide | GLFGAIAGFIENGWEGMIDGR QIKIWFQNRRMKW KK-amide | 22 | pH-dependent membrane active peptide |
| HA-K4 | Influenza HA2 subunit of influenza virus X31 strain fusion peptide | GLFGAIAGFIENGWEGMIDG- SSKKKK | 23 | pH-dependent membrane active peptide |
| HA2E4 | Influenza HA2 subunit of influenza virus X31 strain fusion peptide | GLFEAIAGFIENGWEGMIDGG GYC | 24 | pH-dependent membrane active peptide |
| H5WYG | HA2 analogue | GLFHAIAHFIHGGWH GLIHGWYG | 25 | pH-dependent membrane active peptide |
| GALA-INF3-(PEG)6-NH | INF3 fusion peptide | GLFEAIEGFIENGWEGLAEALA EALEALAA- (PEG)6-NH2 | 26 | pH-dependent membrane active peptide |
| CM18-TAT11 | Cecropin-A-Melittin₂₋₁₂ (CM₁₈) fusion peptide | KWKLFKKIGAVLKVLTTG- YGRKKRRQRRR | 27 | pH-dependent membrane active peptide |

In some cases, the endosomolytic moiety comprises a Bak BH3 polypeptide which induces apoptosis through antagonization of suppressor targets such as Bcl-2 and/or Bcl-x_{L}. In some instances, the endosomolytic moiety comprises a Bak BH3 polypeptide described in Albarran, et al., "Efficient intracellular delivery of a pro-apoptotic peptide with a pH-responsive carrier," Reactive & Functional Polymers 71: 261-265 (2011).

In some instances, the endosomolytic moiety comprises a polypeptide (e.g., a cell-penetrating polypeptide) as described in PCT Publication Nos. WO2013/166155 or WO2015/069587.

### Endosomolytic Polymers

In some embodiments, a molecule of Formula (V): A-(X¹-B)ₙ or Formula (VI): A-X¹-(B-X²-C)ₙ is further conjugated with an endosomolytic polymer. As used herein, an endosomolytic polymer comprises a linear, a branched network, a star, a comb, or a ladder type of polymer. In some instances, an endosomolytic polymer is a homopolymer or a copolymer comprising two ro more different types of monomers. In some cases, an endosomolytic polymer is a polycation polymer. In other cases, an endosomolytic polymer is a polyanion polymer.

In some instances, a polycation polymer comprises monomer units that are charge positive, charge neutral, or charge negative, with a net charge being positive. In other cases, a polycation polymer comprises a non-polymeric molecule that contains two or more positive charges. Exemplary cationic polymers include, but are not limited to, poly(L-lysine) (PLL), poly(L-arginine) (PLA), polyethyleneimine (PEI), poly[α-(4-aminobutyl)-L-glycolic acid] (PAGA), 2-(dimethylamino)ethyl methacrylate (DMAEMA), or N,N-Diethylaminoethyl Methacrylate (DEAEMA).

In some cases, a polyanion polymer comprises monomer units that are charge positive, charge neutral, or charge negative, with a net charge being negative. In other cases, a polyanion polymer comprises a non-polymeric molecule that contains two or more negative charges. Exemplary anionic polymers include p(alkylacrylates) (e.g., poly(propyl acrylic acid) (PPAA)) or poly(N-isopropylacrylamide) (NIPAM). Additional examples include PP75, a L-phenylalanine-poly(L-lysine isophthalamide) polymer described in Khormaee, et al., "Edosomolytic anionic polymer for the cytoplasmic delivery of siRNAs in localized in vivo applications," Advanced Functional Materials 23: 565-574 (2013).

In some embodiments, an endosomolytic polymer described herein is a pH-responsive endosomolytic polymer. A pH-responsive polymer comprises a polymer that increases in size (swell) or collapses depending on the pH of the environment. Polyacrylic acid and chitosan are examples of pH-responsive polymers.

In some instances, an endosomolytic moiety described herein is a membrane-disruptive polymer. In some cases, the membrane-disruptive polymer comprises a cationic polymer, a neutral or hydrophobic polymer, or an anionic polymer. In some instances, the membrane-disruptive polymer is a hydrophilic polymer.

In some instances, an endosomolytic moiety described herein is a pH-responsive membrane-disruptive polymer. Exemplary pH-responsive membrane-disruptive polymers include p(alkylacrylic acids), poly(N-isopropylacrylamide) (NIPAM) copolymers, succinylated p(glycidols), and p(β-malic acid) polymers.

In some instances, p(alkylacrylic acids) include poly(propylacrylic acid) (polyPAA), poly(methacrylic acid) (PMAA), poly(ethylacrylic acid) (PEAA), and poly(propyl acrylic acid) (PPAA). In some instances, a p(alkylacrylic acid) include a p(alkylacrylic acid) described in Jones, et al., Biochemistry Journal 372: 65-75 (2003).

In some embodiments, a pH-responsive membrane-disruptive polymer comprises p(butyl acrylate-co-methacrylic acid). *(see* Bulmus, et al., Journal of Controlled Release 93: 105-120 (2003); and Yessine, et al., Biochimica et Biophysica Acta 1613: 28-38 (2003))

In some embodiments, a pH-responsive membrane-disruptive polymer comprises p(styrene-alt-maleic anhydride). *(see* Henry, et al., Biomacromolecules 7: 2407-2414 (2006))

In some embodiments, a pH-responsive membrane-disruptive polymer comprises pyridyldisulfide acrylate (PDSA) polymers such as poly(MAA-co-PDSA), poly(EAA-co-PDSA), poly(PAA-co-PDSA), poly(MAA-*co*-BA-*co*-PDSA), poly(EAA-*co*-BA-*co*-PDSA), or poly(PAA-co-BA-co-PDSA) polymers. *(see* El-Sayed, et al., "Rational design of composition and activity correlations for pH-responsive and glutathione-reactive polymer therapeutics," Journal of Controlled Release 104: 417-427 (2005); or Flanary et al., "Antigen delivery with poly(propylacrylic acid) conjugation enhanced MHC-1 presentation and T-cell activation," Bioconjugate Chem. 20: 241-248 (2009))

In some embodiments, a pH-responsive membrane-disruptive polymer comprises a lytic polymer comprising the base structure of:

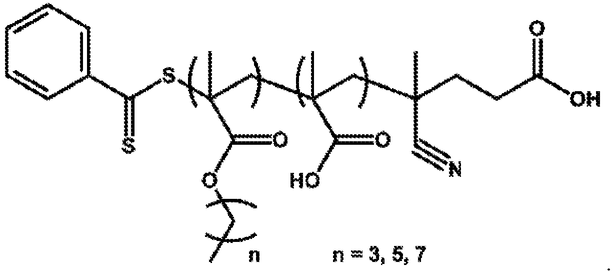

In some instances, an endosomolytic moiety described herein is further conjugated to an additional conjugate, e.g., a polymer (e.g., PEG), or a modified polymer (e.g., cholesterol-modified polymer).

In some instances, the additional conjugate comprises a detergent (e.g., Triton X-100). In some instances, an endosomolytic moiety described herein comprises a polymer (e.g., a poly(amidoamine)) conjugated with a detergent (e.g., Triton X-100). In some instances, an endosomolytic moiety described herein comprises poly(amidoamine)-Triton X-100 conjugate (Duncan, et al., "A polymer-Triton X-100 conjugate capable of pH-dependent red blood cell lysis: a model system illustrating the possibility of drug delivery within acidic intracellular compartments," Journal of Drug Targeting 2: 341-347 (1994)).

### Endosomolytic Lipids

In some embodiments, the endosomolytic moiety is a lipid (e.g., a fusogenic lipid). In some embodiments, a molecule of Formula (V): A-(X¹-B)ₙ or Formula (VI): A-X¹-(B-X²-C)ₙ is further conjugated with an endosomolytic lipid (e.g., fusogenic lipid). Exemplary fusogenic lipids include 1,2-dileoyl-sn-3-phosphoethanolamine (DOPE), phosphatidylethanolamine (POPE), palmitoyloleoylphosphatidylcholine (POPC), (6Z,9Z,28Z,31Z)-heptatriaconta-6,9,28,31-tetraen-19-ol (Di-Lin), N-methyl(2,2-di((9Z,12Z)-octadeca-9,12-dienyl)-1,3-dioxolan-4-yl)methanamine (DLin-k-DMA) and N-methyl-2-(2,2-di((9Z,12Z)-octadeca-9,12-dienyl)-1,3-dioxolan-4-yl)ethanamine (XTC).

In some instances, an endosomolytic moiety is a lipid (e.g., a fusogenic lipid) described in PCT Publication No. WO09/126,933.

### Endosomolytic Small Molecules

In some embodiments, the endosomolytic moiety is a small molecule. In some embodiments, a molecule of Formula (I): A-(X¹-B)ₙ or Formula (II): A-X¹-(B-X²-C)ₙ is further conjugated with an endosomolytic small molecule. Exemplary small molecules suitable as endosomolytic moieties include, but are not limited to, quinine, chloroquine, hydroxychloroquines, amodiaquins (carnoquines), amopyroquines, primaquines, mefloquines, nivaquines, halofantrines, quinone imines, or a combination thereof. In some instances, quinoline endosomolytic moieties include, but are not limited to, 7-chloro-4-(4-diethylamino-1-methylbutyl-amino)quinoline (chloroquine); 7-chloro-4-(4-ethyl-(2-hydroxyethyl)-amino-1-methylbutyl-amino)quinoline (hydroxychloroquine); 7-fluoro-4-(4-diethylamino-1-methylbutyl-amino)quinoline; 4-(4-diethylamino-1-methylbutylamino) quinoline; 7-hydroxy-4-(4-diethyl-amino-1-methylbutylamino)quinoline; 7-chloro-4-(4-diethylamino-1-butylamino)quinoline (desmethylchloroquine); 7-fluoro-4-(4-diethylamino-1-butylamino)quinoline); 4-(4-diethyl-amino-1-butylamino)quinoline; 7-hydroxy-4-(4-diethylamino-1-butylamino)quinoline; 7-chloro-4-(1-carboxy-4-diethylamino-1-butylamino)quinoline; 7-fluoro-4-(1-carboxy-4-diethyl-amino-1-butylamino)quinoline; 4-(1-carboxy-4-diethylamino-1-butylamino) quinoline; 7-hydroxy-4-(1-carboxy-4-diethylamino-1-butylamino)quinoline; 7-chloro-4-(1-carboxy-4-diethylamino-1-methylbutylamino)quinoline; 7-fluoro-4-(1-carboxy-4-diethyl-amino-1-methylbutylamino)quinoline; 4-(1-carboxy-4-diethylamino-1-methylbutylamino)quinoline; 7-hydroxy-4-(1-carboxy-4-diethylamino-1-methylbutylamino)quinoline; 7-fluoro-4-(4-ethyl-(2-hydroxyethyl)-amino-1-methylbutylamino)quinoline; 4-(4-ethyl-(2-hydroxy-ethyl)-amino-1-methylbutylamino-)quinoline; 7-hydroxy-4-(4-ethyl-(2-hydroxyethyl)-amino-1-methylbutylamino)quinoline; hydroxychloroquine phosphate; 7-chloro-4-(4-ethyl-(2-hydroxyethyl-1)-amino-1-butylamino)quinoline (desmethylhydroxychloroquine); 7-fluoro-4-(4-ethyl-(2-hydroxyethyl)-amino-1-butylamino)quinoline; 4-(4-ethyl-(2-hydroxyethyl)-amino-1-butylamino)quinoline; 7-hydroxy-4-(4-ethyl-(2-hydroxyethyl)-amino-1-butylamino) quinoline; 7-chloro-4-(1-carboxy-4-ethyl-(2-hydroxyethyl)-amino-1-butylamino)quinoline; 7-fluoro-4-(1-carboxy-4-ethyl-(2-hydroxyethyl)-amino-1-butylamino)quinoline; 4-(1-carboxy-4-ethyl-(2-hydroxyethyl)-amino-1-butylamino)quinoline; 7-hydroxy-4-(1-carboxy-4-ethyl-(2-hydroxyethyl)-amino-1-butylamino)quinoline; 7-chloro-4-(1-carboxy-4-ethyl-(2-hydroxyethyl)-amino-1-methylbutylamino)quinoline; 7-fluoro-4-(1-carboxy-4-ethyl-(2-hydroxyethyl)-amino-1-methylbutylamino)quinoline; 4-(1-carboxy-4-ethyl-(2-hydroxyethyl)-amino-1-methylbutylamino)quinoline; 7-hydroxy-4-(1-carboxy-4-ethyl-(2-hydroxyethyl)-amino-1-methylbutylamino)quinoline; 8-[(4-aminopentyl)amino-6-methoxydihydrochloride quinoline; 1-acetyl-1,2,3,4-tetrahydroquinoline; 8-[(4-aminopentyl)amino]-6-methoxyquinoline dihydrochloride; 1-butyryl-1,2,3,4-tetrahydroquinoline; 3-chloro-4-(4-hydroxy-alpha,alpha'-bis(2-methyl-1-pyrrolidinyl)-2,5-xylidinoquinoline, 4-[(4-diethyl-amino)-1-methylbutyl-amino]-6-methoxyquinoline; 3-fluoro-4-(4-hydroxy-alpha,alpha'-bis(2-methyl-1-pyrrolidinyl)-2,5-xylidinoquinoline, 4-[(4-diethylamino)-1-methylbutyl-amino]-6-methoxyquinoline; 4-(4-hydroxy-alpha,alpha'-bis(2-methyl-1-pyrrolidinyl)-2,5-xylidinoquinoline; 4-[(4-diethylamino)-1-methylbutyl-amino]-6-methoxyquinoline; 3,4-dihydro-1-(2H)-quinolinecarboxyaldehyde; 1,1'-pentamethylene diquinoleinium diiodide; 8-quinolinol sulfate and amino, aldehyde, carboxylic, hydroxyl, halogen, keto, sulfhydryl and vinyl derivatives or analogs thereof. In some instances, an endosomolytic moiety is a small molecule described in Naisbitt et al (1997, J Pharmacol Exp Therapy 280:884-893) and in U.S. Patent No. 5,736,557.
In some embodiments, the endosomolytic moiety is nigericin or a conjugate thereof, e.g., such as a folate-nigericin ester conjugate, a folate-nigericin amide conjugate, or a folate-nigericin carbamate conjugate. In some instances, the endosomolytic moiety is nigericin described in Rangasamy, et. al., "New mechanism for release of endosomal contents: osmotic lysis via nigericin-mediated K+/H+ exchange," Bioconjugate Chem. 29:1047-1059 (2018).

### Linkers

In some embodiments, a linker described herein is a cleavable linker or a non-cleavable linker. In some instances, the linker is a cleavable linker. In other instances, the linker is a non-cleavable linker.

In some cases, the linker is a non-polymeric linker. A non-polymeric linker refers to a linker that does not contain a repeating unit of monomers generated by a polymerization process. Exemplary non-polymeric linkers include, but are not limited to, C₁-C₆ alkyl group (e.g., a C₅, C₄, C₃, C₂, or C₁ alkyl group), homobifunctional cross linkers, heterobifunctional cross linkers, peptide linkers, traceless linkers, self-immolative linkers, maleimide-based linkers, or combinations thereof. In some cases, the non-polymeric linker comprises a C₁-C₆ alkyl group (e.g., a C₅, C₄, C₃, C₂, or C₁ alkyl group), a homobifunctional cross linker, a heterobifunctional cross linker, a peptide linker, a traceless linker, a self-immolative linker, a maleimide-based linker, or a combination thereof. In additional cases, the non-polymeric linker does not comprise more than two of the same type of linkers, e.g., more than two homobifunctional cross linkers, or more than two peptide linkers. In further cases, the non-polymeric linker optionally comprises one or more reactive functional groups.

In some instances, the non-polymeric linker does not encompass a polymer that is described above. In some instances, the non-polymeric linker does not encompass a polymer encompassed by the polymer moiety C. In some cases, the non-polymeric linker does not encompass a polyalkylene oxide (e.g., PEG). In some cases, the non-polymeric linker does not encompass a PEG.

In some instances, the linker comprises a homobifunctional linker. Exemplary homobifunctional linkers include, but are not limited to, Lomant's reagent dithiobis (succinimidylpropionate) DSP, 3'3'-dithiobis(sulfosuccinimidyl proprionate (DTSSP), disuccinimidyl suberate (DSS), bis(sulfosuccinimidyl)suberate (BS), disuccinimidyl tartrate (DST), disulfosuccinimidyl tartrate (sulfo DST), ethylene glycobis(succinimidylsuccinate) (EGS), disuccinimidyl glutarate (DSG), N,N'-disuccinimidyl carbonate (DSC), dimethyl adipimidate (DMA), dimethyl pimelimidate (DMP), dimethyl suberimidate (DMS), dimethyl-3,3'-dithiobispropionimidate (DTBP), 1,4-di-3'-(2'-pyridyldithio)propionamido)butane (DPDPB), bismaleimidohexane (BMH), aryl halide-containing compound (DFDNB), such as e.g. 1,5-difluoro-2,4-dinitrobenzene or 1,3-difluoro-4,6-dinitrobenzene, 4,4'-difluoro-3,3'-dinitrophenylsulfone (DFDNPS), bis-[β-(4-azidosalicylamido)ethyl]disulfide (BASED), formaldehyde, glutaraldehyde, 1,4-butanediol diglycidyl ether, adipic acid dihydrazide, carbohydrazide, o-toluidine, 3,3'-dimethylbenzidine, benzidine, α,α'-p-diaminodiphenyl, diiodo-p-xylene sulfonic acid, N,N'-ethylene-bis(iodoacetamide), or N,N'-hexamethylene-bis(iodoacetamide).

In some embodiments, the linker comprises a heterobifunctional linker. Exemplary heterobifunctional linker include, but are not limited to, amine-reactive and sulfhydryl cross-linkers such as N-succinimidyl 3-(2-pyridyldithio)propionate (sPDP), long-chain N-succinimidyl 3-(2-pyridyldithio)propionate (LC-sPDP), water-soluble-long-chain N-succinimidyl 3-(2-pyridyldithio) propionate (sulfo-LC-sPDP), succinimidyloxycarbonyl-α-methyl-α-(2-pyridyldithio)toluene (sMPT), sulfosuccinimidyl-6-[α-methyl-α-(2-pyridyldithio)toluamido]hexanoate (sulfo-LC-sMPT), succinimidyl-4-(N-maleimidomethyl)cyclohexane-1-carboxylate (sMCC), sulfosuccinimidyl-4-(N-maleimidomethyl)cyclohexane-1-carboxylate (sulfo-sMCC), m-maleimidobenzoyl-N-hydroxysuccinimide ester (MBs), m-maleimidobenzoyl-N-hydroxysulfosuccinimide ester (sulfo-MBs), N-succinimidyl(4-iodoacteyl)aminobenzoate (sIAB), sulfosuccinimidyl(4-iodoacteyl)aminobenzoate (sulfo-sIAB), succinimidyl-4-(p-maleimidophenyl)butyrate (sMPB), sulfosuccinimidyl-4-(p-maleimidophenyl)butyrate (sulfo-sMPB), N-(y-maleimidobutyryloxy)succinimide ester (GMBs), N-(γ-maleimidobutyryloxy)sulfosuccinimide ester (sulfo-GMBs), succinimidyl 6-((iodoacetyl)amino)hexanoate (sIAX), succinimidyl 6-[6-(((iodoacetyl)amino)hexanoyl)amino]hexanoate (sIAXX), succinimidyl 4-(((iodoacetyl)amino)methyl)cyclohexane-1-carboxylate (sIAC), succinimidyl 6-((((4-iodoacetyl)amino)methyl)cyclohexane-1-carbonyl)amino) hexanoate (sIACX), p-nitrophenyl iodoacetate (NPIA), carbonyl-reactive and sulfhydryl-reactive cross-linkers such as 4-(4-N-maleimidophenyl)butyric acid hydrazide (MPBH), 4-(N-maleimidomethyl)cyclohexane-1-carboxyl-hydrazide-8 (M₂C₂H), 3-(2-pyridyldithio)propionyl hydrazide (PDPH), amine-reactive and photoreactive cross-linkers such as N-hydroxysuccinimidyl-4-azidosalicylic acid (NHs-AsA), N-hydroxysulfosuccinimidyl-4-azidosalicylic acid (sulfo-NHs-AsA), sulfosuccinimidyl-(4-azidosalicylamido)hexanoate (sulfo-NHs-LC-AsA), sulfosuccinimidyl-2-(p-azidosalicylamido)ethyl-1,3'-dithiopropionate (sAsD), N-hydroxysuccinimidyl-4-azidobenzoate (HsAB), N-hydroxysulfosuccinimidyl-4-azidobenzoate (sulfo-HsAB), N-succinimidyl-6-(4'-azido-2'-nitrophenylamino)hexanoate (sANPAH), sulfosuccinimidyl-6-(4'-azido-2'-nitrophenylamino)hexanoate (sulfo-sANPAH), N-5-azido-2-nitrobenzoyloxysuccinimide (ANB-NOs), sulfosuccinimidyl-2-(m-azido-o-nitrobenzamido)-ethyl-1,3'-dithiopropionate (sAND), N-succinimidyl-4(4-azidophenyl) 1,3'-dithiopropionate (sADP), N-sulfosuccinimidyl(4-azidophenyl)-1,3'-dithiopropionate (sulfo-sADP), sulfosuccinimidyl 4-(ρ-azidophenyl)butyrate (sulfo-sAPB), sulfosuccinimidyl 2-(7-azido-4-methylcoumarin-3-acetamide)ethyl-1,3'-dithiopropionate (sAED), sulfosuccinimidyl 7-azido-4-methylcoumain-3-acetate (sulfo-sAMCA), ρ-nitrophenyl diazopyruvate (ρNPDP), ρ-nitrophenyl-2-diazo-3,3,3-trifluoropropionate (PNP-DTP), sulfhydryl-reactive and photoreactive cross-linkers such as1-(ρ-Azidosalicylamido)-4-(iodoacetamido)butane (AsIB), N-[4-(ρ-azidosalicylamido)butyl]-3'-(2'-pyridyldithio)propionamide (APDP), benzophenone-4-iodoacetamide, benzophenone-4-maleimide carbonyl-reactive and photoreactive cross-linkers such as ρ-azidobenzoyl hydrazide (ABH), carboxylate-reactive and photoreactive cross-linkers such as 4-(ρ-azidosalicylamido)butylamine (AsBA), and arginine-reactive and photoreactive cross-linkers such as ρ-azidophenyl glyoxal (APG).

In some instances, the linker comprises a reactive functional group. In some cases, the reactive functional group comprises a nucleophilic group that is reactive to an electrophilic group present on a binding moiety. Exemplary electrophilic groups include carbonyl groups-such as aldehyde, ketone, carboxylic acid, ester, amide, enone, acyl halide or acid anhydride. In some embodiments, the reactive functional group is aldehyde. Exemplary nucleophilic groups include hydrazide, oxime, amino, hydrazine, thiosemicarbazone, hydrazine carboxylate, and arylhydrazide.

In some embodiments, the linker comprises a maleimide goup. In some instances, the maleimide group is also referred to as a maleimide spacer. In some instances, the maleimide group further encompasses a caproic acid, forming maleimidocaproyl (mc). In some cases, the linker comprises maleimidocaproyl (mc). In some cases, the linker is maleimidocaproyl (mc). In other instances, the maleimide group comprises a maleimidomethyl group, such as succinimidyl-4-(N-maleimidomethyl)cyclohexane-1-carboxylate (sMCC) or sulfosuccinimidyl-4-(N-maleimidomethyl)cyclohexane-1-carboxylate (sulfo-sMCC) described above.

In some embodiments, the maleimide group is a self-stablizing maleimide. In some instances, the self-stablizing maleimide utilizes diaminopropionic acid (DPR) to incorporate a basic amino group adjacent to the maleimide to provide intramolecular catalysis of tiosuccinimide ring hydrolysis, thereby eliminating maleimide from undergoing an elimination reaction through a retro-Michael reaction. In some instances, the self-stabilizing maleimide is a maleimide group described in Lyon, et al., "Self-hydrolyzing maleimides improve the stability and pharmacological properties of antibody-drug conjugates," Nat. Biotechnol. 32(10):1059-1062 (2014). In some instances, the linker comprises a self-stablizing maleimide. In some instances, the linker is a self-stablizing maleimide.

In some embodiments, the linker comprises a peptide moiety. In some instances, the peptide moiety comprises at least 2, 3, 4, 5, or 6 more amino acid residues. In some instances, the peptide moiety comprises at most 2, 3, 4, 5, 6, 7, or 8 amino acid residues. In some instances, the peptide moiety comprises about 2, about 3, about 4, about 5, or about 6 amino acid residues. In some instances, the peptide moiety is a cleavable peptide moiety (e.g., either enzymatically or chemically). In some instances, the peptide moiety is a non-cleavable peptide moiety. In some instances, the peptide moiety comprises Val-Cit (valine-citrulline), Gly-Gly-Phe-Gly, Phe-Lys, Val-Lys, Gly-Phe-Lys, Phe-Phe-Lys, Ala-Lys, Val-Arg, Phe-Cit, Phe-Arg, Leu-Cit, Ile-Cit, Trp-Cit, Phe-Ala, Ala-Leu-Ala-Leu, or Gly-Phe-Leu-Gly. In some instances, the linker comprises a peptide moiety such as: Val-Cit (valine-citrulline), Gly-Gly-Phe-Gly, Phe-Lys, Val-Lys, Gly-Phe-Lys, Phe-Phe-Lys, Ala-Lys, Val-Arg, Phe-Cit, Phe-Arg, Leu-Cit, Ile-Cit, Trp-Cit, Phe-Ala, Ala-Leu-Ala-Leu, or Gly-Phe-Leu-Gly. In some cases, the linker comprises Val-Cit. In some cases, the linker is Val-Cit.

In some embodiments, the linker comprises a benzoic acid group, or its derivatives thereof. In some instances, the benzoic acid group or its derivatives thereof comprise paraaminobenzoic acid (PABA). In some instances, the benzoic acid group or its derivatives thereof comprise gamma-aminobutyric acid (GABA).

In some embodiments, the linker comprises one or more of a maleimide group, a peptide moiety, and/or a benzoic acid group, in any combination. In some embodiments, the linker comprises a combination of a maleimide group, a peptide moiety, and/or a benzoic acid group. In some instances, the maleimide group is maleimidocaproyl (mc). In some instances, the peptide group is val-cit. In some instances, the benzoic acid group is PABA. In some instances, the linker comprises a mc-val-cit group. In some cases, the linker comprises a val-cit-PABA group. In additional cases, the linker comprises a mc-val-cit-PABA group.

In some embodiments, the linker is a self-immolative linker or a self-elimination linker. In some cases, the linker is a self-immolative linker. In other cases, the linker is a self-elimination linker (e.g., a cyclization self-elimination linker). In some instances, the linker comprises a linker described in U.S. Patent No. 9,089,614 or PCT Publication No. WO2015038426.

In some embodiments, the linker is a dendritic type linker. In some instances, the dendritic type linker comprises a branching, multifunctional linker moiety. In some instances, the dendritic type linker is used to increase the molar ratio of polynucleotide B to the binding moiety A. In some instances, the dendritic type linker comprises PAMAM dendrimers.

In some embodiments, the linker is a traceless linker or a linker in which after cleavage does not leave behind a linker moiety (e.g., an atom or a linker group) to a binding moiety A, a polynucleotide B, a polymer C, or an endosomolytic moiety D. Exemplary traceless linkers include, but are not limited to, germanium linkers, silicium linkers, sulfur linkers, selenium linkers, nitrogen linkers, phosphorus linkers, boron linkers, chromium linkers, or phenylhydrazide linker. In some cases, the linker is a traceless aryl-triazene linker as described in Hejesen, et al., "A traceless aryl-triazene linker for DNA-directed chemistry," Org Biomol Chem 11(15): 2493-2497 (2013). In some instances, the linker is a traceless linker described in Blaney, et al., "Traceless solid-phase organic synthesis," Chem. Rev. 102: 2607-2024 (2002). In some instances, a linker is a traceless linker as described in U.S. Patent No. 6,821,783.

In some instances, the linker is a linker described in U.S. Patent Nos. 6,884,869; 7,498,298; 8,288,352; 8,609,105; or 8,697,688; U.S. Patent Publication Nos. 2014/0127239; 2013/028919; 2014/286970; 2013/0309256; 2015/037360; or 2014/0294851; or PCT Publication Nos. WO2015057699; WO2014080251; WO2014197854; WO2014145090; or WO2014177042.

In some embodiments, X, Y, and L are independently a bond or a linker. In some instances, X, Y, and L are independently a bond. In some cases, X, Y, and L are independently a linker.

In some instances, X is a bond or a linker. In some instances, X is a bond. In some instances, X is a linker. In some instances, the linker is a C₁-C₆ alkyl group. In some cases, X is a C₁-C₆ alkyl group, such as for example, a C₅, C₄, C₃, C₂, or C₁ alkyl group. In some cases, the C₁-C₆ alkyl group is an unsubstituted C₁-C₆ alkyl group. As used in the context of a linker, and in particular in the context of X, alkyl means a saturated straight or branched hydrocarbon radical containing up to six carbon atoms. In some instances, X is a non-polymeric linker. In some instances, X includes a homobifunctional linker or a heterobifunctional linker described *supra.* In some cases, X includes a heterobifunctional linker. In some cases, X includes sMCC. In other instances, X includes a heterobifunctional linker optionally conjugated to a C₁-C₆ alkyl group. In other instances, X includes sMCC optionally conjugated to a C₁-C₆ alkyl group. In additional instances, X does not include a homobifunctional linker or a heterobifunctional linker described *supra.*

In some instances, Y is a bond or a linker. In some instances, Y is a bond. In other cases, Y is a linker. In some embodiments, Y is a C₁-C₆ alkyl group. In some instances, Y is a homobifunctional linker or a heterobifunctional linker described *supra.* In some instances, Y is a homobifunctional linker described *supra.* In some instances, Y is a heterobifunctional linker described *supra.* In some instances, Y comprises a maleimide group, such as maleimidocaproyl (mc) or a self-stabilizing maleimide group described above. In some instances, Y comprises a peptide moiety, such as Val-Cit. In some instances, Y comprises a benzoic acid group, such as PABA. In additional instances, Y comprises a combination of a maleimide group, a peptide moiety, and/or a benzoic acid group. In additional instances, Y comprises a mc group. In additional instances, Y comprises a mc-val-cit group. In additional instances, Y comprises a val-cit-PABA group. In additional instances, Y comprises a mc-val-cit-PABA group.

In some instances, L is a bond or a linker. In some cases, L is a bond. In other cases, L is a linker. In some embodiments, L is a C₁-C₆ alkyl group. In some instances, L is a homobifunctional linker or a heterobifunctional linker described *supra.* In some instances, L is a homobifunctional linker described *supra.* In some instances, L is a heterobifunctional linker described *supra.* In some instances, L comprises a maleimide group, such as maleimidocaproyl (mc) or a self-stabilizing maleimide group described above. In some instances, L comprises a peptide moiety, such as Val-Cit. In some instances, L comprises a benzoic acid group, such as PABA. In additional instances, L comprises a combination of a maleimide group, a peptide moiety, and/or a benzoic acid group. In additional instances, L comprises a mc group. In additional instances, L comprises a mc-val-cit group. In additional instances, L comprises a val-cit-PABA group. In additional instances, L comprises a mc-val-cit-PABA group.

In some embodiments, X¹ and X² are each independently a bond or a non-polymeric linker. In some instances, X¹ and X² are each independently a bond. In some cases, X¹ and X² are each independently a non-polymeric linker.

In some instances, X¹ is a bond or a non-polymeric linker. In some instances, X¹ is a bond. In some instances, X¹ is a non-polymeric linker. In some instances, the linker is a C₁-C₆ alkyl group. In some cases, X¹ is a C₁-C₆ alkyl group, such as for example, a C₅, C₄, C₃, C₂, or C₁ alkyl group. In some cases, the C₁-C₆ alkyl group is an unsubstituted C₁-C₆ alkyl group. As used in the context of a linker, and in particular in the context of X¹, alkyl means a saturated straight or branched hydrocarbon radical containing up to six carbon atoms. In some instances, X¹ includes a homobifunctional linker or a heterobifunctional linker described *supra.* In some cases, X¹ includes a heterobifunctional linker. In some cases, X¹ includes sMCC. In other instances, X¹ includes a heterobifunctional linker optionally conjugated to a C₁-C₆ alkyl group. In other instances, X¹ includes sMCC optionally conjugated to a C₁-C₆ alkyl group. In additional instances, X¹ does not include a homobifunctional linker or a heterobifunctional linker described *supra.*

In some instances, X² is a bond or a linker. In some instances, X² is a bond. In other cases, X² is a linker. In additional cases, X² is a non-polymeric linker. In some embodiments, X² is a C₁-C₆ alkyl group. In some instances, X² is a homobifunctional linker or a heterobifunctional linker described *supra.* In some instances, X² is a homobifunctional linker described *supra.* In some instances, X² is a heterobifunctional linker described *supra.* In some instances, X² comprises a maleimide group, such as maleimidocaproyl (mc) or a self-stabilizing maleimide group described above. In some instances, X² comprises a peptide moiety, such as Val-Cit. In some instances, X² comprises a benzoic acid group, such as PABA. In additional instances, X² comprises a combination of a maleimide group, a peptide moiety, and/or a benzoic acid group. In additional instances, X² comprises a mc group. In additional instances, X² comprises a mc-val-cit group. In additional instances, X² comprises a val-cit-PABA group. In additional instances, X² comprises a mc-val-cit-PABA group.

### Pharmaceutical Formulation

In some embodiments, the pharmaceutical formulations described herein are administered to a subject by multiple administration routes, including but not limited to, parenteral (e.g., intravenous, subcutaneous, intramuscular), oral, intranasal, buccal, rectal, or transdermal administration routes. In some instances, the pharmaceutical composition describe herein is formulated for parenteral (e.g., intravenous, subcutaneous, intramuscular, intra-arterial, intraperitoneal, intrathecal, intracerebral, intracerebroventricular, or intracranial) administration. In other instances, the pharmaceutical composition describe herein is formulated for oral administration. In still other instances, the pharmaceutical composition describe herein is formulated for intranasal administration.

In some embodiments, the pharmaceutical formulations include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations (e.g., nanoparticle formulations), and mixed immediate and controlled release formulations.

In some instances, the pharmaceutical formulation includes multiparticulate formulations. In some instances, the pharmaceutical formulation includes nanoparticle formulations. In some instances, nanoparticles comprise cMAP, cyclodextrin, or lipids. In some cases, nanoparticles comprise solid lipid nanoparticles, polymeric nanoparticles, self-emulsifying nanoparticles, liposomes, microemulsions, or micellar solutions. Additional exemplary nanoparticles include, but are not limited to, paramagnetic nanoparticles, superparamagnetic nanoparticles, metal nanoparticles, fullerene-like materials, inorganic nanotubes, dendrimers (such as with covalently attached metal chelates), nanofibers, nanohorns, nano-onions, nanorods, nanoropes and quantum dots. In some instances, a nanoparticle is a metal nanoparticle, e.g., a nanoparticle of scandium, titanium, vanadium, chromium, manganese, iron, cobalt, nickel, copper, zinc, yttrium, zirconium, niobium, molybdenum, ruthenium, rhodium, palladium, silver, cadmium, hafnium, tantalum, tungsten, rhenium, osmium, iridium, platinum, gold, gadolinium, aluminum, gallium, indium, tin, thallium, lead, bismuth, magnesium, calcium, strontium, barium, lithium, sodium, potassium, boron, silicon, phosphorus, germanium, arsenic, antimony, and combinations, alloys or oxides thereof.

In some instances, a nanoparticle includes a core or a core and a shell, as in a core-shell nanoparticle.

In some instances, a nanoparticle is further coated with molecules for attachment of functional elements (e.g., with one or more of a polynucleic acid molecule or binding moiety described herein). In some instances, a coating comprises chondroitin sulfate, dextran sulfate, carboxymethyl dextran, alginic acid, pectin, carragheenan, fucoidan, agaropectin, porphyran, karaya gum, gellan gum, xanthan gum, hyaluronic acids, glucosamine, galactosamine, chitin (or chitosan), polyglutamic acid, polyaspartic acid, lysozyme, cytochrome C, ribonuclease, trypsinogen, chymotrypsinogen, α-chymotrypsin, polylysine, polyarginine, histone, protamine, ovalbumin or dextrin or cyclodextrin. In some instances, a nanoparticle comprises a graphene-coated nanoparticle.

In some cases, a nanoparticle has at least one dimension of less than about 500nm, 400nm, 300nm, 200nm, or 100nm.

In some instances, the nanoparticle formulation comprises paramagnetic nanoparticles, superparamagnetic nanoparticles, metal nanoparticles, fullerene-like materials, inorganic nanotubes, dendrimers (such as with covalently attached metal chelates), nanofibers, nanohorns, nano-onions, nanorods, nanoropes or quantum dots. In some instances, a polynucleic acid molecule or a binding moiety described herein is conjugated either directly or indirectly to the nanoparticle. In some instances, at least 1, 5, 10, 15, 20, 30, 40, 50, 60, 70, 80, 90, 100 or more polynucleic acid molecules or binding moieties described herein are conjugated either directly or indirectly to a nanoparticle.

In some embodiments, the pharmaceutical formulation comprise a delivery vector, e.g., a recombinant vector, the delivery of the polynucleic acid molecule into cells. In some instances, the recombinant vector is DNA plasmid. In other instances, the recombinant vector is a viral vector. Exemplary viral vectors include vectors derived from adeno-associated virus, retrovirus, adenovirus, or alphavirus. In some instances, the recombinant vectors capable of expressing the polynucleic acid molecules provide stable expression in target cells. In additional instances, viral vectors are used that provide for transient expression of polynucleic acid molecules.

In some embodiments, the pharmaceutical formulations include a carrier or carrier materials selected on the basis of compatibility with the composition disclosed herein, and the release profile properties of the desired dosage form. Exemplary carrier materials include, e.g., binders, suspending agents, disintegration agents, filling agents, surfactants, solubilizers, stabilizers, lubricants, wetting agents, diluents, and the like. Pharmaceutically compatible carrier materials include, but are not limited to, acacia, gelatin, colloidal silicon dioxide, calcium glycerophosphate, calcium lactate, maltodextrin, glycerine, magnesium silicate, polyvinylpyrrollidone (PVP), cholesterol, cholesterol esters, sodium caseinate, soy lecithin, taurocholic acid, phosphotidylcholine, sodium chloride, tricalcium phosphate, dipotassium phosphate, cellulose and cellulose conjugates, sugars sodium stearoyl lactylate, carrageenan, monoglyceride, diglyceride, pregelatinized starch, and the like. See, e.g., Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

In some instances, the pharmaceutical formulations further include pH adjusting agents or buffering agents which include acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

In some instances, the pharmaceutical formulation includes one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In some instances, the pharmaceutical formulations further include diluent which are used to stabilize compounds because they provide a more stable environment. Salts dissolved in buffered solutions (which also provide pH control or maintenance) are utilized as diluents in the art, including, but not limited to a phosphate buffered saline solution. In certain instances, diluents increase bulk of the composition to facilitate compression or create sufficient bulk for homogenous blend for capsule filling. Such compounds include e.g., lactose, starch, mannitol, sorbitol, dextrose, microcrystalline cellulose such as Avicel^{®}; dibasic calcium phosphate, dicalcium phosphate dihydrate; tricalcium phosphate, calcium phosphate; anhydrous lactose, spray-dried lactose; pregelatinized starch, compressible sugar, such as Di-Pac^{®} (Amstar); mannitol, hydroxypropylmethylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like.

In some cases, the pharmaceutical formulations include disintegration agents or disintegrants to facilitate the breakup or disintegration of a substance. The term "disintegrate" include both the dissolution and dispersion of the dosage form when contacted with gastrointestinal fluid. Examples of disintegration agents include a starch, e.g., a natural starch such as corn starch or potato starch, a pregelatinized starch such as National 1551 or Amijel^{®}, or sodium starch glycolate such as Promogel^{®} or Explotab^{®}, a cellulose such as a wood product, methylcrystalline cellulose, e.g., Avicel^{®}, Avicel^{®} PH101, Avicel^{®} PH102, Avicel^{®} PH105, Elcema^{®} P100, Emcocel^{®}, Vivacel^{®}, Ming Tia^{®}, and Solka-Floc^{®}, methylcellulose, croscarmellose, or a cross-linked cellulose, such as cross-linked sodium carboxymethylcellulose (Ac-Di-Sol^{®}), cross-linked carboxymethylcellulose, or cross-linked croscarmellose, a cross-linked starch such as sodium starch glycolate, a cross-linked polymer such as crospovidone, a cross-linked polyvinylpyrrolidone, alginate such as alginic acid or a salt of alginic acid such as sodium alginate, a clay such as Veegum^{®} HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like.

In some instances, the pharmaceutical formulations include filling agents such as lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol, sodium chloride, polyethylene glycol, and the like.

Lubricants and glidants are also optionally included in the pharmaceutical formulations described herein for preventing, reducing or inhibiting adhesion or friction of materials. Exemplary lubricants include, e.g., stearic acid, calcium hydroxide, talc, sodium stearyl fumerate, a hydrocarbon such as mineral oil, or hydrogenated vegetable oil such as hydrogenated soybean oil (Sterotex^{®}), higher fatty acids and their alkali-metal and alkaline earth metal salts, such as aluminum, calcium, magnesium, zinc, stearic acid, sodium stearates, glycerol, talc, waxes, Stearowet^{®}, boric acid, sodium benzoate, sodium acetate, sodium chloride, leucine, a polyethylene glycol (e.g., PEG-4000) or a methoxypolyethylene glycol such as Carbowax^{™}, sodium oleate, sodium benzoate, glyceryl behenate, polyethylene glycol, magnesium or sodium lauryl sulfate, colloidal silica such as Syloid^{™}, Cab-O-Sil^{®}, a starch such as corn starch, silicone oil, a surfactant, and the like.

Plasticizers include compounds used to soften the microencapsulation material or film coatings to make them less brittle. Suitable plasticizers include, e.g., polyethylene glycols such as PEG 300, PEG 400, PEG 600, PEG 1450, PEG 3350, and PEG 800, stearic acid, propylene glycol, oleic acid, triethyl cellulose and triacetin. Plasticizers also function as dispersing agents or wetting agents.

Solubilizers include compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins, ethanol, n-butanol, isopropyl alcohol, cholesterol, bile salts, polyethylene glycol 200-600, glycofurol, transcutol, propylene glycol, and dimethyl isosorbide and the like.

Stabilizers include compounds such as any antioxidation agents, buffers, acids, preservatives and the like.

Suspending agents include compounds such as polyvinylpyrrolidone, e.g., polyvinylpyrrolidone K12, polyvinylpyrrolidone K17, polyvinylpyrrolidone K25, or polyvinylpyrrolidone K30, vinyl pyrrolidone/vinyl acetate copolymer (S630), polyethylene glycol, e.g., the polyethylene glycol has a molecular weight of about 300 to about 6000, or about 3350 to about 4000, or about 7000 to about 5400, sodium carboxymethylcellulose, methylcellulose, hydroxypropylmethylcellulose, hydroxymethylcellulose acetate stearate, polysorbate-80, hydroxyethylcellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

Surfactants include compounds such as sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic^{®} (BASF), and the like. Additional surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40. Sometimes, surfactants is included to enhance physical stability or for other purposes.

Viscosity enhancing agents include, e.g., methyl cellulose, xanthan gum, carboxymethyl cellulose, hydroxypropyl cellulose, hydroxypropylmethyl cellulose, hydroxypropylmethyl cellulose acetate stearate, hydroxypropylmethyl cellulose phthalate, carbomer, polyvinyl alcohol, alginates, acacia, chitosans and combinations thereof.

Wetting agents include compounds such as oleic acid, glyceryl monostearate, sorbitan monooleate, sorbitan monolaurate, triethanolamine oleate, polyoxyethylene sorbitan monooleate, polyoxyethylene sorbitan monolaurate, sodium docusate, sodium oleate, sodium lauryl sulfate, sodium doccusate, triacetin, Tween 80, vitamin E TPGS, ammonium salts and the like.

### Therapeutic Regimens

In some embodiments, the pharmaceutical compositions described herein are administered for therapeutic applications. In some embodiments, the pharmaceutical composition is administered once per day, twice per day, three times per day or more. The pharmaceutical composition is administered daily, every day, every alternate day, five days a week, once a week, every other week, two weeks per month, three weeks per month, once a month, twice a month, three times per month, or more. The pharmaceutical composition is administered for at least 1 month, 2 months, 3 months, 4 months, 5 months, 6 months, 7 months, 8 months, 9 months, 10 months, 11 months, 12 months, 18 months, 2 years, 3 years, or more.

In some embodiments, one or more pharmaceutical compositions are administered simultaneously, sequentially, or at an interval period of time. In some embodiments, one or more pharmaceutical compositions are administered simultaneously. In some cases, one or more pharmaceutical compositions are administered sequentially. In additional cases, one or more pharmaceutical compositions are administered at an interval period of time (e.g., the first administration of a first pharmaceutical composition is on day one followed by an interval of at least 1, 2, 3, 4, 5, or more days prior to the administration of at least a second pharmaceutical composition).

In some embodiments, two or more different pharmaceutical compositions are coadministered. In some instances, the two or more different pharmaceutical compositions are coadministered simultaneously. In some cases, the two or more different pharmaceutical compositions are coadministered sequentially without a gap of time between administrations. In other cases, the two or more different pharmaceutical compositions are coadministered sequentially with a gap of about 0.5 hour, 1 hour, 2 hour, 3 hour, 12 hours, 1 day, 2 days, or more between administrations.

In the case wherein the patient's status does improve, upon the doctor's discretion the administration of the composition is given continuously; alternatively, the dose of the composition being administered is temporarily reduced or temporarily suspended for a certain length of time (i.e., a "drug holiday"). In some instances, the length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday is from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, can be reduced, as a function of the symptoms, to a level at which the improved disease, disorder or condition is retained.

In some embodiments, the amount of a given agent that correspond to such an amount varies depending upon factors such as the particular compound, the severity of the disease, the identity (e.g., weight) of the subject or host in need of treatment, but nevertheless is routinely determined in a manner known in the art according to the particular circumstances surrounding the case, including, e.g., the specific agent being administered, the route of administration, and the subject or host being treated. In some instances, the desired dose is conveniently presented in a single dose or as divided doses administered simultaneously (or over a short period of time) or at appropriate intervals, for example as two, three, four or more sub-doses per day.

The foregoing ranges are merely suggestive, as the number of variables in regard to an individual treatment regime is large, and considerable excursions from these recommended values are not uncommon. Such dosages is altered depending on a number of variables, not limited to the activity of the compound used, the disease or condition to be treated, the mode of administration, the requirements of the individual subject, the severity of the disease or condition being treated, and the judgment of the practitioner.

In some embodiments, toxicity and therapeutic efficacy of such therapeutic regimens are determined by standard pharmaceutical procedures in cell cultures or experimental animals, including, but not limited to, the determination of the LD50 (the dose lethal to 50% of the population) and the ED50 (the dose therapeutically effective in 50% of the population). The dose ratio between the toxic and therapeutic effects is the therapeutic index and it is expressed as the ratio between LD50 and ED50. Compounds exhibiting high therapeutic indices are preferred. The data obtained from cell culture assays and animal studies are used in formulating a range of dosage for use in human. The dosage of such compounds lies preferably within a range of circulating concentrations that include the ED50 with minimal toxicity. The dosage varies within this range depending upon the dosage form employed and the route of administration utilized.

### Kits/Article of Manufacture

Disclosed herein, in certain embodiments, are kits and articles of manufacture for use with one or more of the compositions and methods described herein. Such kits include a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. In one embodiment, the containers are formed from a variety of materials such as glass or plastic.

The articles of manufacture provided herein contain packaging materials. Examples of pharmaceutical packaging materials include, but are not limited to, blister packs, bottles, tubes, bags, containers, bottles, and any packaging material suitable for a selected formulation and intended mode of administration and treatment.

For example, the container(s) include target nucleic acid molecule described herein. Such kits optionally include an identifying description or label or instructions relating to its use in the methods described herein.

A kit typically includes labels listing contents and/or instructions for use, and package inserts with instructions for use. A set of instructions will also typically be included.

In one embodiment, a label is on or associated with the container. In one embodiment, a label is on a container when letters, numbers or other characters forming the label are attached, molded or etched into the container itself; a label is associated with a container when it is present within a receptacle or carrier that also holds the container, e.g., as a package insert. In one embodiment, a label is used to indicate that the contents are to be used for a specific therapeutic application. The label also indicates directions for use of the contents, such as in the methods described herein.

In certain embodiments, the pharmaceutical compositions are presented in a pack or dispenser device which contains one or more unit dosage forms containing a compound provided herein. The pack, for example, contains metal or plastic foil, such as a blister pack. In one embodiment, the pack or dispenser device is accompanied by instructions for administration. In one embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved product insert. In one embodiment, compositions containing a compound provided herein formulated in a compatible pharmaceutical carrier are also prepared, placed in an appropriate container, and labeled for treatment of an indicated condition.

### Certain Terminology

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. Hence "about 5 µL" means "about 5 µL" and also "5 µL." Generally, the term "about" includes an amount that would be expected to be within experimental error.

The section headings used herein are for organizational purposes only and are not to be construed as limiting the subject matter described.

As used herein, the terms "individual(s)", "subject(s)" and "patient(s)" mean any mammal. In some embodiments, the mammal is a human. In some embodiments, the mammal is a non-human. None of the terms require or are limited to situations characterized by the supervision (e.g. constant or intermittent) of a health care worker (e.g. a doctor, a registered nurse, a nurse practitioner, a physician's assistant, an orderly or a hospice worker).

### EXAMPLES

These examples are provided for illustrative purposes only and not to limit the scope of the claims provided herein.

### Example 1. Antisense oligonucleotide sequences and synthesis

Phosphorodiamidate morpholino oligomers (PMO), phosphorothioate antisense oligonucleotides (PS ASO), and antisense oligonucleotides (ASOs) were synthesized.

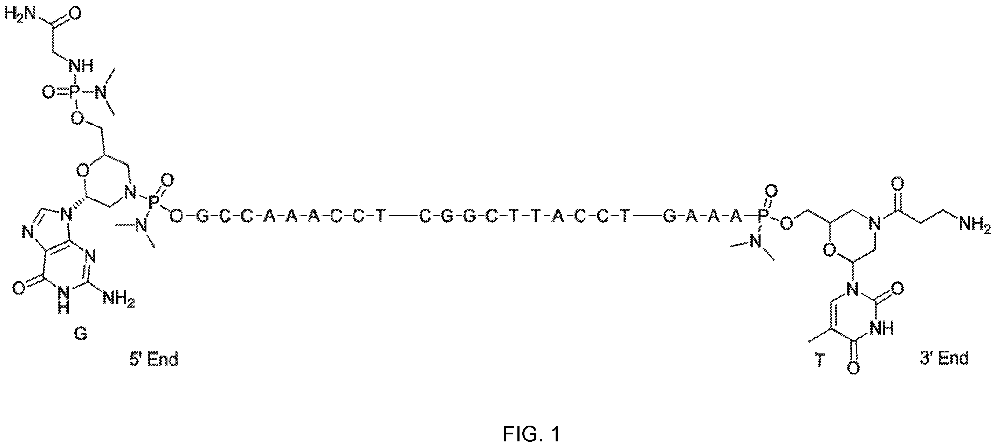

The PMO sequence was 5'GGCCAAACCTCGGCTTACCTGAAAT3' Primary amine (SEQ ID NO: 28) and can be seen in **Fig. 1** with end nucleotides expanded. The PMO contains a C3-NH₂ conjugation handle at the 3' end of the molecule for conjugation. PMOs were fully assembled on solid phase using standard solid phase synthesis protocols and purified over HPLC.

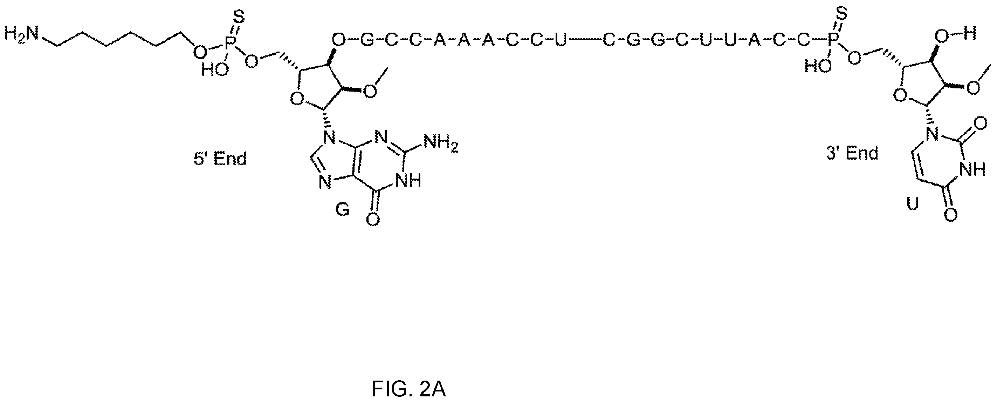

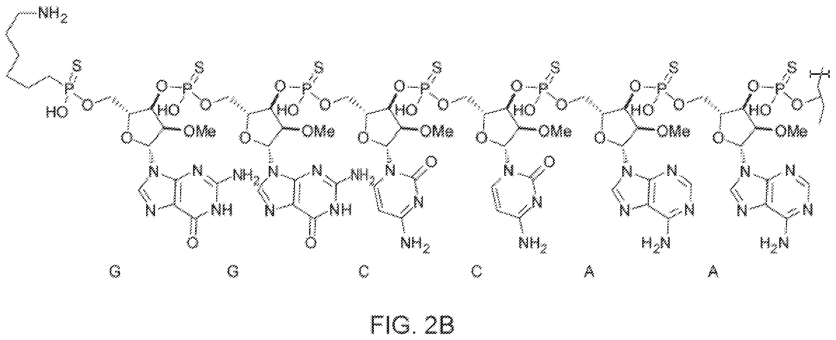

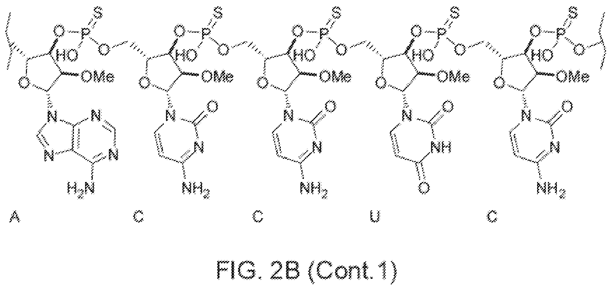

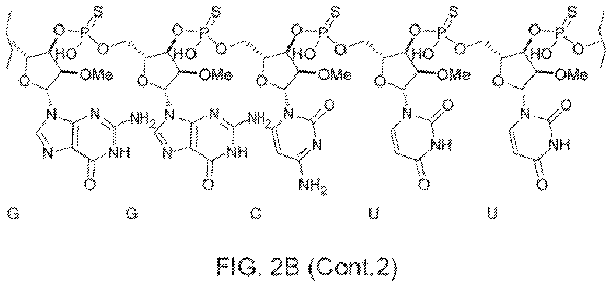

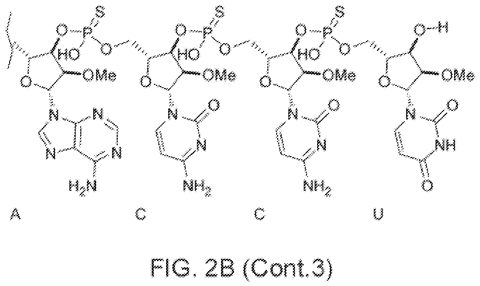

The PS ASO sequence was Amine-C6-GGCCAAACCUCGGCUUACCU (SEQ ID NO: 29) and can be seen in **Figs. 2A-2B** with end nucleotides expanded. The structure of the PS ASO comprised a phosphate backbone that was 100% phosphorothioate linkages and all the ribose sugars contained a 2' 2'OMe modification. The PS ASO also contained a C6-NH₂ conjugation handle at the 5' end of the molecule for conjugation. The PS ASOs were fully assembled on the solid phase using standard solid phase phosphoramidite chemistry and purified over HPLC.

ASOs were fully assembled on the solid phase using standard solid phase phosphoramidite chemistry and purified over HPLC. ASOs contained a C6-NH₂ conjugation handle at the 5' end of the molecule for conjugation.

### Example 2. Detection of DMD exon skipping

### Methods for Determining DMD Exon 23 Skipping in Differentiated C1C12 Cells

Mouse myoblast C2C12 cells were plated at 50,000-100,000/well in 24-well plates in 0.5 mL 10% FBS RPMI 1640 media and incubated at 37 °C with 5% CO₂ overnight. On the second day, cells were switched to differentiation media (2% horse serum RPMI 1640 and 1 µM insulin) and incubated for 3-5 days. Following incubation, samples were added and incubated for 24 hours. After the sample treatment, 1 mL of fresh media (with no compounds) was changed every day for 2 more days. At 72 hours after the start of treatments, cells were harvested. RNAs were isolated using InviTrap RNA Cell HTS 96 Kit (B-Bridge International #7061300400) and reverse transcribed using High Capacity cDNA Reverse transcription Kit (ThermoFisher #4368813). PCR reactions were performed using DreamTaqTM PCR Mastermix (ThermoFisher #K1072). The primary PCR used primers in exon 20 (Ex20F 5'-CAGAATTCTGCCAATTGCTGAG) (SEQ ID NO: 30) and exon 26 (Ex26R 5'-TTCTTCAGCTTGTGTCATCC) (SEQ ID NO: 31) to amplify both skipped and unskipped molecules using the protocol in **Table 2.**

**Table 2. PCR Protocol**

| | |
|---|---|
| Hot Start | 95 °C for 2 minutes |
| Denaturation | 95 °C for 0.5 minute |
| Annealing of primers | 50°C for 0.5 minute |
| Primer extension | 72°C for 1 minute |
| Final extension | 72°C for 5 minutes |
| Number of Cycles | 10 |

For the nested PCR, primary PCR reactions were diluted with water 100X, and 5 µl was used for nested PCR reaction (50 µl total reaction volume). Nested PCR used primers in exon 20 (Ex20F2: 5'-ACCCAGTCTACCACCCTATC) (SEQ ID NO: 32) and exon 25 (Ex25R: 5'-CTCTTTATCTTCTGCCCACCTT) (SEQ ID NO: 33) to amplify both skipped and unskipped molecules using the protocol in Table 3.

**Table 3. Nested PCR Protocol**

| | |
|---|---|
| Hot Start | 95 °C for 2 minutes |
| Denaturation | 95 °C for 0.5 minute |
| Annealing of primers | 50°C for 0.5 minute |
| Primer extension | 72°C for 1 minute |
| Final extension | 72°C for 5 minutes |
| Number of Cycles | 35 |

PCR reactions were analyzed using 4% TAE agarose gels. The wild-type (WT) *DMD* product had an expected size of 788 base pairs and the skipped *DMD* Δ23 of 575 base pairs.

### Animals

All animal studies were conducted following protocols in accordance with the Institutional Animal Care and Use Committee (IACUC) at Explora BioLabs, which adhere to the regulations outlined in the USDA Animal Welfare Act as well as the "Guide for the Care and Use of Laboratory Animals" (National Research Council publication, 8th Ed., revised in 2011). All mice were obtained from either Charles River Laboratories or Harlan Laboratories.

### In vivo mouse model

WT CD-1 mice (4-6 weeks old) were dosed via intravenous (iv) injection with the indicated antisense conjugates (ASCs) and doses. The "naked" PMO or ASO were dosed via intramuscular injection at the indicated doses. After 4, 7, or 14 days, heart and gastrocnemius muscle tissues were harvested and snap-frozen in liquid nitrogen. RNAs were isolated with Trizol and RNeasy Plus 96 Kit (Qiagen, #74192) and reversed transcribed using High Capacity cDNA Reverse transcription Kit (ThennoFisher #4368813). Nested PCR reactions were performed as described. PCR reactions were analyzed in 4% TAE agarose gels which were quantitated by densitometry.

To confirm exon 23 skipping in treated mice, DNA fragments were isolated from the 4% agarose gels and sequenced.

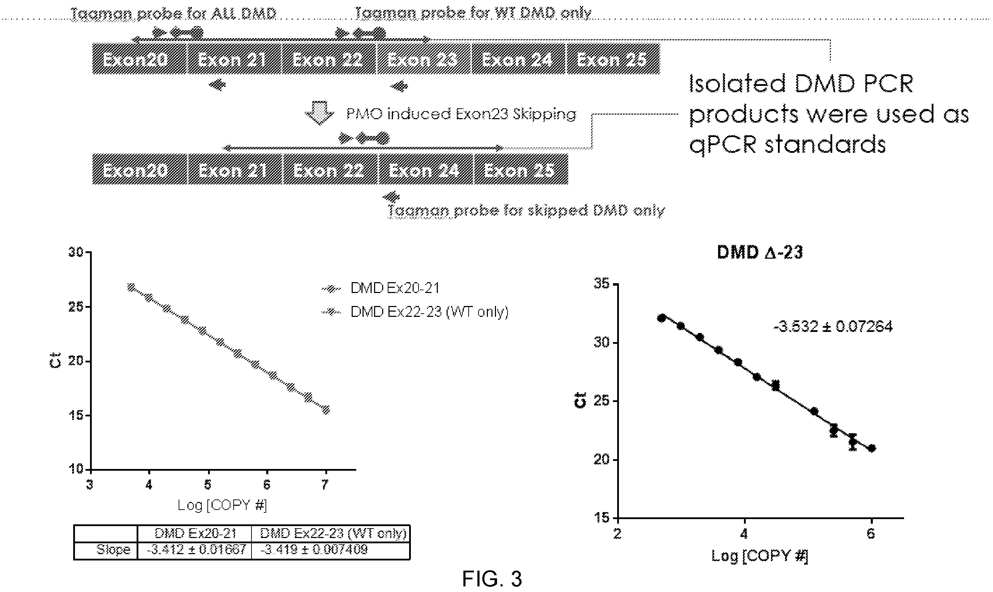

To quantitatively determine the skipped *DMD* mRNA copy number, qPCR primer/probe sets were designed to quantify skipped and WT *DMD* mRNA **(****Fig. 3****). qPCR** quantification standards were designed and produced via PCR using designed PCR primers as seen in **Table 4.** For the qPCR standard for WT and DMD, following PCR a 733 base pair fragment was isolated from the agarose gel. For qPCR standard for skipped DMA, the nested primers were used.

The amplification efficiency of the qPCR primer/probes were determined to be within 10% of expected efficiency. qPCR reactions were performed in QuantStudio 7 and TaqmanTM PCR Universal Mastermix II (ThermoFisher #4440041) according to manufacturer's instructions.

**Table 4.**

| | **SEQ ID NO** | **Primer/Probe** | **Sequence** |
|---|---|---|---|
| DMD Δ-23, for Ex23 skipping | 34 | Forward Primer | 5' GCGCTATCAGGAGACAATGAG |
| | 35 | Reverse Primer | 5' GTTTTTATGTGATTCTGTAATTTCCC |
| | 36 | Probe | 5' CTCTCTGTACCTTATCTTAGTGTT |
| DMD Ex22-23, for WT DMD only | 37 | Forward Primer | 5' TGGAGGAGAGACTCGGGAAA |
| | 38 | Reverse Primer | 5' TTGAAGCCATTTTGTTGCTCTTT |
| | 39 | Probe | 5' ACAGGCTCTGCAAAGT |
| DMD Ex20-21, for All DMD | 40 | Forward Primer | 5' AACAGATGACAACTACTGCCGAAA |
| | 41 | Reverse Primer | 5' TTGGCTCTGATAGGGTGGTAGAC |
| | 42 | Probe | 5' CTTGTTGAAAACCC |
| qPCR standard for WT and all DMD | 43 | Forward Primer | 5' TGAGGGTGTTAATGCTGAAAGTA |
| | 44 | Reverse Primer | 5' CACCAACTGGGAGGAAAGTT |

### Example 3: Conjugate Synthesis

### Analytical and Purification Methods

Analytical and purification methods were performed according to Tables 5-11.

**Table 5. Size exclusion chromatography (SEC) methods**

| **Size Exclusion Chromatography (SEC) Method** | **Column** | **Mobile Phase** | **Flow Rate** |
|---|---|---|---|
| method 1 | TOSOH Biosciences, TSKgelG3000SW XL, 7.8 X 300 mm, 5 µM | 150 mM phosphate buffer | 1.0 mL/minute for 20 minutes |
| method 2 | TOSOH Biosciences, TSKgelG3000SW, 21.5 X 600 mm, 5 µM | PBS pH 7.4 | 1.0 mL/minute for 180 minutes |

**Table 6. Hydrophobic interaction chromatography (HIC) method 1**

| **Column** | **Solvent** | **Gradient** | | |
|---|---|---|---|---|
| | | Column Volume | %A | %B |
| GE, HiScreen Butyl HP, 4.7 mL | **Solvent A**: 50 mM phosphate buffer, 0.8M Ammonium Sulfate, pH 7.0 | 1.00 | 95 | 5 |
| | | 30 | 0 | 100 |
| | **Solvent B**: 80% 50 mM phosphate buffer, 20% IPA, pH 7.0 | 5 | 0 | 100 |
| | **Flow Rate**: 1.0 mL/minute | | | |

**Table 7. Hydrophobic interaction chromatography (HIC) method 2**

| **Column** | **Solvent** | **Gradient** | | |
|---|---|---|---|---|
| | | Time | %A | %B |
| Thermo Scientific, MAbPac HIC-20, 4.6 mm ID X 10 cm, 5 um | **Solvent A:** 100 mM phosphate buffer, 1.8 M Ammonium Sulfate, pH 7.0 | 0.00 | 100 | 0 |
| | | 2.00 | 100 | 0 |
| | **Solvent B:** 80% 100 mM phosphate buffer, 20% IPA, pH 7.0 | 22.00 | 0 | 100 |
| | | 25.00 | 0 | 100 |
| | **Flow Rate:** 0.7 mL/minute | 26.00 | 100 | 0 |
| | | 30.00 | 100 | 0 |

**Table 8. Hydrophobic interaction chromatography (HIC) method 3**

| **Column** | **Solvent** | **Gradient** | | |
|---|---|---|---|---|
| | | Column Volume | %A | %B |
| GE, HiScreen Butyl HP, 4.7 mL | **Solvent A:** 50 mM phosphate buffer, 0.8 M Ammonium Sulfate, pH 7.0 | 1 | 100 | 0 |
| | | 25 | 0 | 80 |
| | **Solvent B:** 80% 50 mM phosphate buffer, 20% IPA, pH 7.0 | 1 | 0 | 100 |
| | | 2 | 0 | 100 |
| | **Flow Rate:** 1.0 mL/minute | | | |

**Table 9. Hydrophobic interaction chromatography (HIC) method 4**

| **Column** | **Solvent** | **Gradient** | | |
|---|---|---|---|---|
| | | Time | %A | %B |
| Thermo Scientific, MAbPac HIC-20, 4.6 mm ID X 10 cm, 5 um | **Solvent A:** 100 mM phosphate buffer, 1.8 M Ammonium Sulfate, pH 7.0 | 0.00 | 100 | 0 |
| | | 5.00 | 100 | 0 |
| | **Solvent B:** 80% 100 mM phosphate buffer, 20% IPA, pH 7.0 | 20.00 | 0 | 100 |
| | | 25.00 | 0 | 100 |
| | **Flow Rate:** 0.5 mL/minute | 26.00 | 100 | 0 |
| | | 30.00 | 100 | 0 |

**Table 10. Strong anion exchange chromatography (SAX) method 1**

| Column | Solvent | Gradient | | |
|---|---|---|---|---|
| | | Column Volume | %A | %B |
| Tosoh Bioscience, TSKGel SuperQ-5PW, 21.5 mm ID X 15 cm, 13 um | **Solvent** A: 20 mM TRIS buffer, pH 8.0; | 0.5 | 100 | 0 |
| | Solvent B: 20 mM TRIS, 1.5 M NaCl, pH 8.0 | 0.5 | 80 | 20 |
| | | 17 | 20 | 80 |
| | Flow Rate: 6.0 mL/minute | 0.5 | 0 | **100** |
| | | 0.5 | 0 | 100 |

**Table 11. Strong anion exchange chromatography (SAX) method 2**

| **Column** | **Solvent** | **Gradient** | | |
|---|---|---|---|---|
| | | Time | %A | %B |
| Thermo Scientific, ProPac^{™} SAX-10, Bio LC^{™}, 4 X 250 mm | **Solvent A:** 80% 10 mM TRIS pH 8, 20% ethanol | 0.0 | 90 | 10 |
| | | 3.00 | 90 | 10 |
| | **Solvent B:** 80% 10 mM TRIS pH 8, 20% ethanol, 1.5 M NaCl | 17.00 | 0 | 100 |
| | | 21.00 | 0 | 100 |
| | **Flow Rate:** 0.75 mL/minute | 22.00 | 90 | 10 |
| | | 25.00 | 90 | 10 |

### Anti-transferrin receptor antibody

Anti-mouse transferrin receptor antibody or anti-CD71 mAb that was used was a rat IgG2a subclass monoclonal antibody that binds mouse CD71 or mouse transferrin receptor 1 (mTfR1). The antibody was produced by BioXcell and it is commercially available (Catalog # BE0175).

### Anti-CD71 antibody morpholino antisense oligonucleotide conjugate (anti-CD71 mAb-PMO)

### Anti-CD71 mAb-PMO conjugation

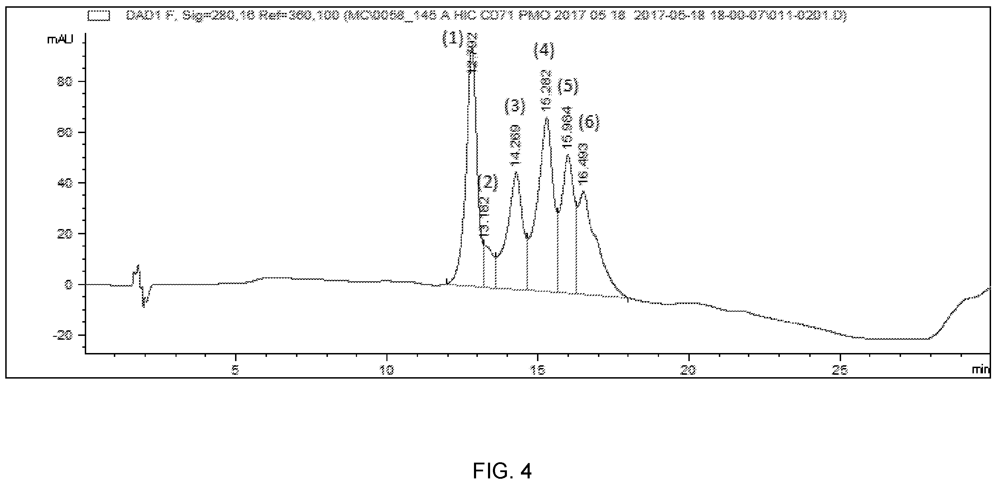

Anti-CD71 antibody (10 mg/mL) in borate buffer (25 mM sodium tetraborate, 25 mM NaCl, 1 mM Diethylene triamine pentaacetic acid, pH 8.0) was reduced by adding 4 equivalents of tris(2-carboxyethyl)phosphine (TCEP) in water and incubating at 37 °C for 4 hours. 4(N-Maleimidomethyl)cyclohexanecarboxylic acid N-hydroxysuccinimide ester (SMCC) was coupled to the primary amine on the 3' end of the phosphorodiamidate morpholino oligomer (PMO) by incubating the PMO (50 mg/mL) in DMSO with 10 equivalents of SMCC (10 mg/mL) in DMSO for one hour. Unconjugated SMCC was removed by ultrafiltration using Amicon Ultra-15 centrifugal filter units with a MWCO of 3 kDa. The PMO-SMCC was washed three times with acetate buffer (10 mM sodium acetate, pH 6.0) and used immediately. The reduced antibody was mixed with 2.25 equivalents of PMO-SMCC and incubated overnight at 4 °C. The pH of the reaction mixture was then reduced to 7.5, and 8 equivalents of N-Ethylmaleimide was added to the mixture at room temperature for 30 minutes to quench unreacted cysteines. Analysis of the reaction mixture by hydrophobic interaction chromatography (HIC) method 2 showed antibody-PMO conjugates along with unreacted antibody and PMO (Fig. 4). Fig. 4 shows a chromatogram of anti-CD71 mAb-PMO reaction mixture produced with HIC method 2 showing free antibody peak (1), free PMO (2), DAR 1 (3), DAR 2 (4), DAR 3 (5), DAR > 3 (6). "DAR" refers to a drug-to-antibody ratio. The number in parentheses refers to the peak in the chromatogram.

### Purification

The reaction mixture was purified with an AKTA Explorer FPLC using HIC method 1. Fractions containing conjugates with a drug to antibody ratio of one (DAR 1) and two (DAR 2) were combined and concentrated with Amicon Ultra-15 centrifugal filter units with a MWCO of 50 kDa separately from conjugates with a DAR greater than 2. Concentrated conjugates were buffer exchanged with PBS (pH 7.4) using Amicon Ultra-15 centrifugal filter units prior to analysis.

### Analysis of the purified conjugate

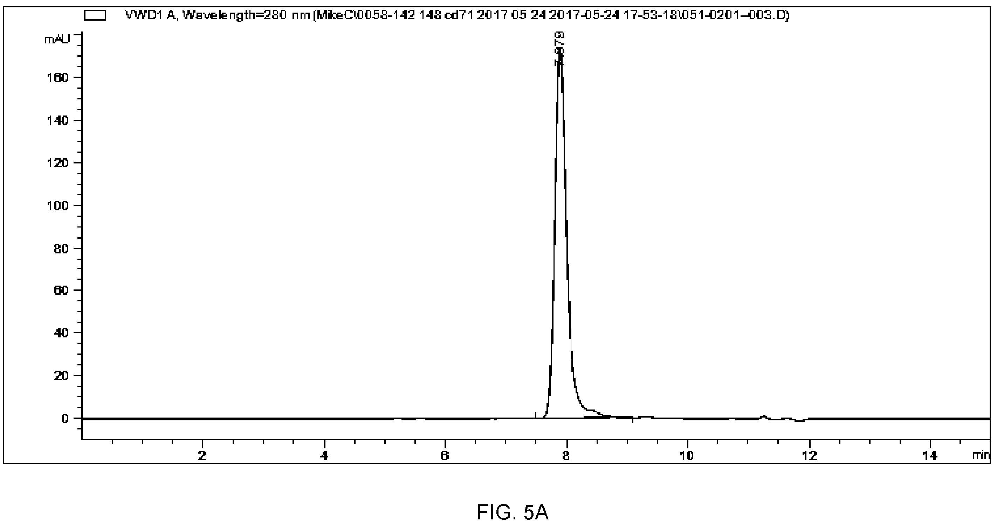

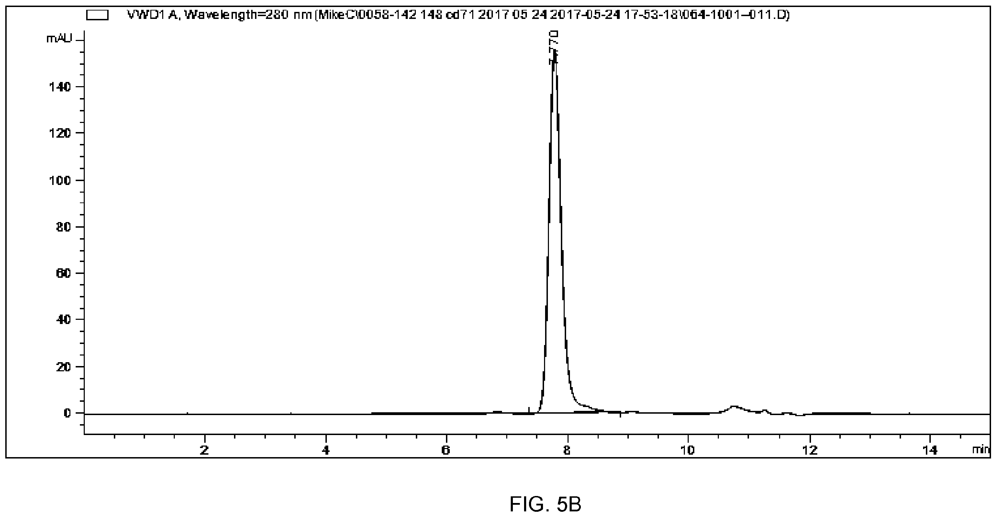

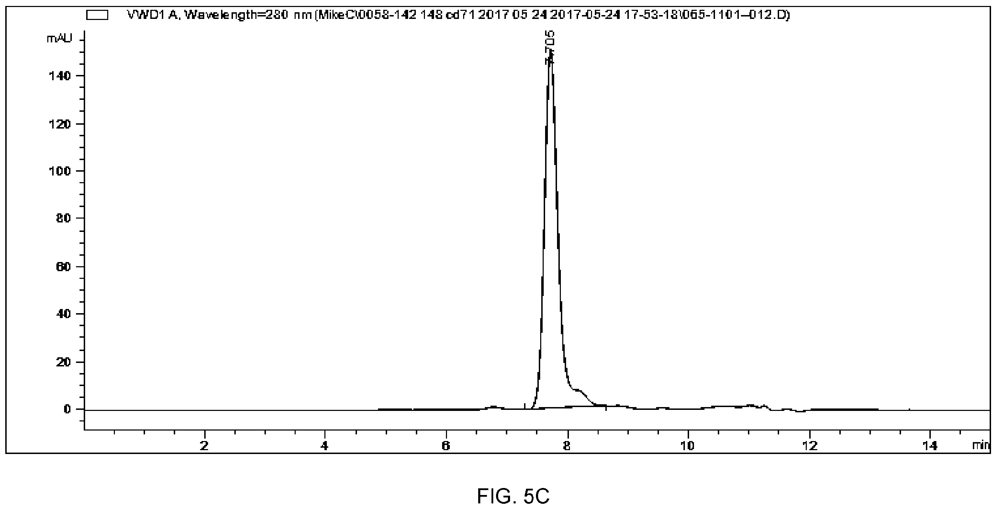

The isolated conjugates were characterized by size exclusion chromatography (SEC) and HIC. SEC method 1 was used to confirm the absence of high molecular weight aggregates and unconjugated PMOs **(****Figs. 5A-5C****).** **Fig. 5A** shows a chromatogram of anti-CD71 mAb produced using SEC method 1. **Fig. 5B** shows a chromatogram of anti-CD71 mAb-PMO DAR 1,2 produced using SEC method 1. **Fig. 5C** shows a chromatogram of anti-CD71 mAb-PMO DAR greater than 2 produced using SEC method 1. "DAR" refers to a drug-to-antibody ratio.

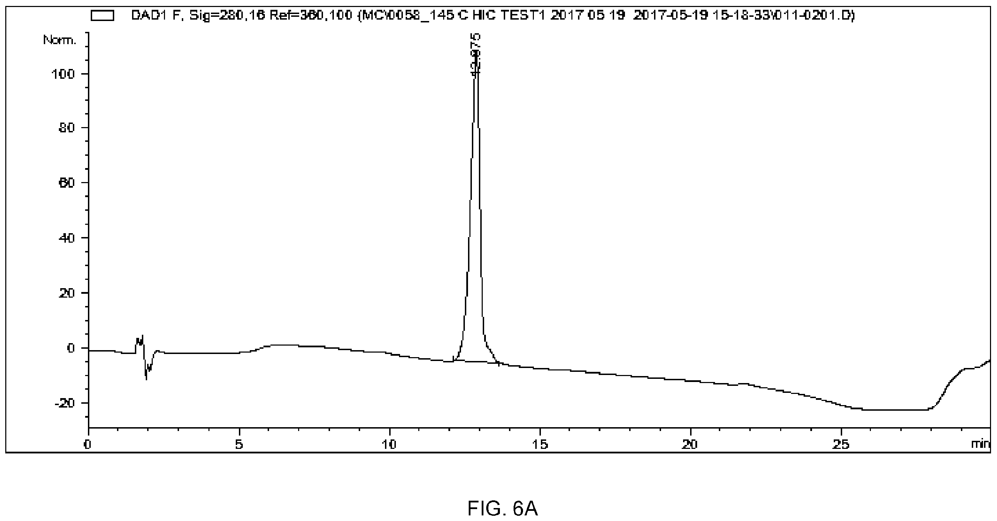

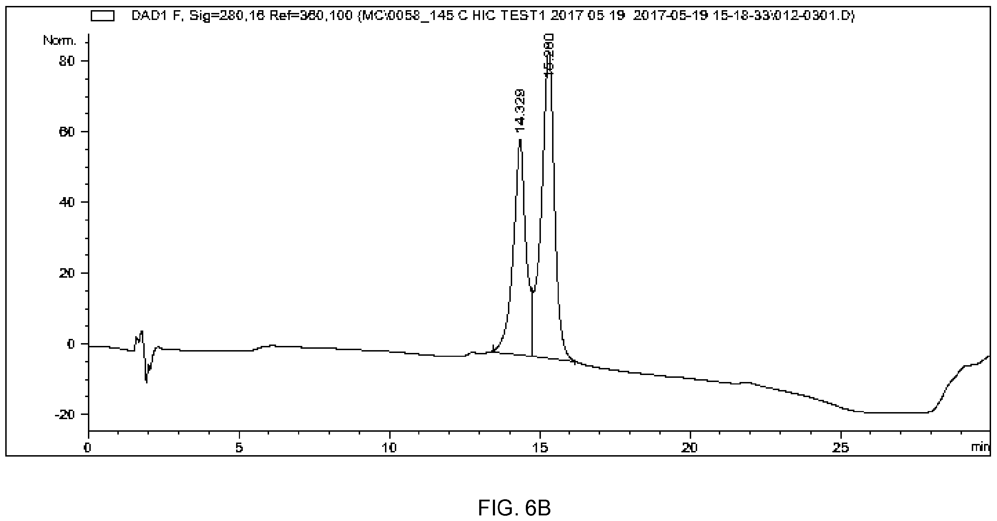

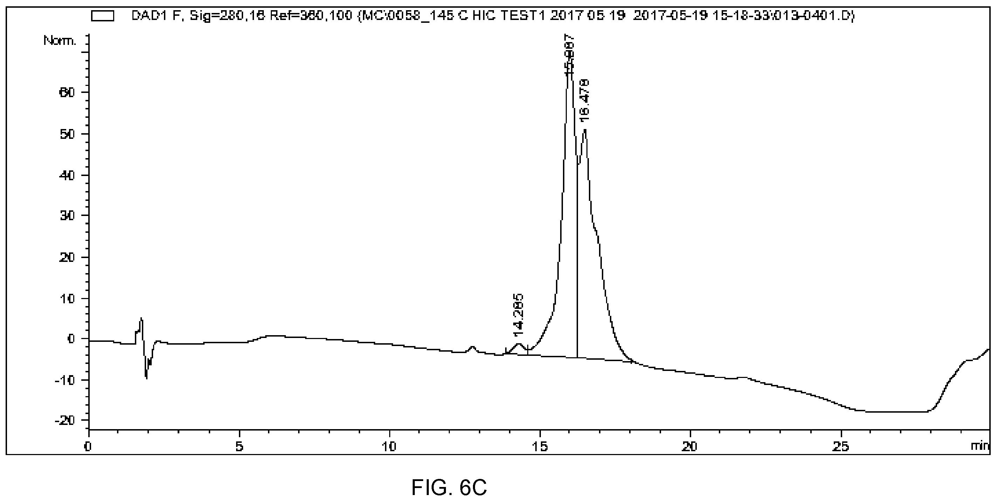

The purity of the conjugate was assessed by analytical HPLC using HIC **method 2** (Figs. **6A-****6C).** **Fig. 6A** shows a chromatogram of anti-CD71 mAb produced using HIC method 2. **Fig. 6B** shows a chromatogram of purified anti-CD71 mAb-PMO DAR 1,2 conjugate produced using HIC method 2. **Fig. 6C** shows a chromatogram of purified anti-CD71 mAb-PMO DAR >2 conjugate produced using HIC method 2. The 260/280nm UV absorbance ratio of each sample was compared to a standard curve of known ratios of PMO and antibody to confirm DAR. The DAR 1,2 sample had an average DAR of ~1.6 while the **DAR** greater than 2 sample had an average DAR of ~3.7. "DAR" refers to a drug-to-antibody ratio.

### Anti-CD71 Fab morpholino antisense oligonucleotide conjugate (anti-CD71 Fab-PMO)

### Antibody digestion with pepsin

Anti-CD71 antibody (5 mg/mL) in 20 mM acetate buffer (pH 4.0) was incubated with immobilized pepsin for 3 hours at 37 °C. The resin was removed and the reaction mixture was washed with PBS (pH 7.4) using Amicon Ultra-15 centrifugal filter units with a MWCO of 30 kDa. The retentate was collected and purified using size exclusion chromatography (SEC) method 2 to isolate the F(ab')2 fragment.

### Anti-CD71 (Fab)-PMO conjugation

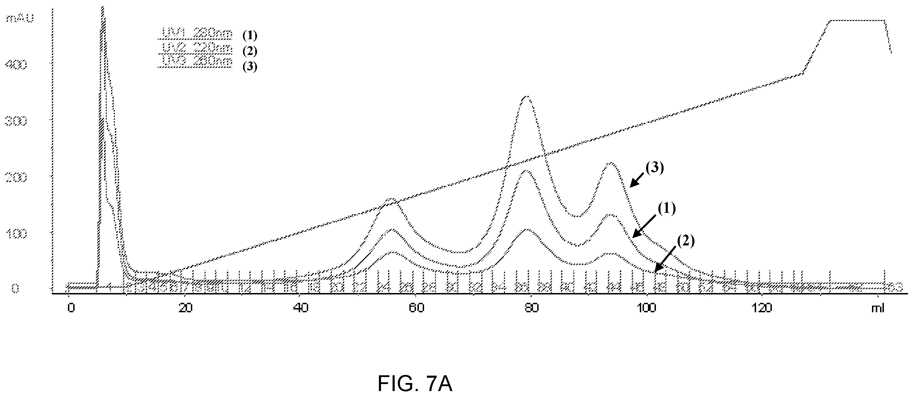

The F(ab')2 fragment (15 mg/mL) in borate buffer (pH 8.0) was reduced by adding 10 equivalents of TCEP in water and incubating at 37 °C for 2 hours. SMCC was added to the primary amine on the 3' end of the PMO by incubating the PMO (50 mg/mL) in DMSO with 10 equivalents of SMCC (10 mg/mL) in DMSO for 1 hour. Unconjugated SMCC was removed by ultrafiltration using Amicon Ultra-15 centrifugal filter units with a MWCO of 3 kDa. The PMO-SMCC was washed three times with acetate buffer (pH 6.0) and used immediately. The reduced F(ab') fragment (Fab) was buffer exchanged into borate buffer (pH 8.0) using Amicon Ultra-15 Centrifugal Filter Units with a MWCO of 10 kDa, and 1.75 equivalents of PMO-SMCC was added and incubated overnight at 4 °C. The pH of the reaction mixture was then reduced to 7.5, and 6 equivalents of N-Ethylmaleimide was added to the mixture at room temperature for 30 minutes to quench unreacted cysteines. Analysis of the reaction mixture by hydrophobic interaction chromatography (HIC) method 3 showed anti-CD71 (Fab)-PMO conjugates along with unreacted Fab **(****Fig. 7A). Fig. 7A** shows a chromatogram of FPLC purification of anti-CD71 Fab-PMO using HIC method 3.

### Purification

The reaction mixture was purified with an AKTA Explorer FPLC using HIC method 3. Fractions containing conjugates with a DAR of one, two and three were combined and concentrated separately. Concentrated conjugates were buffer exchanged with PBS (pH 7.4) using Amicon Ultra-15 centrifugal filter units with a MWCO of 10 kDa prior to analysis.

### Analysis of the purified conjugate

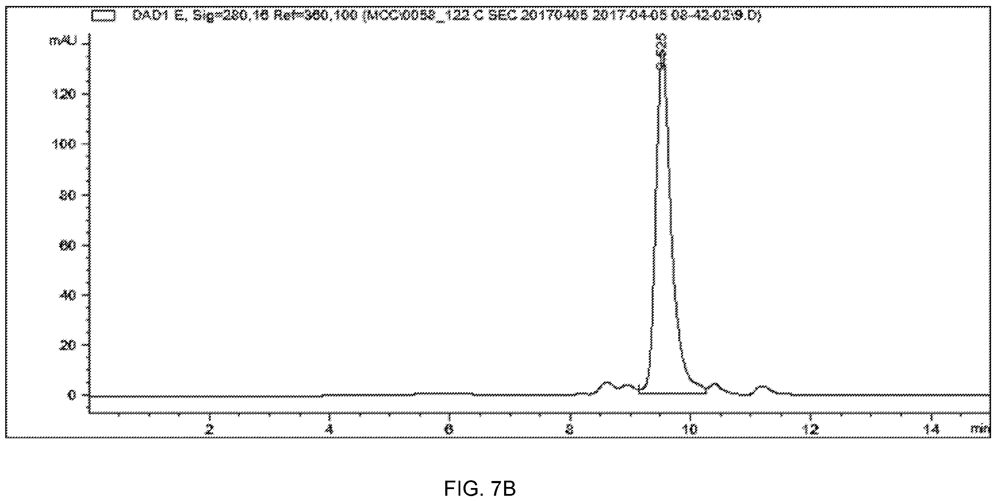

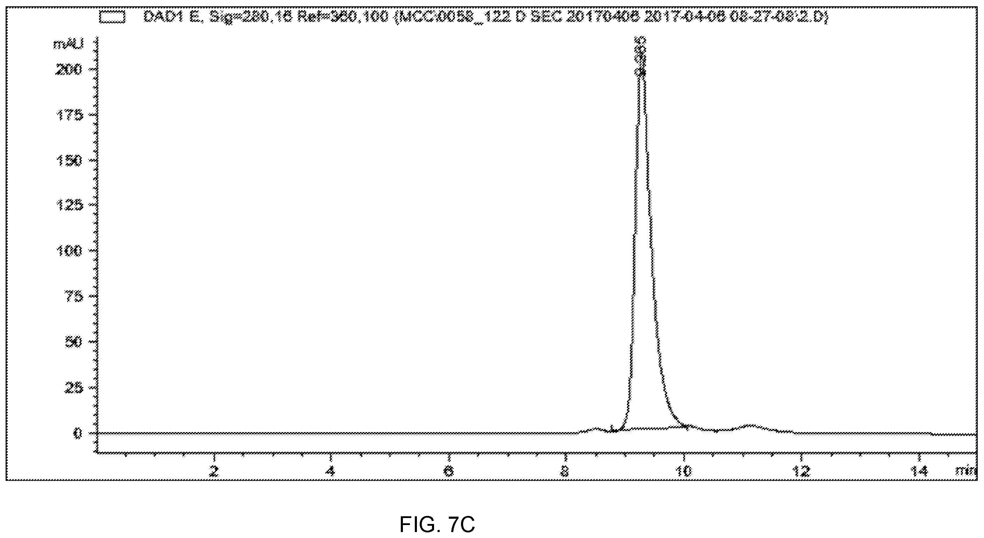

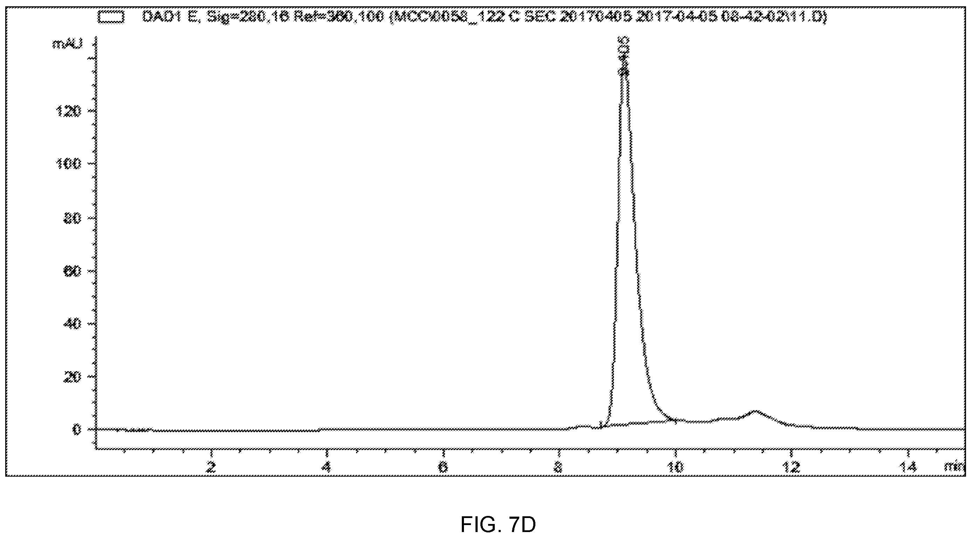

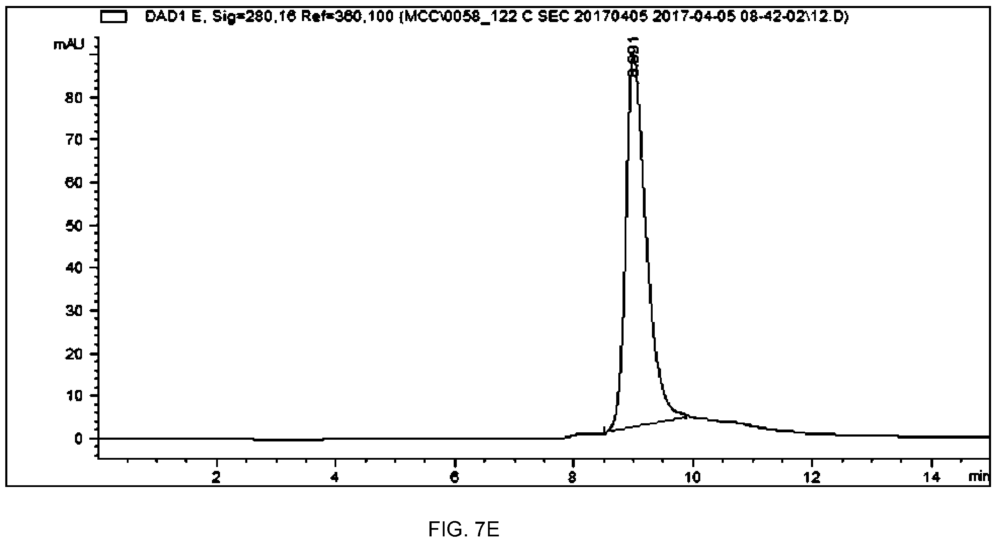

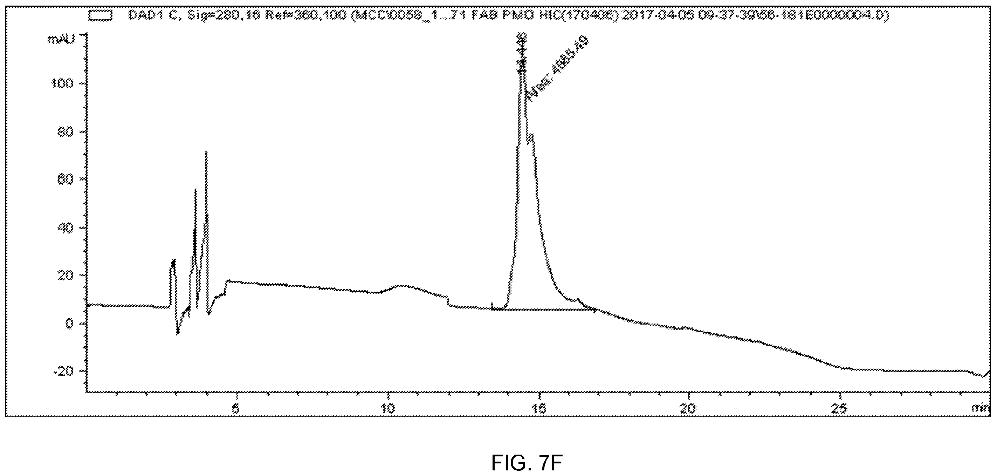

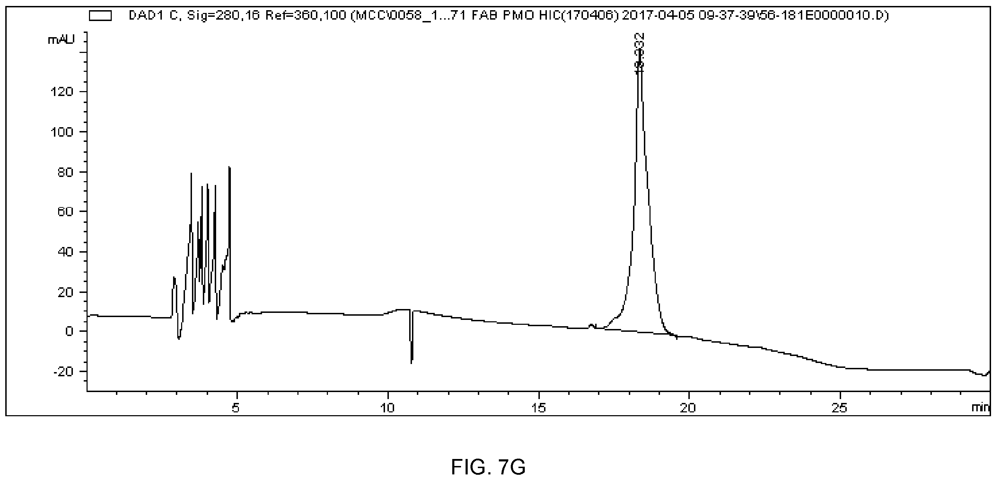

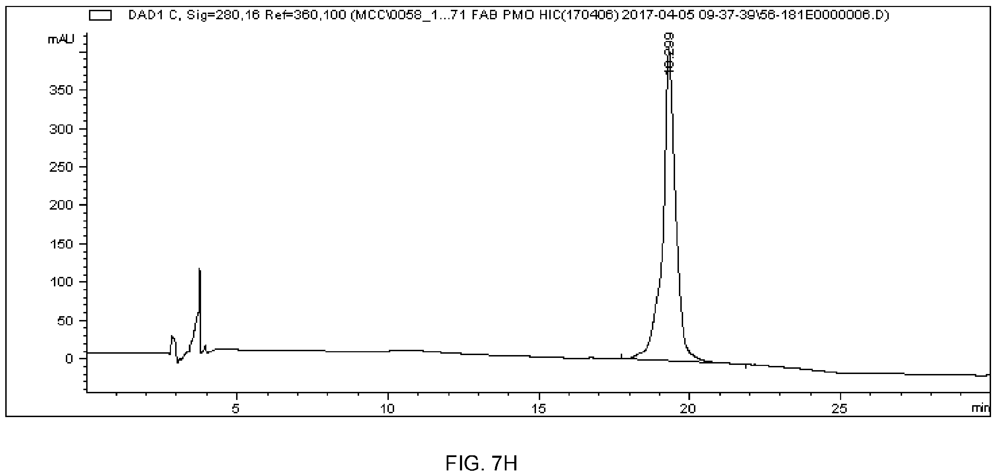

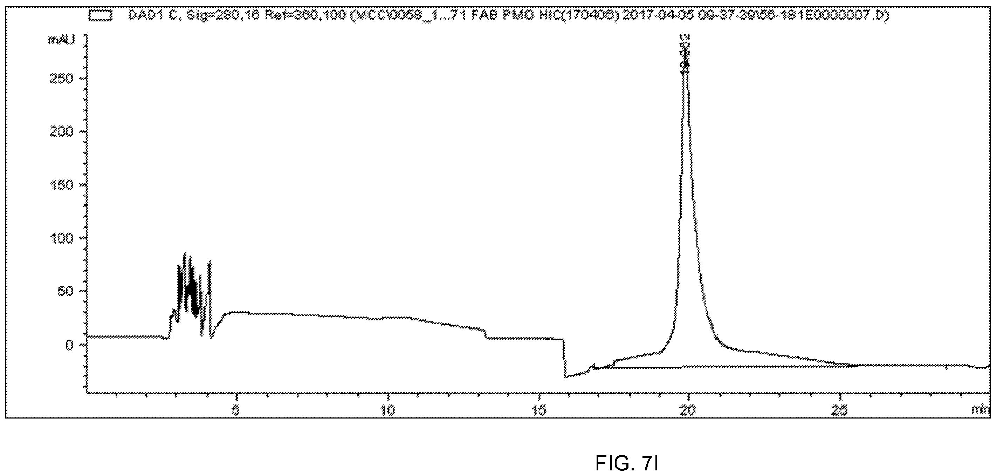

The isolated conjugates were characterized by SEC, and HIC. SEC method 1 was used to confirm the absence of high molecular weight aggregates and unconjugated PMO. *See* ***Figs. 7B-7E******.*** **Fig. 7B** shows a chromatogram of anti-CD71 Fab produced using SEC method 1. **Fig.** 7C shows a chromatogram of anti-CD71 Fab-PMO DAR 1 conjugate produced using SEC method 1. **Fig. 7D** shows a chromatogram of anti-CD71 Fab-PMO DAR 2 conjugate produced using SEC method 1. **Fig. 7E** shows a chromatogram of anti-CD71 Fab-PMO DAR 3 conjugate produced using SEC method 1. The purity of the conjugate was assessed by analytical HPLC using HIC method 4. *See* ***Figs. 7F-7I******.*** **Fig. 7F** shows a chromatogram of anti-CD71 Fab produced using HIC method 4. **Fig.** 7G shows a chromatogram of anti-CD71 Fab-PMO DAR 1 conjugate produced using HIC method 4. **Fig. 7H** shows a chromatogram of anti-CD71 Fab-PMO DAR 2 conjugate produced using HIC method 4. **Fig. 7I** shows a chromatogram of anti-CD71 Fab-PMO DAR 3 conjugate produced using HIC method 4. "DAR" refers to drug-to-antibody ratio. The 260/280nm UV absorbance ratio of each sample was compared to a standard curve of known ratios of PMO and Fab to confirm DAR.

### Anti-CD71 antibody phosphorothioate antisense oligonucleotide conjugate (anti-CD71 mAb-PS ASO)

### Anti-CD71 mAb-PS ASO

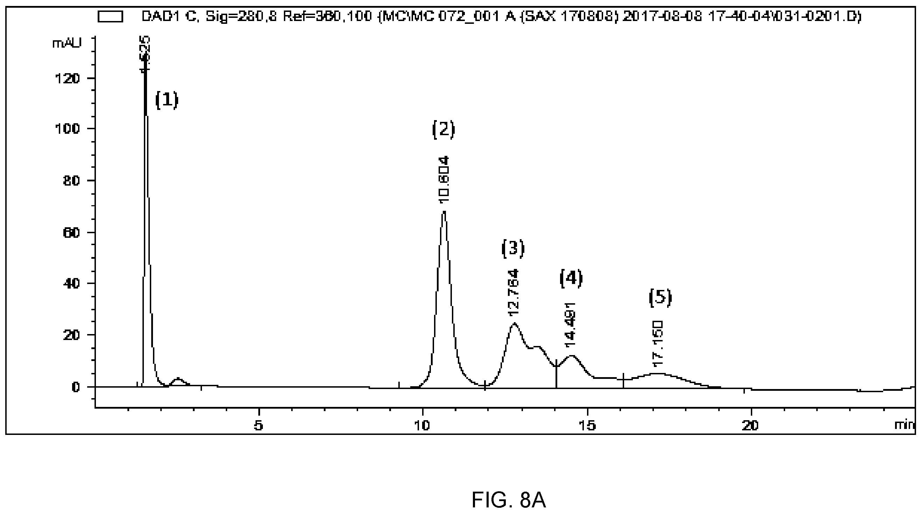

Anti-CD71 antibody (10 mg/mL) in borate buffer (pH 8.0) was reduced by adding 4 equivalents of TCEP in water and incubating at 37°C for 4 hours. 4(N-Maleimidomethyl)cyclohexanecarboxylic acid N-hydroxysuccinimide ester (SMCC) was added to the primary amine on the 5' end of the PS-ASO by incubating the PS ASO (50 mg/mL) in 1:1 mixture of 250 mM PB (pH 7.5) and DMSO with 10 equivalents of SMCC (10 mg/mL) in DMSO for 1 hour. Unconjugated SMCC was removed by ultrafiltration using Amicon Ultra-15 centrifugal filter units with a MWCO of 3 kDa. The PS ASO-SMCC was washed three times with acetate buffer (pH 6.0) and used immediately. The reduced antibody was mixed with 1.7 equivalents of PS ASO-SMCC and incubated overnight at 4°C. The pH of the reaction mixture was then reduced to 7.4, and 8 equivalents of N-Ethylmaleimide was added to the mixture at room temperature for 30 minutes to quench unreacted cysteines. Analysis of the reaction mixture by strong anion exchange chromatography (SAX) method 2 showed antibody-PS ASO conjugates along with unreacted antibody and ASO **(****Fig. 8A). Fig. 8A** shows a chromatogram of anti-CD71 mAb-PS ASO reaction mixture produced with SAX method 2 showing free antibody peak (1), free PS ASO (5), DAR 1 (2), DAR 2 (3), DAR > 2 (4). "DAR" refers to a drug-to-antibody ratio. The number in parentheses refers to the peak.

### Purification

The reaction mixture was purified with an AKTA Explorer FPLC using SAX method 1. Fractions containing conjugates with a drug-to-antibody ratio (DAR) of one, two and three were combined and concentrated separately and buffer exchanged with PBS (pH 7.4) using Amicon Ultra-15 centrifugal filter units with a MWCO of 50 kDa prior to analysis.

### Analysis of the purified conjugate

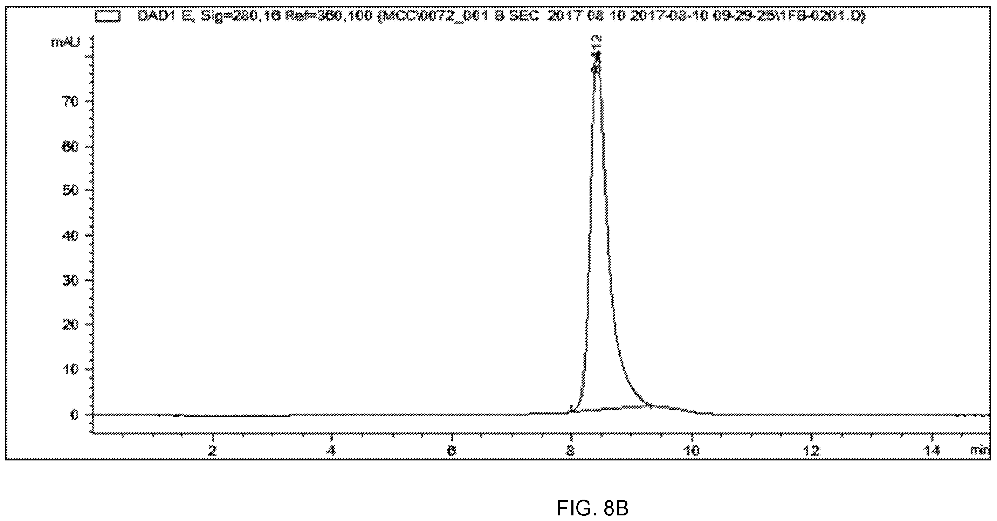

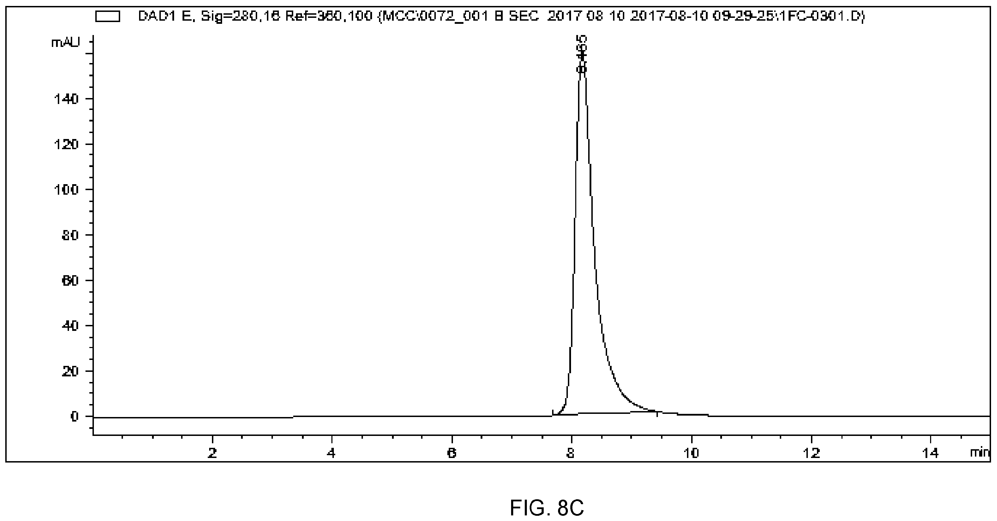

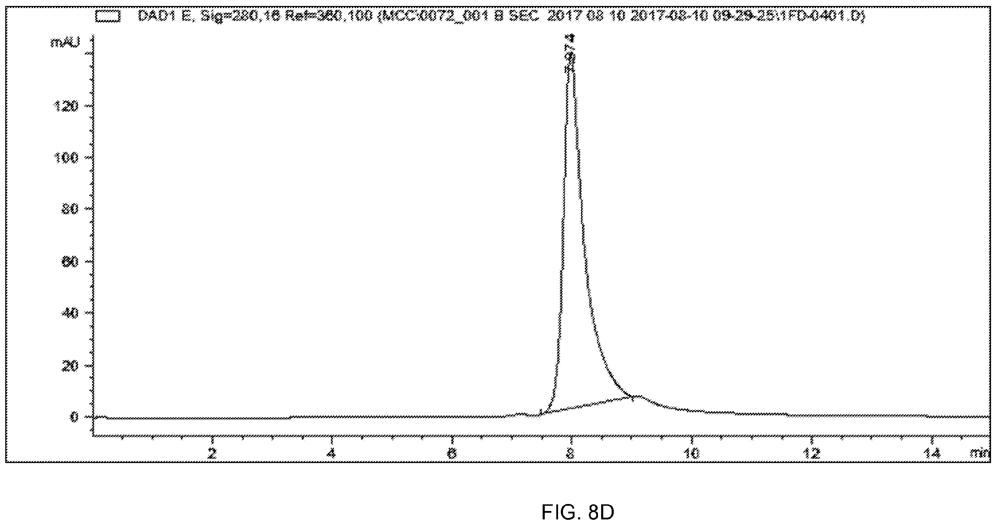

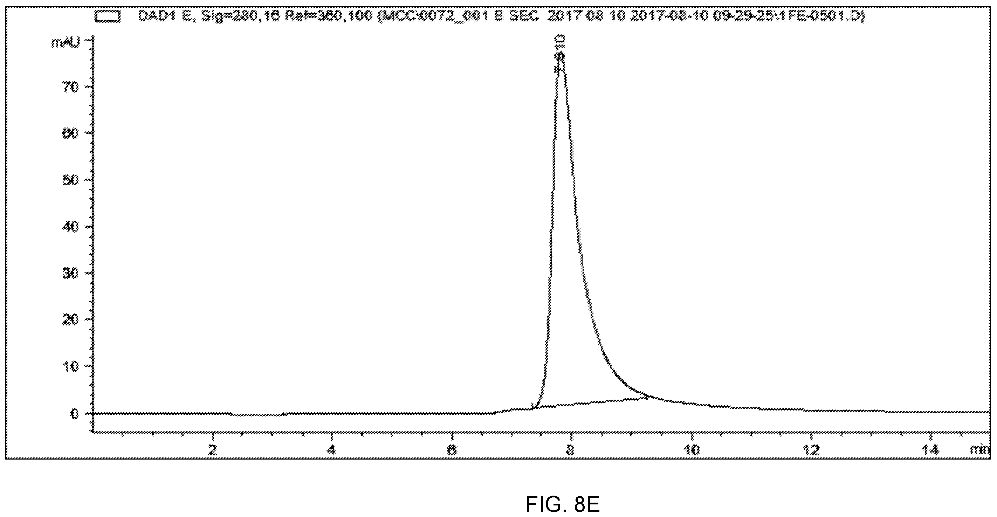

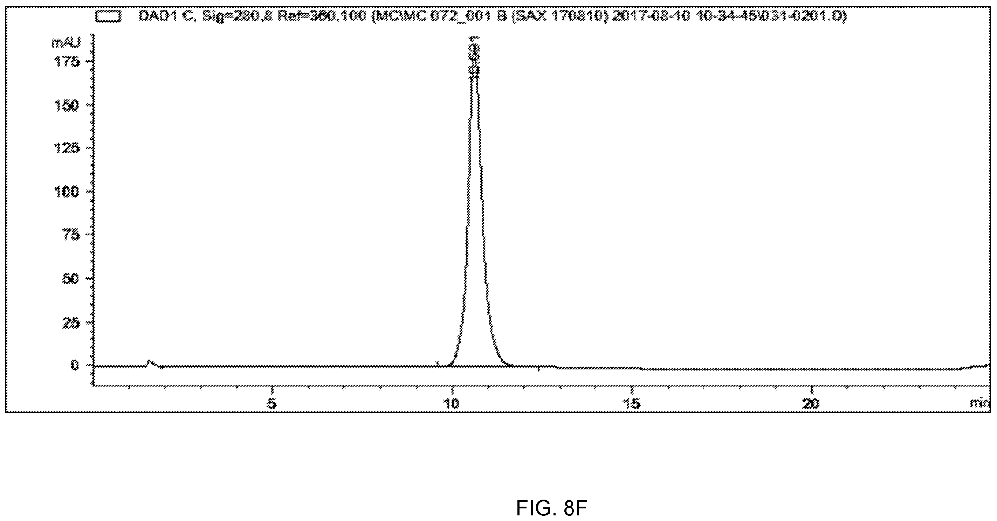

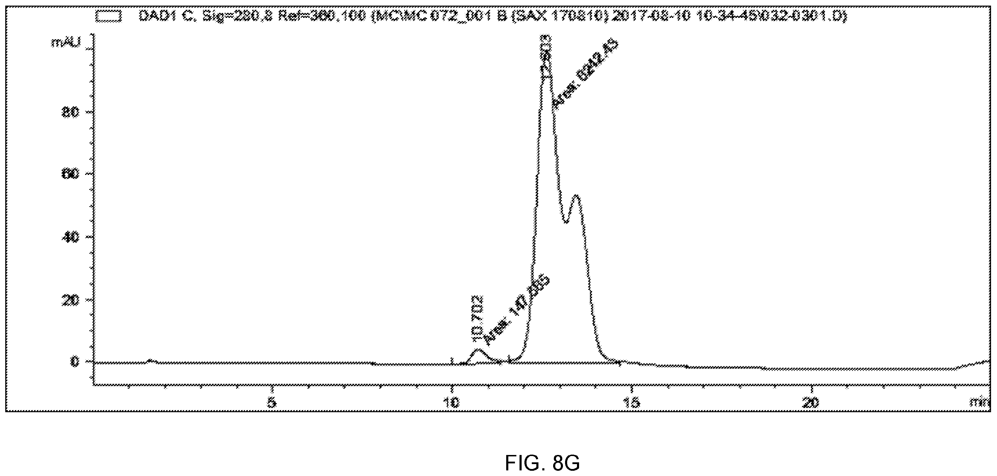

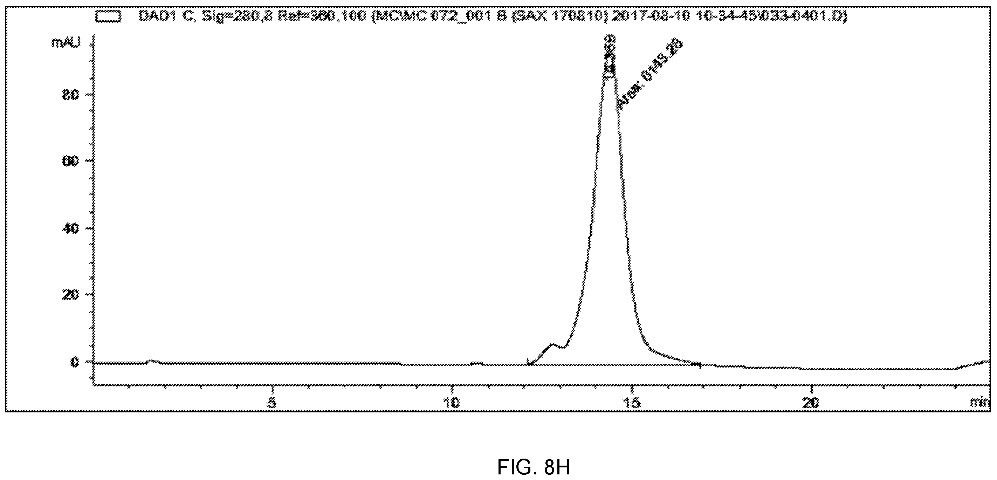

The isolated conjugates were characterized by size exclusion chromatography (SEC) and SAX. Size exclusion chromatography method 1 was used to confirm the absence of high molecular weight aggregates and unconjugated ASO. *See* *Figs. 8B-8E**.* **Fig. 8B** shows a chromatogram of anti-CD71 mAb produced using SEC method 1. **Fig. 8C** shows a chromatogram of anti-CD71 mAb-PS ASO DAR 1 conjugate produced using SEC method 1. **Fig. 8D** shows a chromatogram of anti-CD71 mAb-PS ASO DAR 2 conjugate produced using SEC method 1. **Fig. 8E** shows a chromatogram of anti-CD71 mAb-PS ASO DAR 3 conjugate produced using SEC method 1. The purity of the conjugate was assessed by analytical HPLC using SAX method 2. *See* *Figs. 8F-8H**.* Fig. **8F** shows a chromatogram of anti-CD71 mAb-PS ASO DAR 1 conjugate produced using SAX method 2. **Fig. 8G** shows a chromatogram of anti-CD71 mAb-PS ASO DAR 2 conjugate produced using SAX method 2. **Fig. 8H** shows a chromatogram of anti-CD71 mAb-PS ASO DAR 3 conjugate produced using SAX method 2. The 260/280nm UV absorbance ratio of each sample was compared to a standard curve of known ratios of ASO and antibody to confirm drug-to-antibody ratio (DAR).

### Example 4: In vitro activity of anti-CD71 mAb-PMO conjugate

The anti-CD71 mAb-PMO conjugate was made and characterized as described in Example 3. The conjugate was assessed for its ability to mediate exon skipping in vitro in differentiated C2C12 cells using nested PCR using methods similar to Example 2. Briefly, the potency of "naked" morpholino ASO ("PMO") was compared to an anti-CD71 mAb-PMO conjugate at multiple concentrations with the relevant vehicle controls. Controls included vehicle ("Veh"), scramble morpholino at 50 uM ("Scr50"), and no antibody ("Neg-Ab"). The concentrations of PMO used included 50 uM, 1 uM, and 0.02 uM. The concentrations of anti-CD71 mAB-PMO DAR 1,2 used included 200 nM, 20 nM, and 2 nM. "DAR" refers to drug-to-antibody ratio.

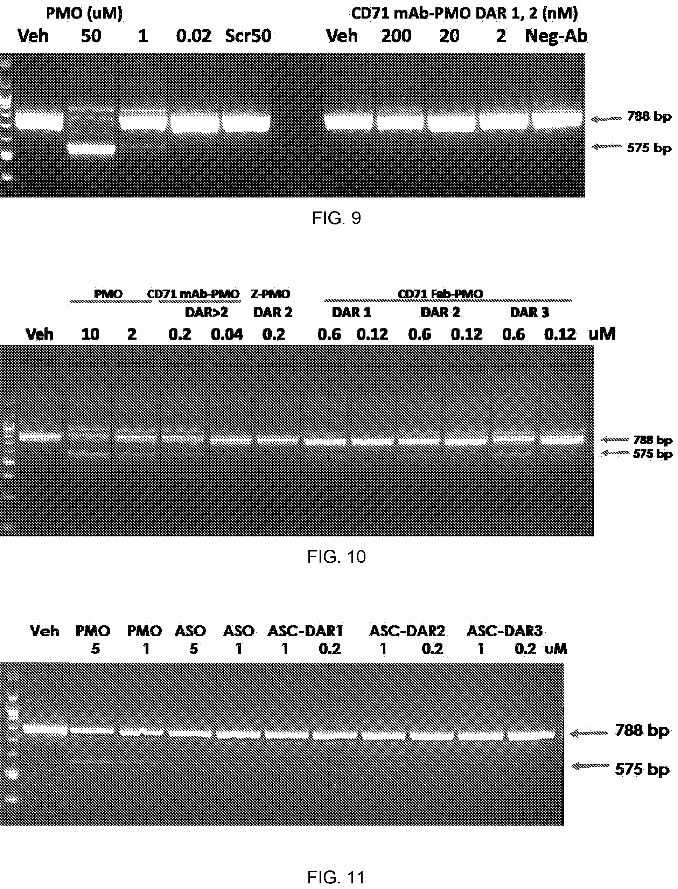

Following cDNA synthesis, two rounds of PCR amplification (primary and nested PCR) were used to detect exon-skipping. PCR reactions were analyzed in a 4% TAE agarose gel (Fig. 9).

Referring to **Fig. 9****,** anti-CD71 mAb-PMO conjugate produced measurable exon 23 skipping in differentiated C2C12 cells and lower concentrations than the "naked" PMO control. The wild-type product had an expected size of 788 base pairs and the skipped DMD Δ23 of 575 base pairs.

A second experiment included an anti-CD71 Fab-PMO conjugate and a PMO targeted with an anti-EGFR ("Z-PMO") as a negative control **(****Fig. 10****).** The concentrations of PMO used included 10 uM and 2 uM. The concentrations of anti-CD71 mAb-PMO used included 0.2 uM and 0.04 uM. Anti-CD71 mAb-PMO had a DAR of 2. Z-PMO was used at a concentration of 0.2 uM and had a DAR of 2. Concentrations of anti-CD71 Fab-PMO included 0.6 uM and 0.12 uM. DAR of 1, 2, and 3 for anti-CD71 mAb-PMO at 0.6 uM and 0.12 uM were assayed.

Referring to Fig. **10****,** Receptor mediated uptake utilizing the transferrin receptor, the anti-CD71 mAb-PMO, and anti-CD71 Fab-PMO conjugates resulted in measurable exon 23 skipping in C2C12 cells and lower concentrations than the "naked" PMO control. There was no measurable exon 23 skipping from the Z-PMO at the concentration tested, which produced skipping from the anti-CD71 conjugates.

### Example 5. In vitro activity of anti-CD71-ASO mAb PS conjugate

The anti-CD71 mAb-PS ASO conjugate was made and characterized as described in Example 3. The conjugate was assessed for its ability to mediate exon skipping in vitro in differentiated C2C12 cells using nested PCR using similar methods as described in Example 2. Briefly, the potency of "naked" phosphorothioate ASO (PS ASO) was compared to an anti-CD71 mAb-PS ASO conjugate at multiple concentrations, with the relevant vehicle control. Two rounds of of PCR amplification (primary and nested PCR) were performed following cDNA synthesis to detect exon-skipping. PCR reactions were analyzed in a 4% TAE agarose gel **(****Fig. 11). Fig. 11** shows an agarose gel of PMO, ASO, conjugated anti-CD71 mAb-ASO of DAR1 ("ASC-DAR1"), conjugated anti-CD71 mAb-ASO of DAR2 ("ASC-DAR2"), and conjugated anti-CD71 mAb-ASO of DAR3 ("ASC-DAR3"). "PMO" and "ASO" refers to free PMO and ASO, unconjugated to antibody. "Veh" refers to vehicle only. The concentrations tested included 0.2, 1, and 5 micromolar (µM).

Referring to **Fig. 11****,** the anti-CD71 mAb-PS ASO conjugate produced measurable exon 23 skipping in differentiated C2C12 cells and lower concentrations than the "naked" PS ASO control. The wild-type product had an expected size of 788 base pairs and the skipped DMD Δ23 of 575 base pairs.

### Example 6: In vivo activity of anti-CD71 mAb-PMO conjugate

The anti-CD71 mAb-PMO conjugate was made and characterized as described in Example 3. The conjugate anti-CD71 mAb-PMO DAR1,2 anti-CD71 and mAb-PMO DAR>2 were assessed for its ability to mediate exon skipping *in vivo* in wild-type CD-1 mice using similar methods as described in Example 2. "DAR" refers to drug-to-antibody ratio.

Mice were dosed via intravenous (iv) injection with the mAb, vehicle control, and antisense conjugates (ASCs) at the doses as provided in **Table 12.** "DAR" refers to drug-to-antibody ratio. The "naked" PMO was dosed via intramuscular injection into the gastrocnemius muscle at the doses provided in **Table 12.** After 4, 7, or 14 days, heart and gastrocnemius muscle tissues were harvested and snap-frozen in liquid nitrogen. RNAs were isolated, reversed transcribed and a nested PCR reactions were performed. PCR reactions were analyzed in 4% TAE agarose gels which were then quantitated by densitometry.

**Table 12. In vivo study design**

| **Group** | **Test Article** | **N** | **mAb dose (mg/kg)** | **PMO Dose (mg/kg)** | **PMO: mAb Ratio (mol/mol)** | **Harvest Time (h)** |
|---|---|---|---|---|---|---|
| 1 | anti-CD71 mAb-PMO, DAR1,2 | 3 | 50 | 4.8 | 1.6 | 96 |
| 2 | anti-CD71 mAb-PMO, DAR1,2 | 3 | 50 | 4.8 | 1.6 | 168 |
| 3 | anti-CD71 mAb-PMO, DAR1,2 | 3 | 50 | 4.8 | 1.6 | 336 |
| 4 | anti-CD71 mAb-PMO, DAR>2 | 3 | 50 | 10.5 | 3.7 | 96 |
| 5 | anti-CD71 mAb-PMO, DAR>2 | 3 | 50 | 10.5 | 3.7 | 168 |
| 6 | anti-CD71 mAb-PMO, DAR>2 | 3 | 50 | 10.5 | 3.7 | 336 |
| 7 | anti-CD71 mAb | 3 | 50 | | | 96 |
| 8 | anti-CD71 mAb | 3 | 50 | | | 168 |
| 9 | anti-CD71 mAb | 3 | 50 | | | 336 |
| 10 | PMO | 3 | 40 ug/inj. | | | 96 |
| 11 | PMO | 3 | 40 ug/inj. | | | 168 |
| 12 | PMO | 3 | 40 ug/inj. | | | 336 |
| 13 | Vehicle | 3 | | | | 96 |
| 14 | Vehicle | 3 | | | | 168 |
| 15 | Vehicle | 3 | | | | 336 |

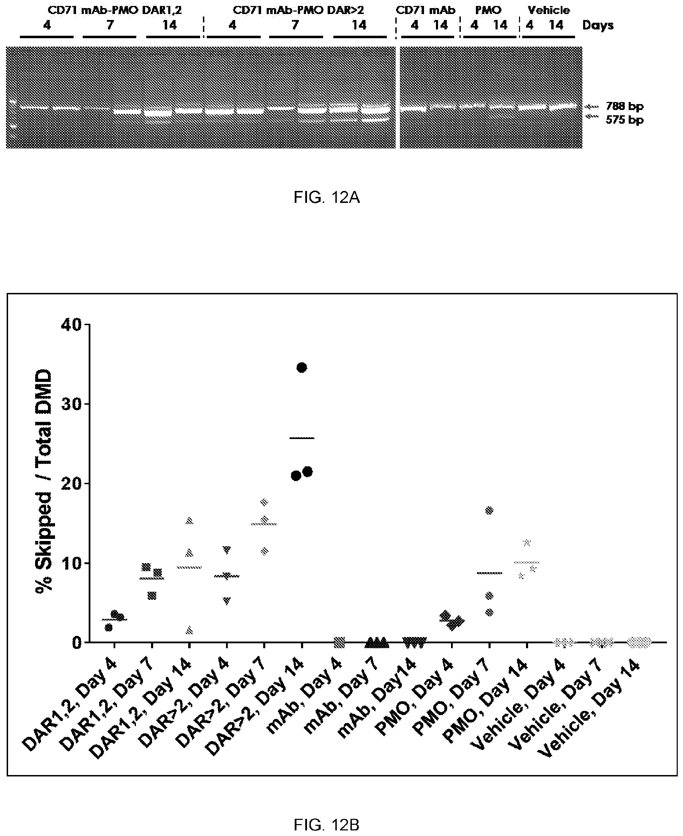

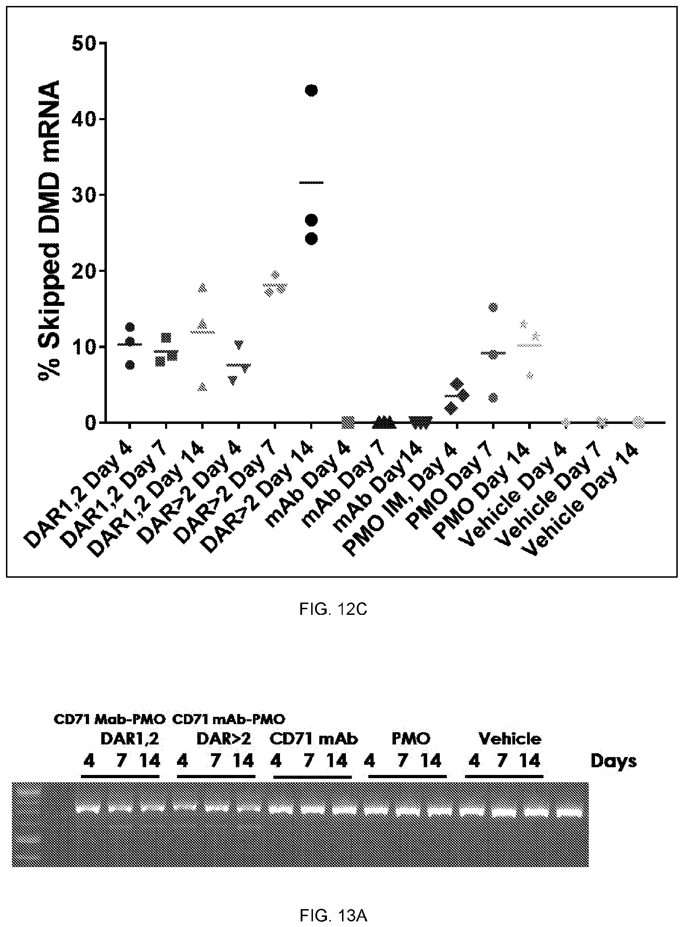

**Fig. 12A** shows a gel electrophoresis of gastrocnemius muscle samples from mice administered anti-CD71 mAb-PMO DAR 1,2, anti-CD71 mAb-PMO DAR>2, anti-CD71 mAb, PMO, and vehicle for 4, 7, or 14 days. The wild-type product had an expected size of 788 base pairs and the skipped DMD Δ23 of 575 base pairs. Anti-CD71 mAb-PMO DAR 1,2 and anti-CD71 mAb-PMO DAR>2 produced measurable exon 23 skipping in gastrocnemius muscle and lower concentrations than the "naked" PMO control. The intensity of the bands on the gel **(****FIG. 12A****)** was quantitated by densitometry as seen in **Fig. 12B****.** **Fig. 12C** shows the quantification of *in vivo* exon skipping in wild-type mice gastrocnemius muscle using Taqman qPCR.

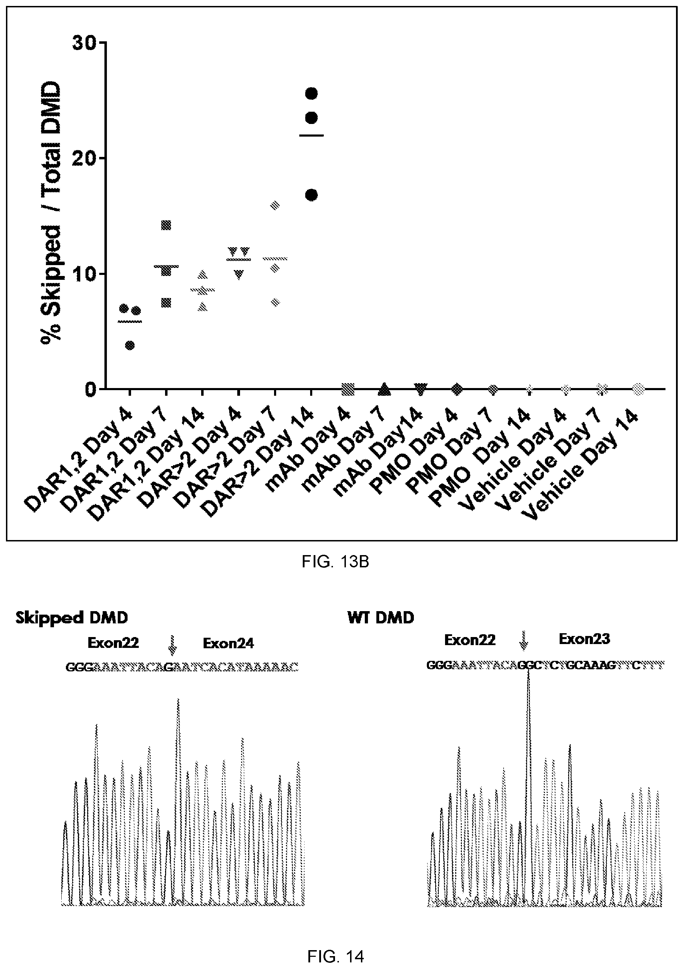

**Fig. 13A** shows a gel electrophoresis of heart samples from mice administered anti-CD71 mAb-PMO DAR 1,2, anti-CD71 mAb-PMO DAR>2, anti-CD71 mAb, PMO, and vehicle for 4, 7, or 14 days. The wild-type product had an expected size of 788 base pairs and the skipped DMD Δ23 of 575 base pairs. The intensity of the bands on the gel **(****FIG. 13A****)** was quantitated by densitometry as seen in **Fig. 13B****.** Similar results as with the gastrocnemius muscle samples were obtained. Anti-CD71 mAb-PMO DAR 1,2 and anti-CD71 mAb-PMO DAR>2 produced measurable exon 23 skipping in gastrocnemius muscle and lower concentrations than the "naked" PMO control.

DNA fragments were then isolated from the 4% agarose gels and sequenced. The sequencing data confirmed the correct sequence in the skipped and wild-type products as seen in **Fig. 14****.**

### Example 7. Sequences

**Table 13** illustrates exemplary target sequences to induce insertion, deletion, duplications, or alteration in the *DMD* gene using compositions and methods as described herein. **Table 14** illustrates exemplary nucleotide sequences to induce an insertion, deletion, duplication, or alteration in the *DMD* gene using compositions and methods as described herein. **Table 15** and **Table 16** illustrate exemplary target sequences in several genes for inducing an insertion, deletion, duplications, or alteration in the gene. **Table 17** illustrates exemplary sequences, including sequences in the *DMD* gene to induce an insertion, deletion, duplication, or alteration in the gene using compositions and methods as described herein.

**Table 13.**

| **Target Exon** | **Antisense Sequence** | **SEQ ID NO.** |
|---|---|---|
| 19 | 5' GCCUGAGCUGAUCUGCUGGCAUCUUGCAGUU 3' | 45 |
| 19 or 20 | 5'GCAGAAUUCGAUCCACCGGCUGUUCAAGCCUGAGC UGAUCUGCUCGCAUCUUGCAGU3' | 46 |
| 20 | 5' CAGCAGUAGUUGUCAUCUGCUC 3' | 47 |
| 21 | 5' CACAAAGUCUGCAUCCAGGAACAUGGGUC 3' | 48 |
| 22 | 5' CUGCAAUUCCCCGAGUCUCUGC 3' | 49 |
| 51 | 5' CUCAUACCUUCUGCUUGAUGAUC 3' | 50 |
| 52 | 5' UCCAACUGGGGACGCCUCUGUUCCAAAUCC 3' | 51 |

**Table 14.**

| **Gene** | **Target Location** | **Nucleotide Sequence (5'-3')** | **SEQ ID NO.** |
|---|---|---|---|
| *DMD* | H8A(-06+18) | GAUAGGUGGUAUCAACAUCUGUAA | 52 |
| *DMD* | H8A(-03+18) | GAUAGGUGGUAUCAACAUCUG | 53 |
| *DMD* | H8A(-07+18) | GAUAGGUGGUAUCAACAUCUGUAAG | 54 |
| *DMD* | H8A(-06+14) | GGUGGUAUCAACAUCUGUAA | 55 |
| *DMD* | H8A(-10+10) | GUAUCAACAUCUGUAAGCAC | 56 |
| *DMD* | H7A(+45+67) | UGCAUGUUCCAGUCGUUGUGUGG | 57 |
| *DMD* | H7A(+02+26) | CACUAUUCCAGUCAAAUAGGUCUGG | 58 |
| *DMD* | H7D(+15-10) | AUUUACCAACCUUCAGGAUCGAGUA | 59 |
| *DMD* | H7A(-18+03) | GGCCUAAAACACAUACACAUA | 60 |
| *DMD* | C6A(-10+10) | CAUUUUUGACCUACAUGUGG | 61 |
| *DMD* | C6A(-14+06) | UUUGACCUACAUGUGGAAAG | 62 |
| *DMD* | C6A(-14+12) | UACAUUUUUGACCUACAUGUGGAAAG | 63 |
| *DMD* | C6A(-13+09) | AUUUUUGACCUACAUGGGAAAG | 64 |
| *DMD* | CH6A(+69+91) | UACGAGUUGAUUGUCGGACCCAG | 65 |
| *DMD* | C6D(+12-13) | GUGGUCUCCUUACCUAUGACUGUGG | 66 |
| *DMD* | C6D(+06-11) | GGUCUCCUUACCUAUGA | 67 |
| *DMD* | H6D(+04-21) | UGUCUCAGUAAUCUUCUUACCUAU | 68 |
| *DMD* | H6D(+18-04) | UCUUACCUAUGACUAUGGAUGAGA | 69 |
| *DMD* | H4A(+13+32) | GCAUGAACUCUUGUGGAUCC | 70 |
| *DMD* | H4D(+04-16) | CCAGGGUACUACUUACAUUA | 71 |
| *DMD* | H4D(-24-44) | AUCGUGUGUCACAGCAUCCAG | 72 |
| *DMD* | H4A(+11+40) | UGUUCAGGGCAUGAACUCUUGUGGAUCCUU | 73 |
| *DMD* | H3A(+30+60) | UAGGAGGCGCCUCCCAUCCUGUAGGUCACUG | 74 |
| *DMD* | H3A(+35+65) | AGGUCUAGGAGGCGCCUCCCAUCCUGUAGGU | 75 |
| *DMD* | H3A(+30+54) | GCGCCUCCCAUCCUGUAGGUCACUG | 76 |
| *DMD* | H3D(+46-21) | CUUCGAGGAGGUCUAGGAGGCGCCUC | 77 |
| *DMD* | H3A(+30+50) | CUCCCAUCCUGUAGGUCACUG | 78 |
| *DMD* | H3D(+19-03) | UACCAGUUUUUGCCCUGUCAGG | 79 |
| *DMD* | H3A(-06+20) | UCAAUAUGCUGCUUCCCAAACUGAAA | 80 |
| *DMD* | H3A(+37+61) | CUAGGAGGCGCCUCCCAUCCUGUAG | 81 |
| *DMD* | H5A(+20+50) | UUAUGAUUUCCAUCUACGAUGUCAGUACUUC | 82 |
| *DMD* | H5D(+25-05) | CUUACCUGCCAGUGGAGGAUUAUAUUCCAAA | 83 |
| *DMD* | H5D(+10-15) | CAUCAGGAUUCUUACCUGCCAGUGG | 84 |
| *DMD* | H5A(+10+34) | CGAUGUCAGUACUUCCAAUAUUCAC | 85 |
| *DMD* | H5D(-04-21) | ACCAUUCAUCAGGAUUCU | 86 |
| *DMD* | H5D(+16-02) | ACCUGCCAGUGGAGGAUU | 87 |
| *DMD* | H5A(-07+20) | CCAAUAUUCACUAAAUCAACCUGUUAA | 88 |
| *DMD* | H5D(+18-12) | CAGGAUUGUUACCUGCCAGUGGAGGAUUAU | 89 |
| *DMD* | H5A(+05+35) | ACGAUGUCAGUACUUCCAAUAUUCACUAAAU | 90 |
| *DMD* | H5A(+15+45) | AUUUCCAUCUACGAUGUCAGUACUUCCAAUA | 91 |
| *DMD* | H10A(-05+16) | CAGGAGCUUCCAAAUGCUGCA | 92 |
| *DMD* | H10A(-05+24) | CUUGUCUUCAGGAGCUUCCAAAUGCUGCA | 93 |
| *DMD* | H10A(+98+119) | UCCUCAGCAGAAAGAAGCCACG | 94 |
| *DMD* | H10A(+130+149) | UUAGAAAUCUCUCCUUGUGC | 95 |
| *DMD* | H10A(-33-14) | UAAAUUGGGUGUUACACAAU | 96 |
| *DMD* | H11D(+26+49) | CCCUGAGGCAUUCCCAUCUUGAAU | 97 |
| *DMD* | H11D(+11-09) | AGGACUUACUUGCUUUGUUU | 98 |
| *DMD* | H11A(+118+140) | CUUGAAUUUAGGAGAUUCAUCUG | 99 |
| *DMD* | H11A(+75+97) | CAUCUUCUGAUAAUUUUCCUGUU | 100 |
| *DMD* | H12A(+52+75) | UCUUCUGUUUUUGUUAGCCAGUCA | 101 |
| *DMD* | H12A(-10+10) | UCUAUGUAAACUGAAAAUUU | 102 |
| *DMD* | H12A(+11+30) | UUCUGGAGAUCCAUUAAAAC | 103 |
| *DMD* | H13A(+77+100) | CAGCAGUUGCGUGAUCUCCACUAG | 104 |
| *DMD* | H13A(+55+75) | UUCAUCAACUACCACCACCAU | 105 |
| *DMD* | H13D(+06-19) | CUAAGCAAAAUAAUCUGACCUUAAG | 106 |
| *DMD* | H14A(+37+64) | CUUGUAAAAGAACCCAGCGGUCUUCUGU | 107 |
| *DMD* | H14A(+14+35) | CAUCUACAGAUGUUUGCCCAUC | 108 |
| *DMD* | H14A(+51+73) | GAAGGAUGUCUUGUAAAAGAACC | 109 |
| *DMD* | H14D(-02+18) | ACCUGUUCUUCAGUAAGACG | 110 |
| *DMD* | H14D(+14-10) | CAUGACACACCUGUUCUUCAGUAA | 111 |
| *DMD* | H14A(+61+80) | CAUUUGAGAAGGAUGUCUUG | 112 |
| *DMD* | H14A(-12+12) | AUCUCCCAAUACCUGGAGAAGAGA | 113 |
| *DMD* | H15A(-12+19) | GCCAUGCACUAAAAAGGCACUGCAAGACAUU | 114 |
| *DMD* | H15A(+48+71) | UCUUUAAAGCCAGUUGUGUGAAUC | 115 |
| *DMD* | H15A(+08+28) | UUUCUGAAAGCCAUGCACUAA | 116 |
| *DMD* | H15D(+17-08) | GUACAUACGGCCAGUUUUUGAAGAC | 117 |
| *DMD* | H16A(-12+19) | CUAGAUCCGCUUUUAAAACCUGUUAAAACAA | 118 |
| *DMD* | H16A(-06+25) | UCUUUUCUAGAUCCGCUUUUAAAACCUGUUA | 119 |
| *DMD* | H16A(-06+19) | CUAGAUCCGCUUUUAAAACCUGUUA | 120 |
| *DMD* | H16A(+87+109) | CCGUCUUCUGGGUCACUGACUUA | 121 |
| *DMD* | H16A(-07+19) | CUAGAUCCGCUUUUAAAACCUGUUAA | 122 |
| *DMD* | H16A(-07+13) | CCGCUUUUAAAACCUGUUAA | 123 |
| *DMD* | H16A(+12+37) | UGGAUUGCUUUUUCUUUUCUAGAUCC | 124 |
| *DMD* | H16A(+92+116) | CAUGCUUCCGUCUUCUGGGUCACUG | 125 |
| *DMD* | H16A(+45+67) | GAUCUUGUUUGAGUGAAUACAGU | 126 |
| *DMD* | H16A(+105+126) | GUUAUCCAGCCAUGCUUCCGUC | 127 |
| *DMD* | H16D(+05-20) | UGAUAAUUGGUAUCACUAACCUGUG | 128 |
| *DMD* | H16D(+12-11) | GUAUCACUAACCUGUGCUGUAC | 129 |
| *DMD* | H19A(+35+53) | CUGCUGGCAUCUUGCAGUU | 130 |
| *DMD* | H19A(+35+65) | GCCUGAGCUGAUCUGCUGGCAUCUUGCAGUU | 131 |
| *DMD* | H20A(+44+71) | CUGGCAGAAUUCGAUCCACCGGCUGUUC | 132 |
| *DMD* | H20A(+147+168) | CAGCAGUAGUUGUCAUCUGCUC | 133 |
| *DMD* | H20A(+185+203) | UGAUGGGGUGGUGGGUUGG | 134 |
| *DMD* | H20A(-08+17) | AUCUGCAUUAACACCCUCUAGAAAG | 135 |
| *DMD* | H20A(+30+53) | CCGGCUGUUCAGUUGUUCUGAGGC | 136 |
| *DMD* | H20A(-11+17) | AUCUGCAUUAACACCCUCUAGAAAGAAA | 137 |
| *DMD* | H20D(+08-20) | GAAGGAGAAGAGAUUCUUACCUUACAAA | 138 |
| *DMD* | H20A(+44+63) | AUUCGAUCCACCGGCUGUUC | 139 |
| *DMD* | H20A(+149+168 | CAGCAGUAGUUGUCAUCUGC | 140 |
| *DMD* | H21A(-06+16) | GCCGGUUGACUUCAUCCUGUGC | 141 |
| *DMD* | H21A(+85+106) | CUGCAUCCAGGAACAUGGGUCC | 142 |
| *DMD* | H21A(+85+108) | GUCUGCAUCCAGGAACAUGGGUC | 143 |
| *DMD* | H21A(+08+31) | GUUGAAGAUCUGAUAGCCGGUUGA | 144 |
| *DMD* | H21D(+18-07) | UACUUACUGUCUGUAGCUCUUUCU | 145 |
| *DMD* | H22A(+22+45) | CACUCAUGGUCUCCUGAUAGCGCA | 146 |
| *DMD* | H22A(+125+106) | CUGCAAUUCCCCGAGUCUCUGC | 147 |
| *DMD* | H22A(+47+69) | ACUGCUGGACCCAUGUCCUGAUG | 148 |
| *DMD* | H22A(+80+101) | CUAAGUUGAGGUAUGGAGAGU | 149 |
| *DMD* | H22D(+13-11) | UAUUCACAGACCUGCAAUUCCCC | 150 |
| *DMD* | H23A(+34+59) | ACAGUGGUGCUGAGAUAGUAUAGGCC | 151 |
| *DMD* | H23A(+18+39) | UAGGCCACUUUGUUGCUCUUGC | 152 |
| *DMD* | H23A(+72+90) | UUCAGAGGGCGCUUUCUUC | 153 |
| *DMD* | H24A(+48+70) | GGGCAGGCCAUUCCUCCUUCAGA | 154 |
| *DMD* | H24A(-02+22) | UCUUCAGGGUUUGUAUGUGAUUCU | 155 |
| *DMD* | H25A(+9+36) | CUGGGCUGAAUUGUCUGAAUAUCACUG | 156 |
| *DMD* | H25A(+131+156) | CUGUUGGCACAUGUGAUCCCACUGAG | 157 |
| *DMD* | H25D(+16-08) | GUCUAUACCUGUUGGCACAUGUGA | 158 |
| *DMD* | H26A(+132+156) | UGCUUUCUGUAAUUCAUCUGGAGUU | 159 |
| *DMD* | H26A(-07+19) | CCUCCUUUCUGGCAUAGACCUUCCAC | 160 |
| *DMD* | H26A(+68+92) | UGUGUCAUCCAUUCGUGCAUCUCUG | 161 |
| *DMD* | H27A(+82+106) | UUAAGGCCUCUUGUGCUACAGGUGG | 162 |
| *DMD* | H27A(-4+19) | GGGGCUCUUCUUUAGCUCUCUGA | 163 |
| *DMD* | H27D(+19-03) | GACUUCCAAAGUCUUGCAUUUC | 164 |
| *DMD* | H28A(-05+19) | GCCAACAUGCCCAAACUUCCUAAG | 165 |
| *DMD* | H28A(+99+124) | CAGAGAUUUCCUCAGCUCCGCCAGGA | 166 |
| *DMD* | H28D(+16-05) | CUUACAUCUAGCACCUCAGAG | 167 |
| *DMD* | H29A(+57+81) | UCCGCCAUCUGUUAGGGUCUGUGCC | 168 |
| *DMD* | H29A(+18+42) | AUUUGGGUUAUCCUCUGAAUGUCGC | 169 |
| *DMD* | H29D(+17-05) | CAUACCUCUUCAUGUAGUUCCC | 170 |
| *DMD* | H30A(+122+147) | CAUUUGAGCUGCGUCCACCUUGUCUG | 171 |
| *DMD* | H30A(+25+50) | UCCUGGGCAGACUGGAUGCUCUGUUC | 172 |
| *DMD* | H30D(+19-04) | UUGCCUGGGCUUCCUGAGGCAUU | 173 |
| *DMD* | H31D(+06-18) | UUCUGAAAUAACAUAUACCUGUGC | 174 |
| *DMD* | H31D(+03-22) | UAGUUUCUGAAAUAACAUAUACCUG | 175 |
| *DMD* | H31A(+05+25) | GACUUGUCAAAUCAGAUUGGA | 176 |
| *DMD* | H31D(+04-20) | GUUUCUGAAAUAACAUAUACCUGU | 177 |
| *DMD* | H32D(+04-16) | CACCAGAAAUACAUACCACA | 178 |
| *DMD* | H32A(+151+170) | CAAUGAUUUAGCUGUGACUG | 179 |
| *DMD* | H32A(+10+32) | CGAAACUUCAUGGAGACAUCUUG | 180 |
| *DMD* | H32A(+49+73) | CUUGUAGACGCUGCUCAAAAUUGGC | 181 |
| *DMD* | H33D(+09-11) | CAUGCACACACCUUUGCUCC | 182 |
| *DMD* | H33A(+53+76) | UCUGUACAAUCUGACGUCCAGUCU | 183 |
| *DMD* | H33A(+30+56) | GUCUUUAUCACCAUUUCCACUUCAGAC | 184 |
| *DMD* | H33A(+64+88) | CCGUCUGCUUUUUCUGUACAAUCUG | 185 |
| *DMD* | H34A(+83+104) | UCCAUAUCUGUAGCUGCCAGCC | 186 |
| *DMD* | H34A(+143+165) | CCAGGCAACUUCAGAAUCCAAAU | 187 |
| *DMD* | H34A(-20+10) | UUUCUGUUACCUGAAAAGAAUUAUAAUGAA | 188 |
| *DMD* | H34A(+46+70) | CAUUCAUUUCCUUUCGCAUCUUACG | 189 |
| *DMD* | H34A(+95+120) | UGAUCUCUUUGUCAAUUCCAUAUCUG | 190 |
| *DMD* | H34D(+10-20) | UUCAGUGAUAUAGGUUUUACCUUUCCCCAG | 191 |
| *DMD* | H34A(+72+96) | CUG UAG CUG CCA GCC AUU CUG UCA AG | 192 |
| *DMD* | H35A(+141+161) | UCU UCU GCU CGG GAG GUG ACA | 193 |
| *DMD* | H35A(+116+135) | CCA GUU ACU AUU CAG AAG AC | 194 |
| *DMD* | H35A(+24+43) | UCU UCA GGU GCA CCU UCU GU | 195 |
| *DMD* | H36A(+26+50) | UGUGAUGUGGUCCACAUUCUGGUCA | 196 |
| *DMD* | H36A(-02+18) | CCAUGUGUUUCUGGUAUUCC | 197 |
| *DMD* | H37A(+26+50) | CGUGUAGAGUCCACCUUUGGGCGUA | 198 |
| *DMD* | H37A(+82+105) | UACUAAUUUCCUGCAGUGGUCACC | 199 |
| *DMD* | H37A(+134+157) | UUCUGUGUGAAAUGGCUGCAAAUC | 200 |
| *DMD* | H38A(-01+19) | CCUUCAAAGGAAUGGAGGCC | 201 |
| *DMD* | H38A(+59+83) | UGCUGAAUUUCAGCCUCCAGUGGUU | 202 |
| *DMD* | H38A(+88+112) | UGAAGUCUUCCUCUUUCAGAUUCAC | 203 |
| *DMD* | H39A(+62+85) | CUGGCUUUCUCUCAUCUGUGAUUC | 204 |
| *DMD* | H39A(+39+58) | GUUGUAAGUUGUCUCCUCUU | 205 |
| *DMD* | H39A(+102+121) | UUGUCUGUAACAGCUGCUGU | 206 |
| *DMD* | H39D(+10-10) | GCUCUAAUACCUUGAGAGCA | 207 |
| *DMD* | H40A(-05+17) | CUUUGAGACCUCAAAUCCUGUU | 208 |
| *DMD* | H40A(+129+153) | CUUUAUUUUCCUUUCAUCUCUGGGC | 209 |
| *DMD* | H42A(-04+23) | AUCGUUUCUUCACGGACAGUGUGCUGG | 210 |
| *DMD* | H42A(+86+109) | GGGCUUGUGAGACAUGAGUGAUUU | 211 |
| *DMD* | H42D(+19-02) | ACCUUCAGAGGACUCCUCUUGC | 212 |
| *DMD* | H43D(+10-15) | UAUGUGUUACCUACCCUUGUCGGUC | 213 |
| *DMD* | H43A(+101+120) | GGAGAGAGCUUCCUGUAGCU | 214 |
| *DMD* | H43A(+78+100) | UCACCCUUUCCACAGGCGUUGCA | 215 |
| *DMD* | H44A(+85+104) | UUUGUGUCUUUCUGAGAAAC | 216 |
| *DMD* | H44D(+10-10) | AAAGACUUACCUUAAGAUAC | 217 |
| *DMD* | H44A(-06+14) | AUCUGUCAAAUCGCCUGCAG | 218 |
| *DMD* | H46D(+16-04) | UUACCUUGACUUGCUCAAGC | 219 |
| *DMD* | H46A(+90+109) | UCCAGGUUCAAGUGGGAUAC | 220 |
| *DMD* | H47A(+76+100) | GCUCUUCUGGGCUUAUGGGAGCACU | 221 |
| *DMD* | H47D(+25-02) | ACCUUUAUCCACUGGAGAUUUGUCUGC | 222 |
| *DMD* | H47A(-9+12) | UUCCACCAGUAACUGAAACAG | 223 |
| *DMD* | H50A(+02+30) | CCACUCAGAGCUCAGAUCUUCUAACUUCC | 224 |
| *DMD* | H50A(+07+33) | CUUCCACUCAGAGCUCAGAUCUUCUAA | 225 |
| *DMD* | H50D(+07-18) | GGGAUCCAGUAUACUUACAGGCUCC | 226 |
| *DMD* | H51A(-01+25) | ACCAGAGUAACAGUCUGAGUAGGAGC | 227 |
| *DMD* | H51D(+16-07) | CUCAUACCUUCUGCUUGAUGAUC | 228 |
| *DMD* | H51A(+111+134) | UUCUGUCCAAGCCCGGUUGAAAUC | 229 |
| *DMD* | H51A(+61+90) | ACAUCAAGGAAGAUGGCAUUUCUAGUUUGG | 230 |
| *DMD* | H51A(+66+90) | ACAUCAAGGAAGAUGGCAUUUCUAG | 231 |
| *DMD* | H51A(+66+95) | CUCCAACAUCAAGGAAGAUGGCAUUUCUAG | 232 |
| *DMD* | H51D(+08-17) | AUCAUUUUUUCUCAUACCUUCUGCU | 233 |
| *DMD* | H51A/D(+08-17) | AUCAUUUUUUCUCAUACCUUCUGCUAG | 234 |
| *DMD* | &(-15+) | GAGCUAAAA | 235 |
| *DMD* | H51A(+175+195) | CACCCACCAUCACCCUCUGUG | 236 |
| *DMD* | H51A(+199+220) | AUCAUCUCGUUGAUAUCCUCAA | 237 |
| *DMD* | H52A(-07+14) | UCCUGCAUUGUUGCCUGUAAG | 238 |
| *DMD* | H52A(+12+41) | UCCAACUGGGGACGCCUCUGUUCCAAAUCC | 239 |
| *DMD* | H52A(+17+37) | ACUGGGGACGCCUCUGUUCCA | 240 |
| *DMD* | H52A(+93+112) | CCGUAAUGAUUGUUCUAGCC | 241 |
| *DMD* | H52D(+05-15) | UGUUAAAAAACUUACUUCGA | 242 |
| *DMD* | H53A(+45+69) | CAUUCAACUGUUGCCUCCGGUUCUG | 243 |
| *DMD* | H53A(+39+62) | CUGUUGCCUCCGGUUCUGAAGGUG | 244 |
| *DMD* | H53A(+39+69) | CAUUCAACUGUUGCCUCCGGUUCUGAAGGUG | 245 |
| *DMD* | H53D(+14-07) | UACUAACCUUGGUUUCUGUGA | 246 |
| *DMD* | H53A(+23+47) | CUGAAGGUGUUCUUGUACUUCAUCC | 247 |
| *DMD* | H53A(+150+176) | UGUAUAGGGACCCUCCUUCCAUGACUC | 248 |
| *DMD* | H53D(+20-05) | CUAACCUUGGUUUCUGUGAUUUUCU | 249 |
| *DMD* | H53D(+09-18) | GGUAUCUUUGAUACUAACCUUGGUUUC | 250 |
| *DMD* | H53A(-12+10) | AUUCUUUCAACUAGAAUAAAAG | 251 |
| *DMD* | H53A(-07+18) | GAUUCUGAAUUCUUUCAACUAGAAU | 252 |
| *DMD* | H53A(+07+26) | AUCCCACUGAUUCUGAAUUC | 253 |
| *DMD* | H53A(+124+145) | UUGGCUCUGGCCUGUCCUAAGA | 254 |
| *DMD* | H46A(+86+115) | CUCUUUUCCAGGUUCAAGUGGGAUACUAGC | 255 |
| *DMD* | H46A(+107+137) | CAAGCUUUUCUUUUAGUUGCUGCUCUUUUCC | 256 |
| *DMD* | H46A(-10+20) | UAUUCUUUUGUUCUUCUAGCCUGGAGAAAG | 257 |
| *DMD* | H46A(+50+77) | CUGCUUCCUCCAACCAUAAAACAAAUUC | 258 |
| *DMD* | H45A(-06+20) | CCAAUGCCAUCCUGGAGUUCCUGUAA | 259 |
| *DMD* | H45A(+91+110) | UCCUGUAGAAUACUGGCAUC | 260 |
| *DMD* | H45A(+125+151) | UGCAGACCUCCUGCCACCGCAGAUUCA | 261 |
| *DMD* | H45D(+ 1 6-04) | CUACCUCUUUUUUCUGUCUG | 262 |
| *DMD* | H45A(+71+90) | UGUUUUUGAGGAUUGCUGAA | 263 |

| | | | |
|---|---|---|---|
| * The first letter designates the species (e.g. H: human, M: murine, C: canine). "#" designates target *DMD* exon number. "A/D" indicates acceptor or donor splice site at the beginning and end of the exon, respectively. (x y) represents the annealing coordinates where "-" or "+" indicate intronic or exonic sequences respectively. | | | |

**Table 15.**

| **Gene** | **Nucleotide Sequence (5' - 3')** | **SEQ ID NO.** |
|---|---|---|
| Bcl-x | TGGTTCTTACCCAGCCGCCG | 264 |
| β-globin 623 | GTTATTCTTTAGAATGGTGC | 265 |
| β-globin 654 | TGCTATTACCTTAACCCAGA | 266 |
| c-myc | CTGTGCTTACCGGGTTTTCCACCTCCC | 267 |
| c-myc | ATCGTCGTGACTGTCTGTTGGAGGG | 268 |
| c-myc | GCTCACGTTGAGGGGCATCG | 269 |
| c-myc | ACGTTGAGGGGCATCGTCGC | 270 |
| c-myc | GGGGCAUCGUCGUGACUGU/CUGUUGGAGGG | 271 |
| c-myc | CGUCGUGACUGUCUGUUGGAGG | 272 |
| c-myc | CGTCGTGACTGTCTGTTGGAGG | 273 |
| c-myc | GGCAUCGUCGCGGGAGGCUGCUGGAGCG | 274 |
| c-myc | CCGCGACAUAGGACGGAGAGCAGAGCCC | 275 |
| c-myc | ACTGTGAGGGCGATCGCTGC | 276 |
| c-myc | ACGATGAGTGGCATAGTCGC | 277 |
| c-myc | GGCATCGTCGCGGGAGGCTG | 278 |
| c-myc | GGGCATCGTCGCGGGAGGCT | 279 |
| c-myc | GGGGCATCGTCGCGGGAGGC | 280 |
| c-myc | AGGGGCATCGTCGCGGGAGG | 281 |
| c-myc | GAGGGGCATCGTCGCGGGAG | 282 |
| c-myc | TGAGGGGCATCGTCGCGGGA | 283 |
| c-myc | TTGAGGGGCATCGTCGCGGG | 284 |
| c-myc | GTTGAGGGGCATCGTCGCGG | 285 |
| c-myc | CGTTGAGGGGCATCGTCGCG | 286 |
| c-myc | ACGTTGAGGGGCATCGTCGC | 287 |
| c-myc | AACGTTGAGGGGCATCGTCG | 288 |
| c-myc | TAACGTTGAGGGGCATCGTC | 289 |
| c-myc | CTAACGTTGAGGGGCATCGT | 290 |
| c-myc | GCTAACGTTGAGGGGCATCG | 291 |
| c-myc | AGCTAACGTTGAGGGGCATC | 292 |
| c-myc | AAGCTAACGTTGAGGGGCAT | 293 |
| c-myc | GAAGCTAACGTTGAGGGGCA | 294 |
| BCL-2 (rat) | CTCCGCAATGCTGAAAGGTG | 295 |
| PCNA-1 (rat) | GGCGUGCCUCAAACAUGGUGGCGG | 296 |

**Table 16.**

| **Gene** | **Target Location** | **Nucleotide Sequence (5'-3')** | **SEQ ID NO.** |
|---|---|---|---|
| Rat c-myc | 2553-79 | CTGTGCTTACCGGGTTTTCCACCTCCC | 297 |
| Rat c-myc | 4140-64 | ATCGTCGTGACTGTCTGTTGGAGGG | 298 |
| Rat c-myc | 4161-80 | GCTCACGTTGAGGGGCATCG | 299 |
| Rat CYP3A2 | 1155-74 | GGTCACTCACCGGTAGAGAA | 300 |
| Rat CYP3A2 | 1526-45 | GGGTTCCAAGTCTATAAAGG | 301 |
| Human androgen receptor exon 2 | 31-44 | TGTGTCTTTTCCAG | 302 |
| Human androgen receptor exon 2 | 45-67 | TTTGGAGACTGCCAGGGACCATG | 303 |
| Human androgen receptor exon 2 | 48-67 | CATGGTCCCTGGCAGTCTCC | 304 |
| Human androgen receptor exon 2 | 45-80 | TCAATGGGCAAAACATGGTCCCTGGCAGTCTCCAAA | 305 |
| Human androgen receptor exon 3 | 28-43 | TTTGTGTTCTCCCAG | 306 |
| Human androgen receptor exon 3 | 44-66 | GGAAACAGAAGTACCTGTGCGCC | 307 |
| Human androgen receptor exon 3 | 49-66 | GGCGCACAGGTACTTCTG | 308 |
| Human androgen receptor exon 3 | 44-79 | AATCATTTCTGCTGGCGCACAGGTACTTCTGTTTCC | 309 |
| Human HCG-β subunit | 1321-38 | CCCCTGCAGCACGCGGGT | 310 |
| Human HCG-β subunit | 1321-57 | GAGGCAGGGCCGGCAGGACCCCCTGCAGCACGCGGGT | 311 |
| Human c-myc | 4506-25 | GGCATCGTCGCGGGAGGCTG | 312 |
| Human c-myc | 4507-26 | GGGCATCGTCGCGGGAGGCT | 313 |
| Human c-myc | 4508-27 | GGGGCATCGTCGCGGGAGGC | 314 |
| Human c-myc | 4509-28 | AGGGGCATCGTCGCGGGAGG | 315 |
| Human c-myc | 4510-29 | GAGGGGCATCGTCGCGGGAG | 316 |
| Human c-myc | 4511-30 | TGAGGGGCATCGTCGCGGGA | 317 |
| Human c-myc | 4512-31 | TTGAGGGGCATCGTCGCGGG | 318 |
| Human c-myc | 4513-32 | GTTGAGGGGCATCGTCGCGG | 319 |
| Human c-myc | 4514-33 | CGTTGAGGGGCATCGTCGCG | 320 |
| Human c-myc | 4515-34 | ACGTTGAGGGGCATCGTCGC | 321 |
| Human c-myc | 4516-35 | AACGTTGAGGGGCATCGTCG | 322 |
| Human c-myc | 4517-36 | TAACGTTGAGGGGCATCGTC | 323 |
| Human c-myc | 4518-37 | CTAACGTTGAGGGGCATCGT | 324 |
| Human c-myc | 4519-38 | GCTAACGTTGAGGGGCATCG | 325 |
| Human c-myc | 4520-39 | AGCTAACGTTGAGGGGCATC | 326 |
| Human c-myc | 4521-40 | AAGCTAACGTTGAGGGGCAT | 327 |
| Human c-myc | 4522-41 | GAAGCTAACGTTGAGGGGCA | 328 |
| Human c-myc | 6656-75 | TCCTCATCTTCTTGTTCCTC | 329 |
| Human c-myc | 6656-91 | AACAACATCGATTTCTTCCTCATCTTCTTGTTCCTC | 330 |
| Human p53 | 11691-708 | CCCGGAAGGCAGTCTGGC | 331 |
| Human p53 | 11689-724 | TCCTCCATGGCAGTGACCCGGAAGGCAGTCTGGCTG | 332 |
| Human abl (ds of bcr-abl fusion point) | 376-94 | CTACTGGCCGCTGAAGGGC | 333 |
| Human abl (ds of bcr-abl fusion point) | 374-409 | GCTCAAAGTCAGATGCTACTGGCCGCTGAAGGGCTT | 334 |
| HW-1 rev | 5517-43 | TCGTCGGTCTCTCCGCTTCTTCTTGCC | 335 |
| HW-1 rev | 7885-7904 | CTCTGGTGGTGGGTAAGGGT | 336 |
| HW-1 rev | 7885-7921 | CGGGTCTGTCGGGTTCCCTCTGGTGGTGGGTAAGGGT | 337 |
| Rat c-myc | 4140-69 | GGGGCAUCGUCGUGACUGUCUGUUGGAGGG | 338 |
| Rat c-myc | 4141-62 | CGUCGUGACUGUCUGUUGGAGG | 339 |
| Rat c-myc | 4141-62 | CGTCGTGACTGTCTGTTGGAGG | 340 |
| Human c-myc | 4498-4505 | GGCAUCGUCGCGGGAGGCUG/CUGGAGCG | 341 |
| Rat c-myc | 4364-91 | CCGCGACAUAGGACGGAGAGCAGAGCCC | 342 |

**Table 17.**

| **Target** | **Nucleotide Sequence (5' - 3')** | **SEQ ID NO.** |
|---|---|---|
| Hu.DMD.Exon44.25.001 | CTGCAGGTAAAAGCATATGGATCAA | 343 |
| Hu.DMD.Exon44.25.002 | ATCGCCTGCAGGTAAAAGCATATGG | 344 |
| Hu.DMD.Exon44.25.003 | GTCAAATCGCCTGCAGGTAAAAGCA | 345 |
| Hu.DMD.Exon44.25.004 | GATCTGTCAAATCGCCTGCAGGTAA | 346 |
| Hu.DMD.Exon44.25.005 | CAACAGATCTGTCAAATCGCCTGCA | 347 |
| Hu.DMD.Exon44.25.006 | TTTCTCAACAGATCTGTCAAATCGC | 348 |
| Hu.DMD.Exon44.25.007 | CCATTTCTCAACAGATCTGTCAAAT | 349 |
| Hu.DMD.Exon44.25.008 | ATAATGAAAACGCCGCCATTTCTCA | 350 |
| Hu.DMD.Exon44.25.009 | AAATATCTTTATATCATAATGAAAA | 351 |
| Hu.DMD.Exon44.25.010 | TGTTAGCCACTGATTAAATATCTTT | 352 |
| Hu.DMD.Exon44.25.011 | AAACTGTTCAGCTTCTGTTAGCCAC | 353 |
| Hu.DMD.Exon44.25.012 | TTGTGTCTTTCTGAGAAACTGTTCA | 354 |
| Hu.DMD.Exon44.25.013 | CCAATTCTCAGGAATTTGTGTCTTT | 355 |
| Hu.DMD.Exon44.25.014 | GTATTTAGCATGTTCCCAATTCTCA | 356 |
| Hu.DMD.Exon44.25.015 | CTTAAGATACCATTTGTATTTAGCA | 357 |
| Hu.DMD.Exon44.25.016 | CTTACCTTAAGATACCATTTGTATT | 358 |
| Hu.DMD.Exon44.25.017 | AAAGACTTACCTTAAGATACCATTT | 359 |
| Hu.DMD.Exon44.25.018 | AAATCAAAGACTTACCTTAAGATAC | 360 |
| Hu.DMD.Exon44.25.019 | AAAACAAATCAAAGACTTACCTTAA | 361 |
| Hu.DMD.Exon44.25.020 | TCGAAAAAACAAATCAAAGACTTAC | 362 |
| Hu.DMD.Exon45.25.001 | CTGTAAGATACCAAAAAGGCAAAAC | 363 |
| Hu.DMD.Exon45.25.002 | CCTGTAAGATACCAAAAAGGCAAAA | 364 |
| Hu.DMD.Exon45.25.002.2 | AGTTCCTGTAAGATACCAAAAAGGC | 365 |
| Hu.DMD.Exon45.25.003 | GAGTTCCTGTAAGATACCAAAAAGG | 366 |
| Hu.DMD.Exon45.25.003.2 | CCTGGAGTTCCTGTAAGATACCAAA | 367 |
| Hu.DMD.Exon45.25.004 | TCCTGGAGTTCCTGTAAGATACCAA | 368 |
| Hu.DMD.Exon45.25.004.2 | GCCATCCTGGAGTTCCTGTAAGATA | 369 |
| Hu.DMD.Exon45.25.005 | TGCCATCCTGGAGTTCCTGTAAGAT | 370 |
| Hu.DMD.Exon45.25.005.2 | CCAATGCCATCCTGGAGTTCCTGTA | 371 |
| Hu.DMD.Exon45.25.006 | CCCAATGCCATCCTGGAGTTCCTGT | 372 |
| Hu.DMD.Exon45.25.006.2 | GCTGCCCAATGCCATCCTGGAGTTC | 373 |
| Hu.DMD.Exon45.25.007 | CGCTGCCCAATGCCATCCTGGAGTT | 374 |
| Hu.DMD.Exon45.25.008 | AACAGTTTGCCGCTGCCCAATGCCA | 375 |
| Hu.DMD.Exon45.25.008.2 | CTGACAACAGTTTGCCGCTGCCCAA | 376 |
| Hu.DMD.Exon45.25.009 | GTTGCATTCAATGTTCTGACAACAG | 377 |
| Hu.DMD.Exon45.25.010 | GCTGAATTATTTCTTCCCCAGTTGC | 378 |
| Hu.DMD.Exon45.25.010.2 | ATTATTTCTTCCCCAGTTGCATTCA | 379 |
| Hu.DMD.Exon45.25.011 | GGCATCTGTTTTTGAGGATTGCTGA | 380 |
| Hu.DMD.Exon45.25.011.2 | TTTGAGGATTGCTGAATTATTTCTT | 381 |
| Hu.DMD.Exon45.25.012 | AATTTTTCCTGTAGAATACTGGCAT | 382 |
| Hu.DMD.Exon45.25.012.2 | ATACTGGCATCTGTTTTTGAGGATT | 383 |
| Hu.DMD.Exon45.25.013 | ACCGCAGATTCAGGCTTCCCAATTT | 384 |
| Hu.DMD.Exon45.25.013.2 | AATTTTTCCTGTAGAATACTGGCAT | 385 |
| Hu.DMD.Exon45.25.014 | CTGTTTGCAGACCTCCTGCCACCGC | 386 |
| Hu.DMD.Exon45.25.014.2 | AGATTCAGGCTTCCCAATTTTTCCT | 387 |
| Hu.DMD.Exon45.25.015 | CTCTTTTTTCTGTCTGACAGCTGTT | 388 |
| Hu.DMD.Exon45.25.015.2 | ACCTCCTGCCACCGCAGATTCAGGC | 389 |
| Hu.DMD.Exon45.25.016 | CCTACCTCTTTTTTCTGTCTGACAG | 390 |
| Hu.DMD.Exon45.25.016.2 | GACAGCTGTTTGCAGACCTCCTGCC | 391 |
| Hu.DMD.Exon45.25.017 | GTCGCCCTACCTCTTTTTTCTGTCT | 392 |
| Hu.DMD.Exon45.25.018 | GATCTGTCGCCCTACCTCTTTTTTC | 393 |
| Hu.DMD.Exon45.25.019 | TATTAGATCTGTCGCCCTACCTCTT | 394 |
| Hu.DMD.Exon45.25.020 | ATTCCTATTAGATCTGTCGCCCTAC | 395 |
| Hu.DMD.Exon45.20.001 | AGATACCAAAAAGGCAAAAC | 396 |
| Hu.DMD.Exon45.20.002 | AAGATACCAAAAAGGCAAAA | 397 |
| Hu.DMD.Exon45.20.003 | CCTGTAAGATACCAAAAAGG | 398 |
| Hu.DMD.Exon45.20.004 | GAGTTCCTGTAAGATACCAA | 399 |
| Hu.DMD.Exon45.20.005 | TCCTGGAGTTCCTGTAAGAT | 400 |
| Hu.DMD.Exon45.20.006 | TGCCATCCTGGAGTTCCTGT | 401 |
| Hu.DMD.Exon45.20.007 | CCCAATGCCATCCTGGAGTT | 402 |
| Hu.DMD.Exon45.20.008 | CGCTGCCCAATGCCATCCTG | 403 |
| Hu.DMD.Exon45.20.009 | CTGACAACAGTTTGCCGCTG | 404 |
| Hu.DMD.Exon45.20.010 | GTTGCATTCAATGTTCTGAC | 405 |
| Hu.DMD.Exon45.20.011 | ATTATTTCTTCCCCAGTTGC | 406 |
| Hu.DMD.Exon45.20.012 | TTTGAGGATTGCTGAATTAT | 407 |
| Hu.DMD.Exon45.20.013 | ATACTGGCATCTGTTTTTGA | 408 |
| Hu.DMD.Exon45.20.014 | AATTTTTCCTGTAGAATACT | 409 |
| Hu.DMD.Exon45.20.015 | AGATTCAGGCTTCCCAATTT | 410 |
| Hu.DMD.Exon45.20.016 | ACCTCCTGCCACCGCAGATT | 411 |
| Hu.DMD.Exon45.20.017 | GACAGCTGTTTGCAGACCTC | 412 |
| Hu.DMD.Exon45.20.018 | CTCTTTTTTCTGTCTGACAG | 413 |
| Hu.DMD.Exon45.20.019 | CCTACCTCTTTTTTCTGTCT | 414 |
| Hu.DMD.Exon45.20.020 | GTCGCCCTACCTCTTTTTTC | 415 |
| Hu.DMD.Exon45.20.021 | GATCTGTCGCCCTACCTCTT | 416 |
| Hu.DMD.Exon45.20.022 | TATTAGATCTGTCGCCCTAC | 417 |
| Hu.DMD.Exon45.20.023 | ATTCCTATTAGATCTGTCGC | 418 |
| Hu.DMD.Exon46.25.001 | GGGGGATTTGAGAAAATAAAATTAC | 419 |
| Hu.DMD.Exon46.25.002 | ATTTGAGAAAATAAAATTACCTTGA | 420 |
| Hu.DMD.Exon46.25.002.2 | CTAGCCTGGAGAAAGAAGAATAAAA | 421 |
| Hu.DMD.Exon46.25.003 | AGAAAATAAAATTACCTTGACTTGC | 422 |
| Hu.DMD.Exon46.25.003.2 | TTCTTCTAGCCTGGAGAAAGAAGAA | 423 |
| Hu.DMD.Exon46.25.004 | ATAAAATTACCTTGACTTGCTCAAG | 424 |
| Hu.DMD.Exon46.25.004.2 | TTTTGTTCTTCTAGCCTGGAGAAAG | 425 |
| Hu.DMD.Exon46.25.005 | ATTACCTTGACTTGCTCAAGCTTTT | 426 |
| Hu.DMD.Exon46.25.005.2 | TATTCTTTTGTTCTTCTAGCCTGGA | 427 |
| Hu.DMD.Exon46.25.006 | CTTGACTTGCTCAAGCTTTTCTTTT | 428 |
| Hu.DMD.Exon46.25.006.2 | CAAGATATTCTTTTGTTCTTCTAGC | 429 |
| Hu.DMD.Exon46.25.007 | CTTTTAGTTGCTGCTCTTTTCCAGG | 430 |
| Hu.DMD.Exon46.25.008 | CCAGGTTCAAGTGGGATACTAGCAA | 431 |
| Hu.DMD.Exon46.25.008.2 | ATCTCTTTGAAATTCTGACAAGATA | 432 |
| Hu.DMD.Exon46.25.009 | AGCAATGTTATCTGCTTCCTCCAAC | 433 |
| Hu.DMD.Exon46.25.009.2 | AACAAATTCATTTAAATCTCTTTGA | 434 |
| Hu.DMD.Exon46.25.010 | CCAACCATAAAACAAATTCATTTAA | 435 |
| Hu.DMD.Exon46.25.010.2 | TTCCTCCAACCATAAAACAAATTCA | 436 |
| Hu.DMDExon46.25.011 | TTTAAATCTCTTTGAAATTCTGACA | 437 |
| Hu.DMD.Exon46.25.012 | TGACAAGATATTCTTTTGTTCTTCT | 438 |
| Hu.DMD.Exon46.25.012.2 | TTCAAGTGGGATACTAGCAATGTTA | 439 |
| Hu.DMD.Exon46.25.013 | AGATATTCTTTTGTTCTTCTAGCCT | 440 |
| Hu.DMD.Exon46.25.013.2 | CTGCTCTTTTCCAGGTTCAAGTGGG | 441 |
| Hu.DMD.Exon46.25.014 | TTCTTTTGTTCTTCTAGCCTGGAGA | 442 |
| Hu.DMD.Exon46.25.014.2 | CTTTTCTTTTAGTTGCTGCTCTTTT | 443 |
| Hu.DMD.Exon46.25.015 | TTGTTCTTCTAGCCTGGAGAAAGAA | 444 |
| Hu.DMD.Exon46.25.016 | CTTCTAGCCTGGAGAAAGAAGAATA | 445 |
| Hu.DMD.Exon46.25.017 | AGCCTGGAGAAAGAAGAATAAAATT | 446 |
| Hu.DMD.Exon46.25.018 | CTGGAGAAAGAAGAATAAAATTGTT | 447 |
| Hu.DMD.Exon46.20.001 | GAAAGAAGAATAAAATTGTT | 448 |
| Hu.DMD.Exon46.20.002 | GGAGAAAGAAGAATAAAATT | 449 |
| Hu.DMD.Exon46.20.003 | AGCCTGGAGAAAGAAGAATA | 450 |
| Hu.DMD.Exon46.20.004 | CTTCTAGCCTGGAGAAAGAA | 451 |
| Hu.DMD.Exon46.20.005 | TTGTTCTTCTAGCCTGGAGA | 452 |
| Hu.DMD.Exon46.20.006 | TTCTTTTGTTCTTCTAGCCT | 453 |
| Hu.DMD.Exon46.20.007 | TGACAAGATATTCTTTTGTT | 454 |
| Hu.DMD.Exon46.20.008 | ATCTCTTTGAAATTCTGACA | 455 |
| Hu.DMD.Exon46.20.009 | AACAAATTCATTTAAATCTC | 456 |
| Hu.DMD.Exon46.20.010 | TTCCTCCAACCATAAAACAA | 457 |
| Hu.DMD.Exon46.20.011 | AGCAATGTTATCTGCTTCCT | 458 |
| Hu.DMD.Exon46.20.012 | TTCAAGTGGGATACTAGCAA | 459 |
| Hu.DMD.Exon46.20.013 | CTGCTCTTTTCCAGGTTCAA | 460 |
| Hu.DMD.Exon46.20.014 | CTTTTCTTTTAGTTGCTGCT | 461 |
| Hu.DMD.Exon46.20.015 | CTTGACTTGCTCAAGCTTTT | 462 |
| Hu.DMD.Exon46.20.016 | ATTACCTTGACTTGCTCAAG | 463 |
| Hu.DMD.Exon46.20.017 | ATAAAATTACCTTGACTTGC | 464 |
| Hu.DMD.Exon46.20.018 | AGAAAATAAAATTACCTTGA | 465 |
| Hu.DMD.Exon46.20.019 | ATTTGAGAAAATAAAATTAC | 466 |
| Hu.DMD.Exon46.20.020 | GGGGGATTTGAGAAAATAAA | 467 |
| Hu.DMD.Exon47.25.001 | CTGAAACAGACAAATGCAACAACGT | 468 |
| Hu.DMD.Exon47.25.002 | AGTAACTGAAACAGACAAATGCAAC | 469 |
| Hu.DMD.Exon47.25.003 | CCACCAGTAACTGAAACAGACAAAT | 470 |
| Hu.DMD.Exon47.25.004 | CTCTTCCACCAGTAACTGAAACAGA | 471 |
| Hu.DMD.Exon47.25.005 | GGCAACTCTTCCACCAGTAACTGAA | 472 |
| Hu.DMD.Exon47.25.006 | GCAGGGGCAACTCTTCCACCAGTAA | 473 |
| Hu.DMD.Exon47.25.007 | CTGGCGCAGGGGCAACTCTTCCACC | 474 |
| Hu.DMD.Exon47.25.008 | TTTAATTGTTTGAGAATTCCCTGGC | 475 |
| Hu.DMD.Exon47.25.008.2 | TTGTTTGAGAATTCCCTGGCGCAGG | 476 |
| Hu.DMD.Exon47.25.009 | GCACGGGTCCTCCAGTTTCATTTAA | 477 |
| Hu.DMD.Exon47.25.009.2 | TCCAGTTTCATTTAATTGTTTGAGA | 478 |
| Hu.DMD.Exon47.25.010 | GCTTATGGGAGCACTTACAAGCACG | 479 |
| Hu.DMD.Exon47.25.010.2 | TACAAGCACGGGTCCTCCAGTTTCA | 480 |
| Hu.DMD.Exon47.25.011 | AGTTTATCTTGCTCTTCTGGGCTTA | 481 |
| Hu.DMD.Exon47.25.012 | TCTGCTTGAGCTTATTTTCAAGTTT | 482 |
| Hu.DMD.Exon47.25.012.2 | ATCTTGCTCTTCTGGGCTTATGGGA | 483 |
| Hu.DMD.Exon47.25.013 | CTTTATCCACTGGAGATTTGTCTGC | 484 |
| Hu.DMD.Exon47.25.013.2 | CTTATTTTCAAGTTTATCTTGCTCT | 485 |
| Hu.DMD.Exon47.25.014 | CTAACCTTTATCCACTGGAGATTTG | 486 |
| Hu.DMD.Exon47.25.014.2 | ATTTGTCTGCTTGAGCTTATTTTCA | 487 |
| Hu.DMD.Exon47.25.015 | AATGTCTAACCTTTATCCACTGGAG | 488 |
| Hu.DMD.Exon47.25.016 | TGGTTAATGTCTAACCTTTATCCAC | 489 |
| Hu.DMD.Exon47.25.017 | AGAGATGGTTAATGTCTAACCTTTA | 490 |
| Hu.DMD.Exon47.25.018 | ACGGAAGAGATGGTTAATGTCTAAC | 491 |
| Hu.DMD.Exon47.20.001 | ACAGACAAATGCAACAACGT | 492 |
| Hu.DMD.Exon47.20.002 | CTGAAACAGACAAATGCAAC | 493 |
| Hu.DMD.Exon47.20.003 | AGTAACTGAAACAGACAAAT | 494 |
| Hu.DMD.Exon47.20.004 | CCACCAGTAACTGAAACAGA | 495 |
| Hu.DMD.Exon47.20.005 | CTCTTCCACCAGTAACTGAA | 496 |
| Hu.DMD.Exon47.20.006 | GGCAACTCTTCCACCAGTAA | 497 |
| Hu.DMD.Exon47.20.007 | CTGGCGCAGGGGCAACTCTT | 498 |
| Hu.DMD.Exon47.20.008 | TTGTTTGAGAATTCCCTGGC | 499 |
| Hu.DMD.Exon47.20.009 | TCCAGTTTCATTTAATTGTT | 500 |
| Hu.DMD.Exon47.20.010 | TACAAGCACGGGTCCTCCAG | 501 |
| Hu.DMD.Exon47.20.011 | GCTTATGGGAGCACTTACAA | 502 |
| Hu.DMD.Exon47.20.012 | ATCTTGCTCTTCTGGGCTTA | 503 |
| Hu.DMD.Exon47.20.013 | CTTATTTTCAAGTTTATCTT | 504 |
| Hu.DMD.Exon47.20.014 | ATTTGTCTGCTTGAGCTTAT | 505 |
| Hu.DMD.Exon47.20.015 | CTTTATCCACTGGAGATTTG | 506 |
| Hu.DMD.Exon47.20.016 | CTAACCTTTATCCACTGGAG | 507 |
| Hu.DMD.Exon47.20.017 | AATGTCTAACCTTTATCCAC | 508 |
| Hu.DMD.Exon47.20.018 | TGGTTAATGTCTAACCTTTA | 509 |
| Hu.DMD.Exon47.20.019 | AGAGATGGTTAATGTCTAAC | 510 |
| Hu.DMD.Exon47.20.020 | ACGGAAGAGATGGTTAATGT | 511 |
| Hu.DMD.Exon48.25.001 | CTGAAAGGAAAATACATTTTAAAAA | 512 |
| Hu.DMD.Exon48.25.002 | CCTGAAAGGAAAATACATTTTAAAA | 513 |
| Hu.DMD.Exon48.25.002.2 | GAAACCTGAAAGGAAAATACATTTT | 514 |
| Hu.DMD.Exon48.25.003 | GGAAACCTGAAAGGAAAATACATTT | 515 |
| Hu.DMD.Exon48.25.003.2 | CTCTGGAAACCTGAAAGGAAAATAC | 516 |
| Hu.DMD.Exon48.25.004 | GCTCTGGAAACCTGAAAGGAAAATA | 517 |
| Hu.DMD.Exon48.25.004.2 | TAAAGCTCTGGAAACCTGAAAGGAA | 518 |
| Hu.DMD.Exon48.25.005 | GTAAAGCTCTGGAAACCTGAAAGGA | 519 |
| Hu.DMD.Exon48.25.005.2 | TCAGGTAAAGCTCTGGAAACCTGAA | 520 |
| Hu.DMD.Exon48.25.006 | CTCAGGTAAAGCTCTGGAAACCTGA | 521 |
| Hu.DMD.Exon48.25.006.2 | GTTTCTCAGGTAAAGCTCTGGAAAC | 522 |
| Hu.DMD.Exon48.25.007 | TGTTTCTCAGGTAAAGCTCTGGAAA | 523 |
| Hu.DMD.Exon48.25.007.2 | AATTTCTCCTTGTTTCTCAGGTAAA | 524 |
| Hu.DMD.Exon48.25.008 | TTTGAGCTTCAATTTCTCCTTGTTT | 525 |
| Hu.DMD.Exon48.25.008 | TTTTATTTGAGCTTCAATTTCTCCT | 526 |
| Hu.DMD.Exon48.25.009 | AAGCTGCCCAAGGTCTTTTATTTGA | 527 |
| Hu.DMD.Exon48.25.010 | AGGTCTTCAAGCTTTTTTTCAAGCT | 528 |
| Hu.DMD.Exon48.25.010.2 | TTCAAGCTTTTTTTCAAGCTGCCCA | 529 |
| Hu.DMD.Exon48.25.011 | GATGATTTAACTGCTCTTCAAGGTC | 530 |
| Hu.DMD.Exon48.25.011.2 | CTGCTCTTCAAGGTCTTCAAGCTTT | 531 |
| Hu.DMD.Exon48.25.012 | AGGAGATAACCACAGCAGCAGATGA | 532 |
| Hu.DMD.Exon48.25.012.2 | CAGCAGATGATTTAACTGCTCTTCA | 533 |
| Hu.DMD.Exon48.25.013 | ATTTCCAACTGATTCCTAATAGGAG | 534 |
| Hu.DMD.Exon48.25.014 | CTTGGTTTGGTTGGTTATAAATTTC | 535 |
| Hu.DMD.Exon48.25.014.2 | CAACTGATTCCTAATAGGAGATAAC | 536 |
| Hu.DMD.Exon48.25.015 | CTTAACGTCAAATGGTCCTTCTTGG | 537 |
| Hu.DMD.Exon48.25.015.2 | TTGGTTATAAATTTCCAACTGATTC | 538 |
| Hu.DMD.Exon48.25.016 | CCTACCTTAACGTCAAATGGTCCTT | 539 |
| Hu.DMD.Exon48.25.016.2 | TCCTTCTTGGTTTGGTTGGTTATAA | 540 |
| Hu.DMD.Exon48.25.017 | AGTTCCCTACCTTAACGTCAAATGG | 541 |
| Hu.DMD.Exon48.25.018 | CAAAAAGTTCCCTACCTTAACGTCA | 542 |
| Hu.DMD.Exon48.25.019 | TAAAGCAAAAAGTTCCCTACCTTAA | 543 |
| Hu.DMD.Exon48.25.020 | ATATTTAAAGCAAAAAGTTCCCTAC | 544 |
| Hu.DMD.Exon48.20.001 | AGGAAAATACATTTTAAAAA | 545 |
| Hu.DMD.Exon48.20.002 | AAGGAAAATACATTTTAAAA | 546 |
| Hu.DMD.Exon48.20.003 | CCTGAAAGGAAAATACATTT | 547 |
| Hu.DMD.Exon48.20.004 | GGAAACCTGAAAGGAAAATA | 548 |
| Hu.DMD.Exon48.20.005 | GCTCTGGAAACCTGAAAGGA | 549 |
| Hu.DMD.Exon48.20.006 | GTAAAGCTCTGGAAACCTGA | 550 |
| Hu.DMD.Exon48.20.007 | CTCAGGTAAAGCTCTGGAAA | 551 |
| Hu.DMD.Exon48.20.008 | AATTTCTCCTTGTTTCTCAG | 552 |
| Hu.DMD.Exon48.20.009 | TTTTATTTGAGCTTCAATTT | 553 |
| Hu.DMD.Exon48.20.010 | AAGCTGCCCAAGGTCTTTTA | 554 |
| Hu.DMD.Exon48.20.011 | TTCAAGCTTTTTTTCAAGCT | 555 |
| Hu.DMD.Exon48.20.012 | CTGCTCTTCAAGGTCTTCAA | 556 |
| Hu.DMD.Exon48.20.013 | CAGCAGATGATTTAACTGCT | 557 |
| Hu.DMD.Exon48.20.014 | AGGAGATAACCACAGCAGCA | 558 |
| Hu.DMD.Exon48.20.015 | CAACTGATTCCTAATAGGAG | 559 |
| Hu.DMD.Exon48.20.016 | TTGGTTATAAATTTCCAACT | 560 |
| Hu.DMD.Exon48.20.017 | TCCTTCTTGGTTTGGTTGGT | 561 |
| Hu.DMD.Exon48.20.018 | CTTAACGTCAAATGGTCCTT | 562 |
| Hu.DMD.Exon48.20.019 | CCTACCTTAACGTCAAATGG | 563 |
| Hu.DMD.Exon48.20.020 | AGTTCCCTACCTTAACGTCA | 564 |
| Hu.DMD.Exon48.20.021 | CAAAAAGTTCCCTACCTTAA | 565 |
| Hu.DMD.Exon48.20.022 | TAAAGCAAAAAGTTCCCTAC | 566 |
| Hu.DMD.Exon48.20.023 | ATATTTAAAGCAAAAAGTTC | 567 |
| Hu.DMD.Exon49.25.001 | CTGGGGAAAAGAACCCATATAGTGC | 568 |
| Hu.DMD.Exon49.25.002 | TCCTGGGGAAAAGAACCCATATAGT | 569 |
| Hu.DMD.Exon49.25.002.2 | GTTTCCTGGGGAAAAGAACCCATAT | 570 |
| Hu.DMD.Exon49.25.003 | CAGTTTCCTGGGGAAAAGAACCCAT | 571 |
| Hu.DMD.Exon49.25.003.2 | TTTCAGTTTCCTGGGGAAAAGAACC | 572 |
| Hu.DMD.Exon49.25.004 | TATTTCAGTTTCCTGGGGAAAAGAA | 573 |
| Hu.DMD.Exon49.25.004.2 | TGCTATTTCAGTTTCCTGGGGAAAA | 574 |
| Hu.DMD.Exon49.25.005 | ACTGCTATTTCAGTTTCCTGGGGAA | 575 |
| Hu.DMD.Exon49.25.005.2 | TGAACTGCTATTTCAGTTTCCTGGG | 576 |
| Hu.DMD.Exon49.25.006 | CTTGAACTGCTATTTCAGTTTCCTG | 577 |
| Hu.DMD.Exon49.25.006.2 | TAGCTTGAACTGCTATTTCAGTTTC | 578 |
| Hu.DMD.Exon49.25.007 | TTTAGCTTGAACTGCTATTTCAGTT | 579 |
| Hu.DMD.Exon49.25.008 | TTCCACATCCGGTTGTTTAGCTTGA | 580 |
| Hu.DMD.Exon49.25.009 | TGCCCTTTAGACAAAATCTCTTCCA | 581 |
| Hu.DMD.Exon49.25.009.2 | TTTAGACAAAATCTCTTCCACATCC | 582 |
| Hu.DMD.Exon49.25.010 | GTTTTTCCTTGTACAAATGCTGCCC | 583 |
| Hu.DMD.Exon49.25.010.2 | GTACAAATGCTGCCCTTTAGACAAA | 584 |
| Hu.DMD.Exon49.25.011 | CTTCACTGGCTGAGTGGCTGGTTTT | 585 |
| Hu.DMD.Exon49.25.011.2 | GGCTGGTTTTTCCTTGTACAAATGC | 586 |
| Hu.DMD.Exon49.25.012 | ATTACCTTCACTGGCTGAGTGGCTG | 587 |
| Hu.DMD.Exon49.25.013 | GCTTCATTACCTTCACTGGCTGAGT | 588 |
| Hu.DMD.Exon49.25.014 | AGGTTGCTTCATTACCTTCACTGGC | 589 |
| Hu.DMD.Exon49.25.015 | GCTAGAGGTTGCTTCATTACCTTCA | 590 |
| Hu.DMD.Exon49.25.016 | ATATTGCTAGAGGTTGCTTCATTAC | 591 |
| Hu.DMD.Exon49.20.001 | GAAAAGAACCCATATAGTGC | 592 |
| Hu.DMD.Exon49.20.002 | GGGAAAAGAACCCATATAGT | 593 |
| Hu.DMD.Exon49.20.003 | TCCTGGGGAAAAGAACCCAT | 594 |
| Hu.DMD.Exon49.20.004 | CAGTTTCCTGGGGAAAAGAA | 595 |
| Hu.DMD.Exon49.20.005 | TATTTCAGTTTCCTGGGGAA | 596 |
| Hu.DMD.Exon49.20.006 | ACTGCTATTTCAGTTTCCTG | 597 |
| Hu.DMD.Exon49.20.007 | CTTGAACTGCTATTTCAGTT | 598 |
| Hu.DMD.Exon49.20.008 | TTTAGCTTGAACTGCTATTT | 599 |
| Hu.DMD.Exon49.20.009 | TTCCACATCCGGTTGTTTAG | 600 |
| Hu.DMD.Exon49.20.010 | TTTAGACAAAATCTCTTCCA | 601 |
| Hu.DMD.Exon49.20.011 | GTACAAATGCTGCCCTTTAG | 602 |
| Hu.DMD.Exon49.20.012 | GGCTGGTTTTTCCTTGTACA | 603 |
| Hu.DMD.Exon49.20.013 | CTTCACTGGCTGAGTGGCTG | 604 |
| Hu.DMD.Exon49.20.014 | ATTACCTTCACTGGCTGAGT | 605 |
| Hu.DMD.Exon49.20.015 | GCTTCATTACCTTCACTGGC | 606 |
| Hu.DMD.Exon49.20.016 | AGGTTGCTTCATTACCTTCA | 607 |
| Hu.DMD.Exon49.20.017 | GCTAGAGGTTGCTTCATTAC | 608 |
| Hu.DMD.Exon49.20.018 | ATATTGCTAGAGGTTGCTTC | 609 |
| Hu.DMD.Exon50.25.001 | CTTTAACAGAAAAGCATACACATTA | 610 |
| Hu.DMD.Exon50.25.002 | TCCTCTTTAACAGAAAAGCATACAC | 611 |
| Hu.DMD.Exon50.25.002.2 | TTCCTCTTTAACAGAAAAGCATACA | 612 |
| Hu.DMD.Exon50.25.003 | TAACTTCCTCTTTAACAGAAAAGCA | 613 |
| Hu.DMD.Exon50.25.003.2 | CTAACTTCCTCTTTAACAGAAAAGC | 614 |
| Hu.DMD.Exon50.25.004 | TCTTCTAACTTCCTCTTTAACAGAA | 615 |
| Hu.DMD.Exon50.25.004.2 | ATCTTCTAACTTCCTCTTTAACAGA | 616 |
| Hu.DMD.Exon50.25.005 | TCAGATCTTCTAACTTCCTCTTTAA | 617 |
| Hu.DMD.Exon50.25.005.2 | CTCAGATCTTCTAACTTCCTCTTTA | 618 |
| Hu.DMD.Exon50.25.006 | AGAGCTCAGATCTTCTAACTTCCTC | 619 |
| Hu.DMD.Exon50.25.006.2 NG-08-0731 | CAGAGCTCAGATCTTCTAACTTCCT | 620 |
| Hu.DMD.Exon50.25.007 | CACTCAGAGCTCAGATCTTCTACT | 621 |
| Hu.DMD.Exon50.25.007.2 | CCTTCCACTCAGAGCTCAGATCTTC | 622 |
| Hu.DMD.Exon50.25.008 | GTAAACGGTTTACCGCCTTCCACTC | 623 |
| Hu.DMD.Exon50.25.009 | CTTTGCCCTCAGCTCTTGAAGTAAA | 624 |
| Hu.DMD.Exon50.25.009.2 | CCCTCAGCTCTTGAAGTAAACGGTT | 625 |
| Hu.DMD.Exon50.25.010 | CCAGGAGCTAGGTCAGGCTGCTTTG | 626 |
| Hu.DMD.Exon50.25.010.2 | GGTCAGGCTGCTTTGCCCTCAGCTC | 627 |
| Hu.DMD.Exon50.25.011 | AGGCTCCAATAGTGGTCAGTCCAGG | 628 |
| Hu.DMD.Exon50.25.011.2 | TCAGTCCAGGAGCTAGGTCAGGCTG | 629 |
| Hu.DMD.Exon50.25.012 AVI-5038 | CTTACAGGCTCCAATAGTGGTCAGT | 630 |
| Hu.DMD.Exon50.25.013 | GTATACTTACAGGCTCCAATAGTGG | 631 |
| Hu.DMD.Exon50.25.014 | ATCCAGTATACTTACAGGCTCCAAT | 632 |
| Hu.DMD.Exon50.25.015 NG-08-0741 | ATGGGATCCAGTATACTTACAGGCT | 633 |
| Hu.DMD.Exon50.25.016 NG-08-0742 | AGAGAATGGGATCCAGTATACTTAC | 634 |
| Hu.DMD.Exon50.20.001 | ACAGAAAAGCATACACATTA | 635 |
| Hu.DMD.Exon50.20.002 | TTTAACAGAAAAGCATACAC | 636 |
| Hu.DMD.Exon50.20.003 | TCCTCTTTAACAGAAAAGCA | 637 |
| Hu.DMD.Exon50.20.004 | TAACTTCCTCTTTAACAGAA | 638 |
| Hu.DMD.Exon50.20.005 | TCTTCTAACTTCCTCTTTAA | 639 |
| Hu.DMD.Exon50.20.006 | TCAGATCTTCTAACTTCCTC | 640 |
| Hu.DMD.Exon50.20.007 | CCTTCCACTCAGAGCTCAGA | 641 |
| Hu.DMD.Exon50.20.008 | GTAAACGGTTTACCGCCTTC | 642 |
| Hu.DMD.Exon50.20.009 | CCCTCAGCTCTTGAAGTAAA | 643 |
| Hu.DMD.Exon50.20.010 | GGTCAGGCTGCTTTGCCCTC | 644 |
| Hu.DMD.Exon50.20.011 | TCAGTCCAGGAGCTAGGTCA | 645 |
| Hu.DMD.Exon50.20.012 | AGGCTCCAATAGTGGTCAGT | 646 |
| Hu.DMD.Exon50.20.013 | CTTACAGGCTCCAATAGTGG | 647 |
| Hu.DMD.Exon50.20.014 | GTATACTTACAGGCTCCAAT | 648 |
| Hu.DMD.Exon50.20.015 | ATCCAGTATACTTACAGGCT | 649 |
| Hu.DMD.Exon50.20.016 | ATGGGATCCAGTATACTTAC | 650 |
| Hu.DMD.Exon50.20.017 | AGAGAATGGGATCCAGTATA | 651 |
| Hu.DMD.Exon51.25.001-44 | CTAAAATATTTTGGGTTTTTGCAAAA | 652 |
| Hu.DMD.Exon51.25.002-45 | GCTAAAATATTTTGGGTTTTTGCAAA | 653 |
| Hu.DMD.Exon51.25.002.2-46 | TAGGAGCTAAAATATTTTGGGTTTTT | 654 |
| Hu.DMD.Exon51.25.003 | AGTAGGAGCTAAAATATTTTGGGTT | 655 |
| Hu.DMD.Exon51.25.003.2 | TGAGTAGGAGCTAAAATATTTTGGG | 656 |
| Hu.DMD.Exon51.25.004 | CTGAGTAGGAGCTAAAATATTTTGGG | 657 |
| Hu.DMD.Exon51.25.004.2 | CAGTCTGAGTAGGAGCTAAAATATT | 658 |
| Hu.DMD.Exon51.25.005 | ACAGTCTGAGTAGGAGCTAAAATATT | 659 |
| Hu.DMD.Exon51.25.005.2 | GAGTAACAGTCTGAGTAGGAGCTAAA | 660 |
| Hu.DMD.Exon51.25.006 | CAGAGTAACAGTCTGAGTAGGAGCT | 661 |
| Hu.DMD.Exon51.25.006.2 | CACCAGAGTAACAGTCTGAGTAGGAG | 662 |
| Hu.DMD.Exon51.25.007 | GTCACCAGAGTAACAGTCTGAGTAG | 663 |
| Hu.DMD.Exon51.25.007.2 | AACCACAGGTTGTGTCACCAGAGTAA | 664 |
| Hu.DMD.Exon51.25.008 | GTTGTGTCACCAGAGTAACAGTCTG | 665 |
| Hu.DMD.Exon51.25.009 | TGGCAGTTTCCTTAGTAACCACAGGT | 666 |
| Hu.DMD.Exon51.25.010 | ATTTCTAGTTTGGAGATGGCAGTTTC | 667 |
| Hu.DMD.Exon51.25.010.2 | GGAAGATGGCATTTCTAGTTTGGAG | 668 |
| Hu.DMD.Exon51.25.011 | CATCAAGGAAGATGGCATTTCTAGTT | 669 |
| Hu.DMD.Exon51.25.011.2 | GAGCAGGTACCTCCAACATCAAGGAA | 670 |
| Hu.DMD.Exon51.25.012 | ATCTGCCAGAGCAGGTACCTCCAAC | 671 |
| Hu.DMD.Exon51.25.013 | AAGTTCTGTCCAAGCCCGGTTGAAAT | 672 |
| Hu.DMD.Exon51.25.013.2 | CGGTTGAAATCTGCCAGAGCAGGTAC | 673 |
| Hu.DMD.Exon51.25.014 | GAGAAAGCCAGTCGGTAAGTTCTGTC | 674 |
| Hu.DMD.Exon51.25.014.2 | GTCGGTAAGTTCTGTCCAAGCCCGG | 675 |
| Hu.DMD.Exon51.25.015 | ATAACTTGATCAAGCAGAGAAAGCCA | 676 |
| Hu.DMD.Exon51.25.015.2 | AAGCAGAGAAAGCCAGTCGGTAAGT | 677 |
| Hu.DMD.Exon51.25.016 | CACCCTCTGTGATTTTATAACTTGAT | 678 |
| Hu.DMD.Exon51.25.017 | CAAGGTCACCCACCATCACCCTCTGT | 679 |
| Hu.DMD.Exon51.25.017.2 | CATCACCCTCTGTGATTTTATAACT | 680 |
| Hu.DMD.Exon51.25.018 | CTTCTGCTTGATGATCATCTCGTTGA | 681 |
| Hu.DMD.Exon51.25.019 | CCTTCTGCTTGATGATCATCTCGTTG | 682 |
| Hu.DMD.Exon51.25.019.2 | ATCTCGTTGATATCCTCAAGGTCACC | 683 |
| Hu.DMD.Exon51.25.020 | TCATACCTTCTGCTTGATGATCATCT | 684 |
| Hu.DMD.Exon51.25.020.2 | TCATTTTTTCTCATACCTTCTGCTTG | 685 |
| Hu.DMD.Exon51.25.021 | TTTTCTCATACCTTCTGCTTGATGAT | 686 |
| Hu.DMD.Exon51.25.022 | TTTTATCATTTTTTCTCATACCTTCT | 687 |
| Hu.DMD.Exon51.25.023 | CCAACTTTTATCATTTTTTCTCATAC | 688 |
| Hu.DMD.Exon51.20.001 | ATATTTTGGGTTTTTGCAAA | 689 |
| Hu.DMD.Exon51.20.002 | AAAATATTTTGGGTTTTTGC | 690 |
| Hu.DMD.Exon51.20.003 | GAGCTAAAATATTTTGGGTT | 691 |
| Hu.DMD.Exon51.20.004 | AGTAGGAGCTAAAATATTTT | 692 |
| Hu.DMD.Exon51.20.005 | GTCTGAGTAGGAGCTAAAAT | 693 |
| Hu.DMD.Exon51.20.006 | TAACAGTCTGAGTAGGAGCT | 694 |
| Hu.DMD.Exon51.20.007 | CAGAGTAACAGTCTGAGTAG | 695 |
| Hu.DMD.Exon51.20.008 | CACAGGTTGTGTCACCAGAG | 696 |
| Hu.DMD.Exon51.20.009 | AGTTTCCTTAGTAACCACAG | 697 |
| Hu.DMD.Exon51.20.010 | TAGTTTGGAGATGGCAGTTT | 698 |
| Hu.DMD.Exon51.20.011 | GGAAGATGGCATTTCTAGTT | 699 |
| Hu.DMD.Exon51.20.012 | TACCTCCAACATCAAGGAAG | 700 |
| Hu.DMD.Exon51.20.013 | ATCTGCCAGAGCAGGTACCT | 701 |
| Hu.DMD.Exon51.20.014 | CCAAGCCCGGTTGAAATCTG | 702 |
| Hu.DMD.Exon51.20.015 | GTCGGTAAGTTCTGTCCAAG | 703 |
| Hu.DMD.Exon51.20.016 | AAGCAGAGAAAGCCAGTCGG | 704 |
| Hu.DMD.Exon51.20.017 | TTTTATAACTTGATCAAGCA | 705 |
| Hu.DMD.Exon51.20.018 | CATCACCCTCTGTGATTTTA | 706 |
| Hu.DMD.Exon51.20.019 | CTCAAGGTCACCCACCATCA | 707 |
| Hu.DMD.Exon51.20.020 | CATCTCGTTGATATCCTCAA | 708 |
| Hu.DMD.Exon51.20.021 | CTTCTGCTTGATGATCATCT | 709 |
| Hu.DMD.Exon51.20.022 | CATACCTTCTGCTTGATGAT | 710 |
| Hu.DMD.Exon51.20.023 | TTTCTCATACCTTCTGCTTG | 711 |
| Hu.DMD.Exon51.20.024 | CATTTTTTCTCATACCTTCT | 712 |
| Hu.DMD.Exon51.20.025 | TTTATCATTTTTTCTCATAC | 713 |
| Hu.DMD.Exon51.20.026 | CAACTTTTATCATTTTTTCT | 714 |
| Hu.DMD.Exon52.25.001 | CTGTAAGAACAAATATCCCTTAGTA | 715 |
| Hu.DMD.Exon52.25.002 | TGCCTGTAAGAACAAATATCCCTTA | 716 |
| Hu.DMD.Exon52.25.002.2 | GTTGCCTGTAAGAACAAATATCCCT | 717 |
| Hu.DMD.Exon52.25.003 | ATTGTTGCCTGTAAGAACAAATATC | 718 |
| Hu.DMD.Exon52.25.003.2 | GCATTGTTGCCTGTAAGAACAAATA | 719 |
| Hu.DMD.Exon52.25.004 | CCTGCATTGTTGCCTGTAAGAACAA | 720 |
| Hu.DMD.Exon52.25.004.2 | ATCCTGCATTGTTGCCTGTAAGAAC | 721 |
| Hu.DMD.Exon52.25.005 | CAAATCCTGCATTGTTGCCTGTAAG | 722 |
| **Hu.DMD.Exon52.25.005.2** | TCCAAATCCTGCATTGTTGCCTGTA | 723 |
| Hu.DMD.Exon52.25.006 | TGTTCCAAATCCTGCATTGTTGCCT | 724 |
| Hu.DMD.Exon52.25.006.2 | TCTGTTCCAAATCCTGCATTGTTGC | 725 |
| Hu.DMD.Exon52.25.007 | AACTGGGGACGCCTCTGTTCCAAAT | 726 |
| Hu.DMD.Exon52.25.007.2 | GCCTCTGTTCCAAATCCTGCATTGT | 727 |
| Hu.DMD.Exon52.25.008 | CAGCGGTAATGAGTTCTTCCAACTG | 728 |
| Hu.DMD.Exon52.25.008.2 | CTTCCAACTGGGGACGCCTCTGTTC | 729 |
| Hu.DMD.Exon52.25.009 | CTTGTTTTTCAAATTTTGGGCAGCG | 730 |
| Hu.DMD.Exon52.25.010 | CTAGCCTCTTGATTGCTGGTCTTGT | 731 |
| Hu.DMD.Exon52.25.010.2 | TTTTCAAATTTTGGGCAGCGGTAAT | 732 |
| Hu.DMD.Exon52.25.011 | TTCGATCCGTAATGATTGTTCTAGC | 733 |
| Hu.DMD.Exon52.25.011.2 | GATTGCTGGTCTTGTTTTTCAAATT | 734 |
| Hu.DMD.Exon52.25.012 | CTTACTTCGATCCGTAATGATTGTT | 735 |
| Hu.DMD.Exon52.25.012.2 | TTGTTCTAGCCTCTTGATTGCTGGT | 736 |
| Hu.DMD.Exon52.25.013 | AAAAACTTACTTCGATCCGTAATGA | 737 |
| Hu.DMD.Exon52.25.014 | TGTTAAAAAACTTACTTCGATCCGT | 738 |
| Hu.DMD.Exon52.25.015 | ATGCTTGTTAAAAAACTTACTTCGA | 739 |
| Hu.DMD.Exon52.25.016 | GTCCCATGCTTGTTAAAAAACTTAC | 740 |
| Hu.DMD.Exon52.20.001 | AGAACAAATATCCCTTAGTA | 741 |
| Hu.DMD.Exon52.20.002 | GTAAGAACAAATATCCCTTA | 742 |
| Hu.DMD.Exon52.20.003 | TGCCTGTAAGAACAAATATC | 743 |
| Hu.DMD.Exon52.20.004 | ATTGTTGCCTGTAAGAACAA | 744 |
| Hu.DMD.Exon52.20.005 | CCTGCATTGTTGCCTGTAAG | 745 |
| Hu.DMD.Exon52.20.006 | CAAATCCTGCATTGTTGCCT | 746 |
| Hu.DMD.Exon52.20.007 | GCCTCTGTTCCAAATCCTGC | 747 |
| Hu.DMD.Exon52.20.008 | CTTCCAACTGGGGACGCCTC | 748 |
| Hu.DMD.Exon52.20.009 | CAGCGGTAATGAGTTCTTCC | 749 |
| Hu.DMD.Exon52.20.010 | TTTTCAAATTTTGGGCAGCG | 750 |
| Hu.DMD.Exon52.20.011 | GATTGCTGGTCTTGTTTTTC | 751 |
| Hu.DMD.Exon52.20.012 | TTGTTCTAGCCTCTTGATTG | 752 |
| Hu.DMD.Exon52.20.013 | TTCGATCCGTAATGATTGTT | 753 |
| Hu.DMD.Exon52.20.014 | CTTACTTCGATCCGTAATGA | 754 |
| Hu.DMD.Exon52.20.015 | AAAAACTTACTTCGATCCGT | 755 |
| Hu.DMD.Exon52.20.016 | TGTTAAAAAACTTACTTCGA | 756 |
| Hu.DMD.Exon52.20.017 | ATGCTTGTTAAAAAACTTAC | 757 |
| Hu.DMD.Exon52.20.018 | GTCCCATGCTTGTTAAAAAA | 758 |
| Hu.DMD.Exon53.25.001 | CTAGAATAAAAGGAAAAATAAATAT | 759 |
| Hu.DMD.Exon53.25.002 | AACTAGAATAAAAGGAAAAATAAAT | 760 |
| Hu.DMD.Exon53.25.002.2 | TTCAACTAGAATAAAAGGAAAAATA | 761 |
| Hu.DMD.Exon53.25.003 | CTTTCAACTAGAATAAAAGGAAAAA | 762 |
| Hu.DMD.Exon53.25.003.2 | ATTCTTTCAACTAGAATAAAAGGAA | 763 |
| Hu.DMD.Exon53.25.004 | GAATTCTTTCAACTAGAATAAAAGG | 764 |
| Hu.DMD.Exon53.25.004.2 | TCTGAATTCTTTCAACTAGAATAAA | 765 |
| Hu.DMD.Exon53.25.005 | ATTCTGAATTCTTTCAACTAGAATA | 766 |
| Hu.DMD.Exon53.25.005.2 | CTGATTCTGAATTCTTTCAACTAGA | 767 |
| Hu.DMD.Exon53.25.006 | CACTGATTCTGAATTCTTTCAACTA | 768 |
| Hu.DMD.Exon53.25.006.2 | TCCCACTGATTCTGAATTCTTTCAA | 769 |
| Hu.DMD.Exon53.25.007 | CATCCCACTGATTCTGAATTCTTTC | 770 |
| Hu.DMD.Exon53.25.008 | TACTTCATCCCACTGATTCTGAATT | 771 |
| Hu.DMD.Exon53.25.008.2 | CTGAAGGTGTTCTTGTACTTCATCC | 772 |
| Hu.DMD.Exon53.25.009 | CGGTTCTGAAGGTGTTCTTGTACT | 773 |
| Hu.DMD.Exon53.25.009.2 | CTGTTGCCTCCGGTTCTGAAGGTGT | 774 |
| Hu.DMD.Exon53.25.010 | TTTCATTCAACTGTTGCCTCCGGTT | 775 |
| Hu.DMD.Exon53.25.010.2 | TAACATTTCATTCAACTGTTGCCTC | 776 |
| Hu.DMD.Exon53.25.011 | TTGTGTTGAATCCTTTAACATTTCA | 777 |
| Hu.DMD.Exon53.25.012 | TCTTCCTTAGCTTCCAGCCATTGTG | 778 |
| Hu.DMD.Exon53.25.012.2 | CTTAGCTTCCAGCCATTGTGTTGAA | 779 |
| Hu.DMD.Exon53.25.013 | GTCCTAAGACCTGCTCAGCTTCTTC | 780 |
| Hu.DMD.Exon53.25.013.2 | CTGCTCAGCTTCTTCCTTAGCTTCC | 781 |
| Hu.DMD.Exon53.25.014 | CTCAAGCTTGGCTCTGGCCTGTCCT | 782 |
| Hu.DMD.Exon53.25.014.2 | GGCCTGTCCTAAGACCTGCTCAGCT | 783 |
| Hu.DMD.Exon53.25.015 | TAGGGACCCTCCTTCCATGACTCAA | 784 |
| Hu.DMD.Exon53.25.016 | TTTGGATTGCATCTACTGTATAGGG | 785 |
| Hu.DMD.Exon53.25.016.2 | ACCCTCCTTCCATGACTCAAGCTTG | 786 |
| Hu.DMD.Exon53.25.017 | CTTGGTTTCTGTGATTTTCTTTTGG | 787 |
| Hu.DMD.Exon53.25.017.2 | ATCTACTGTATAGGGACCCTCCTTC | 788 |
| Hu.DMD.Exon53.25.018 | CTAACCTTGGTTTCTGTGATTTTCT | 789 |
| Hu.DMD.Exon53.25.018.2 | TTTCTTTTGGATTGCATCTACTGTA | 790 |
| Hu.DMD.Exon53.25.019 | TGATACTAACCTTGGTTTCTGTGAT | 791 |
| Hu.DMD.Exon53.25.020 | ATCTTTGATACTAACCTTGGTTTCT | 792 |
| Hu.DMD.Exon53.25.021 | AAGGTATCTTTGATACTAACCTTGG | 793 |
| Hu.DMD.Exon53.25.022 | TTAAAAAGGTATCTTTGATACTAAC | 794 |
| Hu.DMD.Exon53.20.001 | ATAAAAGGAAAAATAAATAT | 795 |
| Hu.DMD.Exon53.20.002 | GAATAAAAGGAAAAATAAAT | 796 |
| Hu.DMD.Exon53.20.003 | AACTAGAATAAAAGGAAAAA | 797 |
| Hu.DMD.Exon53.20.004 | CTTTCAACTAGAATAAAAGG | 798 |
| Hu.DMD.Exon53.20.005 | GAATTCTTTCAACTAGAATA | 799 |
| Hu.DMD.Exon53.20.006 | ATTCTGAATTCTTTCAACTA | 800 |
| Hu.DMD.Exon53.20.007 | TACTTCATCCCACTGATTCT | 801 |
| Hu.DMD.Exon53.20.008 | CTGAAGGTGTTCTTGTACT | 802 |
| Hu.DMD.Exon53.20.009 | CTGTTGCCTCCGGTTCTGAA | 803 |
| Hu.DMD.Exon53.20.010 | TAACATTTCATTCAACTGTT | 804 |
| Hu.DMD.Exon53.20.011 | TTGTGTTGAATCCTTTAACA | 805 |
| Hu.DMD.Exon53.20.012 | CTTAGCTTCCAGCCATTGTG | 806 |
| Hu.DMD.Exon53.20.013 | CTGCTCAGCTTCTTCCTTAG | 807 |
| Hu.DMD.Exon53.20.014 | GGCCTGTCCTAAGACCTGCT | 808 |
| Hu.DMD.Exon53.20.015 | CTCAAGCTTGGCTCTGGCCT | 809 |
| Hu.DMD.Exon53.20.016 | ACCCTCCTTCCATGACTCAA | 810 |
| Hu.DMD.Exon53.20.017 | ATCTACTGTATAGGGACCCT | 811 |
| Hu.DMD.Exon53.20.018 | TTTCTTTTGGATTGCATCTA | 812 |
| Hu.DMD.Exon53.20.019 | CTTGGTTTCTGTGATTTTCT | 813 |
| Hu.DMD.Exon53.20.020 | CTAACCTTGGTTTCTGTGAT | 814 |
| Hu.DMD.Exon53.20.021 | TGATACTAACCTTGGTTTCT | 815 |
| Hu.DMD.Exon53.20.022 | ATCTTTGATACTAACCTTGG | 816 |
| Hu.DMD.Exon53.20.023 | AAGGTATCTTTGATACTAAC | 817 |
| Hu.DMD.Exon53.20.024 | TTAAAAAGGTATCTTTGATA | 818 |
| Hu.DMD.Exon54.25.001 | CTATAGATTTTTATGAGAAAGAGA | 819 |
| Hu.DMD.Exon54.25.002 | AACTGCTATAGATTTTTATGAGAAA | 820 |
| Hu.DMD.Exon54.25.003 | TGGCCAACTGCTATAGATTTTTATG | 821 |
| Hu.DMD.Exon54.25.004 | GTCTTTGGCCAACTGCTATAGATTT | 822 |
| Hu.DMD.Exon54.25.005 | CGGAGGTCTTTGGCCAACTGCTATA | 823 |
| Hu.DMD.Exon54.25.006 | ACTGGCGGAGGTCTTTGGCCAACTG | 824 |
| Hu.DMD.Exon54.25.007 | TTTGTCTGCCACTGGCGGAGGTCTT | 825 |
| Hu.DMD.Exon54.25.008 | AGTCATTTGCCACATCTACATTTGT | 826 |
| Hu.DMD.Exon54.25.008.2 | TTTGCCACATCTACATTTGTCTGCC | 827 |
| Hu.DMD.Exon54.25.009 | CCGGAGAAGTTTCAGGGCCAAGTCA | 828 |
| Hu.DMD.Exon54.25.010 | GTATCATCTGCAGAATAATCCCGGA | 829 |
| Hu.DMD.Exon54.25.010.2 | TAATCCCGGAGAAGTTTCAGGGCCA | 830 |
| Hu.DMD.Exon54.25.011 | TTATCATGTGGACTTTTCTGGTATC | 831 |
| Hu.DMD.Exon54.25.012 | AGAGGCATTGATATTCTCTGTTATC | 832 |
| Hu.DMD.Exon54.25.012.2 | ATGTGGACTTTTCTGGTATCATCTG | 833 |
| Hu.DMD.Exon54.25.013 | CTTTTATGAATGCTTCTCCAAGAGG | 834 |
| Hu.DMD.Exon54.25.013.2 | ATATTCTCTGTTATCATGTGGACTT | 835 |
| Hu.DMD.Exon54.25.014 | CATACCTTTTATGAATGCTTCTCCA | 836 |
| Hu.DMD.Exon54.25.014.2 | CTCCAAGAGGCATTGATATTCTCTG | 837 |
| Hu.DMD.Exon54.25.015 | TAATTCATACCTTTTATGAATGCTT | 838 |
| Hu.DMD.Exon54.25.015.2 | CTTTTATGAATGCTTCTCCAAGAGG | 839 |
| Hu.DMD.Exon54.25.016 | TAATGTAATTCATACCTTTTATGAA | 840 |
| Hu.DMD.Exon54.25.017 | AGAAATAATGTAATTCATACCTTTT | 841 |
| Hu.DMD.Exon54.25.018 | GTTTTAGAAATAATGTAATTCATAC | 842 |
| Hu.DMD.Exon54.20.001 | GATTTTTATGAGAAAGAGA | 843 |
| Hu.DMD.Exon54.20.002 | CTATAGATTTTTATGAGAAA | 844 |
| Hu.DMD.Exon54.20.003 | AACTGCTATAGATTTTTATG | 845 |
| Hu.DMD.Exon54.20.004 | TGGCCAACTGCTATAGATTT | 846 |
| Hu.DMD.Exon54.20.005 | GTCTTTGGCCAACTGCTATA | 847 |
| Hu.DMD.Exon54.20.006 | CGGAGGTCTTTGGCCAACTG | 848 |
| Hu.DMD.Exon54.20.007 | TTTGTCTGCCACTGGCGGAG | 849 |
| Hu.DMD.Exon54.20.008 | TTTGCCACATCTACATTTGT | 850 |
| Hu.DMD.Exon54.20.009 | TTCAGGGCCAAGTCATTTGC | 851 |
| Hu.DMD.Exon54.20.010 | TAATCCCGGAGAAGTTTCAG | 852 |
| Hu.DMD.Exon54.20.011 | GTATCATCTGCAGAATAATC | 853 |
| Hu.DMD.Exon54.20.012 | ATGTGGACTTTTCTGGTATC | 854 |
| Hu.DMD.Exon54.20.013 | ATATTCTCTGTTATCATGTG | 855 |
| Hu.DMD.Exon54.20.014 | CTCCAAGAGGCATTGATATT | 856 |
| Hu.DMD.Exon54.20.015 | CTTTTATGAATGCTTCTCCA | 857 |
| Hu.DMD.Exon54.20.016 | CATACCTTTTATGAATGCTT | 858 |
| Hu.DMD.Exon54.20.017 | TAATTCATACCTTTTATGAA | 859 |
| Hu.DMD.Exon54.20.018 | TAATGTAATTCATACCTTTT | 860 |
| Hu.DMD.Exon54.20.019 | AGAAATAATGTAATTCATAC | 861 |
| Hu.DMD.Exon54.20.020 | GTTTTAGAAATAATGTAATT | 862 |
| Hu.DMD.Exon55.25.001 | CTGCAAAGGACCAAATGTTCAGATG | 863 |
| Hu.DMD.Exon55.25.002 | TCACCCTGCAAAGGACCAAATGTTC | 864 |
| Hu.DMD.Exon55.25.003 | CTCACTCACCCTGCAAAGGACCAAA | 865 |
| Hu.DMD.Exon55.25.004 | TCTCGCTCACTCACCCTGCAAAGGA | 866 |
| Hu.DMD.Exon55.25.005 | CAGCCTCTCGCTCACTCACCCTGCA | 867 |
| Hu.DMD.Exon55.25.006 | CAAAGCAGCCTCTCGCTCACTCACC | 868 |
| Hu.DMD.Exon55.25.007 | TCTTCCAAAGCAGCCTCTCGCTCAC | 869 |
| Hu.DMD.Exon55.25.007.2 | TCTATGAGTTTCTTCCAAAGCAGCC | 870 |
| Hu.DMD.Exon55.25.008 | GTTGCAGTAATCTATGAGTTTCTTC | 871 |
| Hu.DMD.Exon55.25.008.2 | GAACTGTTGCAGTAATCTATGAGTT | 872 |
| Hu.DMD.Exon55.25.009 | TTCCAGGTCCAGGGGGAACTGTTGC | 873 |
| Hu.DMD.Exon55.25.010 | GTAAGCCAGGCAAGAAACTTTTCCA | 874 |
| Hu.DMD.Exon55.25.010.2 | CCAGGCAAGAAACTTTTCCAGGTCC | 875 |
| Hu.DMD.Exon55.25.011 | TGGCAGTTGTTTCAGCTTCTGTAAG | 876 |
| Hu.DMD.Exon55.25.011.2 | TTCAGCTTCTGTAAGCCAGGCAAGA | 877 |
| Hu.DMD.Exon55.25.012 | GGTAGCATCCTGTAGGACATTGGCA | 978 |
| Hu.DMD.Exon55.25.012.2 | GACATTGGCAGTTGTTTCAGCTTCT | 879 |
| Hu.DMD.Exon55.25.013 | TCTAGGAGCCTTTCCTTACGGGTAG | 880 |
| Hu.DMD.Exon55.25.014 | CTTTTACTCCCTTGGAGTCTTCTAG | 881 |
| Hu.DMD.Exon55.25.014.2 | GAGCCTTTCCTTACGGGTAGCATCC | 882 |
| Hu.DMD.Exon55.25.015 | TTGCCATTGTTTCATCAGCTCTTTT | 883 |
| Hu.DMD.Exon55.25.015.2 | CTTGGAGTCTTCTAGGAGCCTTTCC | 884 |
| Hu.DMD.Exon55.25.016 | CTTACTTGCCATTGTTTCATCAGCT | 885 |
| Hu.DMD.Exon55.25.016.2 | CAGCTCTTTTACTCCCTTGGAGTCT | 886 |
| Hu.DMD.Exon55.25.017 | CCTGACTTACTTGCCATTGTTTCAT | 887 |
| Hu.DMD.Exon55.25.018 | AAATGCCTGACTTACTTGCCATTGT | 888 |
| Hu.DMD.Exon55.25.019 | AGCGGAAATGCCTGACTTACTTGCC | 889 |
| Hu.DMD.Exon55.25.020 | GCTAAAGCGGAAATGCCTGACTTAC | 890 |
| Hu.DMD.Exon55.20.001 | AAGGACCAAATGTTCAGATG | 891 |
| Hu.DMD.Exon55.20.002 | CTGCAAAGGACCAAATGTTC | 892 |
| Hu.DMD.Exon55.20.003 | TCACCCTGCAAAGGACCAAA | 893 |
| Hu.DMD.Exon55.20.004 | CTCACTCACCCTGCAAAGGA | 894 |
| Hu.DMD.Exon55.20.005 | TCTCGCTCACTCACCCTGCA | 895 |
| Hu.DMD.Exon55.20.006 | CAGCCTCTCGCTCACTCACC | 896 |
| Hu.DMD.Exon55.20.007 | CAAAGCAGCCTCTCGCTCAC | 897 |
| Hu.DMD.Exon55.20.008 | TCTATGAGTTTCTTCCAAAG | 898 |
| Hu.DMD.Exon55.20.009 | GAACTGTTGCAGTAATCTAT | 899 |
| Hu.DMD.Exon55.20.010 | TTCCAGGTCCAGGGGGAACT | 900 |
| Hu.DMD.Exon55.20.011 | CCAGGCAAGAAACTTTTCCA | 901 |
| Hu.DMD.Exon55.20.012 | TTCAGCTTCTGTAAGCCAGG | 902 |
| Hu.DMD.Exon55.20.013 | GACATTGGCAGTTGTTTCAG | 903 |
| Hu.DMD.Exon55.20.014 | GGTAGCATCCTGTAGGACAT | 904 |
| Hu.DMD.Exon55.20.015 | GAGCCTTTCCTTACGGGTAG | 905 |
| Hu.DMD.Exon55.20.016 | CTTGGAGTCTTCTAGGAGCC | 906 |
| Hu.DMD.Exon55.20.017 | CAGCTCTTTTACTCCCTTGG | 907 |
| Hu.DMD.Exon55.20.018 | TTGCCATTGTTTCATCAGCT | 908 |
| Hu.DMD.Exon55.20.019 | CTTACTTGCCATTGTTTCAT | 909 |
| Hu.DMD.Exon55.20.020 | CCTGACTTACTTGCCATTGT | 910 |
| Hu.DMD.Exon55.20.021 | AAATGCCTGACTTACTTGCC | 911 |
| Hu.DMD.Exon55.20.022 | AGCGGAAATGCCTGACTTAC | 912 |
| Hu.DMD.Exon55.20.023 | GCTAAAGCGGAAATGCCTGA | 913 |
| H50A(+02+30)-AVI-5656 | CCACTCAGAGCTCAGATCTTCTAACTTCC | 914 |
| H50D(+07-18)-AVI-5915 | GGGATCCAGTATACTTACAGGCTCC | 915 |
| H50A(+07+33) | CTTCCACTCAGAGCTCAGATCTTCTAA | 916 |
| H51A(+61+90)-AVI-4657 | ACATCAAGGAAGATGGCATTTCTAGTTTGG | 917 |
| H51A(+66+95)-AVI-4658 | CTCCAACATCAAGGAAGATGGCATTTCTAG | 918 |
| H51A(+111+134) | TTCTGTCCAAGCCCGGTTGAAATC | 919 |
| H51A(+175+195) | CACCCACCATCACCCTCYGTG | 920 |
| H51A(+199+220) | ATCATCTCGTTGATATCCTCAA | 921 |
| H51A(+66+90) | ACATCAAGGAAGATGGCATTTCTAG | 922 |
| H51A(-01+25) | ACCAGAGTAACAGTCTGAGTAGGAGC | 923 |
| h51AON1 | TCAAGGAAGATGGCATTTCT | 924 |
| h51AON2 | CCTCTGTGATTTTATAACTTGAT | 925 |
| H51D(+08-17) | ATCATTTTTTCTCATACCTTCTGCT | 926 |
| H51D(+16-07) | CTCATACCTTCTGCTTGATGATC | 927 |
| hAON#23 | TGGCATTTCTAGTTTGG | 928 |
| hAON#24 | CCAGAGCAGGTACCTCCAACATC | 929 |
| H44A(+61+84) | TGTTCAGCTTCTGTTAGCCACTGA | 930 |
| H44A(+85+104) | TTTGTGTCTTTCTGAGAAAC | 931 |
| h44AON1 | CGCCGCCATTTCTCAACAG | 932 |
| H44A(-06+14) | ATCTGTCAAATCGCCTGCAG | 933 |
| H45A(+71+90) | TGTTTTTGAGGATTGCTGAA | 934 |
| h45AON1 | GCTGAATTATTTCTTCCCC | 935 |
| h45AON5 | GCCCAATGCCATCCTGG | 936 |
| H45A(-06+20) | CCAATGCCATCCTGGAGTTCCTGTAA | 937 |
| H53A(+39+69) | CATTCAACTGTTGCCTCCGGTTCTGAAGGTG | 938 |
| H53A(+23+47) | CTGAAGGTGTTCTTGTACTTCATCC | 939 |
| h53AON1 | CTGTTGCCTCCGGTTCTG | 940 |
| H53A(-12+10) | ATTCTTTCAACTAGAATAAAAG | 941 |
| huEx45.30.66 | GCCATCCTGGAGTTCCTGTAAGATACCAAA | 942 |
| huEx45.30.71 | CCAATGCCATCCTGGAGTTCCTGTAAGATA | 943 |
| huEx45.30.79 | GCCGCTGCCCAATGCCATCCTGGAGTTCCT | 944 |
| huEx45.30.83 | GTTTGCCGCTGCCCAATGCCATCCTGGAGT | 945 |
| huEx45.30.88 | CAACAGTTTGCCGCTGCCCAATGCCATCCT | 946 |
| huEx45.30.92 | CTGACAACAGTTTGCCGCTGCCCAATGCCA | 947 |
| huEx45.30.96 | TGTTCTGACAACAGTTTGCCGCTGCCCAAT | 948 |
| huEx45.30.99 | CAATGTTCTGACAACAGTTTGCCGCTGCCC | 949 |
| huEx45.30.103 | CATTCAATGTTCTGACAACAGTTTGCCGCT | 950 |
| huEx45.30.120 | TATTTCTTCCCCAGTTGCATTCAATGTTCT | 951 |
| huEx45.30.127 | GCTGAATTATTTCTTCCCCAGTTGCATTCA | 952 |
| huEx45.30.132 | GGATTGCTGAATTATTTCTTCCCCAGTTGC | 953 |
| huEx45.30.137 | TTTGAGGATTGCTGAATTATTTCTTCCCCA | 954 |
| huEx53.30.84 | GTACTTCATCCCACTGATTCTGAATTCTTT | 955 |
| huEx53.30.88 | TCTTGTACTTCATCCCACTGATTCTGAATT | 956 |
| huEx53.30.91 | TGTTCTTGTACTTCATCCCACTGATTCTGA | 957 |
| huEx53.30.103 | CGGTTCTGAAGGTGTTCTTGTACTTCATCC | 958 |
| huEx53.30.106 | CTCCGGTTCTGAAGGTGTTCTTGTACTTCA | 959 |
| huEx53.30.109 | TGCCTCCGGTTCTGAAGGTGTTCTTGTACT | 960 |
| huEx53.30.112 | TGTTGCCTCCGGTTCTGAAGGTGTTCTTGT | 961 |
| huEx53.30.115 | AACTGTTGCCTCCGGTTCTGAAGGTGTTCT | 962 |
| huEx53.30.118 | TTCAACTGTTGCCTCCGGTTCTGAAGGTGT | 963 |

### Step 1: Antibody coniugation with maleimide-PEG-NHS followed by siRNA-DMD conjugates

Anti-dystrophin antibody is exchanged with 1X Phosphate buffer (pH 7.4) and made up to 5mg/ml concentration. To this solution, 2 equivalents of SMCC linker or maleimide-PEGxkDa-NHS (x = 1, 5, 10, 20) is added and rotated for 4 hours at room temperature. Unreacted maleimide-PEG is removed by spin filtration using 50 kDa MWCO Amicon spin filters and PBS pH 7.4. The antibody-PEG-Mal conjugate is collected and transferred into a reaction vessel. Various siRNA conjugates are synthesized using sequences listed in **Tables 13-17.** siRNA-DMD conjugates (2 equivalents) is added at RT to the antibody-PEG-maleimide in PBS and rotated overnight. The reaction mixture is analyzed by analytical SAX column chromatography and conjugate along with unreacted antibody and siRNA is seen.

### Step 2: Purification

The crude reaction mixture is purified by AKTA explorer FPLC using anion exchange chromatography. Fractions containing the antibody-PEG-DMD conjugate are pooled, concentrated and buffer exchanged with PBS, pH 7.4. Antibody siRNA conjugates with SMCC linker, PEG1kDa, PEG5kDa and PEG10kDa are separated based on the siRNA loading.

### Step-3: Analysis of the purified conjugate

The isolated conjugate is characterized by either mass spec or SDS-PAGE. The purity of the conjugate is assessed by analytical HPLC using anion exchange chromatography.

### Example 8. Additional Sequences

**Table 18 illustrates additional polynucleic acid molecule sequences described herein.**

| **Exon** | **AO name (h,H:Human; M:mouse)** | **Location from acceptor site** | **Sequence** | **SEQ ID NO:** |
|---|---|---|---|---|
| 2 | hEx2_Ac12 | 12 | CCA UUU UGU GAA UGU UUU CUU UUG AAC AUC | 964 |
| 2 | hEx2_Ac19 | 19 | CCC AU U UUG UGA AUG UUU UCU UUU | 965 |
| 2 | hEx2_Ac32 | 32 | UUG UGC AUU UAC CCA UUU UGU G | 966 |
| 2 | hEx2_Ac35 | 35 | GAA AAU UGU GCA UUU ACC CAU UUU | 967 |
| 3 | hEx3_Ac20 | 20 | GUA GGU CAC UGA AGA GGU UCU | 968 |
| 4 | hEx4_Ac11 | 11 | UGU UCA GGG CAU GAA CUC UUG UGG AUC CUU | 969 |
| 5 | hEx5_Ac25 | 25 | UCA GUU UAU GAU UUC CAU CUA CGA UGU CAG U | 970 |
| 6 | hEx6_Ac69 | 69 | UAC GAG UUG AUU GUC GGA CCC AG | 971 |
| 7 | hEx_Ac45 | 45 | UGC AUG UUC CAG UCG UUG UGU GG | 972 |
| 8 | hEx8_Ac-6 | -6 | GAU AGG UGG UAU CAA CAU CUG UAA | 973 |
| 8 | hEx8_Ac26 | 26 | CUU CCU GGA UGG CUU CAA U | 974 |
| 8 | hEx8_Ac84 | 84 | GUA CAU UAA GAU GGA CUU C | 975 |
| 9 | hEx9_Ac-6 | -6 | CCC UGU GCU AGA CUG ACC GUG AUC UGC AG | 976 |
| 10 | hEx10_Ac-5 | -5 | CAG GAG CUU CCA AAU GCU GCA | 977 |
| 10 | hEx10_Ac98 | 98 | UCC UCA GCA GAA AGA AGC CAC G | 978 |
| 11 | hEx11_Ac75 | 75 | CAU CUU CUG AUA AUU UUC CUG UU | 979 |
| 12 | hEx12_Ac52 | 52 | UCU UCU GUU UUU GUU AGC CAG UCA | 980 |
| 13 | hEx13_Ac77 | 77 | CAG CAG UUG CGU GAU CUC CAC UAG | 981 |
| 14 | hEx14_Ac32 | 32 | GUA AAA GAA CCC AGC GGU CUU CUG UCC AUC | 982 |
| 15 | hEx15_Ac48 | 48 | UCU UUA AAG CCA GUU GUG UGA AUC | 983 |
| 16 | hEx16_Ac12 | 12 | CUA GAU CCG CUU UUA AAA CCU GUU AAA ACA A | 984 |
| 16 | hEx16_Ac11 | 11 | GAU UGC UUU UUC UUU UCU AGA UCC G | 985 |
| 17 | hEx17_Ac-7 | -7 | UGA CAG CCU GUG AAA UCU GUG AG | 986 |
| 17 | hEx17_Ac36 | 36 | CCA UUA CAG UUG UCU GUG UU | 987 |
| 17 | hEx17_Ac132 | 132 | UAA UCU GCC UCU UCU UUU GG | 988 |
| 18 | hEx18_Ac24 | 24 | CAG CUU CUG AGC GAG UAA UCC AGC UGU GAA | 989 |
| 19 | hEx19_Ac35 | 35 | GCC UGA GCU GAU CUG CUG GCA UCU UGC AGU U | 990 |
| 19 | hEx19_Ac39 | 39 | UCU GCU GGC AUC UUG C | 991 |
| 20 | hEx20_Ac23 | 23 | GUU CAG UUG UUC UGA GGC UUG UUU G | 992 |
| 20 | mEx20 _Ac23 | 23 | GUU CAG UUG UUC UGA AGC UUG UCU G | 993 |
| 20 | hEx20_Ac44 | 44 | CUG GCA GAA UUC GAU CCA CCG GCU GUU C | 994 |
| 20 | mEx20_Ac44 | 44 | UUG GCA GAA UUC UGU CCA CCG GCU GUU C | 995 |
| 20 | hEx20_Ac140 | 140 | AGU AGU UGU CAU CUG CUC CAA UUG U | 996 |
| 20 | mEx20_Ac140 | 140 | AGU AGU UGU CAU CUG UUC CAA UUG U | 997 |
| 20 | hEx20_Ac1147 | 147 | CAG CAG UAG UUG UCA UCU GCU C | 998 |
| 20 | mEx20_Ac147 | 147 | CGG CAG UAG UUG UCA UCU GUU C | 999 |
| 21 | hEx21_Ac85 | 85 | CUG CAU CCA GGA ACA UGG GUC C | 1000 |
| 21 | mEx21_Ac85 | 85 | CUG CAU CCA GAA ACA UUG GCC C | 1001 |
| 21 | hEx21_Ac86 | 86 | GUC UGC AUC CAG GAA CAU GGG UC | 1002 |
| 22 | mEx22_Ac8 | 8 | AUG UCC ACA GAC CUG UAA UU | 1003 |
| 22 | hEx22_Ac8 | 8 | AUA UUC ACA GAC CUG CAA UU | 1004 |
| 22 | hEx22_Ac125 | 125 | CUG CAA UUC CCC GAG UCU CUG C | 1005 |
| 22 | mEx22_Ac125 | 125 | CUG UAA UUU CCC GAG UCU CUC C | 1006 |
| 23 | mEx23_Ac7 | 7 | GGC CAA ACC UCG GCU UAC CUG AAA U | 1007 |
| 23 | hEx23 _Ac7 | 7 | AGU AAA AUC UUG AAU UAC CUG AAU U | 1008 |
| 23 | hEx23_Ac69 | 69 | CGG CUA AUU UCA GAG GGC GCU UUC UUC GAC | 1009 |
| 23 | mEx23_Ac69 | 69 | UGG CAU AUU UCU GAA GGU GCU UUC UUG GCC | 1010 |
| 24 | mEx24_Ac16 | 16 | CAA CUU CAG CCA UCC AUU UCU GUA A | 1011 |
| 24 | hEx24_Ac16 | 16 | CAA CUU CAG CCA UCC AUU UCU UCA G | 1012 |
| 24 | hEx24_Ac51 | 51 | CAA GGG CAG GCC AUU CCU CCU UC | 1013 |
| 24 | mEx24_Ac51 | 51 | CCA GGG CAG GCC AUU CCU CUU UC | 1014 |
| 24 | mEx24_Ac78 | 78 | GAG CUG UUU UUU CAG GAU UUC AGC A | 1015 |
| 24 | hEx24_Ac78 | 78 | CAG CUG CUU UUU UAG AAU UUC UGA A | 1016 |
| 25 | hEx25_Ac95 | 95 | UUG AGU UCU GUC UCA AGU CUC GAA G | 1017 |
| 25 | mEx25_Ac95 | 95 | CUA AGU UCU GUC UCC AGU CUG GAU G | 1018 |
| 26 | hEx26_Ac-7 | -7 | CCU CCU UUC UGG CAU AGA CCU UCC AC | 1019 |
| 27 | hEx27_Ac82 | 82 | UUA AGG CCU CUU GUG CUA CAG GUG G | 1020 |
| 28 | hEx28_Ac99 | 99 | CAG AGA UUU CCU CAG CUC CGC CAG GA | 1021 |
| 29 | hEx29_Ac15 | 15 | UAU CCU CUG AAU GUC GCA UC | 1022 |
| 29 | hEx29_Ac18 | 18 | GGU UAU CCU CUG AAU GUC GC | 1023 |
| 29 | hEx29_Ac45 | 45 | UCU GUG CCA AUA UGC GAA UC | 1024 |
| 29 | hEx29_Ac57 | 57 | UCC GCC AUC UGU UAG GGU CUG UGC C | 1025 |
| 29 | hEx29_Ac59 | 59 | CCA UCU GUU AGG GUC UGU G | 1026 |
| 29 | hEx29_Ac105 | 105 | UUA AAU GUC UCA AGU UCC | 1027 |
| 29 | hEx29_Ac127 | 127 | GUA GUU CCC UCC AAC G | 1028 |
| 29 | hEx29_Ac131 | 131 | CAU GUA GUU CCC UCC | 1029 |
| 30 | hEx30_Ac25 | 25 | UCC UGG GCA GAC UGG AUG CUC UGU UC | 1030 |
| 31 | hEx31_Ac3 | 3 | UAG UUU CUG AAA UAA CAU AUA CCU G | 1031 |
| 32 | hEx32_Ac44 | 44 | CUU GUA GAC GCU GCU CAA AAU UGG CUG GUU | 1032 |
| 33 | hEx33_Ac64 | 64 | CCG UCU GCU UUU UCU GUA CAA UCU G | 1033 |
| 34 | hEx34_Ac46 | 46 | CAU UCA UUU CCU UUC GCA UCU UAC G | 1034 |
| 34 | hEx34_Ac95 | 95 | AUC UCU UUG UCA AUU CCA UAU CUG UA | 1035 |
| 35 | hEx35_Ac24 | 24 | UCU GUG AUA CUC UUC AGG UGC ACC UUC UGU | 1036 |
| 36 | hEx36_Ac22 | 22 | UGU GAU GUG GUC CAC AUU CUG GUC AAA AGU | 1037 |
| 37 | hEx37_Ac134 | 134 | UUC UGU GUG AAA UGG CUG CAA AUC | 1038 |
| 38 | hEx38_Ac88 | 88 | UGA AGU CUU CCU CUU UCA GAU UCA C | 1039 |
| 39 | hEx39_Ac62 | 62 | UUU CCU CUC GCU UUC UCU CAU CUG UGA UUC | 1040 |
| 40 | hEx40_Ac-5 | -5 | CUU UGA GAC CUC AAA UCC UGU U | 1041 |
| 40 | hEx40_Ac13 | 13 | GAG CCU UUU UUC UUC UUU G | 1042 |
| 40 | hEx40_Ac127 | 127 | UCC UUU CAU CUC UGG GCU C | 1043 |
| 41 | hEx41_Ac44 | 44 | CAA GCC CUC AGC UUG CCU ACG CAC UG | 1044 |
| 41 | hEx41_Ac18 | 18 | CUC CUC UUU CUU CUU CUG C | 1045 |
| 41 | hEx41_Ac145 | 145 | CUU CGA AAC UGA GCA AAU UU | 1046 |
| 42 | hEx42_Ac4 | 4 | AUC GUU UCU UCA CGG ACA GUG UGC UGG | 1047 |
| 42 | hEx42_Ac90 | 90 | CUU GUG AGA CAU GAG UG | 1048 |
| 42 | hEx42_Ac175 | 175 | CAG AGA CUC CUC UUG CUU | 1049 |
| 43 | hEx43_Ac52 | 52 | UGC UGC UGU CUU CUU GCU | 1050 |
| 43 | hEx43_Ac90 | 90 | CUG UAG CUU CAC CCU UUC C | 1051 |
| 43 | hEx43_Ac101 | 101 | GGA GAG AGC UUC CUG UAG CU | 1052 |
| 43 | hEx43_Ac132 | 132 | UGU UAA CUU UUU CCC AUU GG | 1053 |
| 43 | hEx43_Ac134 | 134 | UUG UUA ACU UUU UCC AUU | 1054 |
| 43 | hEx43_Acl37 | 137 | CAU UUU GUU AAC UUU UUC CC | 1055 |
| 44 | hEx44_Ac0 | 0 | CGC CAT TTC TCA ACA GAT CTG TCA AAT CGC | 1056 |
| 44 | hEx44_Ac1 | 1 | CCG CCA TTT CTC AAC AGA  TCTGTC AAA TCG | 1057 |
| 44 | hEx44_Ac2 | 2 | GCC GCC ATT TCT CAA CAG ATC TGT CAA ATC | 1058 |
| 44 | hEx44_Ac3 | 3 | AGC CGC CAT TTC TCA ACA GAT CTG TCA AAT | 1059 |
| 44 | hEx44_Ac4 | 4 | AAG CCG CCA TTT CTC AAC AGA TCT GTC AAA | 1060 |
| 44 | hEx44_Ac5 | 5 | AAA GCC GCC ATT TCT CAA CAG ATC TGT CAA | 1061 |
| 44 | hEx44_Ac6 | 6 | AAA AGC CGC CAT TTC TCA ACA GAT CTG TCA | 1062 |
| 44 | hEx44_Ac7 | 7 | AAA ACG CCG CCA TTT CTC AAC AGA TCT GTC | 1063 |
| 44 | hEx44_Ac8 | 8 | GAA AAC GCC GCC ATT TCT CAA CAG ATC TGT | 1064 |
| 44 | hEx44_Ac9 | 9 | TGA AAA CGC CGC CAT TTC TCA ACA GAT CTG | 1065 |
| 44 | hEx44_Ac10 | 10 | ATG AAA ACG CCG CCA TTT CTC AAC AGA TCT | 1066 |
| 44 | hEx44_Ac14 | 14 | CAT AAT GAA AAC GCC GCC ATT TCT CAA CAG | 1067 |
| 44 | hEx44_Ac15 | 15 | CGC CGC CAU UUC UCA ACA G | 1068 |
| 44 | hEx44_Ac18 | 18 | ATA TCA TAA TGA AAA CGC CGC CAT TTC TCA | 1069 |
| 44 | hEx44_Ac19 | 19 | TAT ATC ATA ATG AAA ACG CCG CCA TTT CTC | 1070 |
| 44 | hEx44_54 | 54 | TGT TCA GCT TCT GTT AGC CAC TGA TTA AAT | 1071 |
| 44 | hEx44_Ac56 | 56 | ACT GTT CAG CTT CTG TTA GCC ACT GAT TAA | 1072 |
| 44 | hEx44_Ac59 | 59 | GAA ACT GTT CAG CTT CTG TTA GCC ACT GAT | 1073 |
| 44 | hEx44_Ac61 | 61 | UGU UCA GCU UCU GUU AGC CAC UGA | 1074 |
| 44 | hEx44_Ac69 | 69 | GTC TTT CTG AGA AAC TGT TCA GCT TCT GTT | 1075 |
| 44 | hEx44_Ac87 | 87 | UUU GUA UUU AGC AUG UUC CC | 1076 |
| 45 | hEx45_Ac-6 | -6 | CCA AUG CCA UCC UGG AGU UCC UGU AA | 1077 |
| 45 | hEx45 _Ac0 | 0 | TTG CCG CTG CCC AAT GCC ATC CTG GAG TTC | 1078 |
| 45 | hEx45_Ac1 | 1 | TTT GCC GCT GCC CAA TGC CAT CCT GGA GTT | 1079 |
| 45 | hEx45_Ac2 | 2 | GTT TGC CGC TGC CCA ATG CCA TCC TGG AGT | 1080 |
| 45 | hEx45_Ac3 | 3 | AGT TTG CCG CTG CCC AAT GCC ATC CTG GAG | 1081 |
| 45 | hEx45_Ac4 | 4 | CAG TTT GCC GCT GCC CAA TGC CAT CCT GGA | 1082 |
| 45 | hEx45_Ac6 | 6 | GCC CAA UGC CAU CCU GG | 1083 |
| 45 | hEx45_Ac7 | 7 | CAA CAG TTT GCC GCT GCC CAA TGC CAT CCT | 1084 |
| 45 | hEx45_Ac8 | 8 | ACA ACA GTT TGC CGC TGC CCA ATG CCA TCC | 1085 |
| 45 | hEx45_Ac9 | 9 | GAC AAC AGT TTG CCG CTG CCC AAT GCC ATC | 1086 |
| 45 | hEx45_Ac10 | 10 | TGA CAA CAG TTT GCC GCT GCC CAA TGC CAT | 1087 |
| 45 | hEx45_Ac11 | 11 | CTG ACA ACA GTT TGC CGC TGC CCA ATG CCA | 1088 |
| 45 | hEx45_Ac12 | 12 | TCT GAC AAC AGT TTG CCG CTG CCC AAT GCC | 1089 |
| 45 | hEx45_Ac58 | 58 | GCU GAA UUA UUU CUU CCC C | 1090 |
| 45 | hEx45_Ac75 | 75 | UCU GUU UUU GAG GAU UGC | 1091 |
| 45 | hEx45_Ac122 | 122 | CCA CCG CAG AUU CAG GC | 1092 |
| 45 | hEx45_Ac137 | 137 | UUU GCA GAC CUC CUG CC | 1093 |
| 45 | hEx45_Ac154 | 154 | UUU UUC UGU CUG ACA GCU G | 1094 |
| 46 | hEx46_Ac14 | 14 | CUG ACA AGA UAU UCU U | 1095 |
| 46 | hEx46_Ac15 | 15 | GAA AUU CUG ACA AGA UAU UCU | 1096 |
| 46 | hEx46_Ac45 | 45 | CTT CCT CCA ACC ATA AAA CAA ATT CAT TTA | 1097 |
| 46 | hEx46_Ac46 | 46 | GCT TCC TCC AAC CAT AAA ACA AAT TCA TTT | 1098 |
| 46 | hEx46_Ac47 | 47 | TGC TTC CTC CAA CCA TAA AAC AAA TTC ATT | 1099 |
| 46 | hEx46Ac47 | 47 | UAA AAC AAA UUC AUU | 1100 |
| 46 | hEx46_Ac48 | 48 | CTG CTT CCT CCA ACC ATA AAA CAA ATT CAT | 1101 |
| 46 | hEx46_Ac49 | 49 | TCT GCT TCC TCC AAC CAT AAA ACA AAT TCA | 1102 |
| 46 | hEx46_Ac50 | 50 | ATC TGC TTC CTC CAA CCA TAA AAC AAA TTC | 1103 |
| 46 | hEx46_Ac51 | 51 | TAT CTG CTT CCT CCA ACC ATA AAA CAA ATT | 1104 |
| 46 | hEx46_Ac52 | 52 | TTA TCT GCT TCC TCC AAC CAT AAA ACA AAT | 1105 |
| 46 | hEx46_Ac53 | 53 | GTT ATC TGC TTC CTC CAA CCA TAA AAC AAA | 1106 |
| 46 | hEx46_Ac54 | 54 | TGT TAT CTG CTT CCT CCA ACC ATA AAA CAA | 1107 |
| 46 | hEx46_Ac55 | 55 | ATG TTA TCT GCT TCC TCC AAC CAT AAA ACA | 1108 |
| 46 | hEx46_Ac56 | 56 | AAT GTT ATC TGC TTC CTC CAA CCA TAA AAC | 1109 |
| 46 | hEx46_Ac57 | 57 | CAA TGT TAT CTG CTT CCT CCA ACC ATA AAA | 1110 |
| 46 | hEx46_Ac58 | 58 | GCA ATG TTA TCT GCT TCC TCC AAC CAT AAA | 1111 |
| 46 | hEx46_Ac59 | 59 | AGC AAT GTT ATC TGC TTC CTC CAA CCA TAA | 1112 |
| 46 | hEx46 Ac60 | 60 | TAG CAA TGT TAT CTG CTT CCT CCA ACC ATA | 1113 |
| 46 | hEx46_Ac61 | 61 | CTA GCA ATG TTA TCT GCT TCC TCC AAC CAT | 1114 |
| 46 | hEx46_Ac62 | 62 | ACT AGC AAT GTT ATC TGC TTC CTC CAA CCA | 1115 |
| 46 | hEx46_Ac63 | 63 | GUU AUC UGC UUC CUC CAA CC | 1116 |
| 46 | hEx46_Ac88 | 88 | AGG UUC AAG UGG GAU ACU A | 1117 |
| 46 | hEx46_Ac90 | 90 | UCC AGG UUC AAG UGG GAU AC | 1118 |
| 46 | hEx46_Ac96 | 96 | UUC CAG GUU CAA GUG | 1119 |
| 46 | hEx46_Ac107 | 107 | CAA GCU UUU CUU UUA GUU GCU GCU CUU UUC C | 1120 |
| 46 | hEx46_Ac111 | 111 | UUA GUU GCU GCU CUU | 1121 |
| 46 | hEx46_Ac115 | 115 | GCU UUU CUU UUA GUU GCU GC | 1122 |
| 46 | hEx46_Ac122 | 122 | UCA AGC UUU UCU UUU AG | 1123 |
| 47 | hEx47_Ac-6 | -6 | CAG GGG CAA CUC UUC CAC CAG UAA CUG AAA | 1124 |
| 47 | hEx47_Ac39 | 39 | UCC AGU UUC AUU UAA UUG UUU G | 1125 |
| 47 | hEx47_Ac63 | 63 | AGC ACU UAC AAG CAC GGG U | 1126 |
| 47 | hEx47_Ac87 | 87 | UCU UGC UCU UCU GGG CUU | 1127 |
| 47 | hEx47_Ac94 | 94 | UUC AAG UUU AUC UUG CUC UUC | 1128 |
| 47 | hEx47_Ac101 | 101 | CUU GAG CUU AUU UUC AAG UUU | 1129 |
| 47 | hEx47_Ac103 | 103 | CUG CUU GAG CUU AUU UUC AAG UU | 1130 |
| 48 | hEx48_Ac-7 | -7 | UUC UCA GGU AAA GCU CUG GAA ACC UGA AAG | 1131 |
| 48 | hEx48_Ac2 | 2 | CUU CAA GCU UUU UUU CAA GCU | 1132 |
| 48 | hEx48_Ac19 | 19 | UUU CUC CUU GUU UCU C | 1133 |
| 48 | hEx48__Ac23 | 23 | GCU UCA AUU UCU CCU UGU U | 1134 |
| 48 | hEx48_Ac32 | 32 | UUU AUU UGA GCU UCA AUU U | 1135 |
| 48 | hEx48_Ac37 | 37 | GGU CUU UUA UUU GAG CUU C | 1136 |
| 48 | hEx48_Ac48 | 48 | GCU GCC CAA GGU CUU UU | 1137 |
| 48 | hEx48_Ac71 | 71 | CUU CAA GGU CUU CAA GCU UUU | 1138 |
| 48 | hEx48_Ac79 | 79 | UAA CUG CUC UUC AAG GUC UUC | 1139 |
| 48 | hEx48_Ac133 | 133 | UUA UAA AUU UCC AAC UGA UUC | 1140 |
| 49 | hEx49_Ac-11 | -11 | CUG CUA UUU CAG UUU CCU GGG GAA AAG | 1141 |
| 49 | hEx49_Ac25 | 25 | CUU CCA CAU CCG GUU GUU U | 1142 |
| 49 | hEx49_Ac60 | 60 | GUG GCU GGU UUU UCC UUG U | 1143 |
| 50 | hEx50_Ac2 | 2 | CCA CUC AGA GCU CAG AUC UUC UAA CUU CC | 1144 |
| 50 | hEx50_Ac11 | 11 | CUC AGA GCU CAG AUC UU | 1145 |
| 50 | hEx50_Ac36 | 36 | GGC UGC UUU GCC CUC | 1146 |
| 51 | hEx51_Ac0 | 0 | GTG TCA CCA GAG TAA CAG TCT GAG TAG GAG | 1147 |
| 51 | hEx51_Ac5 | 5 | AGG TTG TGT CAC CAG AGT AAC AGT CTG AGT | 1148 |
| 51 | hEx51_Ac9 | 9 | CCA CAG GTT GTG TCA CCA GAG TAA CAG TCT | 1149 |
| 51 | hEx51_Ac26 | 26 | GGC AGT TTC CTT AGT AAC CAC AGG TTG TGT | 1150 |
| 51 | hEx51_Ac30 | 30 | AGA TGG CAG TTT CCT TAG TAA CCA CAG GTT | 1151 |
| 51 | hEx51_Ac48 | 48 | ATG GCA TTT CTA GTT TGG AGA TGG CAG TTT | 1152 |
| 51 | hEx51_Ac65 | 65 | CTC CAA CAT CAA GGA AGA TGG CAT TTC TAG | 1153 |
| 51 | hEx51_Ac66 | 66 | ACA UCA AGG AAG AUG GCA UUU CUA G | 1154 |
| 51 | hEx51_Ac67 | 67 | TCA AGG AAG ATG GCA TTT CT | 1155 |
| 51 | hEx51_Ac68 | 68 | UCA AGG AAG AUG GCA UUU CU | 1156 |
| 51 | hEx5_Ac132 | 132 | GAA AGC CAG UCG GUA AGU UC | 1157 |
| 51 | hEx51_Ac141 | 141 | TTA TAA CTT GAT CAA GCA GAG AAA GCC AGT | 1158 |
| 51 | hEx51_Ac160 | 160 | CCU CUG UGA UUU UAU AAC UUG AU | 1159 |
| 51 | hEx31_Ac181 | 181 | CAC CCA CCA UCA CCC | 1160 |
| 51 | hEx51_Ac191 | 191 | UGA UAU CCU CAA GGU CAC CC | 1161 |
| 51 | hEx51_Ac207 | 207 | ATA CCT TCT GCT TGA TGA TCA TCT CGT TGA | 1162 |
| 52 | hEx52_Ac12 | 12 | UCC AAC UGG GGA CGC CUC UGU UCC AAA UCC | 1163 |
| 52 | mEx52_Ac12 | 12 | UCC AAU UGG GGG CGU CUC UGU UCC AAA UCU | 1164 |
| 52 | mEx52_Ac17 | 17 | UCC AAU UGG GGG CGU CUC UGU UCC A | 1165 |
| 52 | hEx52_Ac17 | 17 | UCC AAC UGG GGA CGC CUC UGU UCC A | 1166 |
| 52 | hEx52_Ac18 | 18 | UUC CAA CUG GGG ACG CCU CUG UUC C | 1167 |
| 52 | hEx52_Ac24 | 24 | GGT AAT GAG TTC TTC CAA CTG GGG ACG CCT | 1168 |
| 52 | mEx52_Ac42 | 42 | UUC AAA UUC UGG GCA GCA GUA AUG AGU UCU | 1169 |
| 52 | hEx52_Ac42 | 42 | UUC AAA UUU UGG GCA GCG GUA AUG AGU UCU | 1170 |
| 52 | hEx52_Ac69 | 69 | UUG CUG GUC UUG UUU UUC | 1171 |
| 52 | hEx52_Ac97 | 97 | CCG UAA UGA UUG UUC U | 1172 |
| 53 | hEx53_Ac1 | 1 | ACT TCA TCC CAC TGA TTC TGA ATT CTT TCA | 1173 |
| 53 | hEx53_Ac2 | 2 | TAC TTC ATC CCA CTG ATT CTG AAT TCT TTC | 1174 |
| 53 | hEx53_Ac3 | 3 | GTA CTT CAT CCC ACT GAT TCT GAA TTC TTT | 1175 |
| 53 | hEx53_Ac4 | 4 | TGT ACT TCA TCC CAC TGA TTC TGA ATT CTT | 1176 |
| 53 | mEx53_Ac5 | 5 | UUU UAA AGA UAU GCU UGA CAC UAA CCU UGG | 1177 |
| 53 | hEx53_Ac5 | 5 | UUA AAA AGG UAU CUU UGA UAC UAA CCU UGG | 1178 |
| 53 | hEx53_Ac5 | 5 | TTG TAC TTC ATC CCA CTG ATT CTG AAT TCT | 1179 |
| 53 | hEx53_Ac6 | 6 | CTT GTA CTT CAT CCC ACT GAT TCT GAA TTC | 1180 |
| 53 | hEx53 _Ac7 | 7 | TCT TGT ACT TCA TCC CAC TGA TTC TGA ATT | 1181 |
| 53 | hEx53 _Ac8 | 8 | TTC TTG TAC TTC ATC CCA CTG ATT CTG AAT | 1182 |
| 53 | hEx53_Ac9 | 9 | GTT CTT GTA CTT CAT CCC ACT GAT TCT GAA | 1183 |
| 53 | hEx53_Ac10 | 10 | TGT TCT TGT ACT TCA TCC CAC TGA TTC TGA | 1184 |
| 53 | hEx53_Ac11 | 11 | GTG TTC TTG TAC TTC ATC CCA CTG ATT CTG | 1185 |
| 53 | hEx53_Ac12 | 12 | GGT GTT CTT GTA CTT CAT CCC ACT GAT TCT | 1186 |
| 53 | hEx53_Ac13 | 13 | AGG TGT TCT TGT ACT TCA TCC CAC TGA TTC | 1187 |
| 53 | hEx53_Ac14 | 14 | AAG GTG TTC TTG TAC TTC ATC CCA CTG ATT | 1188 |
| 53 | hEx53_Ac15 | 15 | GAA GGT GTT CTT GTA CTT CAT CCC ACT GAT | 1189 |
| 53 | hEx53_Ac16 | 16 | TGA AGG TGT TCT TGT ACT TCA TCC CAC TGA | 1190 |
| 53 | hEx53_Ac7 | 17 | CTG AAG GTG TTC TTG TAC TTC ATC CCA CTG | 1191 |
| 53 | hEx53_Ac18 | 18 | TCT GAA GGT GTT CTT GTA CTT CAT CCC ACT | 1192 |
| 53 | hEx53_Ac19 | 19 | TTC TGA AGG TGT TCT TGT ACT TCA TCC CAC | 1193 |
| 53 | hEx53_Ac20 | 20 | GTT CTG AAG GTG TTC TTG TAC TTC ATC CCA | 1194 |
| 53 | hEx53_Ac21 | 21 | GGT TCT GAA GGT GTT CTT GTA CTT CAT CCC | 1195 |
| 53 | hEx53_Ac22 | 22 | CGG TTC TGA AGG TGT TCT TGT ACT TCA TCC | 1196 |
| 53 | hEx53_Ac23 | 23 | CCG GTT CTG AAG GTG TTC TTG TAC TTC ATC | 1197 |
| 53 | hEx53_Ac24 | 24 | TCC GGT TCT GAA GGT GTT CTT GTA CTT CAT | 1198 |
| 53 | hEx53_Ac25 | 25 | CTC CGG TTC TGA AGG TGT TCT TGT ACT TCA | 1199 |
| 53 | hEx53_Ac26 | 26 | CCT CCG GTT CTG AAG GTG TTC TTG TAC TTC | 1200 |
| 53 | hEx53_Ac27 | 27 | GCC TCC GGT TCT GAA GGT GTT CTT GTA CTT | 1201 |
| 53 | hEx53_Ac28 | 28 | TGC CTC CGG TTC TGA AGG TGT TCT TGT ACT | 1202 |
| 53 | hEx53_Ac20 | 29 | TTG CCT CCG GTT CTG AAG GTG TTC TTG TAC | 1203 |
| 53 | hEx53_Ac30 | 30 | GTT GCC TCC GGT TCT GAA GGT GTT CTT GTA | 1204 |
| 53 | hEx53_Ac39 | 39 | CAU UCA ACU GUU GCC UCC GGU UCU GAA GGU G | 1205 |
| 53 | mEx53_Ac39 | 39 | CAU UCA ACU GUU GUC UCC UGU UCU GCA GCU G | 1206 |
| 53 | hEx53_Ac45 | 45 | CUG UUG CCU CCG GUU CUG | 1207 |
| 53 | hEx53_Ac69 | 69 | CAG CCA UUG UGU UGA AUC CUU UAA CAU UUC | 1208 |
| 53 | hEx53_Ac128 | 128 | UUG GCU CUG GCC UGU CCU | 1209 |
| 53 | mEx53_Ac151 | 151 | CUA CUG UGU GAG GAC CUU CUU UCC AUG AGU | 1210 |
| 53 | mEx53_Ac176 | 176 | UCU GUG AUC UUC UUU UGG AUU GCA UCU ACU | 1211 |
| 54 | hEx54_Ac21 | 21 | UAC AUU UGU CUG CCA CUG G | 1212 |
| 54 | hEx54_Ac42 | 42 | GAG AAG TTT CAG GGC CAA GTC ATT TGC CAC | 1213 |
| 54 | hEx54_Ac58 | 58 | CCC GGA GAA GUU UCA GGG | 1214 |
| 54 | hEx54_Ac67 | 67 | UCU GCA GAA UAA UCC CGG AGA AG | 1215 |
| 55 | hEx55_Ac0 | 0 | TCT TCC AAA GCA GCC TCT CGC TCA CTC ACC | 1216 |
| 55 | hEx55_Ac29 | 29 | UGC AGU AAU CUA UGA GUU UC | 1217 |
| 55 | hEx55_Ac33 | 33 | CUG UUG CAG UAA UCU AUG AG | 1218 |
| 55 | hEx55_Ac104 | 104 | UCC UGU AGG ACA UUG GCA GU | 1219 |
| *55* | hEx55_A139 | 139 | GAG UCU UCU AGG AGC CUU | 1220 |
| 55 | hEx55_Ac141 | 141 | CUU GGA GUC UUC UAG GAG CC | 1221 |
| 55 | hEx55_Ac167 | 167 | UGC CAU UGU UUC AUC AGC UCU UU | 1222 |
| 56 | hEx56_Ac48 | 48 | UUU UUU GGC UGU UUU CAU CC | 1223 |
| 56 | hEx56_Ac69 | 69 | CCU UCC AGG GAU CUC AGG | 1224 |
| 56 | hEx56_Ac102 | 102 | GUU AUC CAA ACG UCU UUG UAA CAG G | 1225 |
| 56 | hEx56_Ac129 | 129 | GUU CAC UCC ACU UGA AGU UC | 1226 |
| 57 | hEx57_Ac-12 | -12 | CUG GCU UCC AAA UGG GAC CUG AAA AAG AAC | 1227 |
| 57 | hEx57_Ac64 | 64 | UUC AGC UGU AGC CAC ACC | 1228 |
| 57 | hEx57_Ac97 | 97 | UAG GUG CCu GCC GGC UU | 1229 |
| 57 | hEx57_Ac118 | 118 | CUG AAC UGC UGG AAA GUC GCC | 1230 |
| 58 | hEx58_Ac9 | 9 | UUC UUU AGU UUU CAA UUC CCU C | 1231 |
| 58 | hEx58_Ac21 | 21 | ACU CAU GAU UAC ACG UUC UUU AGU U | 1232 |
| 58 | hEx58_Ac86 | 86 | GAG UUU CUC UAG UCC UUC C | 1233 |
| 59 | hEx59_Ac6 | 6 | UCC UCA GGA GGC AGC UCU AAA U | 1234 |
| 59 | hEx59_Ac66 | 66 | GAG UUU CUC UAG UCC UUC C | 1235 |
| 59 | hEx59 _Ac134 | 134 | UUG AAG UUC CUG GAG UCU U | 1236 |
| 60 | hEx60 _Ac19 | 19 | GUU CUC UUU CAG AGG CGC | 1237 |
| 60 | hEx60_Ac37 | 37 | CUG GCG AGC AAG GUC CUU GAC GUG GCU CAC | 1238 |
| 60 | hEx60_Ac92 | 92 | GUG CUG AGG UUA UAC GGU G | 1239 |
| 61 | hEx61_Ac10 | 10 | GGG CUU CAU GCA GCU GCC UGA CUC GGU CCU C | 1240 |
| 61 | hEx61_Ac31 | 31 | GUC CCU GUG GGC UUC AUG | 1241 |
| 61 | hEx61_Ac51 | 51 | GUG CUG AGA UGC UGG ACC | 1242 |
| 62 | hEx62 _Ac8 | 8 | GAG AUG GCU CUC UCC CAG GGA CCC UGG | 1243 |
| 62 | hEx62_Ac15 | 15 | UGG CUC UCU CCC AGG G | 1244 |
| 62 | hEx62_Ac37 | 37 | GGG CAC UUU GUU UGG CG | 1245 |
| 63 | hEx63_Ac11 | 11 | UGG GAU GGU CCC AGC AAG UUG UUU G | 1246 |
| 63 | hEx63_Ac11 | 11 | GGU CCC AGC AAG UUG UUU G | 1247 |
| 63 | hEx63_Ac33 | 33 | GUA GAG CUC UGU CAU UUU GGG | 1248 |
| 64 | hEx64_Ac47 | 47 | GCA AAG GGC CUU CUG CAG UCU UCG GAG | 1249 |
| 65 | hEx65_Ac-11 | -11 | GCU CAA GAG AUC CAC UGC AAA AAA C | 1250 |
| 65 | mEx65_Ac-11 | -11 | GCU CAA GAG AUC CAC UGC AAA AAA G | 1251 |
| 65 | hEx65_Ac15 | 15 | GCC AUA CGU ACG UAU CAU AAA CAU UC | 1252 |
| 65 | hEx65_Ac26 | 26 | GUU GUG CUG GUC CAA GGC AUC ACA U | 1253 |
| 65 | mEx65_Ac26 | 26 | GUU GUG CUG GUC CAG GGC AUC ACA U | 1254 |
| 65 | hEx65_Ac63 | 63 | UCU GCA GGA UAU CCA UGG GCU GGU C | 1255 |
| 65 | hEx65_Ac63 | 63 | UCU GCA GGA UAU CCA UGG GCU GGU C | 1256 |
| 66 | hEx66_Ac-8 | -8 | GAU CCU CCC UGU UCG UCC CCU AUU AUG | 1257 |
| 67 | hEx67_Ac22 | 22 | GCG CUG GUC ACA AAA UCC UGU UGA AC | 1258 |
| 68 | hEx68_Ac22 | 22 | CAU CCA GUC UAG GAA GAG GGC CGC UUC | 1259 |
| 69 | hEx69 _Ac-6 | -6 | UGC UUU AGA CUC CUG UAC CUG AUA | 1260 |
| 70 | hEx70_Ac98 | 98 | CCU CUA AGA CAG UCU GCA CUG GCA | 1261 |
| 71 | hEx71_Ac-3 | -3 | AAG UUG AUC AGA GUA ACG GGA CUG | 1262 |
| 71 | hEx71_Ac8 | 8 | GCC AGA AGU UGA UCA GAG U | 1263 |
| 71 | hEx71_Ac16 | 16 | UCU ACU GGC CAG AAG UUG | 1264 |
| 72 | hEx72_Ac2 | 2 | GUG UGA AAG CUG AGG GGA CGA GGC AGG | 1265 |
| 72 | hEx72_Ac20 | 20 | UGA GUA UCA UCG UGU GAA AG | 1266 |
| 72 | hEx7 _Ac42 | 42 | GCA UAA UGU UCA AUG CGU G | 1267 |
| 73 | hEx73_Ac6 | 6 | GAU CCA UUG CUG UUU UCC AUU UCU G | 1268 |
| 73 | **hEx73_**Ac13 | 13 | GAU CCA UUG CUG UUU UCC | 1269 |
| 73 | hEx73_Ac31 | 31 | GAG AUG CUA UCA UUU AGA UAA | 1270 |
| 74 | hEx74_Ac48 | 48 | CGA GGC UGG CUC AGG GGG GAG UCC U | 1271 |
| 74 | hEx74_Ac51 | 51 | CUG GCU CAG GGG GGA GU | 1272 |
| 74 | hEx74_Ac72 | 72 | UCC CCU CUU UCC UCA CUC U | 1273 |
| 75 | hEx75_Ac34 | 34 | GGA CAG GCC UUU AUG UUC GUG CUG C | 1274 |
| 75 | hEx75_Ac33 | 33 | CCU UUA UGU UCG UGC UGC U | 1275 |
| 75 | hEx75_Ac144 | 144 | GGC GGC CUU UGU GUU GAC | 1276 |
| 76 | hEx76_Ac53 | 53 | GCU GAC UGC UGU CGG ACC UCU GUA GAG | 1277 |
| 76 | hEx76_Ac37 | 37 | GAG AGG UAG AAG GAG AGG A | 1278 |
| 76 | hEx76_Ac65 | 65 | AUA GGC UGA CUG CUG UCG G | 1279 |
| 77 | hEx77_Ac16 | 16 | CUG UGC UUG UGU CCU GGG GAG GAC UGA | 1280 |
| 77 | hEx77_Ac20 | 20 | UUG UGU CCU GGG GAG GA | 1281 |
| 77 | hEx77_A47 | 47 | UGC UCC AUC ACC UCC UCU | 1282 |
| 78 | hEx78_Ac4 | 4 | UCU CAU UGG CUU UCC AGG GGU AUU UC | 1283 |
| 78 | hEx78_Ac4 | 4 | GCU UUC CAG GGG UAU UUC | 1284 |
| 78 | hEx78_Ac10 | 10 | CAU UGG CUU UCC AGG GG | 1285 |

### Example 9. Screening of DMD exon 44 and 45 skipping PMOs in transfected primary human skeletal muscle cells

Primary, pre-differentiated human skeletal muscle cells (Gibco, #A11440) were plated on collagen Type 1 coated 24-well plates (Gibco, #1970788) in DMEM supplemented with 2% horse serum) and 1x ITS (Gibco, #1933286) according to the manufacturer's instructions. Cells were grown in 37°C + 5% CO₂ for 2 days to establish myotubes. These cells were then treated with defined concentrations of PMOs in water and 2 uM Endo-Porter (Gene Tools, #EP6P1-1) to facilitate PMO uptake into cells. Cell were harvested 48 hours after treatment by aspirating the culture medium and addition of 300 ul TRIZOL per well. Cells were frozen at -80°C before RNA was prepared using Direct-zol^{™}-96 RNA kit (Zymo Research, #R2056). Total RNA concentration was quantified spectroscopically. Between 100-200 ng total RNA was reverse transcribed using High Capacity cDNA Reverse Transcription kit (Applied Biosystems, #4368813). RT PCR reactions were incubated at 25°C for 10 min, 37°C for 120 min, 85°C for 5 min, and then held at 4°C. Reactions were diluted 1:1 with water. For quantification of exon skipping by gel electrophoresis DNA fragments representing total (non-skipped +skipped) and skipped mRNAs were amplified by qPCR using Taqman Fast Advanced Master mix (Applied Biosystems, #4444558) and specific primer pairs (see Table 19). qPCR reactions were incubated at 95°C for 20 sec, followed by 32 cycles of 95°C for 1 sec and 60°C for 20 sec using a QuantStudio 7 Flex (Applied Biosystems). PCR products were diluted 4:1 with TAE loading buffer and loaded onto 24-well 4% TAE gels (Embi Tec, #GG3807) containing GelGreen. PCR products were separated by electrophoresis (50 V for 2 hrs). The intensity of bands corresponding to total DMD and skipped DMD products were quantified by densiometry using ChemiDoc ^{™} XRS+ (Bio-Rad).

### Taqman qPCR primers and probes are illustrated in Table 19.

| | | |
|---|---|---|
| **hDMD Ex44 skipped** | Forward: | 5'-CTGTGGAAAGGGTGAAGCTA-3' |
| | Reverse: | 5'-GACAAGGGAACTCCAGGATG-3 |
| | Probe: | 5'-AGCTCTCTCCCAGCTTGATTTCCA-3' |
| **hDMD Ex45 skipped** | Forward: | 5'-CAGTGGCTAACAGAAGCTGA-3' |
| | Reverse: | 5'-CAAATGGTATCTTAAGGCTAGAAGAAC-3' |
| | Probe: | 5'-ACACAAATTCCTGAGAATTGGGAACATGC-3' |

### hDMD total Hs01049401_m1, human DMD VIC-MGR, 360 rxns (Thermo Fisher Scientific)

**Table 20A illustrates exon skipping activity of PMOs (30mer) targeting DMD exon 45 in transfected primary human skeletal muscle cells.**

| | **PMO conc** | **% Skipping (skipped/total)** | |
|---|---|---|---|
| | uM | AVG | STDEV |
| hEx45_Ac1 | 10.0 | 43.5 | 6.4 |
| | 3.0 | 38.5 | 9.2 |
| | 1.0 | 29.5 | 3.5 |
| hEx45_Ac2 | 10.0 | 67.0 | 14.1 |
| | 3.0 | 71.5 | 14.8 |
| | 1.0 | 38.0 | 7.8 |
| | 0.1 | 10.0 | |
| hEx45_Ac3 | 10.0 | 69.5 | 2.1 |
| | 3.0 | 56.5 | 10.6 |
| | 1.0 | 34.0 | 8.5 |
| hEx45_Ac4 | 10.0 | 51.7 | 10.4 |
| | 3.0 | 49.0 | 1.4 |
| | 1.0 | 34.0 | 5.3 |
| | 0.1 | 18.0 | |
| hEx45_Ac7 | 10.0 | 72.0 | 11.4 |
| | 3.0 | 62.5 | 2.1 |
| | 1.0 | 43.3 | 4.9 |
| | 0.1 | 18.0 | |
| hEx45_Ac8 | 10.0 | 76.0 | 8.5 |
| | 3.0 | 69.5 | 12.0 |
| | 1.0 | 43.5 | 19.1 |
| hEx45_Ac9 | 10.0 | 73.7 | 6.0 |
| | 3.0 | 62.5 | 9.2 |
| | 1.0 | 47.3 | 8.3 |
| | 0.1 | 20.0 | |
| hEx45_Ac10 | 10.0 | 53.0 | 0.0 |
| | 3.0 | 56.5 | 10.6 |
| | 1.0 | 35.5 | 0.7 |
| hEx45_Ac11 | 10.0 | 54.5 | 2.1 |
| | 3.0 | 53.0 | 1.4 |
| | 1.0 | 34.0 | 4.2 |
| hEx45_Ac2 | 10.0 | 52.0 | 21.2 |
| | 3.0 | 40.0 | 14.1 |
| | 1.0 | 26.5 | 10.6 |
| No PMO | 0 | 10.5 | 6.4 |

**Table 20B illustrates exon skipping activity of PMOs (30mer) targeting DMD exon 44 in transfected primary human skeletal muscle cells.**

| | **PMO conc** | **% Skipping (skipped/total)** | |
|---|---|---|---|
| | uM | AVG | STDEV |
| hEx44_Ac0 | 10 | 83.8 | 11.3 |
| | 3 | 79.7 | 3.5 |
| | 1 | 67.5 | 7.8 |
| | 0.1 | 31.5 | 0.7 |
| hEx44_Ac1 | 10 | 77.7 | 8.3 |
| | 3 | 79.5 | 0.7 |
| | 1 | 68.3 | 8.5 |
| | 0.1 | 32.0 | |
| hEx44_Ac2 | 10 | 88.7 | 4.5 |
| | 3 | 96.0 | 7.1 |
| | 1 | 70.0 | 13.2 |
| | 0.1 | 31.0 | |
| hEx44_Ac3 | 10 | 75.0 | 14.1 |
| | 3 | 89.0 | |
| | 1 | 62.0 | 8.5 |
| | 0.1 | 26.0 | |
| hEx44_Ac4 | 10 | 84.0 | 17.0 |
| | 3 | 88.0 | |
| | 1 | 67.0 | 15.6 |
| | 0.1 | 23.0 | |
| hEx44_Ac5 | 10 | 63.0 | 0.0 |
| | 3 | 68.0 | |
| | 1 | 54.0 | 8.5 |
| | 0.1 | 18.0 | |
| hEx44_Ac6 | 10 | 74.0 | 12.7 |
| | 3 | 81.0 | |
| | 1 | 58.5 | 17.7 |
| | 0.1 | 20.0 | |
| hEx44_Ac7 | 10 | 84.3 | 19.5 |
| | 3 | 85.0 | 4.2 |
| | 1 | 59.3 | 13.0 |
| | 0.1 | 23.0 | |
| hEx44_Ac8 | 10 | 76.0 | 0.0 |
| | 3 | 70.0 | |
| | 1 | 53.5 | 2.1 |
| | 0.1 | 27.0 | |
| hEx44_Ac9 | 10 | 76.5 | 2.1 |
| | 3 | 73.0 | |
| | 1 | 59.0 | 15.6 |
| | 0.1 | 32.0 | |
| hEx44_Ac10 | 10 | 85.0 | 18.4 |
| | 3 | 79.0 | |
| | 1 | 45.5 | 6.4 |
| | 0.1 | 23.0 | |
| hEx44_Ac14 | 10 | 86.5 | 19.1 |
| | 3 | 80.0 | 11.8 |
| | 1 | 62.0 | 9.0 |
| | 0.1 | 31.5 | 0.7 |
| No PMO | | 8.3 | 3.8 |

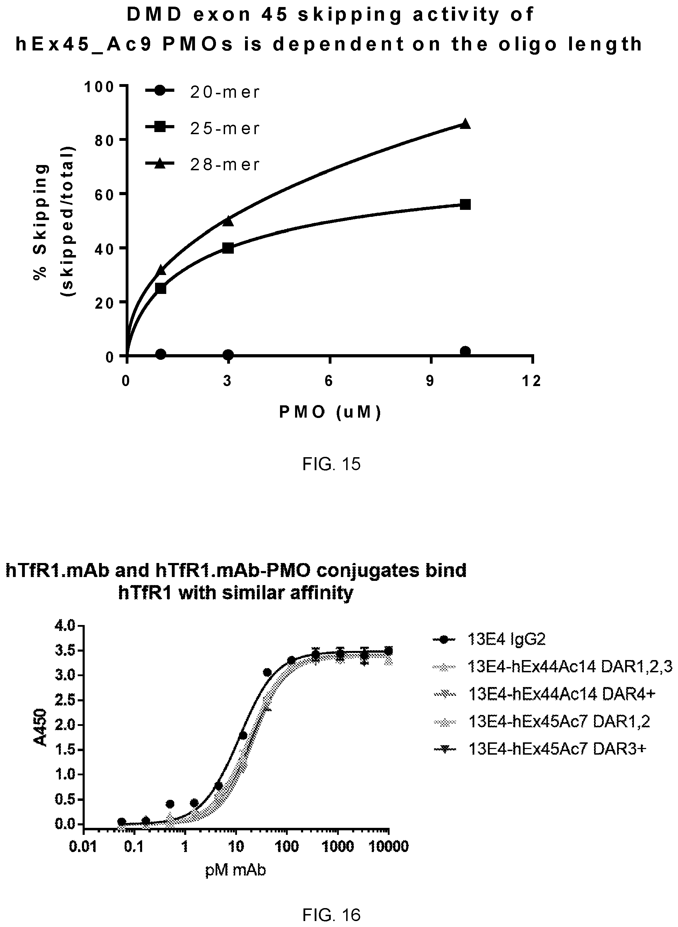

Fig. 15 illustrates exon skipping activity of different lengths of hEx45_Ac9 PMOs in transfected primary human skeletal muscle cells.

### Example 10. Synthesis and purification of human TfR1 PMO conjugates

An anti-human transferrin receptor antibody was produced. PMOs (28-mers) were synthesized by GeneTools. Antibody (10 mg/ml) in borate buffer (25 mM sodium tetraborate, 25 mM NaCl, 1 mM Diethylene triamine pentaacetic acid, pH 8.0) was reduced by adding 4 equivalents of tris(2-carboxyethyl)phosphine (TCEP) in water and incubating at 37°C for 4 hours. 4(N-Maleimidomethyl)cyclohexanecarboxylic acid N-hydroxysuccinimide ester (SMCC) was coupled to the primary amine on the 3' end of the PMO by incubating the PMO (50 mg/ml) in DMSO with 10 equivalents of SMCC (10 mg/ml) in DMSO for one hour. Unconjugated SMCC was removed by ultrafiltration using Amicon Ultra-15 centrifugal filter units with a MWCO of 3 kDa. The PMO-SMCC was washed three times with acetate buffer (10 mM sodium acetate, pH 6.0) and used immediately. The reduced antibody was mixed with 2.25 equivalents of PMO-SMCC and incubated overnight at 4°C. The pH of the reaction mixture was then reduced to 7.5 and 8 equivalents of N-Ethylmaleimide was added to the mixture at room temperature for 30 minutes to quench unreacted cysteines. Analysis of the reaction mixture by hydrophobic interaction chromatography (HIC) method-2 showed antibody-PMO conjugates along with unreacted antibody and PMO.

The reaction mixture was purified with an AKTA Explorer FPLC using HIC method-1. Dependent on the conjugate, fractions containing either conjugates with a drug to antibody ratio of one (DAR 1), two (DAR 2), and three (DAR 3), or fractions containing conjugates with a drug to antibody ratio of 3+ (DAR 3+), or 4+ (DAR 4+) were combined and concentrated with Amicon Ultra-15 centrifugal filter units with a MWCO of 50 kDa. Concentrated conjugates were buffer exchanged with PBS (pH 7.4) using Amicon Ultra-15 centrifugal filter units prior to analysis.

### Hydrophobic interaction chromatography (HIC) method-1.

1. Column: GE, HiScreen Butyl HP, 4.7ml
2. Solvent A: 50 mM phosphate buffer, 0.7M Ammonium Sulfate, pH 7.0; Solvent B: 80% 50 mM phosphate buffer, 20% IPA, pH 7.0; Flow Rate: 1.0 ml/min
3. Gradient:

| a. | %A | %B | Column Volume |
|---|---|---|---|
| b. | 100 | 0 | 1 |
| c. | 70 | 30 | 25 |
| d. | 0 | 100 | 1 |
| e. | 0 | 100 | 2 |

### Binding of hTfR1.mAb-PMO conjugates to human Transferrin Receptor

Antibody conjugate (AOC) binding was measured by ELISA. Recombinant human Transferrin Receptor (Sino Biological 11020-H07H) was coated onto high bind plates (Costar 3690) at 1 ng/uL in PBS overnight. Plates were washed and AOC or mAb samples were added at concentrations up to 10 nM. Color was developed through HRP conjugated secondary antibody (Jackson Immunoresearch 109-035-006) and TMB substrate (ThermoFisher 34028) stopped with 2N sulfuric acid. Kd was determined using GraphPad Prism.

Fig. 16 illustrates binding of hTfR1.mAb-PMO conjugates to human Transferrin Receptor in vitro.

### Activity of TfR1 mAb-PMO conjugates in primary human skeletal muscle cells

Primary, pre-differentiated human skeletal muscle cells (Gibco, #A11440) were plated on collagen Type 1 coated 24-well plates (Gibco, #1970788) in DMEM supplemented with 2% horse serum and 1x ITS (Gibco, #1933286) according to the manufacturer's instructions. Cells were grown in 37°C + 5% CO₂ for 2 days to establish myotubes. Immortalized human skeletal muscle cells from healthy donors (Myology Institute Paris) were plated on collagen Type 1 coated 24-well plates (Gibco, #1970788) in Skeletal Muscle Cell Growth medium (Promocell, C-23160) supplemented with 5 % FBS. After myoblasts reached confluency, myotube formation was induced in differentiation medium containing DMEM supplemented with gentamycin (50 ug/ml) (Invitrogen, 15750-045) and insulin (10 ug/ml) (sigma, 91077). Myotubes were then treated with defined concentrations of AOCs in the respective medium. Cell were harvested 72 hours after treatment by aspirating the culture medium, followed by addition of 300 ul TRIZOL per well. RNA isolation and quantification of DMD exon skipping was performed as detailed in example 9.

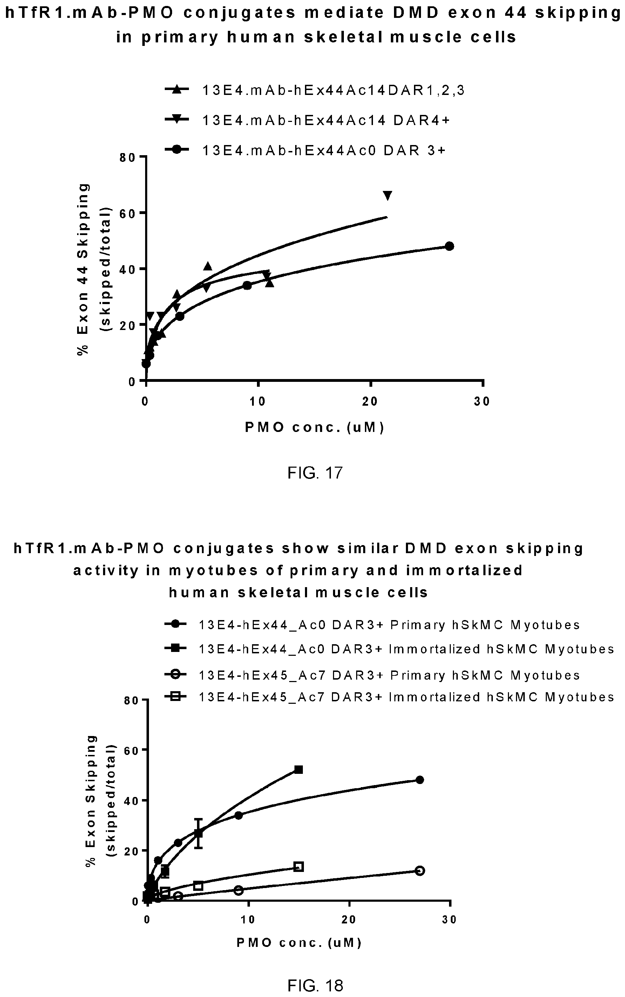

Fig. 17 illustrates exon skipping activity of hTfR1.mAb-PMO (28-mer) conjugates in primary human skeletal muscle cells.

Fig. 18 illustrates exon skipping activity of hTfR1.mAb-PMO conjugates in myotubes of primary and immortalized human skeletal muscle cells.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

The following clauses, describing aspects of the invention, are part of the description
1. A polynucleic acid conjugate comprising a target cell binding moiety binding to at least one polynucleic acid molecule that hybridizes to a target region of a pre-mRNA transcript of *DMD* gene, wherein the at least one polynucleic acid molecule induces splicing out of an exon from a pre-mRNA transcript to generate a mRNA transcript that encodes a functional dystrophin protein.
2. The polynucleic acid conjugate of clause 1, wherein the functional dystrophin protein is a truncated form of the dystrophin protein.
3. The polynucleic acid conjugate of clause 1, wherein the target region is at an exon-intron junction, wherein the exon is the exon that is to be spliced out.
4. The polynucleic acid conjugate of clause 3, wherein the exon is exon 8, 23, 35, 43, 44, 45, 50, 51, 52, 53, or 55.
5. The polynucleic acid conjugate of clause 3, wherein the exon-intron junction is located at the 5' of the exon that is to be spliced out.
6. The polynucleic acid conjugate of clause 5, wherein the target region is an intronic region upstream of the exon-intron junction.
7. The polynucleic acid conjugate of clause 5 or 6, wherein the target region is about 500, 450, 400, 350, 300, 250, 200, 150, 100, 90, 80, 70, 60, 50, 40, 30, 20, or 10 nucleotides upstream of the exon-intron junction.
8. The polynucleic acid conjugate of clause 3, wherein the exon-intron junction is located at the 3' of the exon that is to be spliced out.
9. The polynucleic acid conjugate of clause 8, wherein the target region is an intronic region downstream of the exon-intron junction.
10. The polynucleic acid conjugate of clause 8 or 9, wherein the target region is about 500, 450, 400, 350, 300, 250, 200, 150, 100, 90, 80, 70, 60, 50, 40, 30, 20, or 10 nucleotides downstream of the exon-intron junction.
11. The polynucleic acid conjugate of any one of the clauses 1-10, wherein the target cell binding moiety binds to two or more, three or more, four or more, five or more, six or more, or eight or more polynucleic acid molecules.
12. The polynucleic acid conjugate of any one of the clauses 1-10, wherein the polynucleic acid molecule is from about 10 to about 50 nucleotides in length.
13. The polynucleic acid conjugate of any one of the clauses 1-12, wherein the polynucleic acid molecule comprises about 80%, 85%, 90%, 95%, 96%, 97%, 98%, or 99% sequence identity to a sequence selected from SEQ ID NOs: 964-1285.
14. The polynucleic acid conjugate of any one of the clauses 1-13, wherein the polynucleic acid molecule comprises at least 10, 15, 16, 17, 18, 19, 20, 21, 22, 23, 24, 25, 26, or more contiguous bases of a base sequence selected from SEQ ID NOs: 964-1285.
15. The polynucleic acid conjugate of any one of the clauses 1-14, wherein the polynucleic acid molecule further comprises 1, 2, 3, or 4 mismatches.
16. The polynucleic acid conjugate of any one of the clauses 1-15, wherein the polynucleic acid molecule comprises at least 10, 15, 16, 17, 18, 19, 20, 21, 22, 23, 24, 25, 26, or more contiguous bases of a base sequence selected from SEQ ID NOs: 1056-1094, 1147-1162, or 1173-1211.
17. The polynucleic acid conjugate of clause 16, wherein the polynucleic acid molecule comprises at least 10, 15, 16, 17, 18, 19, 20, 21, 22, 23, 24, 25, 26, or more contiguous bases of a base sequence selected from SEQ ID NOs: 1056-1076.
18. The polynucleic acid conjugate of clause 16, wherein the polynucleic acid molecule comprises at least 10, 15, 16, 17, 18, 19, 20, 21, 22, 23, 24, 25, 26, or more contiguous bases of a base sequence selected from SEQ ID NOs: 1077-1094.
19. The polynucleic acid conjugate of clause 16, wherein the polynucleic acid molecule comprises at least 10, 15, 16, 17, 18, 19, 20, 21, 22, 23, 24, 25, 26, or more contiguous bases of a base sequence selected from SEQ ID NOs: 1147-1162.
20. The polynucleic acid conjugate of clause 16, wherein the polynucleic acid molecule comprises at least 10, 15, 16, 17, 18, 19, 20, 21, 22, 23, 24, 25, 26, or more contiguous bases of a base sequence selected from SEQ ID NOs: 1173-1211.
21. The polynucleic acid conjugate of any one of the clauses 1-19, wherein the binding moiety comprises an antibody.
22. The polynucleic acid conjugate of clause 21, wherein the antibody comprises an anti-transferrin antibody.
23. The polynucleic acid conjugate of any one of the clauses 1-19, wherein the binding moiety comprises a plasma protein.
24. The polynucleic acid conjugate of any one of the clauses 1-23, wherein the polynucleic acid conjugate comprises

   A-(X¹-B)ₙ Formula (V)

   wherein,
   A comprises the binding moiety;
   B consists of the polynucleic acid molecule;
   X¹ consists of a bond or first non-polymeric linker; and
   n is an averaged value selected from 1-12.
25. The polynucleic acid conjugate of any one of the clauses 1-24, wherein the polynucleic acid molecule comprises a passenger strand and a guide strand.
26. The polynucleic acid conjugate of clause 25, wherein the guide strand comprises at least one modified internucleotide linkage, at least one inverted abasic moiety, at least one 5'-vinylphosphonate modified non-natural nucleotide, or a combination thereof.
27. The polynucleic acid conjugate of clause 25, wherein the guide strand comprises about 2, 3, 4, 5, 6, 7, 8, or 9 phosphorothioate-modified non-natural nucleotides.
28. The polynucleic acid conjugate of clause 25, wherein the guide strand comprises 1 phosphorothioate-modified non-natural nucleotide.
29. The polynucleic acid conjugate of any one of the clauses 26-28, wherein the phosphorothioate modified non-natural nucleotide is located at an internucleotide linkage of the polynucleotide.
30. The polynucleic acid conjugate of clause 26, wherein the at least one 5'-vinylphosphonate modified non-natural nucleotide is located about 1, 2, 3, 4, or 5 bases away from the 5' terminus of the guide strand.
31. The polynucleic acid conjugate of clause 26 or 30, wherein the at least one 5'-vinylphosphonate modified non-natural nucleotide is further modified at the 2'-position.
32. The polynucleic acid conjugate of clause 31, wherein the 2'-modification is selected from 2'-O-methyl, 2'-O-methoxyethyl (2'-O-MOE), 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-O-DMAP), T-O-dimethylaminoethyloxyethyl (2'-O-DMAEOE), or 2'-O-N-methylacetamido (2'-O-NMA) modified nucleotide.
33. The polynucleic acid conjugate of clause 25, wherein the passenger strand comprises at least 6, 7, 8, 9, 10, 11, 12, 13, 14, 15, 16, 17, 18, 19, 20, or more phosphorodiamidate morpholino oligomer-modified non-natural nucleotides.
34. The polynucleic acid conjugate of clause 25, wherein the passenger strand comprises 100% phosphorodiamidate morpholino oligomer-modified non-natural nucleotides.
35. The polynucleic acid conjugate of clause 25, wherein the passenger strand is shorter in length than the guide strand, thereby generating a 5' overhang, a 3' overhang, or a combination thereof.
36. The polynucleic acid conjugate of clause 25, wherein the passenger strand is equal in length to the guide strand, thereby generating a blunt end at each terminus of the polynucleic acid molecule.
37. The polynucleic acid conjugate of clause 35 or 36, wherein the polynucleic acid molecule is a phosphorodiamidate morpholino oligomer/RNA hetero-duplex.
38. The polynucleic acid conjugate of clause 25, wherein the passenger strand comprises at least 6, 7, 8, 9, 10, 11, 12, 13, 14, 15, 16, 17, 18, 19, 20, or more peptide nucleic acid-modified non-natural nucleotides.
39. The polynucleic acid conjugate of clause 25, wherein the passenger strand comprises 100% peptide nucleic acid-modified non-natural nucleotides.
40. The polynucleic acid conjugate of clause 25, wherein the passenger strand is shorter in length than the guide strand, thereby generating a 5' overhang, a 3' overhang, or a combination thereof.
41. The polynucleic acid conjugate of clause 25, wherein the passenger strand is equal in length to the guide strand, thereby generating a blunt end at each terminus of the polynucleic acid molecule.
42. The polynucleic acid conjugate of clause 40 or 41, wherein the polynucleic acid molecule is a peptide nucleic acid/RNA hetero-duplex.
43. The polynucleic acid conjugate of clause 25, wherein the passenger strand is conjugated to A- X¹.
44. The polynucleic acid conjugate of clause 43, wherein A- X¹ is conjugated to the 5' end of the passenger strand.
45. The polynucleic acid conjugate of clause 43, wherein A- X¹ is conjugated to the 3' end of the passenger strand.
46. The polynucleic acid conjugate of any one of the clauses 24 or 43-45, wherein X¹ is a bond.
47. The polynucleic acid conjugate of any one of the clauses 24 or 43-45, wherein X¹ is a C₁-C₆ alkyl group.
48. The polynucleic acid conjugate of any one of the clauses 24 or 43-45, wherein X¹ is a homobifuctional linker or a heterobifunctional linker, optionally conjugated to a C₁-C₆ alkyl group.
49. The polynucleic acid conjugate of clause 1, further comprising C.
50. The polynucleic acid conjugate of clause 49, wherein C is polyethylene glycol.
51. The polynucleic acid conjugate of any one of the clauses 24-50, wherein C is directly conjugated to B via X².
52. The polynucleic acid conjugate of clause 51, wherein X² consists of a bond or second non-polymeric linker.
53. The polynucleic acid conjugate of clause 52, wherein X² is a bond.
54. The polynucleic acid conjugate of clause 52, wherein X² is a C₁-C₆ alkyl group.
55. The polynucleic acid conjugate of clause 52, wherein X² is a homobifuctional linker or a heterobifunctional linker, optionally conjugated to a C₁-C₆ alkyl group.
56. The polynucleic acid conjugate of any one of the clauses 1-55, wherein the passenger strand is conjugated to A- X¹ and X²-C.
57. The polynucleic acid conjugate of any one of the clauses 1-56, wherein A- X¹ is conjugated to the 5' end of the passenger strand and X²-C is conjugated to the 3' end of the passenger strand.
58. The polynucleic acid conjugate of any one of the clauses 1-56, wherein X²-C is conjugated to the 5' end of the passenger strand and A- X¹ is conjugated to the 3' end of the passenger strand.
59. The polynucleic acid conjugate of any one of the clauses 1-58, wherein the polynucleic acid conjugate comprises:

   A-X¹-(B-X²-C)ₙ Formula (VI)

   wherein,
   A comprises the binding moiety;
   B consists of the polynucleic acid molecule;
   C consists of a polymer;
   X¹ consists a bond or first non-polymeric linker;
   X² consists of a bond or second non-polymeric linker; and
   n is an averaged value selected from 1-12.
60. The polynucleic acid conjugate of clause 1, further comprising D.
61. The polynucleic acid conjugate of clause 60, wherein D is an endosomolytic moiety.
62. A polynucleic acid molecule comprising at least 23 contiguous bases of a base sequence selected from SEQ ID NOs: 1056-1058 or 1087-1089, wherein the polynucleic acid molecule comprises no more than 50 nucleotides in length.
63. A polynucleic acid molecule comprising SEQ ID NOs: 1056-1058, wherein the polynucleic acid molecule comprises no more than 50 nucleotides in length.
64. A polynucleic acid molecule comprising SEQ ID NOs: 1087-1089, wherein the polynucleic acid molecule comprises no more than 50 nucleotides in length.
65. A pharmaceutical composition, comprising:
   a polynucleic acid conjugate of clauses 1-61 or a polynucleic acid molecule of clauses 62-64; and
   a pharmaceutically acceptable excipient.
66. The pharmaceutical composition of clause 65, wherein the pharmaceutical composition is formulated for systemic delivery.
67. The pharmaceutical composition of clause 65 or 66, wherein the pharmaceutical composition is formulated for parenteral administration.
68. A method of treating a disease or condition characterized with a defective mRNA in a subject in need thereof, comprising:
   administering to the subject a polynucleic acid conjugate of clauses 1-61 or a polynucleic acid molecule of clauses 62-64 to induce skipping of an exon that leads to the defective mRNA to generate a processed mRNA encoding a functional protein, thereby treating the disease or condition in the subject.
69. The method of clause 68, wherein the disease or condition is a neuromuscular disease, a genetic disease, cancer, a hereditary disease, or a cardiovascular disease.
70. The method of clause 69, wherein the neuromuscular disease is a muscular dystrophy.
71. The method of clause 70, wherein the muscular dystrophy is Duchenne muscular dystrophy, Becker muscular dystrophy, facioscapulohumeral muscular dystrophy, congenital muscular dystrophy, or myotonic dystrophy.
72. A method of treating a muscular dystrophy in a subject in need thereof, comprising:
   administering to the subject a polynucleic acid conjugate of clauses 1-61 or a polynucleic acid molecule of clauses 62-64, thereby treating the muscular dystrophy in the subject.
73. The method of clause 72, wherein the muscular dystrophy is Duchenne muscular dystrophy.
74. The method of any one of the preceding clauses, wherein the subject is a human.
75. A kit comprising a polynucleic acid conjugate of clauses 1-61 or a polynucleic acid molecule of clauses 62-64.

## Claims

1. A polynucleic acid conjugate comprising a target cell binding moiety bound to at least one polynucleic acid molecule that hybridizes to a target region of a pre-mRNA transcript of a DMD gene, wherein the at least one polynucleic acid molecule induces splicing out of an exon from a pre-mRNA transcript to generate a mRNA transcript that encodes a functional dystrophin protein, and wherein the binding moiety comprises an anti-transferrin antibody.

2. The polynucleic acid conjugate of claim 1, wherein the functional dystrophin protein is a truncated form of the dystrophin protein.

3. The polynucleic acid conjugate of claim 1, wherein the target region is at the exon-intron junction, wherein the exon is the exon that is to be spliced out.

4. The polynucleic conjugate of claim 1, wherein the target region is an intronic region upstream or downstream of an exon-intron junction.

5. The polynucleic acid conjugate of claim 3 or 4, wherein the exon is exon 8, 23, 35, 43, 44, 45, 50, 51, 52, 53, or 55.

6. The polynucleic acid conjugate of claim 5, wherein the exon is exon 44.

7. The polynucleic acid conjugate of any one of claims 1-6, wherein the polynucleic acid molecule is from about 10 to about 50 nucleotides in length.

8. The polynucleic acid conjugate of any one of claims 1-7, wherein the polynucleic acid molecule is a single stranded polynucleotide.

9. The polynucleic acid conjugate of any one of claims 1-8, wherein the polynucleic acid molecule is a phosphorodiamidate morpholino oligomer.

10. The polynucleic acid conjugate of any one of the claims 1-9, wherein the polynucleic acid conjugate comprises
A-(X¹-B)n Formula (V)
wherein
A comprises the anti-transferrin antibody or its binding fragment thereof;
B consists of the polynucleic acid molecule;
X¹ consists of a bond or first non-polymeric linker; and
n is an averaged value selected from 1-12.

11. A polynucleic acid conjugate of any one of claims 1-10 for use in a treatment of a muscular dystrophy in a subject.

12. The polynucleic acid conjugate for use of claim 11, wherein the muscular dystrophy is Duchenne muscular dystrophy, Becker muscular dystrophy, facioscapulohumeral muscular dystrophy, congenital muscular dystrophy, or myotonic dystrophy.

13. The polynucleic acid conjugate for use of claim 11, wherein the muscular dystrophy is Duchenne muscular dystrophy.

14. The polynucleic acid conjugate for use of any one of claims 11-13, wherein the subject is a human.
